# EUROPEAN PATENT SPECIFICATION

(11) **EP 2 670 764 B1**
(45) Date of publication and mention of the grant of the patent: **02.09.2015**
(21) Application number: 12702946.0
(22) Date of filing: 27.01.2012
(51) Int. Cl.: C07J 63/00, A61K 31/56, A61P 31/18

(54) **C-28 AMINES OF C-3 MODIFIED BETULINIC ACID DERIVATIVES AS HIV MATURATION INHIBITORS**
C-28-AMINE AUS MODIFIZIERTEN C-3-BETULINSÄUREDERIVATEN ALS HIV-REIFUNGSHEMMER
AMINES EN C28 DE DÉRIVÉS D'ACIDE BÉTULINIQUE MODIFIÉ EN C-3 EN TANT QU'INHIBITEURS DE MATURATION DU VIH

(30) Priority: 31.01.2011 US 201161437870 P
(43) Date of publication of application: 11.12.2013
(73) Proprietor: Bristol-Myers Squibb Company, Princeton, NJ 08543 (US)
(72) Inventor: REGUEIRO-REN, Alicia, Wallingford, Connecticut 06492 (US); SWIDORSKI, Jacob, Wallingford, Connecticut 06492 (US); SIT, Sing-Yuen, Wallingford, Connecticut 06492 (US); CHEN, Yan, Wallingford, Connecticut 06492 (US); CHEN, Jie, Wallingford, Connecticut 06492 (US); MEANWELL, Nicholas A., Wallingford, Connecticut 06492 (US); LIU, Zheng, Wallingford, Connecticut 06492 (US)
(74) Representative: Reitstötter Kinzebach
(86) International application number: PCT/US2012/022847
(87) International publication number: WO 2012/106188

(56) References cited:
- WO-A1-2011/153315
- WO-A1-2011/153319
- WO-A2-2006/053255
- ZHU Y-M ET AL: "Synthesis and Anti-HIV Activity of Oleanolic Acid Derivatives", BIOORGANIC & MEDICINAL CHEMISTRY LETTERS, PERGAMON, ELSEVIER SCIENCE, GB, vol. 11, no. 24, 1 December 2001 (2001-12-01), pages 3115-3118, XP002984538, ISSN: 0960-894X, DOI: 10.1016/S0960-894X(01)00647-3

## Description

### CROSS REFERENCE TO RELATED APPLICATIONS

### FIELD OF THE INVENTION

The present invention relates to novel compounds useful against HIV, and more particularly, to compounds derived from betulinic acid and other structurally related compounds which are useful as HIV maturation inhibitors, and to pharmaceutical compositions containing same, as well as to methods for their preparation.

### BACKGROUND OF THE INVENTION

HIV-1 (human immunodeficiency virus -1) infection remains a major medical problem, with an estimated 45 million people infected worldwide at the end of 2007. The number of cases of HIV and AIDS (acquired immunodeficiency syndrome) has risen rapidly. In 2005, approximately 5.0 million new infections were reported, and 3.1 million people died from AIDS. Currently available drugs for the treatment of HIV include nucleoside reverse transcriptase (RT) inhibitors or approved single pill combinations: zidovudine (or AZT or Retrovir^{®}), didanosine (or Videx^{®}), stavudine (or Zerit^{®}), lamivudine (or 3TC or Epivir^{®}), zalcitabine (or DDC or Hivid^{®}), abacavir succinate (or Ziagen^{®}), Tenofovir disoproxil fumarate salt (or Viread^{®}), emtricitabine (or FTC - Emtriva^{®}),Combivir^{®} (contains -3TC plus AZT), Trizivir^{®} (contains abacavir, lamivudine, and zidovudine), Epzicom^{®} (contains abacavir and lamivudine), Truvada^{®} (contains Viread^{®} and Emtriva^{®}); non-nucleoside reverse transcriptase inhibitors: nevirapine (or Viramune^{®}), delavirdine (or Rescriptor^{®}) and efavirenz (or Sustiva^{®}), Atripla^{®} (Truvada^{®} + Sustiva^{®}), and etravirine, and peptidomimetic protease inhibitors or approved formulations: saquinavir, indinavir, ritonavir, nelfinavir, amprenavir, lopinavir, Kaletra^{®}(lopinavir and Ritonavir), darunavir, atazanavir (Reyataz^{®}) and tipranavir (Aptivus^{®}), and integrase inhibitors such as raltegravir (Isentress^{®}), and entry inhibitors such as enfuvirtide (T-20) (Fuzeon^{®}) and maraviroc (Selzentry^{®}).

Each of these drugs can only transiently restrain viral replication if used alone. However, when used in combination, these drugs have a profound effect on viremia and disease progression. In fact, significant reductions in death rates among AIDS patients have been recently documented as a consequence of the widespread application of combination therapy. However, despite these impressive results, 30 to 50% of patients may ultimately fail combination drug therapies. Insufficient drug potency, non-compliance, restricted tissue penetration and drug-specific limitations within certain cell types (e.g. most nucleoside analogs cannot be phosphorylated in resting cells) may account for the incomplete suppression of sensitive viruses. Furthermore, the high replication rate and rapid turnover of HIV-1 combined with the frequent incorporation of mutations, leads to the appearance of drug-resistant variants and treatment failures when sub-optimal drug concentrations are present. Therefore, novel anti-HIV agents exhibiting distinct resistance patterns, and favorable pharmacokinetic as well as safety profiles are needed to provide more treatment options. Improved HIV fusion inhibitors and HIV entry coreceptor antagonists are two examples of new classes of anti-HIV agents further being studied by a number of investigators.

HIV attachment inhibitors are a further subclass of antiviral compounds that bind to the HIV surface glycoprotein gp120, and interfere with the interaction between the surface protein gp120 and the host cell receptor CD4. Thus, they prevent HIV from attaching to the human CD4 T-cell, and block HIV replication in the first stage of the HIV life cycle. The properties of HIV attachment inhibitors have been improved in an effort to obtain compounds with maximized utility and efficacy as antiviral agents. In particular, US 7,354, 924 and US 2005/0209246 are illustrative of HIV attachment inhibitors.

Another emerging class of HIV treatment compounds are called HIV maturation inhibitors. Maturation is the last of as many as 10 or more steps in HIV replication or the HIV life cycle, in which HIV becomes infectious as a consequence of several HIV protease-mediated cleavage events in the gag protein that ultimately results in release of the capsid (CA) protein. Maturation inhibitors prevent the HIV capsid from properly assembling and maturing, from forming a protective outer coat, or from emerging from human cells. Instead, non-infectious viruses are produced, preventing subsequent cycles of HIV infection.

Certain derivatives of betulinic acid have now been shown to exhibit potent anti-HIV activity as HIV maturation inhibitors. For example, US 7,365,221 discloses monoacylated betulin and dihydrobetuline derivatives, and their use as anti-HIV agents. As discussed in the '221 reference, esterification of betulinic acid (1) with certain substituted acyl groups, such as 3',3'-dimethylglutaryl and 3',3'-dimethylsuccinyl groups produced derivatives having enhanced activity (Kashiwada, Y., et al., J. Med. Chem. 39:1016-1017 (1996)). Acylated betulinic acid and dihydrobetulinic acid derivatives that are potent anti-HIV agents are also described in U.S. Pat. No. 5,679,828. Esterification of the hydroxyl in the 3 carbon of betulin with succinic acid also produced a compound capable of inhibiting HIV-1 activity (Pokrovskii, A. G., et al., Gos. Nauchnyi Tsentr Virusol. Biotekhnol. "Vector" 9:485-491 (2001)).

Other references to the use of treating HIV infection with compounds derived from betulinic acid include US 2005/0239748 and US 2008/0207573, as well as WO 2006/053255, WO2009/100532, and WO 2011/007230.

One HIV maturation compound that has been in development has been identified as Bevirimat or PA-457, with the chemical formula of C₃₆H₅₆O₆ and the IUPAC name of 3β- (3-carboxy-3-methyl-butanoyloxy) lup-20(29)-en-28-oic acid.

Reference is also made herein to the applications by Bristol-Myers Squibb entitled "MODIFIED C-3 BETULINIC ACID DERIVATIVES AS HIV MATURATION INHIBITORS" USSN 13/151,706 filed on June 2, 2011 (published as US-A-2012/142707) and "C-28

### AMIDES OF MODIFIED C-3 BETULINIC ACID DERIVATIVES AS HIV

MATURATION INHIBITORS" USSN 13/151,722, filed on June 2, 2011 (published as US-A-2012/142653).

What is now needed in the art are new compounds which are useful as HIV maturation inhibitors, as well as new pharmaceutical compositions containing these compounds.

### SUMMARY OF THE INVENTION

The present invention provides compounds of Formulas I, II, and III below, including pharmaceutically acceptable salts thereof, their pharmaceutical formulations, and their use in patients suffering from or susceptible to a virus such as HIV. The compounds of Formulas I - III are effective antiviral agents, particularly as inhibitors of HIV. They are useful for the treatment of HIV and AIDS.

One embodiment of the present invention is directed to a compound, including pharmaceutically acceptable salts thereof, which is selected from the group of:
a compound of formula I 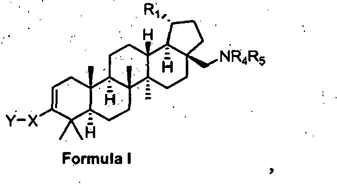
a compound of formula II 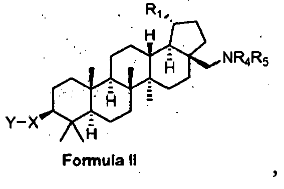 and
a compound of formula III 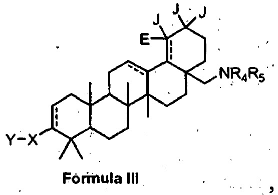 wherein R₁ is isopropenyl or isopropyl;
   J and E are independently -H or -CH₃ and E is absent when the double bond is present;
   X is a phenyl or heteroaryl ring substituted with A, wherein A is at least one member selected from the group of -H, -halo, -hydroxyl, -C₁₋₆alkyl, -C₁₋₆ alkoxy, and-COOR₂;
   R₂ is -H, -C₁₋₆ alkyl, -alkylsubstituted C₁₋₆ alkyl or -arylsubstituted C₁₋₆ alkyl;
   Y is selected from the group of -COOR₂, -C(O)NR2SO₂R₃, - C(O)NHSO₂NR₂R₂,-NR₂SO₂R₂, -SO₂NR₂R₂, -C₃₋₆cycloalkyl-COOR₂, -C₁₋₆ alkenyl-COOR₂, -C₁₋₆ alkynyl-COOR₂, -C₁₋₆alkyl-COOR₂, -NHC(O)(CH₂)ₙ-COOR₂, -SO₂NR₂C(O)R₂, -tetrazole, and -CONHOH, wherein n=1-6;
   R₃ is -C₁₋₆ alkyl or alkylsubstituted C₁₋₆ alkyl;
   R₄ is selected from the group of H, -C₁₋₆alkyl, -C₃₋₆cycloalkyl, -C₁₋₆substituted alkyl, -C₁₋₆alkyl-heteroaryl, -C₁₋₆alkyl-substitutedheteroaryl, -C₁₋₆alkyl-NR₆R₇, -C₁₋₆alkyl-CONR₈R₉, -C₃₋₆cycloalkyl-CONR₈R₉, -C₃₋₆cycloalkyl-(CH₂)₁₋₃-NR₆R₇, -(CH₂)₁₋₃-C₃₋₆ cycloalkyl-NR₆R₇; -(CH₂) ₁₋₃-C₃₋₆ cycloalkyl-(CH₂)₁₋₃-NR₆R₇; -C₁₋₆ alkyl-Q₁, C₃₋₆ cycloalkyl-Q₁, -COR₁₀,-SO₂R₃ and -SO₂NR₂R₂;
   Q₁ = -hydroxy, -COOR₂, -halo, -SO₂Rₐ;
   Ra= C₁₋₆ alkyl, NR₂R₂, 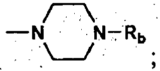
   R_{b}= -H, -C₁₋₆ alkyl, -COR₃, -SO₂R₃, -SONR₃R₃;
   R₄ can also be selected from the group of: 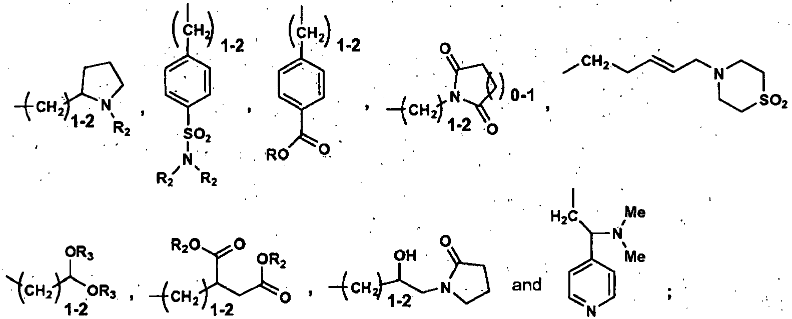
   R₅ is selected from the group of -H, -C₁₋₆ alkyl, -C₃₋₆ cycloalkyl, -C₁₋₆ alkylsubstituted alkyl, -COR₁₀,-SO₂R₃ and -SO₂NR₂R₂;
   with the proviso that only one of R₄ or R₅ can be selected from the group of -COR₁₀,-SO₂R₃ and -SO₂NR₂R₂;
   or R₄ and R₅ are taken together with the adjacent N to form a cycle such as 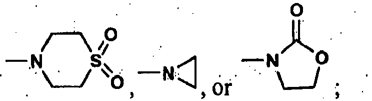
   R₁₀ is selected from the group of -H, -C₁₋₆ alkyl, -C₁₋₆ alkyl-NR₆R₇, -NR₁₁R₁₂, -OR₁₃, -C₁₋₆ alkyl-Q2_{,} -C₃₋₆ cycloalkyl-Q₂, aryl-Q₂, wherein n =1-6,
   wherein Q₂ = hydroxy, -COOR₂, -halo, SO₂Rₐ, -CONHSO₂R₃, -CONHSO₂NR₂R₂;
   R₁₀ can also be selected from the group of: 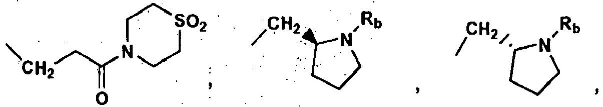 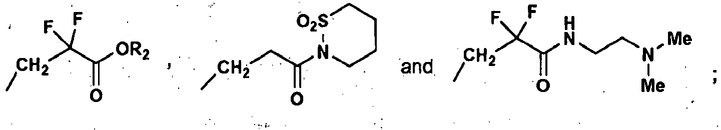
   R₆ and R₇ are independently selected from the group of -H, -C₁₋₆ alkyl, -C₁₋₆ substituted alkyl, aryl, heteroaryl, substituted aryl, substituted heteroaryl, and -C₁₋₆ alkyl-Q₁,
   or R₆ and R₇ are taken together with the adjacent N to form a cycle selected from the group of 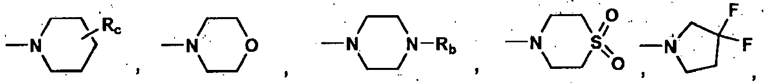 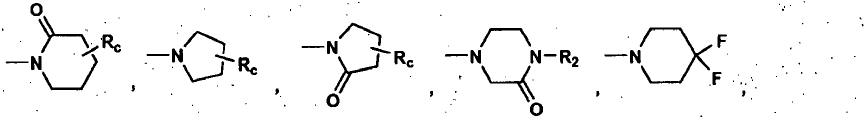 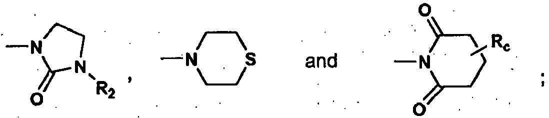
   R_{c}= C₁₋₆ alkyl, NR₂R₂, -COOR₃;
   R₈ and R₉ are independently selected from the group of -H, -C₁₋₆ alkyl, -C₃₋₆ cycloalkyl, -C₁₋₆ substituted alkyl, -C₁₋₆ alkyl-heteroaryl, -C₁₋₆ alkyl-substitutedheteroaryl, -C₁₋₆ alkyl-NR₂R₂, -C₁₋₆ alkyl-CONR₂R₂, -C₁₋₆ alkyl-Q₁, C₃₋₆ cycloalkyl-Q₁,
   or R₈ and R₉ can also be independently selected from the group of 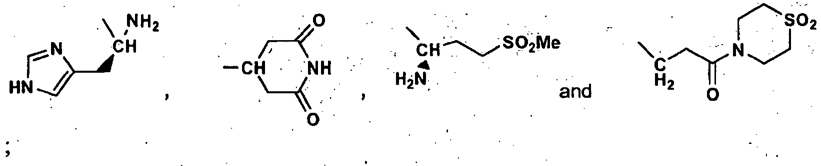
   or R₈ and R₉ are taken together with the adjacent N to form a cycle selected from the group of: 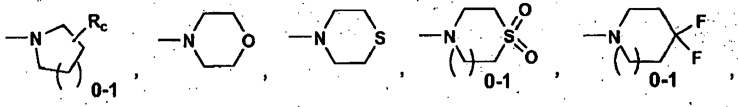 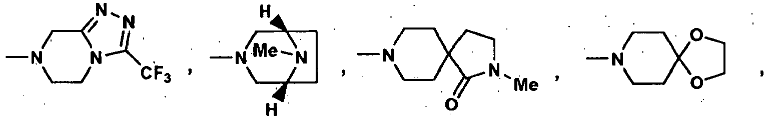 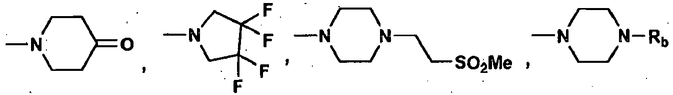 and 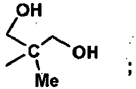
   and R₁₁ and R₁₂ are independently selected from the group of -H, -C₁₋₆ alkyl, -C₃₋₆ cycloalkyl, and -C₁₋₆ alkylsubstituted alkyl;
   or R₁₁ and R₁₂ are taken together with the adjacent N to form a cycle selected from the group of 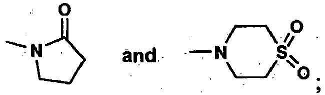 and
   R₁₃ is selected from the group of -H, -C₁₋₆ alkyl, -C₁₋₆ alkylsubstituted alkyl, and -C₁₋₆ alkyl NR₁₄R₁₅, wherein
   R₁₄ and R₁₅ are independently selected from the group of-H, -C₁₋₆ alkyl, and -C₁₋₆ alkylsubstituted alkyl, or R₁₄ and R₁₅ are taken together with the adjacent N to form a cycle selected from the group of

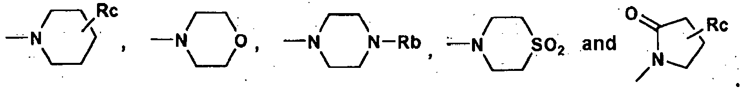

The compounds of the invention asre used for treating mammals infected with a virus, especially wherein said virus is HIV, comprising administering to said mammal an antiviral effective amount of a compound which is selected from the group of compounds of Formulas I, II, III above, and one or more pharmaceutically acceptable carriers, excipients or diluents. Optionally, the compound of Formulas I, II, and/or III can be administered in combination with an antiviral effective amount of another- AIDS treatment agent selected from the group consisting of: (a) an AIDS antiviral agent; (b) an anti-infective agent; (c) an immunomodulator; and (d) other HIV entry inhibitors.

Another embodiment of the present invention is a pharmaceutical composition comprising an antiviral effective amount of a compound which is selected from the group of compounds of Formulas I, II, and III, and one or more pharmaceutically acceptable carriers, excipients, and diluents; and optionally in combination with an antiviral effective amount of another AIDS treatment agent selected from the group consisting of: (a) an AIDS antiviral agent; (b) an anti-infective agent; (c) an immunomodulator; and (d) other HIV entry inhibitors.

In another embodiment of the invention there is provided one or more methods for making the compounds of Formulas I, II, and III.

Also provided herein are intermediate compounds useful in making the Compounds of Formulas I, II, and III.

The present invention is directed to these, as well as other important ends, hereinafter described.

### DETAILED DESCRIPTION OF THE EMBODIMENTS

### Definitions

Unless otherwise specifically set forth elsewhere in the application, one or more of the following terms may be used herein, and shall have the following meanings:
"H" refers to hydrogen, including its isotopes, such as deuterium.

The term "C₁₋₆ alkyl" as used herein and in the claims (unless specified otherwise) mean straight or branched chain alkyl groups such as methyl, ethyl, propyl, isopropyl, butyl, isobutyl, t-butyl, amyl, hexyl and the like.

"C₁ -C4 fluoroalkyl" refers to F-substituted C₁-C₄ alkyl wherein at least one H atom is substituted with F atom, and each H atom can be independently substituted by F atom;

"Halogen" refers to chlorine, bromine, iodine or fluorine.

An "aryl" or "Ar" group refers to an all carbon monocyclic or fused-ring polycyclic (i.e., rings which share adjacent pairs of carbon atoms) groups having a completely conjugated pi-electron system. Examples, without limitation, of aryl groups are phenyl, napthalenyl and anthracenyl. The aryl group may be substituted or unsubstituted. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thiohydroxy, thioaryloxy, thioheteroaryloxy, thioheteroalicycloxy, cyano, halogen, nitro, carbonyl, O-carbamyl, N-carbamyl, C-amido, N-amido, C-carboxy, O-carboxy, sulfinyl, sulfonyl, sulfonamido, trihalomethyl, ureido, amino and -NR^{x}R^{Y}, wherein R^{x} and R^{y} are independently selected from the group consisting of hydrogen, alkyl, cycloalkyl, aryl, carbonyl, C-carboxy, sulfonyl, trihalomethyl, and, combined, a five- or six-member heteroalicyclic ring.

As used herein, a "heteroaryl" group refers to a monocyclic or fused ring (i.e., rings which share an adjacent pair of atoms) group having in the ring(s) one or more atoms selected from the group consisting of nitrogen, oxygen and sulfur and, in addition, having a completely conjugated pi-electron system. Unless otherwise indicated, the heteroaryl group may be attached at either a carbon or nitrogen atom within the heteroaryl group. It should be noted that the term heteroaryl is intended to encompass an N-oxide of the parent heteroaryl if such an N-oxide is chemically feasible as is known in the art. Examples, without limitation, of heteroaryl groups are furyl, thienyl, benzothienyl, thiazolyl, imidazolyl, oxazolyl, oxadiazolyl, thiadiazolyl, benzothiazolyl, triazolyl, tetrazolyl, isoxazolyl, isothiazolyl, pyrrolyl, pyranyl, tetrahydropyranyl, pyrazolyl, pyridyl, pyrimidinyl, quinolinyl, isoquinolinyl, purinyl, carbazolyl, benzoxazolyl, benzimidazolyl, indolyl, isoindolyl, pyrazinyl. diazinyl, pyrazine, triazinyl, tetrazinyl, and tetrazolyl. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thioalkoxy, thiohydroxy, thioaryloxy, thioheteroaryloxy, thioheteroalicycloxy, cyano, halogen, nitro, carbonyl, O-carbamyl, N-carbamyl, C-amido, N-amido, C-carboxy, O-carboxy, sulfinyl, sulfonyl, sulfonamido, trihalomethyl, ureido, amino, and -NR^{x}R^{y}, wherein R^{x} and R^{y} are as defined above.

As used herein, a "heteroalicyclic" group refers to a monocyclic or fused ring group having in the ring(s) one or more atoms selected from the group consisting of nitrogen, oxygen and sulfur. Rings are selected from those which provide stable arrangements of bonds and are not intended to encompass systems which would not exist. The rings may also have one or more double bonds. However, the rings do not have a completely conjugated pi-electron system. Examples, without limitation, of heteroalicyclic groups are azetidinyl, piperidyl, piperazinyl, imidazolinyl, thiazolidinyl, 3-pyrrolidin-1-yl, morpholinyl, thiomorpholinyl and tetrahydropyranyl. When substituted the substituted group(s) is preferably one or more selected from alkyl, cycloalkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroaryloxy, thioheteroalicycloxy, cyano, halogen, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalomethanesulfonamido, trihalomethanesulfonyl, silyl, guanyl, guanidino, ureido, phosphonyl, amino and -NR^{x}R^{Y}, wherein R^{x} and R^{y} are as defined above.

An "alkyl" group refers to a saturated aliphatic hydrocarbon including straight chain and branched chain groups. Preferably, the alkyl group has 1 to 20 carbon atoms (whenever a numerical range; e.g., "1-20", is stated herein, it means that the group, in this case the alkyl group may contain 1 carbon atom, 2 carbon atoms, 3 carbon atoms, etc. up to and including 20 carbon atoms). More preferably, it is a medium size alkyl having 1 to 10 carbon atoms. Most preferably, it is a lower alkyl having 1 to 4 carbon atoms. The alkyl group may be substituted or unsubstituted. When substituted, the substituent group(s) is preferably one or more individually selected from trihaloalkyl, cycloalkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroaryloxy, thioheteroalicycloxy, cyano, halo, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalomethanesulfonamido, trihalomethanesulfonyl, and combined, a five- or six-member heteroalicyclic ring.

A "cycloalkyl" group refers to an all-carbon monocyclic or fused ring (i.e., rings which share and adjacent pair of carbon atoms) group wherein one or more rings does not have a completely conjugated pi-electron system. Examples, without limitation, of cycloalkyl groups are cyclopropane, cyclobutane, cyclopentane, cyclopentene, cyclohexane, cyclohexene, cycloheptane, cycloheptene and adamantane. A cycloalkyl group may be substituted or unsubstituted. When substituted, the substituent group(s) is preferably one or more individually selected from alkyl, aryl, heteroaryl, heteroalicyclic, hydroxy, alkoxy, aryloxy, heteroaryloxy, heteroalicycloxy, thiohydroxy, thioalkoxy, thioaryloxy, thioheteroaryloxy, thioheteroalicycloxy, cyano, halo, nitro, carbonyl, thiocarbonyl, O-carbamyl, N-carbamyl, O-thiocarbamyl, N-thiocarbamyl, C-amido, C-thioamido, N-amido, C-carboxy, O-carboxy, sulfinyl, sulfonyl, sulfonamido, trihalo- methanesulfonamido, trihalomethanesulfonyl, silyl, amidino, guanidino, ureido, phosphonyl, amino and-NR^{x}R^{y} with R^{x} and R^{y} as defined above.

An "alkenyl" group refers to an alkyl group, as defined herein, having at least two carbon atoms and at least one carbon-carbon double bond.

An "alkynyl" group refers to an alkyl group, as defined herein, having at least two carbon atoms and at least one carbon-carbon triple bond.

A "hydroxy" group refers to an -OH group.

An "alkoxy" group refers to both an -O-alkyl and an -O-cycloalkyl, group as defined herein.

An "aryloxy" group refers to both an -O-aryl and an -O-heteroaryl group, as defined herein.

A "heteroaryloxy" group refers to a heteroaryl-O- group with heteroaryl as defined herein.

A "heteroalicycloxy" group refers to a heteroalicyclic-O- group with heteroalicyclic as defined herein.

A "thiohydroxy" group refers to an -SH group.

A "thioalkoxy" group refers to both an S-alkyl and an -S-cycloalkyl group, as defined herein.

A "thioaryloxy" group refers to both an -S-aryl and an -S-heteroaryl group, as defined herein.

A "thioheteroaryloxy" group refers to a heteroaryl-S- group with heteroaryl, as defined herein.

A "thioheteroalicycloxy" group refers to a heteroalicyclic-S- group with heteroalicyclic as defined herein.

A "carbonyl" group refers to a -C(=O)-R" group, where R" is selected from the group consisting of hydrogen, alkyl, alkenyl, alkynyl, cycloalkyl, aryl, heteroaryl (bonded through a ring carbon) and heteroalicyclic (bonded through a ring carbon), as each is defined herein.

An "aldehyde" group refers to a carbonyl group where R" is hydrogen.

A "thiocarbonyl" group refers to a -C(=S)-R" group, with R" as defined herein.

A "Keto" group refers to a --CC(=O)C- group wherein the carbon on either or both sides of the C=O may be alkyl, cycloalkyl, aryl or a carbon of a heteroaryl or heteroalicyclic group.

A "trihalomethanecarbonyl" group refers to a Z₃CC(=O)- group with said Z being a halogen.

A "C-carboxy" group refers to a -C(=O)O-R" groups, with R" as defined herein.

An "O-carboxy" group refers to a R"C(-O)O-group, with R" as defined herein.

A "carboxylic acid" group refers to a C-carboxy group in which R" is hydrogen.

A "trihalomethyl" group refers to a -CZ₃, group wherein Z is a halogen group as defined herein.

A "trihalomethanesulfonyl" group refers to an Z₃CS(=O)₂- groups with Z as defined above.

A "trihalomethanesulfonamido" group refers to a Z₃CS(=O)₂NR^{x}- group with Z as defined above and R^{x} being H or (C₁₋₆)alkyl.

A "sulfinyl" group refers to a -S(=O)-R" group, with R" being (C₁₋₆)alkyl.

A "sulfonyl" group refers to a -S(=O)₂R" group with R" being (C₁₋₆)alkyl.

A "S-sulfonamido" group refers to a -S(=O)₂NR^{x}R^{y}, with R^{x} and R^{y} independently being H or (C₁₋₆)alkyl.

A "N-Sulfonamido" group refers to a R"S(=O)₂NRₓ- group, with Rₓ being H or (C₁₋₆)alkyl.

A "O-carbamyl" group refers to a -OC(=O)NR^{x}R^{y} group, with R^{x} and R^{y} independently being H or (C₁₋₆)alkyl.

A "N-carbamyl" group refers to a R^{x}OC(=O)NR^{y} group, with R^{x} and R^{y} independently being H or (C₁₋₆)alkyl.

A "O-thiocarbamyl" group refers to a -OC(=S)NR^{x}R^{y} group, with R^{x} and R^{y} independently being H or (C₁₋₆)alkyl.

A "N-thiocarbamyl" group refers to a R^{x}OC(=S)NR^{y}- group, with R^{x} and R^{y} independently being H or (C₁₋₆)alkyl.

An "amino" group refers to an -NH₂ group.

A "C-amido" group refers to a -C(=O)NR^{x}R^{y} group, with R^{x} and R^{y} independently being H or (C₁₋₆halkyl.

A "C-thioamido" group refers to a -C(=S)NR^{x}R^{y} group, with R^{x} and R^{y} independently being H or (C₁₋₆)alkyl.

A "N-amido" group refers to a R^{x}C(=O)NR^{y}- group, with R^{x} and R^{y} independently being H or (C₁₋₆)alkyl.

An "ureido" group refers to a NR^{x}C(=O)NR^{y}R^{y2} group, with R^{x}, R^{y}, and R^{y2} independently being H or (C₁₋₆)alkyl.

A "guanidino" group refers to a -R^{x}NC(=N)NR^{y}R^{y2} group, with R^{x}, R^{y}, and R^{y2} independently being H or (C₁₋₆)alkyl.

A "amidino" group refers to a R^{x}R^{y}NC(=N)- group, with R^{x} and R^{y} independently being H or (C₁₋₆)alkyl.

A "cyano" group refers to a -CN group.

A "silyl" group refers to a -Si(R")₃, with R" being (C₁₋₆)alkyl or phenyl.

A "phosphonyl" group refers to a P(=O)(OR^{x})₂ with R^{x} being (C₁₋₆)alkyl.

A "hydrazino" group refers to a -NR^{x}NR^{y}R^{y2} group, with R^{x}, R^{y}, and R^{y2} independently being H or (C₁₋₆)alkyl.

A "4, 5, or 6 membered ring cyclic N-lactam" group refers to

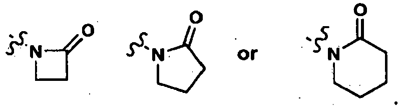

Any two adjacent R groups may combine to form an additional aryl, cycloalkyl, heteroaryl or heterocyclic ring fused to the ring initially bearing those R groups.

It is known in the art that nitrogen atoms in heteroaryl systems can be "participating in a heteroaryl ring double bond", and this refers to the form of double bonds in the two tautomeric structures which comprise five-member ring heteroaryl groups. This dictates whether nitrogens can be substituted as well understood by chemists in the art. The disclosure and claims of the present disclosure are based on the known general principles of chemical bonding. It is understood that the claims do not encompass structures known to be unstable or not able to exist based on the literature.

Pharmaceutically acceptable salts of compounds disclosed herein are within the scope of the invention. The term "pharmaceutically acceptable salt" as used herein and in the claims is intended to include nontoxic base addition salts. Suitable salts include those derived from organic and inorganic acids such as, without limitation, hydrochloric acid, hydrobromic acid, phosphoric acid, sulfuric acid, methanesulfonic acid, acetic acid, tartaric acid, lactic acid, sulfinic acid, citric acid, maleic acid, fumaric acid, sorbic acid, aconitic acid, salicylic acid, phthalic acid, and the like. The term "pharmaceutically acceptable salt" as used herein is also intended to include salts of acidic groups, such as a carboxylate, with such counterions as ammonium, alkali metal salts, particularly sodium or potassium, alkaline earth metal salts, particularly calcium or magnesium, and salts with suitable organic bases such as lower alkylamines (methylamine, ethylamine, cyclohexylamine, and the like) or with substituted lower alkylamines (e.g. hydroxyl-substituted alkylamines such as diethanolamine, triethanolamine or tris(hydroxymethyl)- aminomethane), or with bases such as piperidine or morpholine.

As set forth above, the invention is directed to a compound, including pharmaceutically acceptable salts thereof, which is selected from the group of:
a compound of formula I 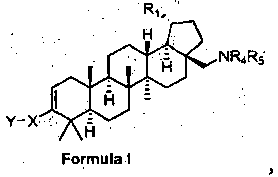
a compound of formula II 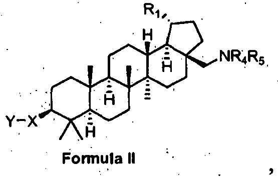 and
a compound of formula III 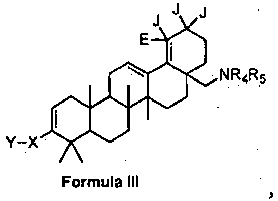 wherein R₁ is isopropenyl or isopropyl;
   J and E are independently -H or -CH₃ and E is absent when the double bond is present;
   X is a phenyl or heteroaryl ring substituted with A, wherein A is at least one member selected from the group of -H, -halo, -hydroxyl, -C₁₋₆alkyl, -C₁₋₆ alkoxy, and-COOR₂;
   R₂ is -H, -C₁₋₆ alkyl or -alkylsubstituted C₁₋₆ alkyl or -arylsubstituted C₁₋₆alkyl;
   Y is selected from the group of -COOR₂, -C(O)NR₂SO₂R₃, - C(O)NHSO₂NR₂R₂,-NR₂SO₂R₂, -SO₂NR₂R₂, -C₃₋₆ cycloalkyl-COOR₂, -C₁₋₆ alkenyl-COOR₂, -C₁₋₆ alkynyl-COOR₂, -C₁₋₆alkyl-COOR₂₎ -NHC(O)(CH₂)ₙ-COOR₂, -SO₂NR₂C(O)R₂, -tetrazole, and -CONHOH, wherein n=1-6;
   R₃ is -C₁₋₆ alkyl or alkylsubstituted C₁₋₆ alkyl;
   R₄ is selected from the group of H, -C₁₋₆ alkyl, -C₃₋₆ cycloalkyl, -C₁₋₆ substituted alkyl, -C₁₋₆ alkyl-heteroaryl, -C₁₋₆alkyl-substitutedheteroaryl, -C₁₋₆alkyl-NR₆R₇, -C₁₋₆alkyl-CONR₈R₉, -C₃₋₆cycloalkyl-CONR₈R₉, -C₃₋₆cycloalkyl-(CH₂)₁₋₃-NR₆R₇, -(CH₂)₁₋₃-C₃₋₆cycloalkyl-NR₆R₇; -(CH₂)₁₋₃-C₃₋₆cycloalkyl-(CH₂)₁₋₃-NR₆R₇; -C₁₋₆alkyl-Q₁, C₃₋₆ cycloalkyl-Q₁, -COR₁₀,-SO₂R₃ and -SO₂NR₂R₂;
   Q₁ = -hydroxy, -COOR₂, -halo, -SO₂Rₐ;
   Rₐ= C₁₋₆ alkyl, NR₂R₂, 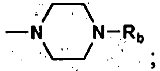
   R_{b}= -H, -C₁₋₆ alkyl, -COR₃, -SO₂R₃, -SONR₃R_{3;}
   R₄ can also be selected from the group of: 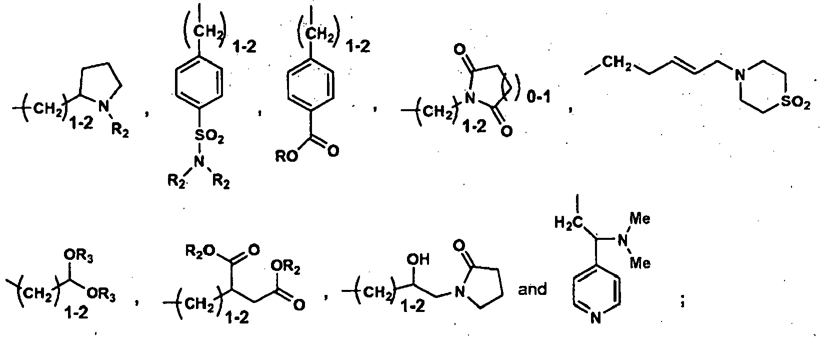
   R₅ is selected from the group of -H, -C₁₋₆ alkyl, -C₃₋₆ cycloalkyl, -C₁₋₆ alkylsubstituted alkyl, -COR₁₀,-SO₂R₃ and -SO₂NR₂R₂;
   with the proviso that only one of R₄ or R₅ can be selected from the group of -COR₁₀,-SO₂R₃ and -SO₂NR₂R₂; or R₄ and R₅ are taken together with the adjacent N to form a cycle such as 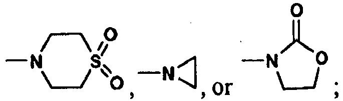
   R₁₀ is selected from the group of -H, -C₁₋₆ alkyl, -C₁₋₆ alkyl-NR₆R₇, -NR₁₁R₁₂, -OR₁₃, -C₁₋₆alkyl-Q₂, -C₃₋₆ cycloalkyl-Q₂, aryl-Q₂, wherein n =1-6,
   wherein Q₂ = hydroxy, -COOR₂, -halo, SO₂Rₐ, -CONHSO₂R₃, -CONHSO₂NR₂R₂;
   R₁₀ can also be selected from the group of: 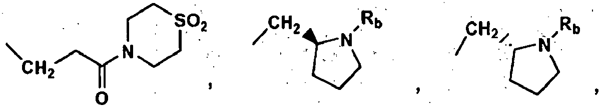 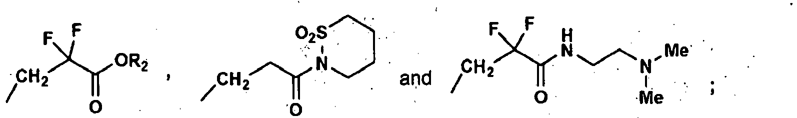
   R₆ and R₇ are independently selected from the group of -H, -C₁₋₆ alkyl, -C₁₋₆ substituted alkyl, aryl, heteroaryl, substituted aryl, substituted heteroaryl, and -C₁₋₆ alkyl-Q₁
   or R₆ and R₇ are taken together with the adjacent N to form a cycle selected from the group of 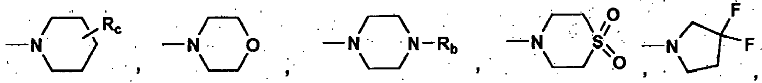 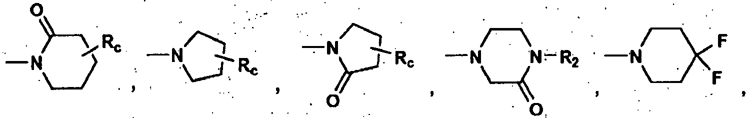 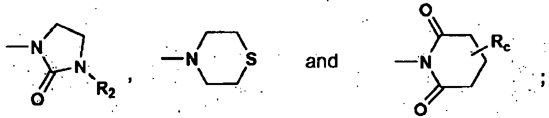
   R_{c}= C₁₋₆ alkyl, NR₂R₂, -COOR₃;
   R₈ and R₉ are independently selected from the group of -H, -C₁₋₆ alkyl, -C₃₋₆ cycloalkyl, -C₁₋₆ substituted alkyl, -C₁₋₆ alkyl-heteroaryl, -C₁₋₆ alkyl-substitutedheteroaryl, -C₁₋₆ alkyl-NR₂R₂, -C₁₋₆ alkyl-CONR₂R₂, -C₁₋₆ alkyl-Q₁, C₃₋₆ cycloalkyl-Q₁,
   or R₈ and R₉ can also be independently selected from the group of 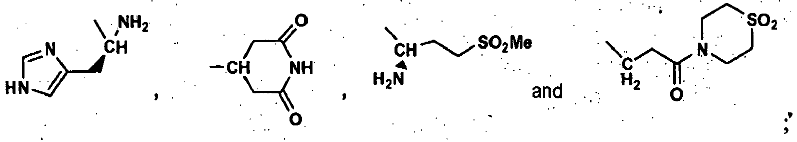
   or R₈ and R₉ are taken together with the adjacent N to form a cycle selected from the group of: 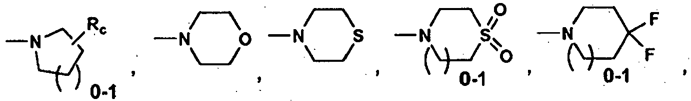 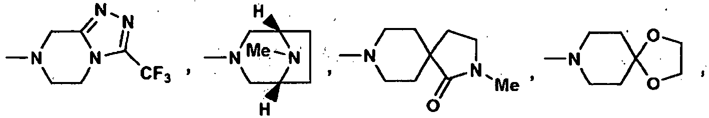 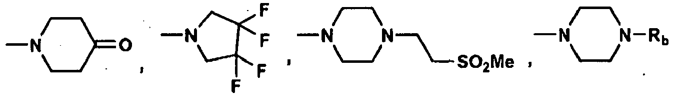 and 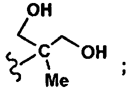
   and R₁₁ and R₁₂ are independently selected from the group of -H, -C₁₋₆ alkyl, -C₃₋₆ cycloalkyl, and -C₁₋₆ alkylsubstituted alkyl;
   or R₁₁ and R₁₂ are taken together with the adjacent N to form a cycle selected from the group of 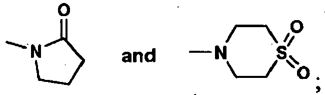 and
   R₁₃ is selected from the group of -H, -C₁₋₆ alkyl, -C₁₋₆ alkylsubstituted alkyl, and -C₁₋₆ alkyl NR₁₄R₁₅, wherein
   R₁₄ and R₁₅ are independently selected from the group of -H, -C₁₋₆ alkyl, and -C₁₋₆ alkylsubstituted alkyl, or R₁₄ and R₁₅ are taken together with the adjacent N to form a cycle selected from the group of

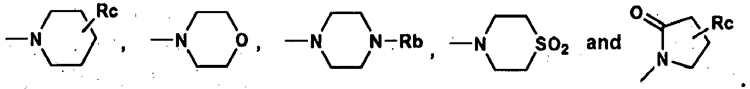

More preferred compounds include those which are encompassed by Formula I. Of these, those wherein X is a phenyl ring are even more preferred. Even more preferred are compounds of Formula I wherein X is a phenyl ring and Y is in the para position.

Also preferred are compounds of Formula I wherein A is at least one member selected from the group of -H, -OH, -halo, -C₁₋₃ alkyl, and -C₁₋₃ alkoxy, wherein -halo is selected from the group of -Cl, -F and -Br, with -F being more preferred.

Also preferred are compounds of Formula I wherein Y is -COOR₂, and more preferably -COOH.

In another preferred embodiment there is provided a compound of Formula Ia below wherein X is a phenyl ring and Y is -COOH in the para position:

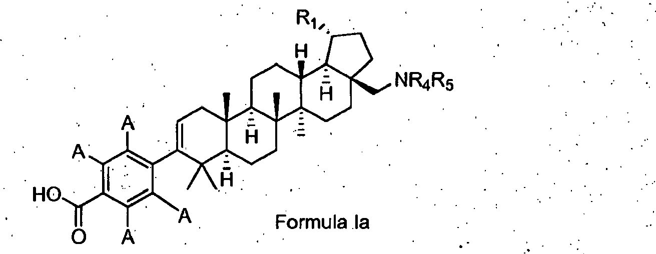

In this embodiment, it is also preferred that A is at least one member selected from the group of -H, -halo, -OH, -C₁₋₃ alkyl and -C₁₋₃ alkoxy. It is particularly preferred that A is at least one member selected from the group of -H, -fluoro, -chloro, -OH, - methyl and -methoxy.

Other compounds which are preferred as part of the invention include the following: 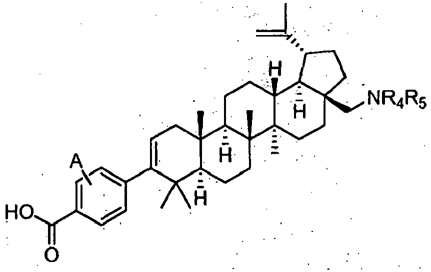 and

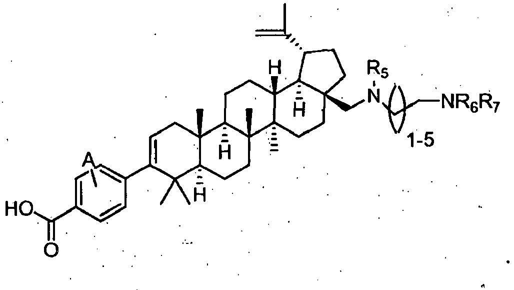

Even more preferred compounds include the following: 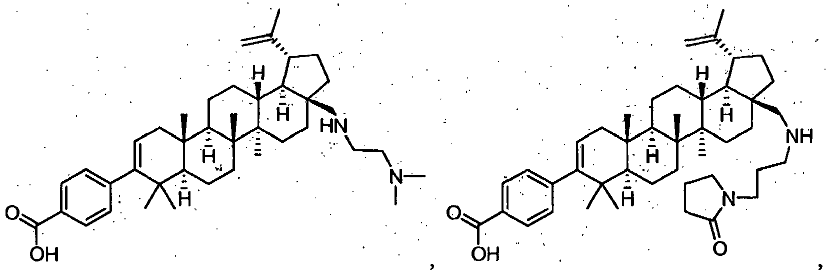 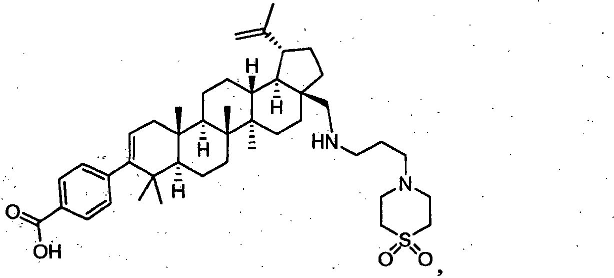 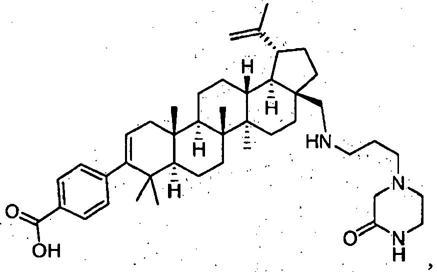 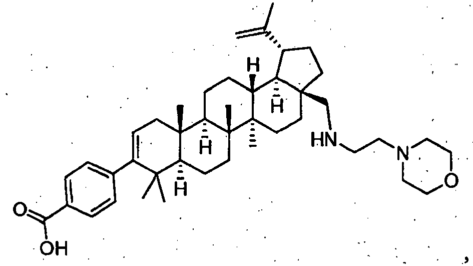 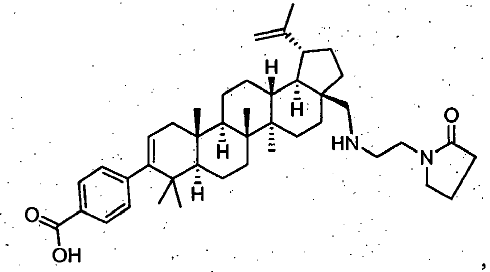 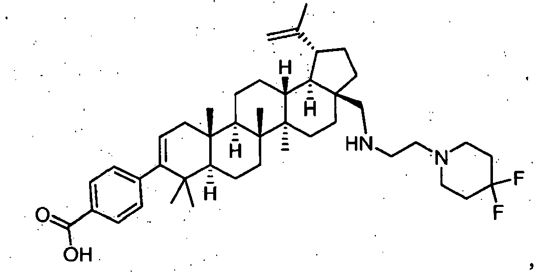 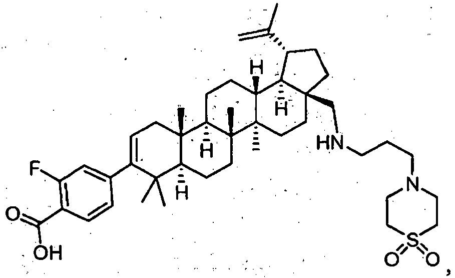 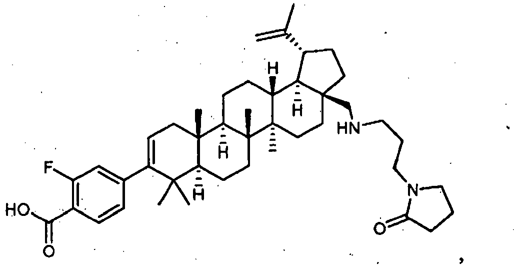 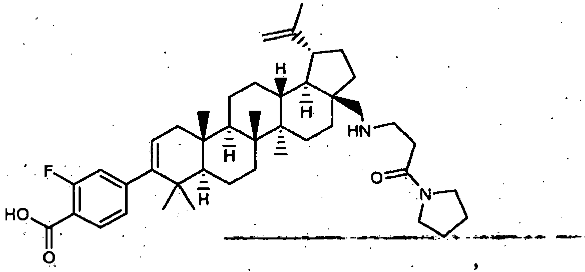 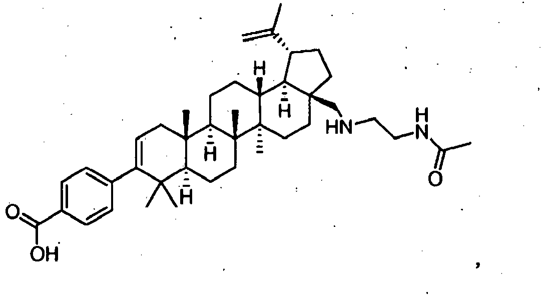 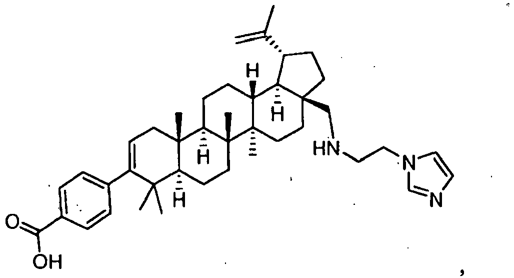 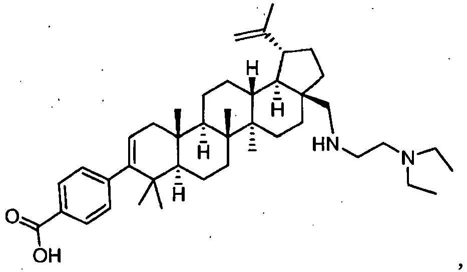 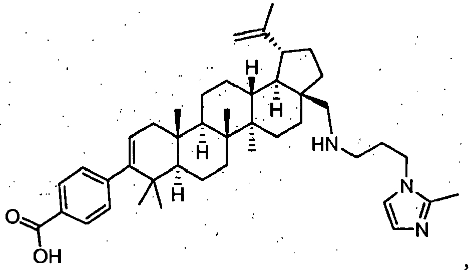 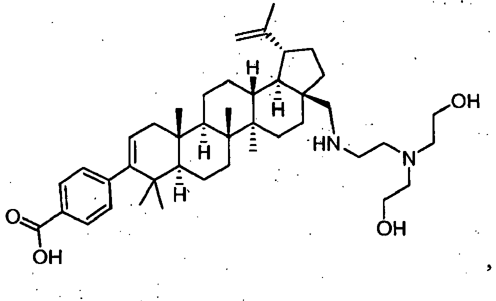 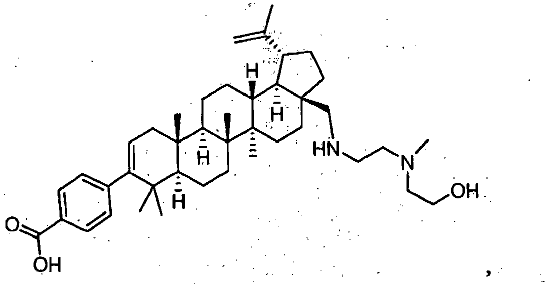 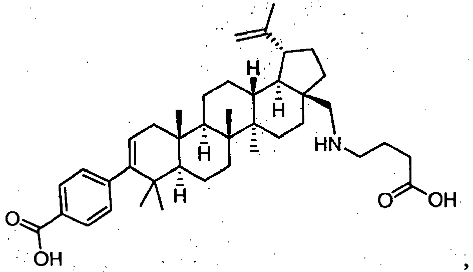 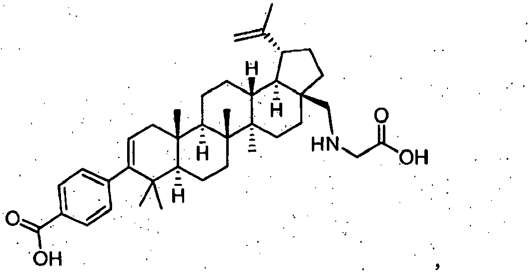 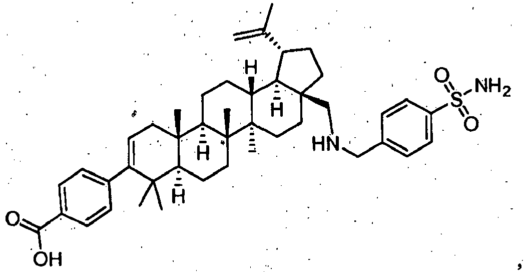 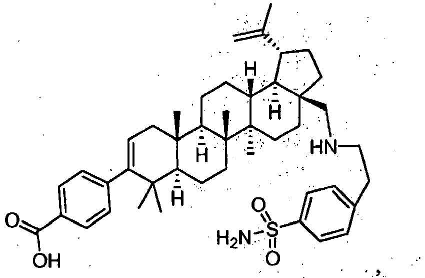 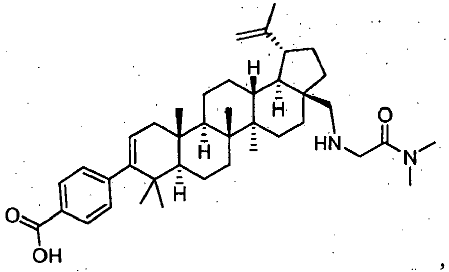 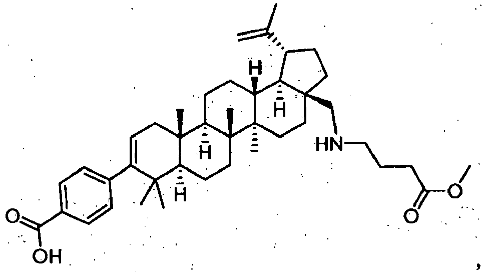 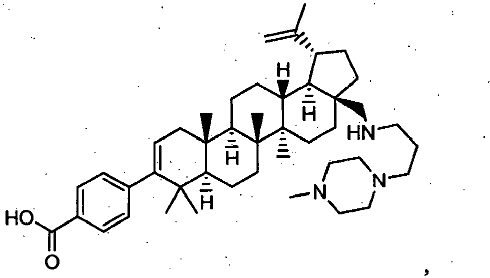 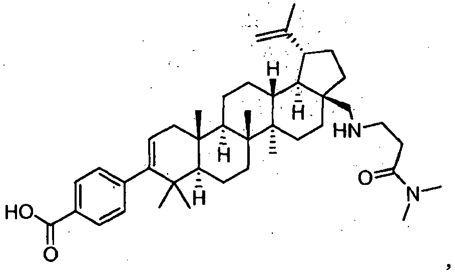 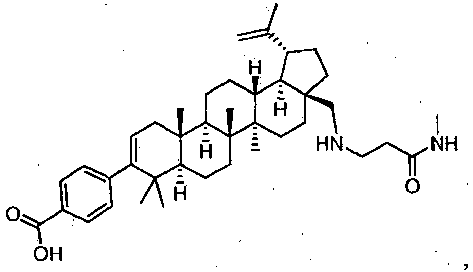 and

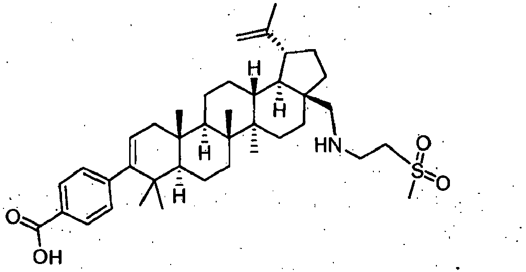

The compounds of the present invention, according to all the various embodiments described above, may be administered orally, parenterally (including subcutaneous injections, intravenous, intramuscular, intrasternal injection or infusion techniques), by inhalation spray, or rectally, and by other means, in dosage unit formulations containing non-toxic pharmaceutically acceptable carriers, excipients and diluents available to the skilled artisan. One or more adjuvants may also be included.

Thus, in accordance with the present invention, there are further provided compounds and a pharmaceutical composition, for treating viral infections such as HIV infection and AIDS. The treatment involves administering to a patient in need of such treatment a pharmaceutical composition which contains an antiviral effective amount of one or more of the compounds of Formulas I, II, and/or III, together with one or more pharmaceutically acceptable carriers, excipients or diluents. As used herein, the term "antiviral effective amount" means the total amount of each active component of the composition and method that is sufficient to show a meaningful patient benefit, i.e., inhibiting, ameliorating, or healing of acute conditions characterized by inhibition of the HIV infection. When applied to an individual active ingredient, administered alone, the term refers to that ingredient alone. When applied to a combination, the term refers to combined amounts of the active ingredients that result in the therapeutic effect, whether administered in combination, serially or simultaneously. The terms "treat, treating, treatment" as used herein and in the claims means preventing, ameliorating or healing diseases associated with HIV infection.

The pharmaceutical compositions of the invention may be in the form of orally administrable suspensions or tablets; as well as nasal sprays, sterile injectable preparations, for example, as sterile injectable aqueous or oleaginous suspensions or suppositories. Pharmaceutically acceptable carriers, excipients or diluents may be utilized in the pharmaceutical compositions, and are those utilized in the art of pharmaceutical preparations.

When administered orally as a suspension, these compositions are prepared according to techniques typically known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents, and lubricants known in the art.

The injectable solutions or suspensions may be formulated according to know art, using suitable non-toxic, parenterally acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

The compounds herein.set forth can be administered orally to humans in a dosage range of about 1 to 100 mg/kg body weight in divided doses, usually over an extended period, such as days, weeks, months, or even years. One preferred dosage range is about 1 to 10 mg/kg body weight orally in divided doses. Another preferred dosage range is about 1 to 20 mg/kg body weight in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Also contemplated herein are combinations of the compounds of Formulas I, II, and /or III herein set forth, together with one or more other agents useful in the treatment of AIDS. For example, the compounds of this disclosure may be effectively administered, whether at periods of pre-exposure and/or post-exposure, in combination with effective amounts of the AIDS antivirals, immunomodulators, antiinfectives, or vaccines, such as those in the following non-limiting table:

| ANTIVIRALS | | |
|---|---|---|
| *Drug Name* | *Manufacturer* | *Indication* |
| 097 | Hoechst/Bayer | HIV. infection, AIDS, ARC (non-nucleoside reverse transcriptase (RT) inhibitor) |
| Amprenavir 141 W94 GW 141 | Glaxo Wellcome | HIV infection, AIDS, ARC (protease inhibitor) |
| Abacavir (1592U89) GW 1592 | Glaxo Wellcome | HIV infection, AIDS, ARC (RT inhibitor) |
| Acemannan | Carrington Labs (Irving, TX) | ARC |
| Acyclovir | Burroughs Wellcome | HIV infection, AIDS, ARC |
| AD-439 | Tanox Biosystems | HIV infection, AIDS, ARC |
| AD-519 | Tanox Biosystems | HIV infection, AIDS, ARC |
| Adefovir dipivoxil AL-721 | Gilead Sciences Ethigen (Los Angeles, CA) | HIV infection ARC, PGL HIV positive, AIDS |
| Alpha Interferon | Glaxo Wellcome | Kaposi's sarcoma, HIV in combination w/Retrovir |
| Ansamycin LM 427 | Adria Laboratories (Dublin, OH) Erbamont (Stamford, CT) | ARC |
| Antibody which Neutralizes pH Labile alpha aberrant Interferon | Advanced Biotherapy Concepts (Rockville, MD) | AIDS, ARC |
| AR177 | Aronex Pharm | HIV infection, AIDS, ARC |
| Beta-fluoro-ddA | Nat'l Cancer Institute | AIDS-associated diseases |
| BMS-234475 (CGP-61755) | Bristol-Myers Squibb/ Novartis | HIV infection, AIDS, ARC (protease inhibitor) |
| CI-1012 | Warner-Lambert | HIV-1 infection |
| Cidofovir | Gilead Science | CMV retinitis, |
| | | herpes, papillomavirus |
| Curdlan sulfate | AJI Pharma USA | HIV infection |
| Cytomegalovirus Immune globin | Medlmmune | CMV retinitis |
| Cytovene | Syntex | Sight threatening |
| Ganciclovir | | CMV peripheral CMV retinitis |
| Darunavir | Tibotec- J & J | HIV infection, AIDS, ARC (protease inhibitor) |
| Delaviridine | Pharmacia-Upjohn | HIV infection, AIDS, ARC (RT inhibitor) |
| Dextran Sulfate | Ueno Fine Chem. Ind. Ltd. (Osaka, Japan) | AIDS, ARC, HIV positive asymptomatic |
| ddC Dideoxycytidine | Hoffman-La Roche | HIV infection, AIDS, ARC |
| ddI Dideoxyinosine | Bristol-Myers Squibb | HIV infection, AIDS, ARC; combination with AZT/d4T |
| DMP-450 | AVID (Camden, NJ) | HIV infection, AIDS, ARC (protease inhibitor) |
| Efavirenz (DMP 266, Sustiva^{®}) (-)6-Chloro-4-(S)-cyclopropylethynyl-4(S)-trifluoro-methyl-,1,4-dihydro-2H-3,1-benzoxazin-2-one, STOCRINE | Bristol Myers Squibb | HIV infection, AIDS, ARC (non-nucleoside RT inhibitor) |
| EL10 | Elan Corp, PLC | HIV infection |
| | (Gainesville, GA) | |
| Etravirine | Tibotec/ J & J | HIV infection, AIDS, ARC (non-nucleoside reverse transcriptase inhibitor) |
| Famciclovir | Smith Kline | herpes zoster, herpes simplex |
| GS 840 | Gilead | HIV infection, AIDS, ARC (reverse transcriptase inhibitor) |
| HBY097 | Hoechst Marion Roussel | HIV. infection, AIDS, ARC (non-nucleoside reverse transcriptase inhibitor) |
| Hypericin | VIMRx Pharm. | HIV infection, AIDS, ARC |
| Recombinant Human Interferon Beta | Triton Biosciences (Almeda, CA) | AIDS, Kaposi's sarcoma, ARC |
| Interferon alfa-n3 | Interferon Sciences | ARC, AIDS |
| Indinavir | Merck | HIV infection, AIDS, ARC, asymptomatic HIV positive, also in combination with AZT/ddI/ddC |
| ISIS 2922 | ISIS Pharmaceuticals | CMV retinitis |
| KNI-272 | Nat'l Cancer Institute | HIV-assoc. diseases |
| Lamivudine, 3TC | Glaxo Wellcome AIDS, | HIV. infection, AIDS, ARC (reverse transcriptase inhibitor); also with AZT |
| Lobucavir | Bristol-Myers Squibb | CMV infection |
| Nelfinavir | Agouron Pharmaceuticals | HIV infection, AIDS, ARC (protease inhibitor) |
| Nevirapine | Boeheringer Ingleheim | HIV. infection, AIDS, ARC (RT inhibitor) |
| Novapren | Novaferon Labs, Inc. (Akron, OH) | HIV inhibitor |
| Peptide T Octapeptide Sequence | Peninsula Labs (Belmont, CA) | AIDS |
| Trisodium Phosphonoformate | Astra Pharm. Products, Inc. | CMV retinitis, HIV infection, other CMV infections |
| PNU-140690 | Pharmacia Upjohn | HIV infection, AIDS, ARC (protease inhibitor) |
| Probucol | Vyrex | HIV infection, AIDS |
| RBC-CD4 | Sheffield Med. Tech (Houston, TX) | HIV infection, AIDS, ARC |
| Ritonavir | Abbott | HIV infection, AIDS,ARC (protease inhibitor) |
| Saquinavir | Hoffmann-LaRoche | HIV infection, AIDS, ARC (protease inhibitor) |
| Stavudine; d4T Didehydrodeoxy-Thymidine | Bristol-Myers Squibb | HIV infection, AIDS, ARC |
| Tipranavir | Boehringer Ingelheim | HIV infection, AIDS, ARC (protease inhibitor) |
| Valaciclovir | Glaxo Wellcome | Genital HSV & CMV infections |
| Virazole Ribavirin | Viratek/ICN (Costa Mesa, CA) | asymptomatic HIV positive, LAS, ARC |
| VX-478 | Vertex | HIV infection, AIDS, ARC |
| Zalcitabine | Hoffmann-LaRoche | HIV infection, AIDS, ARC, with AZT |
| Zidovudine; AZT | Glaxo Wellcome | HIV infection, AIDS, ARC, Kaposi's sarcoma, in combination with other therapies |
| Tenofovir disoproxil, fumarate salt (Viread^{®}) | Gilead | HIV infection, AIDS, (reverse transcriptase inhibitor) |
| Emtriva^{®} (Emtricitabine) (FTC) | Gilead | HIV infection, AIDS, (reverse transcriptase inhibitor) |
| Combivir^{®} | GSK | HIV infection, AIDS, (reverse transcriptase inhibitor) |
| Abacavir succinate (or Ziagen^{®}) | GSK | HIV infection, AIDS, (reverse transcriptase inhibitor) |
| Reyataz^{®} (or atazanavir) | Bristol-Myers Squibb | HIV infection AIDs, protease inhibitor |
| Fuzeon^{®} (Enfuvirtide or T-20) | Roche / Trimeris | HIV infection AIDs, viral Fusion inhibitor |
| Lexiva^{®} | GSK/Vertex | HIV infection |
| (or Fosamprenavir calcium) | | AIDs, viral protease inhibitor |
| Selzentry Maraviroc; (UK 427857) | Pfizer | HIV infection AIDs, (CCR5 antagonist, in development) |
| Trizivir^{®} | GSK | HIV infection AIDs, (three drug combination) |
| Sch-417690 (vicriviroc) | Schering-Plough | HIV infection AIDs, (CCR5 antagonist, in development) |
| TAK-652 | Takeda | HIV infection AIDs, (CCR5 antagonist, in development) |
| GSK 873140 (ONO-4128) | GSK/ONO | HIV infection AIDs, (CCR5 antagonist, in development) |
| Integrase Inhibitor MK-0518 Raltegravir | Merck | HIV infection AIDs |
| Truvada^{®} | Gilead | Combination of Tenofovir disoproxil fumarate salt (Viread^{®}) and Emtriva^{®} (Emtricitabine) |
| Integrase Inhibitor GS917/JTK-303 Elvitegravir | Gilead/Japan Tobacco | HIV Infection AIDs in development |
| Triple drug combination Atripla^{®} | Gilead/Bristol-Myers Squibb | Combination of Tenofovir disoproxil fumarate salt (Viread^{®}), Emtriva^{®} (Emtricitabine), and Sustiva^{®} (Efavirenz) |
| Festinavir^{®} 4'-ethynyl-d4T | Oncolys BioPharma BMS | HIV infection AIDs in development |
| CMX-157 | Chimerix | HIV infection |
| Lipid conjugate of nucleotide tenofovir | | AIDs. |
| GSK1349572 Integrase inhibitor | GSK | HIV infection AIDs |
| | | |

| IMMUNOMODULATORS | | |
|---|---|---|
| *Drug Name* | *Manufacturer* | *Indication* |
| AS-101 | Wyeth-Ayerst | AIDS |
| Bropirimine | Pharmacia Upjohn | Advanced AIDS |
| Acemannan | Carrington Labs, Inc. (Irving, TX) | AIDS, ARC |
| CL246,738 | Wyeth Lederle Labs | AIDS, Kaposi's sarcoma |
| FP-21399 | Fuki ImmunoPharm | Blocks HIV fusion with CD4+ cells |
| Gamma Interferon | Genentech | ARC, in combination w/TNF (tumor necrosis factor) |
| Granulocyte Macrophage Colony Stimulating Factor | Genetics Institute Sandoz | AIDS |
| Granulocyte Macrophage Colony Stimulating Factor | Hoechst-Roussel Immunex | AIDS |
| Granulocyte Macrophage Colony Stimulating Factor | Schering-Plough | AIDS, combination w/AZT |
| HIV Core Particle | Rorer | Seropositive HIV |
| Immunostimulant | | |
| IL-2 Interleukin-2 | Cetus | AIDS, in combination w/AZT |
| IL-2 Interleukin-2 | Hoffman-LaRoche Immunex | AIDS, ARC, HIV, in combination w/AZT |
| IL-2 Interleukin-2 (aldeslukin) | Chiron | AIDS, increase in CD4 cell counts |
| Immune Globulin Intravenous (human) | Cutter Biological (Berkeley, CA) | Pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute | AIDS, ARC |
| Alpha-2 Interferon | Schering Plough | Kaposi's sarcoma w/AZT, AIDS |
| Methionine-Enkephalin | TNI Pharmaceutical (Chicago,IL) | AIDS, ARC |
| MTP-PE Muramyl-Tripeptide | Ciba-Geigy Corp. | Kaposi's sarcoma |
| Granulocyte Colony Stimulating Factor | Amgen | AIDS, in combination w/AZT |
| Remune | Immune Response Corp. | Immunotherapeutic |
| rCD4 Recombinant Soluble Human CD4 | Genentech | AIDS, ARC |
| rCD4-IgG hybrids | | AIDS, ARC |
| Recombinant Soluble Human CD4 | Biogen | AIDS, ARC |
| Interferon Alfa 2a | Hoffman-La Roche | Kaposi's sarcoma AIDS, ARC, in combination w/AZT |
| SK&F106528 Soluble T4 | Smith Kline | HIV infection |
| Thymopentin | Immunobiology Research Institute (Annandale, NJ) | HIV infection |
| Tumor Necrosis Factor; TNF | Genentech | ARC, in combination w/gamma Interferon , |
| | | |

| ANTI-INFECTIVES | | |
|---|---|---|
| *Drug Name* | *Manufacturer* | *Indication* |
| Clindamycin with Primaquine | Pharmacia Upjohn | PCP |
| Fluconazole | Pfizer | Cryptococcal meningitis, candidiasis |
| Pastille Nystatin Pastille | Squibb Corp. | Prevention of oral candidiasis |
| Ornidyl Eflornithine | Merrell Dow | PCP |
| Pentamidine Isethionate (IM & IV) | LyphoMed (Rosemont, IL) | PCP treatment |
| Trimethoprim | | Antibacterial |
| Trimethoprim/sulfa | | Antibacterial |
| Piritrexim | Burroughs Wellcome | PCP treatment |
| Pentamidine Isethionate for Inhalation | Fisons Corporation | PCP prophylaxis |
| Spiramycin | Rhone-Poulenc diarrhea | Cryptosporidial |
| Intraconazole-R51211 | Janssen-Pharm. | Histoplasmosis; cryptococcal meningitis |
| Trimetrexate | Warner-Lambert | PCP |
| Daunorubicin | NeXstar, Sequus | Kaposi's sarcoma |
| Recombinant Human Erythropoietin | Ortho Pharm. Corp. | Severe anemia assoc. with AZT therapy |
| Recombinant Human Growth Hormone | Serono | AIDS-related wasting, cachexia |
| Megestrol Acetate | Bristol-Myers Squibb | Treatment of anorexia assoc. W/AIDS |
| Testosterone | Alza, Smith Kline | AIDS-related wasting |
| Total Enteral Nutrition | Norwich Eaton Pharmaceuticals | Diarrhea and malabsorption related to AIDS |

Additionally, the compounds of the disclosure herein set forth may be used in combination with HIV entry inhibitors. Examples of such HIV entry inhibitors are discussed in DRUGS OF THE FUTURE 1999,24(12), pp. 1355-1362; CELL, Vol. 9, pp. 243-246, Oct. 29,1999; and DRUG DISCOVERY TODAY, Vol. 5, No. 5, May 2000, pp. 183-194 and *Inhibitors of the entry of HIV into host cells.* Meanwell, Nicholas A.; Kadow, John F. Current Opinion in Drug Discovery & Development (2003), 6(4), 451-461. Specifically the compounds can be utilized in combination with attachment inhibitors, fusion inhibitors, and chemokine receptor antagonists aimed at either the CCR5 or CXCR4 coreceptor. HIV attachment inhibitors are also set forth in US 7,354,924 and US 2005/0209246.

It will be understood that the scope of combinations of the compounds of this application with AIDS antivirals, immunomodulators, anti-infectives, HIV entry inhibitors or vaccines is not limited to the list in the above Table but includes, in principle, any combination with any pharmaceutical composition useful for the treatment of AIDS.

Preferred combinations are simultaneous or alternating treatments with a compound of the present disclosure and an inhibitor of HIV protease and/or a non-nucleoside inhibitor of HIV reverse transcriptase. An optional fourth component in the combination is a nucleoside inhibitor of HIV reverse transcriptase, such as AZT, 3TC, ddC or ddI. A preferred inhibitor of HIV protease is Reyataz^{®} (active ingredient Atazanavir). Typically a dose of 300 to 600mg is administered once a day. This may be co-administered with a low dose of Ritonavir (50 to 500mgs). Another preferred inhibitor of HIV protease is Kaletra^{®}. Another useful inhibitor of HIV protease is indinavir, which is the sulfate salt of N-(2(R)-hydroxy-1-(S)-indanyl)-2(R)-phenylmethyl-4-(S)-hydroxy-5-(1-(4-(3-pyridyl-methyl)-2(S)-N'-(t-butylcarboxamido)-piperazinyl))-pentaneamide ethanolate, and is synthesized according to U.S. 5,413,999. Indinavir is generally administered at a dosage of 800 mg three times a day. Other preferred protease inhibitors are nelfinavir and ritonavir. Another preferred inhibitor of HIV protease is saquinavir which is administered in a dosage of 600 or 1200 mg tid. Preferred non-nucleoside inhibitors of HIV reverse transcriptase include efavirenz. These combinations may have unexpected effects on limiting the spread and degree of infection of HIV. Preferred combinations include those with the following (1) indinavir with efavirenz, and, optionally, AZT and/or 3TC and/or ddI and/or ddC; (2) indinavir, and any of AZT and/or ddI and/or ddC and/or 3TC, in particular, indinavir and AZT and 3TC; (3) stavudine and 3TC and/or zidovudine; (4) tenofovir disoproxil fumarate salt and emtricitabine.

In such combinations the compound of the present invention and other active agents may be administered separately or in conjunction. In addition, the administration of one element may be prior to, concurrent to, or subsequent to the administration of other agent(s).

### GENERAL CHEMISTRY (METHODS OF SYNTHESIS)

The present invention comprises compounds of Formulas I, II, and III, their pharmaceutical formulations, and their use in patients suffering from or susceptible to HIV infection. The compounds of Formulas I, II, and III also include pharmaceutically acceptable salts thereof. General procedures to construct compounds of Formulas I, II, and III and intermediates useful for their synthesis are described in the following Schemes (after the Abbreviations).

### Abbreviations

One or more of the following abbreviations, most of which are conventional abbreviations well known to those skilled in the art, may be used throughout the description of the disclosure and the examples:
Bz₂O = benzoic anhydride
TBTU = O-(benzotriazol-1-yl)-*N,N,N',N'*-tetramethyluronium tetrafluoroborate
HATU = 2-(IH-7-azabenzotriazol-1-yl)-1,1,3,3-tetramethyluronium hexafluorophosphate methanaminium
DCE = dichloroethane
DCM = dichloromethane
CDI = carbonyl diimidazole
prep. HPLC = preparative high performance liquid chromatography
rt = room temperature
DIPEA = diisopropylethylamine
DMAP = 4-dimethylaminopyridine
DMSO = dimethylsulfoxide
THF = tetrahydrofuran
KHMDS = potassium bis(trimethylsilyl)amide
min = minute(s)
h = hour(s)
sat. = saturated
TEA = triethylamine
EtOAc = ethyl acetate
TFA = trifluoroacetic acid
PCC = pyridinium chlorochromate
TLC = thin layer chromatography
Tf₂NPh = (trifluoromethylsulfonyl)methanesulfonamide
dioxane = 1,4-dioxane
PG = protective group
atm = atmosphere(s)
mol = mole(s)
mmol= milimole(s)
mg = milligram(s)
µg = microgram(s)
µl = microliter(s)
µm= micrometer(s)
mm= millimeter(s)
HOAc= acetic acid
MeOH= methanol
DMF= *N,N-*dimethylformamide
TBAF= tetrabutylammonium fluoride

The terms "C-3" and "C-28" refer to certain positions of a triterpene core as numbered in accordance with IUPAC rules (positions depicted below with respect to an illustrative triterpene: betulin):

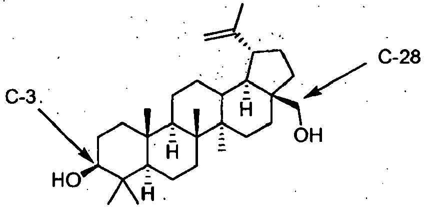

The same numbering is maintained when referring to the compound series in schemes and general descriptions of methods.

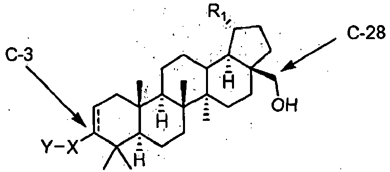

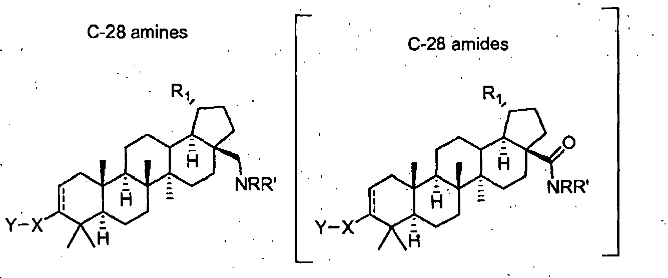

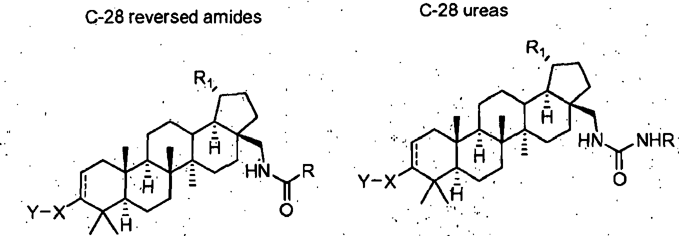

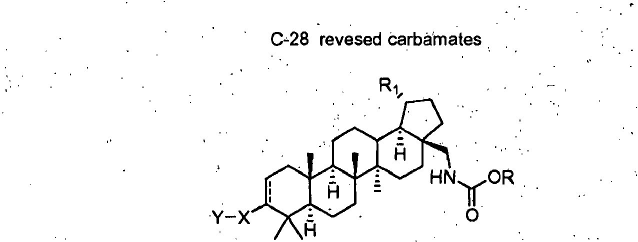

### Preparation of Compounds of Formulas I, II, and III General Chemistry Schemes:

### Preparation of Compounds of Formulas I, II and III General Chemistry Schemes:

Compounds of Formula I can be prepared from commercially available (Aldrich, others) betulinic acid and betulin by chemistry described in the following schemes. Compounds of Formula II and III are described thereafter.

General reaction schemes are set forth as follows:

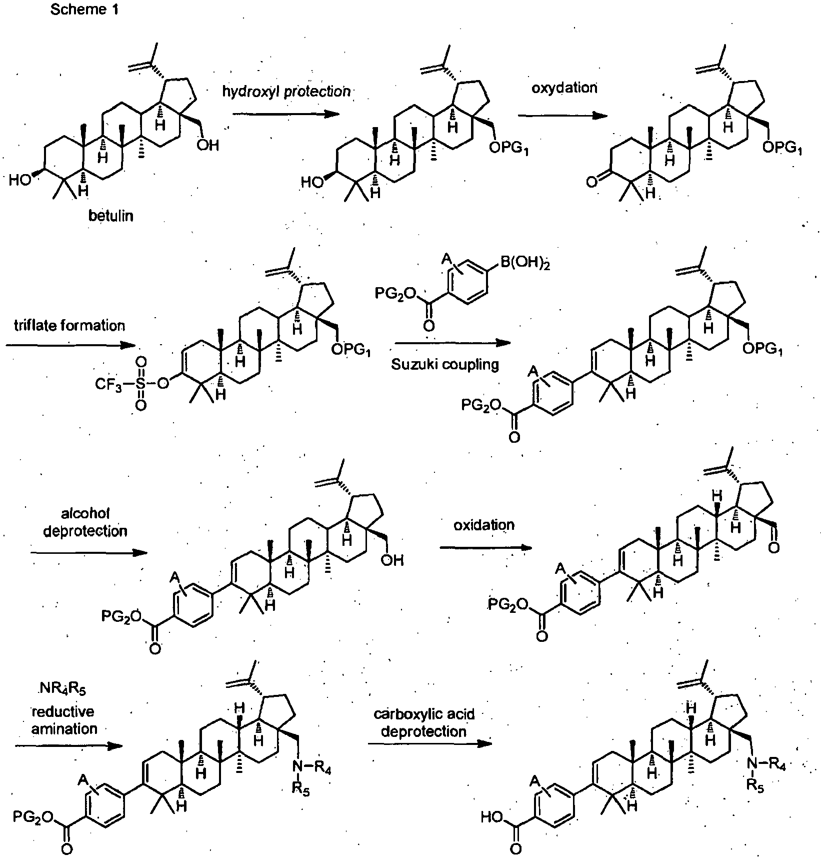

The hydroxyl group in the C-28 position of betulin can be protected with a suitable hydroxyl-protective group. Standard oxidation (i.e. PCC, Dess-Martin) of the C-3 hydroxyl group produces the C-3 ketone which is then converted into the triflate using conditions know to those skilled in the art. Palladium catalyzed cross coupling with boronic acid (Stille coupling using stannanes can also be used) afforded the corresponding C-3 modified betulin derivatives. Deprotection of the hydroxyl group in the C-28 position followed by oxidation under standard conditions (i.e. PCC) affords the corresponding aldehyde. Standard reductive amination of this aldehyde with amines using sodium triacetoxyborohydride (sodium cyanoborohydride can also be used) followed by deprotection of the carboxylic acid affords the desired C-28 amines.

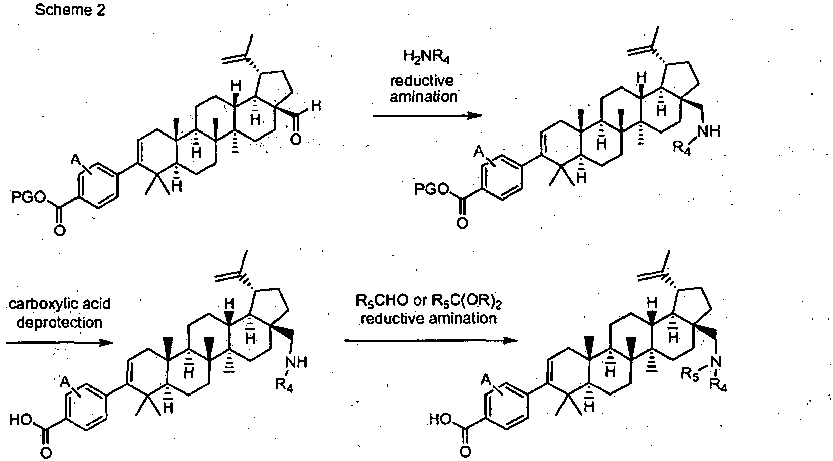

Alternatively, some of the C-28 tertiary amines can be prepared as describe in scheme 2: First, reductive amination of C-28 aldehyde with a primary amine generates a C-28 secondary amine. Deprotection of the carboxylic acid followed by reductive amination under standard conditions of the C-28 secondary amine with an aldehyde of a dialkylacetal affords the desired C-28 tertiary amine.

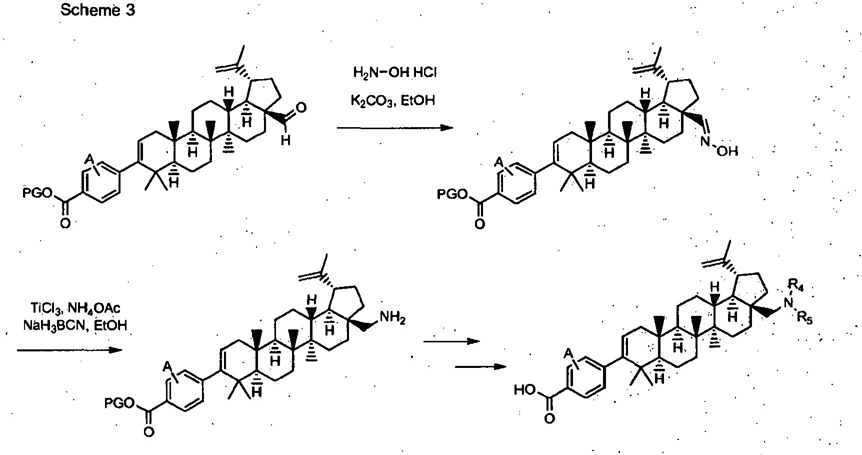

Alternatively, the C-28 amines can be prepared by converting the C-28 aldehyde in the corresponding oxime by treatment with hydroxylamine under standard conditions. Reduction of the hydroxyl amine using sodium cyanoborohydride in the presence of titanium trichloride afforded the C-28 primary amine which can be further derivatized using methods known to those skilled in the art to provide the desired final products.

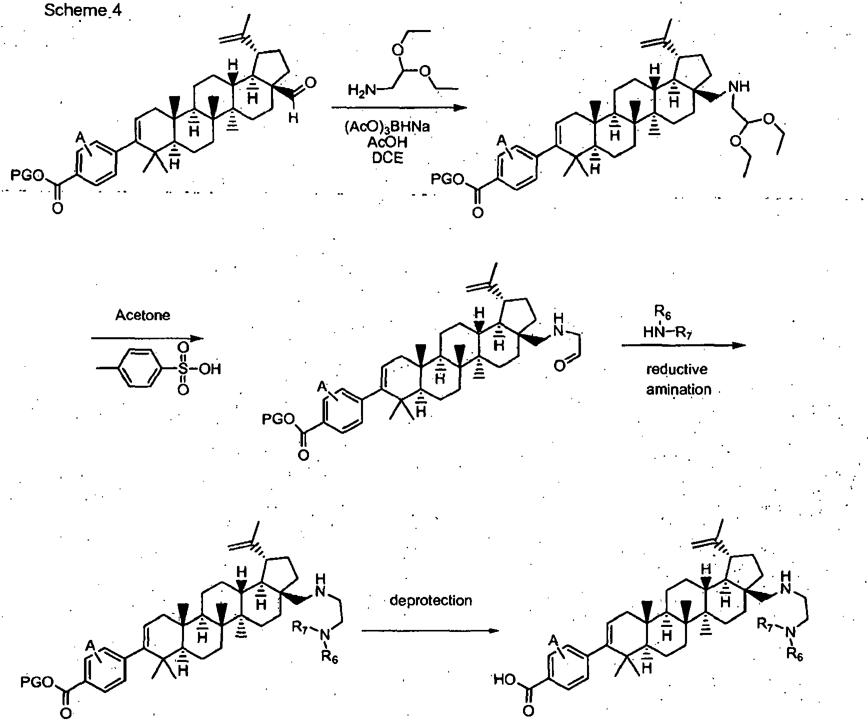

Some of the C-28 amines can be prepared as described in scheme 4: Reductive amination under standard conditions of the C-28 aldehyde in the presence of 2,2-diethoxyethanamine followed by ketal hydrolysis generates a the C-28 amine carrying an aldehyde which can also be submitted to reductive amination under standard conditions. Deprotection of the carboxylic acid produces the desired C-28 amine.

Substituents R₄, R₅, R₆ and R₇ may contain functional groups (i.e. COOH, COOR, OH, NHR) that can be further modified by methods know to those skilled in the art.

The modification can be carried out before or after the final deprotection of the carboxylic acid is performed depending on the nature of the functional group

Alternatively, when R₄, R₅, R₆ and/or R₇ are H, the corresponding amine can be further modified (for example by alkylation, acylation, Michael addition, etc.) by methods known to those skilled in the art. Saturation of the isopropenyl group can be accomplished by hydrogenation under standard conditions of the final products.

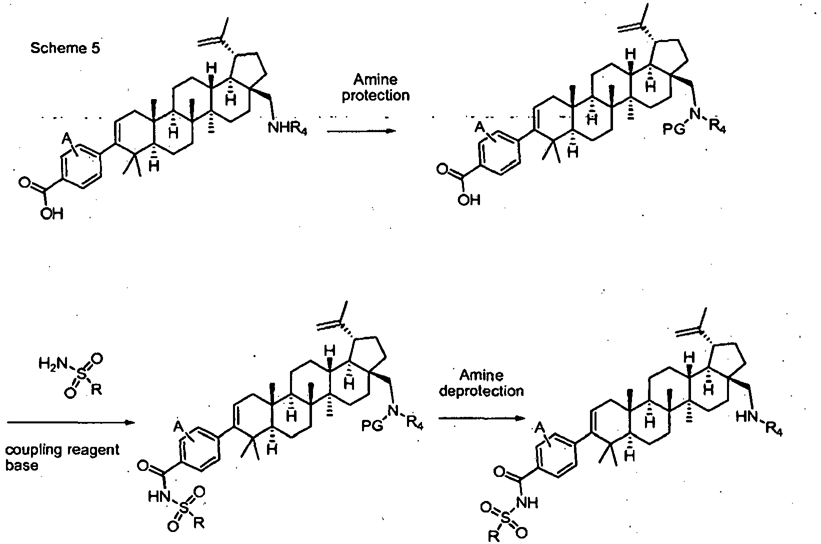

The benzoic acid can be further modified by methods known to those skilled in the art. An example of such modifications is shown in scheme 5: A suitable protective group is installed under standard conditions to mask the free NH in the C-28 position. Then, treatment of the carboxylic acid with the corresponding nucleophile, for example a sulfonyl amide or urea in the presence of a coupling reagent and a base followed by removal of the C-28 amine protective group affords the desired final product.

Compounds of formula I where the modification in the C-3 position is other than benzoic acid can be prepared by selecting the corresponding boronic acid in the palladium cross coupling step shown in scheme 1 (scheme 6) and then using the chemistry methods described in the above schemes.

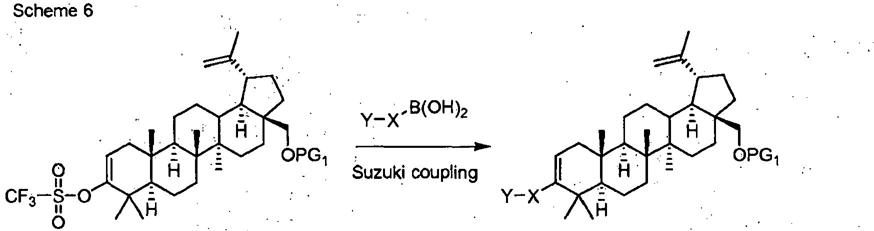

Alternatively, compounds of formula I can be prepared using the method described below in scheme 7. The C-28 primary amine can be treated in the presence of base with 1,2-disubstituted ethane where the substituents are two leaving groups (i.e, tosylate, mesylate, Br, Cl, I), to form the C-28 aziridine. Opening of the aziridine with a nucleophile can be achieve with or without heat to produce the corresponding secondary C-28 amine which can be further modified.

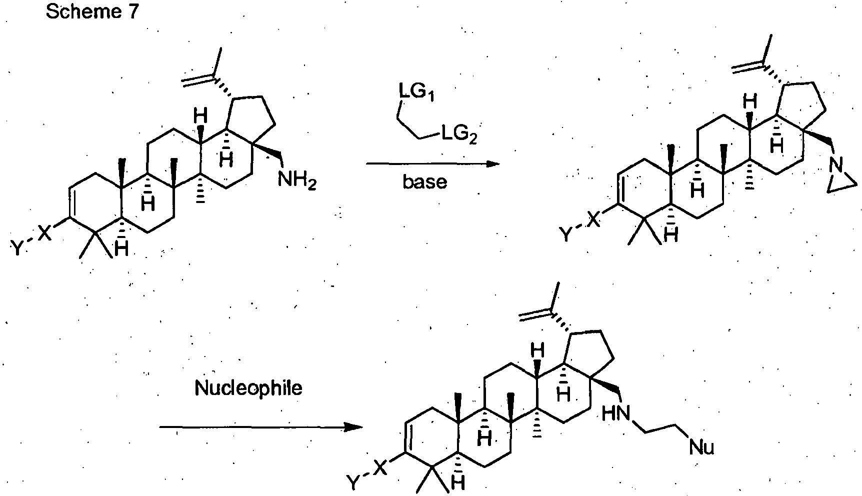

Compounds of formula II can be prepared using the chemistry methods described above for compounds of formula I, with one extra step which consist on the saturation of the double bonds as shown below in scheme 8:

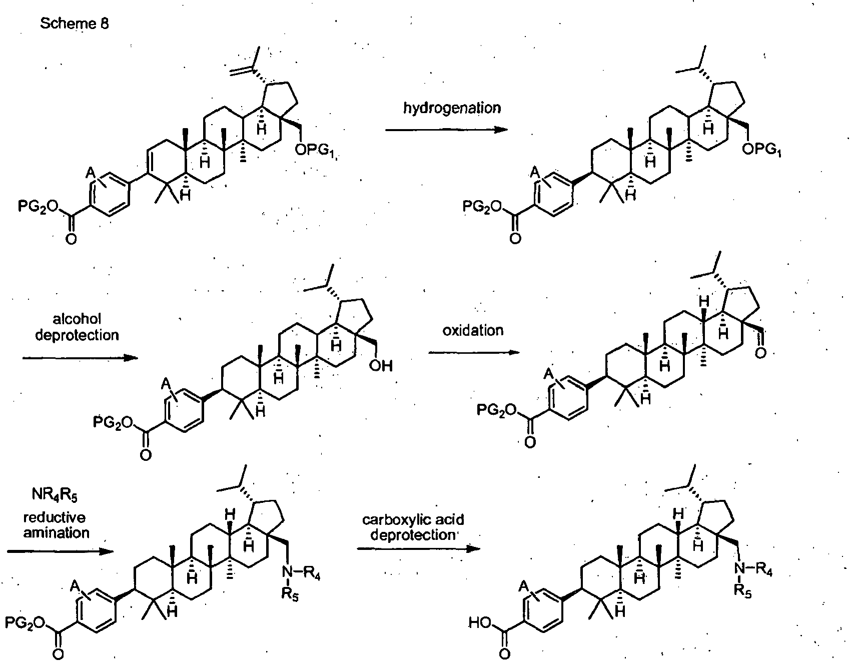

Compound of formula III can be prepared in the same manner described above for compounds of formula I and II using oleanoic or ursolic acid as starting materials instead of betulin.

### EXAMPLES

The following examples illustrate typical syntheses of the compounds of Formulas I, II and III as described generally above. These examples are illustrative only and are not intended to limit the disclosure in any way. The reagents and starting materials are readily available to one of ordinary skill in the art.

### Chemistry

### Typical Procedures and Characterization of Selected Examples:

Unless otherwise stated, solvents and reagents were used directly as obtained from commercial sources, and reactions were performed under a nitrogen atmosphere.

Flash chromatography was conducted on Silica gel 60 (0.040-0.063 particle size; EM Science supply). ¹H NMR spectra were recorded on Bruker DRX-500f at 500 MHz (or Bruker AV 400 MHz, Bruker DPX-300B or Varian Gemini 300 at 300 MHz as stated). The chemical shifts were reported in ppm on the δ scale relative to δTMS = 0. The following internal references were used for the residual protons in the following solvents: CDCl₃ (δ_{H} 7.26), CD₃OD (δ_{H} 3.30), Acetic-d4 *(Acetic Acid d₄*) (δ_{H} 11.6, 2.07), DMSO mix or DMSO-D6_CDCl₃ ((_{H} 2.50 and 8.25) (ratio 75%:25%), and DMSO-D6 (δ_{H} 2.50). Standard acronyms were employed to describe the multiplicity patterns: s (singlet), br. s (broad singlet), d (doublet), t (triplet), q (quartet), m (multiplet), b (broad), app (apparent). The coupling constant (*J*) is in Hertz. All Liquid Chromatography (LC) data were recorded on a Shimadzu LC-10AS liquid chromatograph using a SPD-10AV UV-Vis detector with Mass Spectrometry (MS) data determined using a Micromass Platform for LC in electrospray mode.

### LC/MS methods

### Method 1

Start % B = 20, final % B = 100 over 1 min gradient
Flow Rate = 4 ml/min
Wavelength = 254
Solvent A = 10% MeOH - 90% Water - 0.1% TFA
Solvent B = 90% MeOH - 10% Water - 0.1% TFA
Column 3 = Xbridge Phenyl 4.6 x 50 mm S5

### Method 2

Start % B = 30, final % B = 100 over 1 min gradient
Flow Rate = 0.8 ml/min
Wavelength = 220
Solvent A = 10% Methanol/ 90% Water/ 0.1% TFA
Solvent B = 90% Methanol/ 10% Water/ 0.1% TFA
Column 3 = Xbridge Phenyl 2.1 x 50 mm 2.5µm

### Method 3

Start % B = 20, final % B = 100 over 2 min gradient
Flow Rate = 0.8 ml/min
Wavelength = 254
Solvent A = 10% Methanol/ 90% Water/ 0.1% TFA
Solvent B = 90% Methanol/ 10% Water /0.1% TFA
Column 3 = Xbridge Phenyl 2.1 x 50 mm 2.5 µm

### Method 4

Start %B = 0, final %B = 100 over 2 minute gradient
Flow Rate = 4 ml/min
Wavelength = 220
Solvent A = 95% Water/ 5% Methanol/ 10 mM Ammonium Acetate
Solvent B = 5% Water/ 95% Methanol/ 10 mM Ammonium Acetate
Column = PHENOMENEX- LUNA 3.0 x 50 mm

### Method 5

Start %B = 0, final % B = 100 over 2 minute gradient
Flow Rate = 4 ml/min
Wavelength = 220
Solvent A = 95% Water/ 5% methanol/ 10 mM Ammonium Acetate
Solvent B = 5% Water/ 95% methanol/ 10 mM Ammonium Acetate
Column = Xbridge 4.6 x 50 mm 5µ C18

### Method 6

Start %B = 40, final % B = 100 over 2 minute gradient
Flow Rate = 1 ml/min
Wavelength = 220
Solvent A = 95% Water/ 5% methanol/ 10 mM Ammonium Acetate
Solvent B = 5% Water/ 95% methanol/ 10 mM Ammonium Acetate
Column = PHENOMENEX- LUNA C18,2.0 x 30, µm

### Method 7

Start %B = 0, final % B = 100 over 2 minute gradient
Flow Rate = 5 ml/min
Wavelength = 220
Solvent A = 95% Water/ 5% methanol/ 10 mM TFA
Solvent B = 5% Water/ 95% methanol/ 10 mM TFA
Column = PHENOMENEX- LUNA 3.0 x 50 mm S10

### Method 8

Start%B = 0, Final%B = 100 over 2 minute gradient, hold at 100%B
Flow Rate = 1 mL / min
Wavelength = 220 nm
Solvent A = 95% water, 5% methanol, 10 mM ammonium acetate
Solvent B = 5% water, 95% methanol, 10 mM ammonium acetate
Column = Phenomenex LUNA C18,2.0 x 30 mm, 3 µm

### Method 9

Start%B = 0%, Final%B = 100 over 2 minute gradient, hold at 100%B
Flow Rate = 1.0 mL / min
Wavelength = 220 nm
Solvent A = 90% water, 10% methanol, 0.1% TFA
Solvent B = 10% water, 90% methanol, 0.1% TFA
Column = phenomenex-luna , 2.0 x 30 mm, 3.0 µm

### Preparation of compounds

### Preparation of tert-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

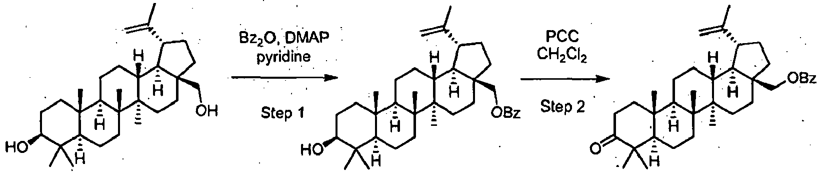

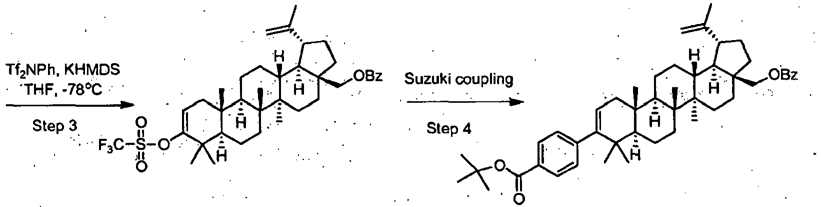

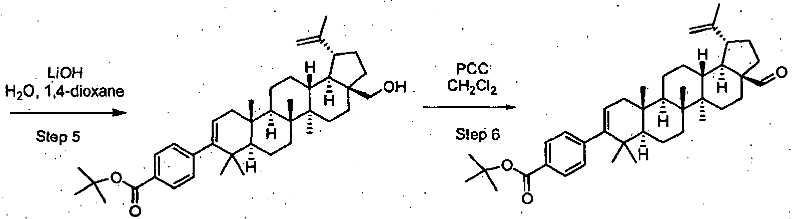

### Step 1. Preparation of ((1R,3aS,5aR,5bR,7aR,9S,11aR,11bR,13aR,13bR)-9-hydroxy-5a,5b,8,8,11a-pentamethyl-1-(prop-1-en-2-yl)icosahydro-1H-cyclopenta[a]chrysen-3a-yl)methyl benzoate.

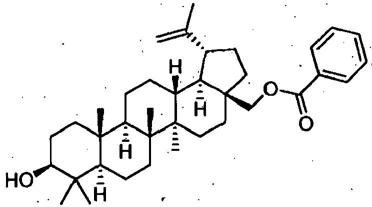

A suspension of (1R,3aS,5aR,5bR,7aR,9S,11aR,11bR,13aR,13bR)-3a-(hydroxymethyl)-5a,5b,8,8,11a-pentamethyl-1-(prop-1-en-2-yl)icosahydro-1H-cyclopenta[a]chrysen-9-ol (10 g, 22.59 mmol) and DMAP (0.552 g, 4.52 mmol) in pyridine (100 ml) was heated to 50 °C. Upon heating all solids dissolved. To the solution was added benzoic anhydride (7.66 g, 33.9 mmol) portion wise (4 portions) over 1 h, each time rinsing the sides of the flask with 5 ml of pyridine. The clear, colorless solution was stirred at 50 °C for 4 h then was cooled to rt and concentrated under reduced pressure. The thick amber residue was diluted with sat. NaHCO₃ (200 ml) and was extracted with dichloromethane (3 x 150 ml). The combined organic layers were dried with Na₂SO₄, the drying agent was removed by filtration and the filtrate was concentrated under reduced pressure. The residue was purified by flash chromatography using a 0-25% EtOAc in hexanes gradient to afford the title compound as a white foam (8.6 g, 15.73 mmol, 69.6 % yield). ¹H NMR (500 MHz, *CHLOROFORM-d*) δ ppm 8.05 (d, *J*=7.02 Hz, 2 H),7.55 (t, *J*=7.32 Hz, 1 H), 7.44 (t, *J*=7.63 Hz, 2 H), 4.71 (s, 1 H), 4.60 (s, 1 H), 4.51 (d, *J*=10.99 Hz, 1 H), 4.09 (d, *J*=10.99 Hz, 1 H), 3.15 - 3.21 (m, 1 H), 2.52 (td, *J*=10.99, 5.80 Hz, 1 H), 1.89 - 2.08 (m, 3 H), 1.70 (s, 3 H), 1.06 (s, 3 H), 1.00 (s, 3 H), 0.96 (s, 3 H), 0.83 (s, 3 H), 0.75 (s, 3 H), 0.63 - 1.81 (m, 21 H).

### Step 2. Preparation of ((1R,3aS,5aR,5bR,7aR,11aR,11bR,13aR,13bR)-5a,5b,8,8,11a-pentamethyl-9-oxo-1-(prop-1-en-2-yl)icosahydro-1H-cyclopenta[a]chrysen-3a-yl)methyl benzoate.

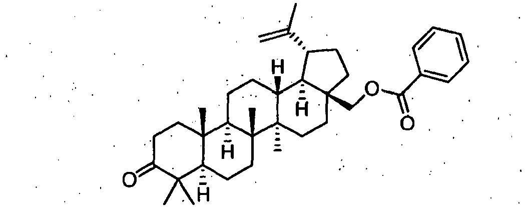

To a solution of ((1R,3aS,5aR,5bR,7aR,9S,11aR,11bR,13aR,13bR)-9-hydroxy-5a,5b,8,8,11a-pentamethyl-1-(prop-1-en-2-yl)icosahydro-1H-cyclopenta[a]chrysen-3a-yl)methyl benzoate (8.6 g, 15.73 mmol) in CH₂Cl₂ (100 ml) was added PCC (5.09 g, 23.59 mmol). After 7.25 h of stirring at rt, the mixture was filtered through a pad of celite and silica gel and was washed with dichloromethane then with 1:1 EtOAc:hexanes. The filtrate was concentrated under reduced pressure to give the title compound as a white foam (8.26 g, 15.16 mmol, 96 % yield). ¹H NMR (500 MHz, *CHLOROFORM-d)* δ ppm 8.05 (d, *J*=7.32 Hz, 2 H),7.56 (t, *J*=7.48 Hz, 1 H), 7.44 (t, *J*=7.63 Hz, 2 H), 4.72 (s, 1 H), 4.61 (s, 1 H), 4.52 (d, *J*=10.99 Hz, 1 H), 4.09 (d, *J*=11.29 Hz, 1 H), 2.45 - 2.58 (m, 2 H), 2.34 - 2.43 (m, 1 H), 1.86 - 2.10 (m, 4 H), 1.70 (s, 3 H), 1.10 (s, 3 H), 1.06 (s, 3 H), 1.04 - 1.82 (m, 18 H), 1.02 (s, 3 H), 1.01 (s, 3 H), 0.94 (s, 3 H).

### Step 3. Preparation of ((1R,3aS,5aR,5bR,7aR,11aR,11bR,13aR,13bR)-5a,5b,8,8,11a-pentamethyl-1-(prop-1-en-2-yl)-9-(trifluoromethylsulfonyloxy)-2,3,3a,4,5,5a,5b,6,7,7a,8,11,11a,11b,12,13,13a,13b-octadecahydro-1H-cyclopenta[a]chrysen-3a-yl)methyl benzoate.

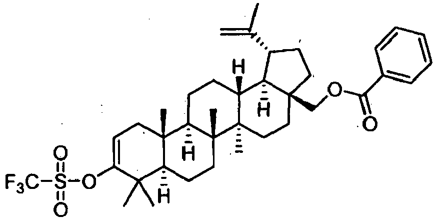

A solution of ((1R,3aS,5aR,5bR,7aR,11aR,11bR,13aR,13bR)-5a,5b,8,8,11a-pentamethyl-9-oxo-1-(prop-1-en-2-yl)icosahydro-1H-cyclopenta[a]chrysen-3a-yl)methyl benzoate (10.1 g, 18.54 mmol) in THF (100 ml) was cooled to -78 °C. To the solution was added KHMDS (0.5M in toluene) (74.2 ml, 37.1 mmol). The mixture was stirred for 15 minutes at -78 °C and a solution of *N*-Phenyl-bis(trifluoromethanesulfonimide) (7.29 g, 20.4 mmol) in THF (20 ml) and toluene (20 ml) was added via cannula. The mixture was stirred for 3.5 h at -78 °C. TLC indicated a trace of starting material was still present so an additional 0.7 g of *N*-phenyl-bis(trifluoromethanesulfonimide) was added to the mixture and stirring continued at-78 °C for 1 h. TLC indicated the reaction was complete. The mixture was diluted with water (75 ml) and extracted with ethyl acetate (3 x 75 ml). The combined organic layers were dried with MgSO₄. The drying agent was removed by filtration and the filtrate was concentrated under reduced pressure. The residue was purified flash chromatography using 0-20% toluene in hexanes gradient, then 20% toluene in hexanes, followed by a 10-15% EtOAc in hexanes to afford the title compound as a white foam (9.85 g, 14.55 mmol, 78 % yield). ¹H NMR (400 MHz, *CHLOROFORM-d*) δ ppm 0.94 (s, 3 H), 1.03 (s, 3 H), 1.04 (s, 3 H), 1.10 - 1.86 (m, 18 H), 1.12 (s, 3 H), 1.14 (s, 3 H), 1.73 (s, 3 H), 1.92 - 2.13 (m, 3 H), 2.18 (dd, *J*=17.07, 6.78 Hz, 1 H), 2.55 (td, *J*=11.11, 5.90 Hz, 1 H), 4.12 (d, *J*=11.04 Hz, 1 H), 4.55 (dd, *J*=11.04*,* 1.25 Hz, 1 H), 4.64 (s, 1 H), 4.75 (d, *J*=2.01 Hz, 1 H), 5.58 (dd, *J*=6.65, 1.88 Hz, 1 H), 7.43 - 7.49 (m, 2 H), 7.55 - 7.60 (m, 1 H), 8.05 - 8.09 (m, 2 H).

### Step 4. Preparation of tert-butyl 4-((1R,3aS,5aR,5bR,7aR,11aS,11bR,13aR,13bR)-3a-(benzoyloxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

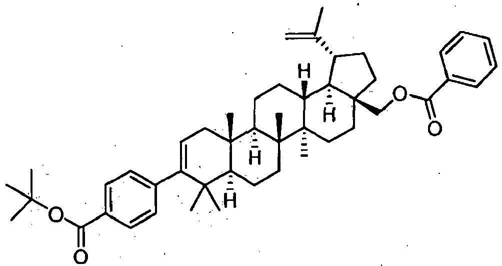

The title compound was prepared via Suzuki coupling as follows:

To a solution of ((1R,3aS,5aR,5bR,7aR,11aR,11bR,13aR,13bR)-5a,5b,8,8,11a-pentamethyl-1-(prop-1-en-2-yl)-9-(trifluoromethylsulfonyloxy)-2,3,3a,4,5,5a,5b,6,7,7a,8,11,11a,11b,12,13,13a,13b-octadecahydro-1H-cyclopenta[a]chrysen-3a-yl)methyl benzoate (9.85 g, 14.55 mmol) in 1,4-dioxane (50 ml) was added 2-propanol (50.0 ml), water (20 ml), sodium carbonate monohydrate (5.41 g, 43.7 mmol), 4-tert-butoxycarbonylphenylboronic acid (4.85 g, 21.83 mmol), and tetrakis(triphenylphospine)palladium(0) (0.504 g, 0.437 mmol). Potassium carbonate and potassium phosphate can also be used instead of sodium carbonate monohydrate. The sides of the flask were rinsed with an additional 20 ml of dioxane and the mixture was attached to a reflux condenser, was flushed with N₂ and was heated to reflux. Upon heating, the solids in the mixture dissolved completely. The solution was heated at reflux for 3.5 h, was cooled to rt and was diluted with 200 ml of water. The mixture was extracted with ethyl acetate (3 x 150 ml) and the combined organic layers were dried with Na₂SO₄. The drying agent was removed by filtration and the filtrate was concentrated under reduced pressure. The residue purified by flash chromatography using a 0-15% EtOAc in hexanes gradient to afford the title compound as a white foam (9.5 g, ∼83% pure based on ¹H NMR integrations). The product was used in the next step with no additional purification.

### Step 5. Preparation of tert-butyl 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

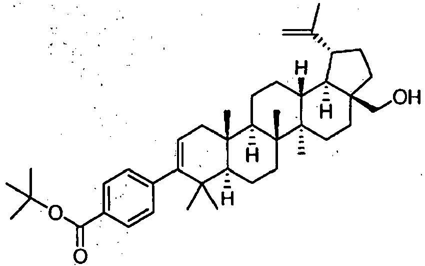

To a solution of *tert*-butyl 4-((1R,3aS,5aR,5bR,7aR,11aS,11bR,13aR,13bR)-3a-(benzoyloxymethyl)-5a,5b,8,8,11apentamethyl-1-(prop-1-en-2-yl)-2,3,3a,4,5,5a,5b,6,7,7a,8,11,11a,11b,12,13,13a,13b-octadecahydro-1H-cyclopenta[ a]chrysen-9-yl)benzoate (9.5 g, 13.47 mmol) in dioxane (200 ml) was added water (25 ml) and lithium hydroxide monohydrate (1.696 g, 40.4 mmol). The mixture was heated to 75 °C. Initially solids were apparent in the mixture, but after 2 h of heating, all solids had dissolved. After 23.5 h of heating, solids were again apparent in the mixture. The mixture was cooled to rt and 250 ml of water were added. The solids that had formed were collected by filtration and were washed with water. The solids were dissolved in ether and dichloromethane and were dried with MgSO₄. The drying agent was removed by filtration and the filtrate was concentrated under reduced pressure to afford the title compound as a white foam (5.6 g, 9.32mmol, 64 % yield for two steps. ¹H NMR (500 MHz, *CHLOROFORM-d*) δ ppm 7.87 (2 H, d, *J*=8.2 Hz), 7.16 (2 H, d, *J*=7.9 Hz), 5.26 (1 H, dd, *J*=6.3, 1.7 Hz), 4.69 (1 H, d, *J*=2.1 Hz), 4.58 (1 H, s), 3.82 (1 H, d, *J*=9.8 Hz), 3.35 (1 H, d, *J*=10.7 Hz), 2.40 (1 H, td, *J*=11.0, 5.8 Hz), 2.09 (1 H, dd, *J*=17.1, 6.1 Hz), 1.69 (3 H, s), 1.58 (9 H, s), 1.08 (3 H, s), 1.01 (3 H, s), 0.97 (3 H, s), 0.91 (6 H, s), 0.83 - 2.03 (21 H, m).

### Step 6. Preparation of tert-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

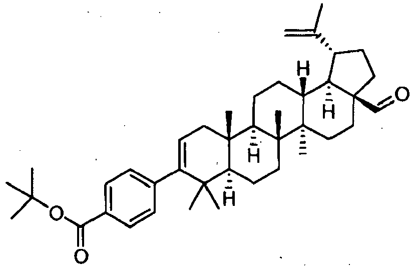

To a solution of *tert*-butyl 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (5.6 g, 9.32 mmol) in dichloromethane (100 ml) was added PCC (3.01 g, 13.98 mmol). The mixture was stirred at rt for 6.5 h then was filtered through a pad of celite and silica gel which was washed with dichloromethane then 1:1 ethyl acetate:hexanes. The filtrate was concentrated under reduced pressure and the residue was purified by flash chromatography using a 0-10% EtOAc in hexanes gradient to afford the title compound as a white solid (4.49 g, 7.50 mmol, 80 % yield). ¹H NMR (500 MHz, *CHLOROFORM-d*) δ ppm 9.68 (1 H, d, *J*=1.5 Hz), 7.87 (2 H, d, *J*=8.2 Hz), 7.16 (2 H, d, *J*=8.2 Hz), 5.26 (1 H, dd, *J*=6.4, 1.8 Hz), 4.76 (1 H, d, *J*=1.8 Hz), 4.63 (1 H, s), 2.88 (1 H, td, *J*=11.1, 5.8 Hz); 2:02-2.15 (3 H, m), 1.70 (3 H, s), 1.58 (9 H, s), 1.00 (3 H, s), 0.97 (3 H, s), 0.97 (3 H, s), 0.91 (6 H, s), 0.83 - 1.94 (19 H,m).

### Preparation of tert-butyl 4-((1R,3aS,5aR,5bR,7aR,11aS,11bR, 13aR, 13bR)-3a-((2-(dimethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

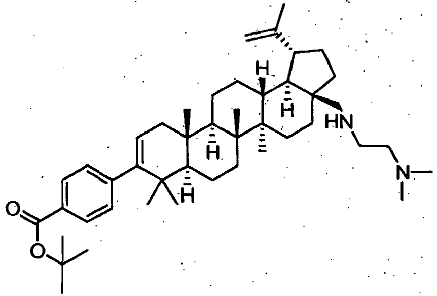

A mixture of tert-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (59 mg, 0.099 mmol), *N*1,*N*1-dimethylethane-1,2-diamine (0.13 ml, 1.2 mmol), sodium triacetoxyborohydride (104 mg, 0.493 mmol) and AcOH (0.023 ml, 0.394 mmol) in DCE (3ml) was stirred at rt for 12 h. The solvent was removed *in vacuo.* LCMS showed desired product and some imine not reduced yet. The residue was redissolved in DCE (3 ml) and treated again with sodium triacetoxyborohydride (104 mg, 0.493 mmol) and AcOH (0.023 ml, 0.394 mmol) at rt for 48 h. Solvent removed *in vacuo* and the residue redissolved in methylene chloride and purified by silica gel column (0-10% MeOH/CH₂Cl₂) to afford the title compound as a white solid (65mg, 98 % yield). ¹H NMR (400 MHz, *CHLOROFORM-d*) δ ppm 7.90 (d, *J*=8.3 Hz, 2 H), 7.19 (d, *J*=8.5 Hz, 2 H), 5.30 (dd, *J*=6.1, 1.6 Hz, 1 H), 4.72 (d, *J*=1.5 Hz, 1 H), 4.63 (s, 1 H), 3.17 - 2.95 (m, 3 H), 2.72 (t, *J*=5.9 Hz, 2 H), 2.54 (d, *J*=12.3 Hz, 1 H), 2.48 - 2.41 (m, 1 H), 2.40 (s, 6 H), 2.21-1.10 (m, 22 H), 1.72 (s, 3 H), 1.61 (s, 9 H), 1.12 (s, 3 H), 1.03 (s, 3 H), 1.00 (s, 3 H), 0.95 (s, 6 H).

### EXAMPLE 1. Preparation of 4-((1R,3aS,5aR,5bR,7aR,11aS,11bR,13aR,13bR)-3a-((2-(dimethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

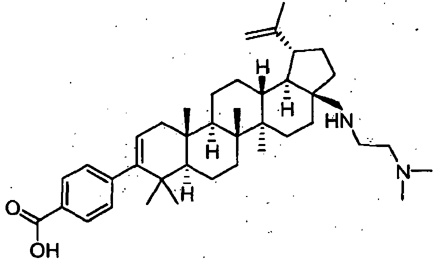

A solution of *tert*-butyl 4-((1R,3aS,5aR,5bR,7aR,11aS,11bR,13aR,13bR)-3a-((2-(dimethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (59 mg, 0.088 mmol) in DCM (2 ml) was treated with TFA (0.6 ml, 7.6 mmol) and the mixture was stirred at rt for 12 h. The solvent was removed *in vacuo* to provide the title compound as a white solid (40 mg, 0.065 mmol, 74.0 % yield). LCMS: m/e 615.7 (M+H)⁺, 2.00 min (method 7). ¹H NMR (400 MHz, *MeOD*) δ ppm 7.94 (d, *J*=8.3 Hz, 2 H), 7.24 (d, *J*=8.0 Hz, 2 H), 5.33 (d, *J*=4.8 Hz, 1 H), 4.78 (s, 1 H), 4.67 (s, 1 H), 3.73 - 3.42 (m, 4 H), 3.33 (m, 1 H), 2.99 - 2.94 (m, 1 H), 2.93 (s, 6 H), 2.53 (td, *J*=10.6, 5.6 Hz, 1 H), 2.99 - 1.29 (m, 2 H), 1.98 - 1.22 (m, 20 H), 1.76 (s, 3 H), 1.20 (s, 3 H), 1.10 (s, 3 H), 1.06 (s, 3 H), 0.99 (s, 3 H), 0.98 (s, 3 H).

### Preparation of tert-butyl 4-((1R,3aS,5aR,5bR,7aR,11aS,11bR,13aR,13bR)-5a,5b,8,8,11a-pentamethyl-3a-((3-morpholinopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

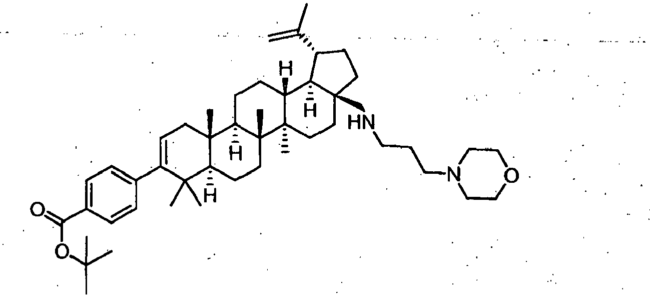

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (1M in DCM) (0.167 ml, 0.167 mmol) and *N*-(3-Aminopropyl)morpholine (0.029 ml, 0.200 mmol). The mixture was stirred for 15 minutes at rt and sodium triacetoxyborohydride (0.071 g, 0.334 mmol) was added. The mixture was stirred at rt for 1.5 h and an additional 0.1 g of sodium triacetoxyborohydride was added. The mixture was stirred overnight at rt then was quenched with 7 ml of sat. NaHCO₃. The mixture was extracted with dichloromethane (3 x 7 ml) and the combined organic layers were dried with Na₂SO₄. The drying agent was removed by filtration and the filtrate was concentrated under reduced pressure. The mixture was purified by flash chromatography using a 0-10% MeOH in dichloromethane gradient. The fractions containing the expected product were combined and concentrated under reduced pressure to give the title compound as a white foam (69 mg, 0.095 mmol, 56.8 % yield). LCMS: m/e 727.5 (M+H)⁺, 2.81 min (method 6).

### EXAMPLE 2. Preparation of 4-((1R,3aS,5aR,5bR,7aR,11aS,11bR,13aR,13bR)-5a,5b,8,8,11a-pentamethyl-3a-((3-morpholinopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

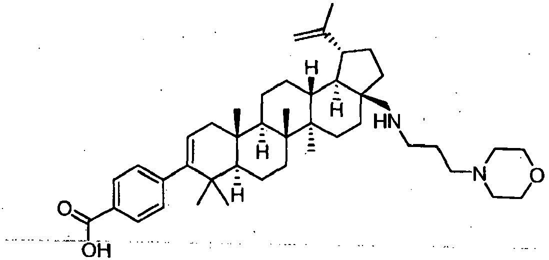

To a solution of *tert*-butyl 4-((1R,3aS,5aR,5bR,7aR,11aS,11bR,13aR,13bR)-5a,5b,8,8,11a-pentamethyl-3a-((3-morpholinopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (69 mg, 0.095 mmol) in DCM (1 ml) was added TFA (0.4 ml, 5.19 mmol). The mixture was stirred at rt for 3 h then was concentrated under reduced pressure. The residue was purified by prep. HPLC. The fractions containing the expected product were combined and concentrated under reduced pressure to afford the title compound (36 mg, 0.054 mmol, 56.5 % yield) as a white foam. LCMS: m/e 671.5 (M+H)⁺, 2.17 min (method 6). ¹H NMR (400 MHz, *CHLOROFORM-d*) δ ppm 7.94 (d, *J*=8.03 Hz, 2 H),7.15 (d, *J*=8.03 Hz, 2 H), 5.29 (d, *J*=4.52 Hz, 1 H), 4.71 (br. s., 1 H), 4.62 (br. s., 1 H), 3.73 (br. s., 4 H), 3.09 - 3.26 (m, 3 H), 2.65 (d, *J*=12.30 Hz, 1 H), 2.56 (br. s., 6 H), 2.42 (br. s., 1 H), 1.69 (s, 3 H), 1.07 (s, 3 H), 1.05 - 2.14 (m, 24 H), 1.00 (s, 3 H), 0.97 (s, 3 H), 0.93 (br. s., 3 H), 0.93 (br. s., 3 H).

### Preparation of tert-butyl 4-((1R,3aS,5aR,5bR,7aR,11aS,11bR,13aR,13bR)-3a-(((1-ethylpyrrolidin-2-yl)methylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

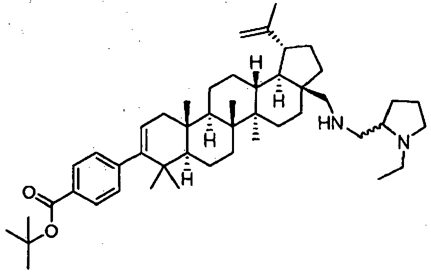

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (1M in DCM) (0.167 ml, 0.167 mmol) and 2-(aminomethyl)-1-ethylpyrrolidine (0.029 ml, 0.200 mmol). The mixture was stirred for 15 minutes at rt and sodium triacetoxyborohydride (0.071 g, 0.334 mmol) was added. The mixture was stirred at rt for 1.5 h then an additional 0.1 g of sodium triacetoxyborohydride was added and the mixture was stirred at rt overnight. After 24 h of stirring, some starting material still remained by TLC. To the mixture was added acetic acid (1M in DCM) (0.167 ml, 0.167 mmol), 2-(aminomethyl)-1-ethylpyrrolidine (0.029 ml, 0.200 mmol), and sodium triacetoxyborohydride (0.071 g, 0.334 mmol). The mixture was stirred at rt for an additional 19 h then was diluted with 7 ml of sat. NaHCO₃. The mixture was extracted with dichloromethane (3 x 7 ml) and the combined organic layers were dried with Na₂SO₄. The drying agent was removed by filtration and the filtrate was concentrated under reduced pressure. The mixture was purified by flash chromatography using a 0-10% MeOH in dichloromethane gradient. Two products were isolated as white foams. LC/MS indicated the same mass for both, with different retention times. ¹H NMR confirmed that two diastereomers were isolated. 44 mg of diastereomer 1 (less polar spot by TLC) was isolated while 55 mg of diastereomer 2 (more polar spot) was isolated. Diastereomer 1: LCMS: m/e 711.4 (M+H)⁺, 3.34 min (method 6). Diastereomer 2: LCMS: m/e 711.6 (M+H)⁺, 3.27 min (method 6).

### EXAMPLE 3 (diastereomer 1) and EXAMPLE 4 (diastereomer 2). Preparation of 4-((1R,3aS,5aR,5bR,7aR,11aS,11bR,13aR,13bR)-3a-(((1-ethylpyrrolidin-2-yl)methylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

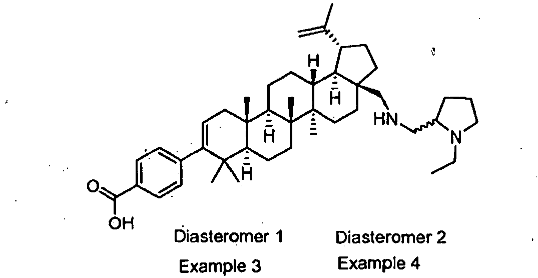

Two reactions were set up in separate vessels: To a solution of each the diastereomers isolated above (0.044 g of isomer 1 (less polar spot)), (0.044g of isomer 2 (more polar spot)) in DCM (1 ml) was added TFA (0.4 ml, 5.19 mmol). The mixtures were stirred at rt for 3 h then were concentrated under reduced pressure. The residues were purified by prep. HPLC. The fractions containing the expected products were concentrated under reduced pressure to give diastereomer 1 (31 mg, 0.047 mmol) and diastereomer 2 (37 mg, 0.056 mmol) as off-white foams. Diastereomer 1: LCMS: m/e 653.5 (M-H)⁻, 2.29 min (method 6). ¹H NMR (400 MHz, *CHLOROFORM-d*) δ ppm 7.96 (d, *J*=8.03 Hz, 2 H), 7.11 - 7.16 (m, 2 H), 5.31 (d, *J*=6.27 Hz, 1 H), 4.71 (br. s., 1 H), 4.60 (s, 1 H), 3.80 - 3.90 (m, 1 H), 2.32 - 3.45 (m, 9 H), 1.70 (s, 3 H), 1.17 (d, *J*=13.30 Hz, 3 H), 1.01 (br. s., 6 H), 0.99 - 2.19 (m, 29 H), 0.97 (s, 3 H), 0.90 (d, *J*=5.27 Hz, 3 H). Diastereomer 2: LCMS: m/e 653.5 (M-H)⁻, 2.30 min (method 6). ¹H NMR (400 MHz, *CHLOROFORM-d*) δ ppm 7.96 (d, *J*=8.03 Hz, 2 H). 7.09 - 7.18 (m, 2 H), 5.31 (d, *J*=6.02 Hz, 1 H), 4.71 (br. s., 1 H), 4.60 (br. s., 1 H), 3.77 - 3.92 (m, 1 H), 2.31 - 3.44 (m, 9 H), 1.70 (s, 3 H), 1.17 (d, *J*=12.05 Hz, 3 H), 1.01 (s, 6 H), 0.99 - 2.20 (m, 29 H), 0.97 (s, 3 H), 0.90 (d, *J*=5.02 Hz, 3 H).

### Preparation of 4-((1R,3aS,5aR,5bR,7aR,11aS,11bR,13aR,13bR)-5a,5b,8,8,11a-pentamethyl-1-(prop-1-en-2-yl)-3a-((2-(pyridin-2-yl)ethylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

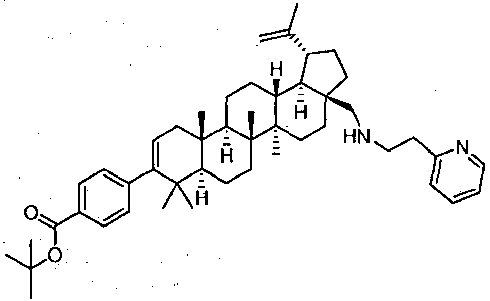

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (1M in DCM) (0.167 ml, 0.167 mmol) and 2-(2-pyridyl)ethylamine (0.024 ml, 0.200 mmol). The mixture was stirred for 15 minutes at rt and sodium triacetoxyborohydride (0.071 g, 0.334 mmol) was added. The mixture was stirred at rt for 1.5 h then an additional 0.1 g of sodium triacetoxyborohydride was added to the mixture and it was stirred for 23 h. To the mixture was added additional acetic acid (1M in DCM) (0.167 ml, 0.167 mmol), 2-(2-pyridyl)ethylamine (0.024 ml, 0.200 mmol), and sodium triacetoxyborohydride (0.071 g, 0.334 mmol). The mixture was stirred at rt for an additional 19 h, was diluted with 7 ml of sat. NaHCO₃ and was extracted with dichloromethane (3 x 7 ml). The combined organic layers were dried with Na₂SO₄, the drying agent was removed by filtration and the filtrate was concentrated under reduced pressure. The mixture was purified by flash chromatography using a 0-10% MeOH in dichloromethane gradient. The fractions containing the expected product were combined and concentrated under reduced pressure to afford the title compound. LCMS: m/e 705.4 (M+H)⁺, 2.90 min (method 6).

### EXAMPLE 5. Preparation of 4-((1R,3aS,5aR,5bR,7aR,11aS,11bR,13aR,13bR)-5a,5b,8,8,11a-pentamethyl-1-(prop-1-en-2-yl)-3a-((2-(pyridin-2-yl)ethylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

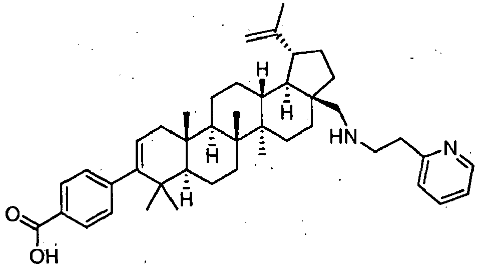

To a solution of *tert-*butyl 4-((1R,3aS,5aR,5bR,7aR,11aS,11bR,13aR,13bR)-5a,5b,8,8,11a-pentamethyl-1-(prop-1-en-2-yl)-3 a-((2-(pyridin-2-yl)ethylamino)methyl)-2,3,3a,4,5,5a,5b,6,7,7a,8,11,11a,11 b,12,13,13a,13b-octadecahydro-1H-cyclopenta[a]chrysen-9-yl)benzoate (0.119 g, 0.127 mmol) in DCM (1 ml) was added TFA (0.4 ml, 5.19 mmol). The mixture was stirred at rt for 3.5 h and the mixture was concentrated under reduced pressure. The residue was purified by prep. HPLC. The fractions containing the expected product were combined and concentrated under reduced pressure to give the title compound as a light-yellow foam (61 mg, 0.094 mmol, 74.3 % yield). LCMS: m/e 647.4 (M-H)⁻, 2.20 min (method 6). ¹H NMR (400 MHz, *CHLOROFORM-d*) δ ppm 8.42 - 8.48 (m, 1 H),7.95 (d, *J*=8.28 Hz, 2 H), 7.65 (td, *J*=7.65, 1.76 Hz, 1 H), 7.17 - 7.24 (m, 2 H), 7.14 (d, *J*=8.03 Hz, 2 H), 5.29 (d, *J*=4.52 Hz, 1 H), 4.71 (br. s., 1 H), 4.61 (br. s., 1 H), 3.45 - 3.59 (m, 2 H), 3.18 - 3.32 (m, 3 H), 2.74 (d, *J*=12.30 Hz, 1 H), 2.45 (br. s., 1 H), 1.70 (s, 3 H), 1.05 (s, 3 H), 1.01 (s, 3 H), 0.98 - 2.13 (m, 24 H), 0.96 (s, 3 H), 0.94 (br. s., 3 H), 0.92 (br. s., 3 H).

### Preparation of tert-butyl 4-((1R,3aS,5aR,5bR,7aR,11aS,11bR,13aR,13bR)-3a-((2-hydroxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

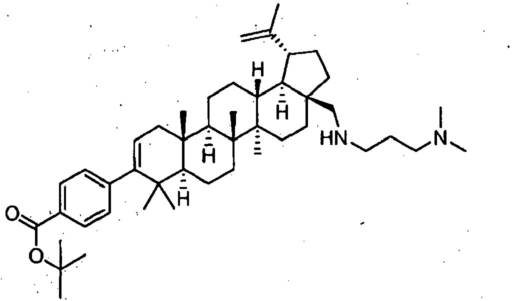

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (0.019 ml, 0.334 mmol) and 3-(dimethylamino)propylamine (0.084 ml, 0.668 mmol). To the mixture was added-sodium triacetoxyborohydride (0.177 g, 0.835 mmol) and it was stirred at rt for 72 h. After 72h of stirring, the reaction was diluted with 7 ml of sat. NaHCO₃ was extracted with dichloromethane (3 x 7 ml) and the combined organic layers were dried with Na₂SO₄. The drying agent was removed by filtration and the filtrate was concentrated under reduced pressure to give the expected product, *tert*-butyl 4-((1R,3aS,5aR,5bR,7aR,11aS,11bR,13aR,13bR)-3a-((3-(dimethylamino)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate. The crude product was used in the next step with no additional purification. LCMS: m/e 685.6 (M-H)⁻, 2.92 min (method 6).

### EXAMPLE 6. Preparation of 4-((1R,3aS,5aR,5bR,7aR,11aS,11bR,13aR,13bR)-3a-((3-(dimethylamino)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

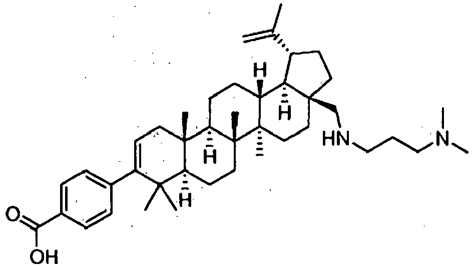

To a solution of *tert*-butyl 4-((1R,3aS,5aR,5bR,7aR,11aS,11bR,13aR,13bR)-3a-((3-(dimethylamino)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (114 mg, 0.167 mmol) in DCM (1 ml) was added TFA (0.4 ml, 5.19 mmol). The mixture was stirred at rt for 5 h and was concentrated under reduced pressure. The residue was purified by prep HPLC. The fractions containing the expected product were combined and concentrated under reduced pressure to afford the title compound as a white solid (77 mg, 0.122 mmol, 73.3 % yield). LCMS: m/e 629.6 (M-H)⁻, 2.22 min (method 6). ¹H NMR (500 MHz, *Acetic acid*) δ ppm 8.03 (d, *J*=8.24 Hz, 2 H)7.30 (d, *J*=8.24 Hz, 2 H), 5.37 (d, *J*=4.58 Hz, 1 H), 4.79 (s, 1 H), 4.68 (s, 1 H), 3.32 - 3.44 (m, 3.H), 3.25 - 3.32 (m, 2 H), 2.96 (d, *J*=13.12 Hz, 1 H), 2.89 - 2.94 (m, 6 H), 2.49 - 2.58 (m, 1 H), 2.30 - 2.38 (m, *J*=8.09, 7.86, 7.74, 7.74 Hz, 2 H), 1.75 (s, 3 H), 1.19 (s, 3 H), 1.13 - 2.23 (m, 22 H), 1.09 (s, 3 H), 1.08 (s, 3 H), 1.02 (s, 3 H), 1.00 (s, 3 H).

### Preparation of tert-butyl 4-((1R,3aS,5aR,5bR,7aR,11aS,11bR,13aR,13bR)-3a-((2-acetamidoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

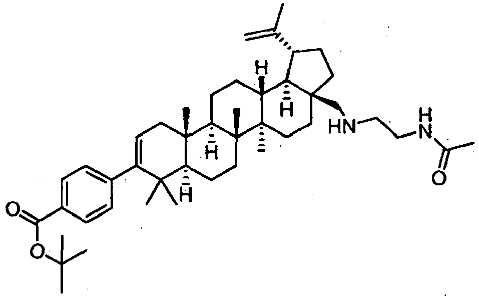

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (0.019 ml, 0.334 mmol) and *N*-acetylethylenediamine (0.048 ml, 0.501 mmol). The mixture was stirred for 2 h at rt then to the mixture was added sodium triacetoxyborohydride (0.177 g, 0.835 mmol). The mixture was stirred at rt for 3 days then was diluted with 7 ml of sat. NaHCO₃ and was extracted with dichloromethane (3 x 7 ml). The combined organic layers were dried with Na₂SO₄, the drying agent was removed by filtration, and the filtrate was concentrated under reduced pressure. The crude material was used in the next step with no additional purification. LCMS: m/e 685.4 (M+H)⁺, 3.86 min (method 6).

### EXAMPLE 7. Preparation of 4-((1R,3aS,5aR,5bR,7aR,11aS,11bR,13aR,13bR)-3a-((2-acetamidoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

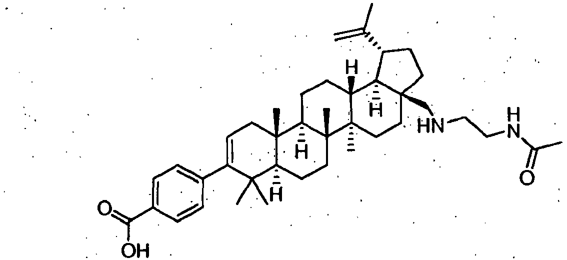

To a solution of *tert*-butyl 4-((1R,3aS,5aR,5bR,7aR,11aS,11bR,13aR,13bR)-3a-((2-acetamidoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (114 mg, 0.167 mmol) in DCM (1 ml) was added TFA (0.4 ml, 5.19 mmol). The mixture was stirred at rt for 3.5 h then was concentrated under reduced pressure. The residue was dissolved in dioxane and MeOH and was purified by pre. HPLC. The fractions containing the expected product were combined and concentrated under reduced pressure to afford the title compound as an off-white solid (40.7 mg, 0.065 mmol, 38.8 % yield). LCMS: m/e 629.5 (M+H)⁺, 2.17 min (method 6). ¹H NMR (500 MHz, *Acetic)* δ ppm 8.03 (d, *J*=8.24 Hz, 2 H),7.30 (d, *J*=8.24 Hz, 2 H), 5.37 (d, *J*=4.58 Hz, 1 H), 4.79 (s, 1 H), 4.68 (s, 1 H), 3.61 - 3.70 (m, 2 H), 3.40 - 3.53 (m, 2 H), 3.37 (d, *J*=13.12 Hz, 1 H), 2.98 (d, *J*=12.82 Hz, 1 H), 2.49 - 2.57 (m, 1 H), 2.09 (s, 3 H), 1.75 (s, 3 H), 1.19 (s, 3 H), 1.14 - 2.24 (m, 22 H), 1.10 (s, 3 H), 1.07 (s, 3 H), 1.02 (s, 3 H), 1.00 (s, 3 H).

### Preparation of tert-butyl 4-(2-(((1R,3aS,5aR,5bR,7aR,11aS,11bR,13aR,13bR)-9-(4-(tert-butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)ethyl)piperazine-1-carboxylate.

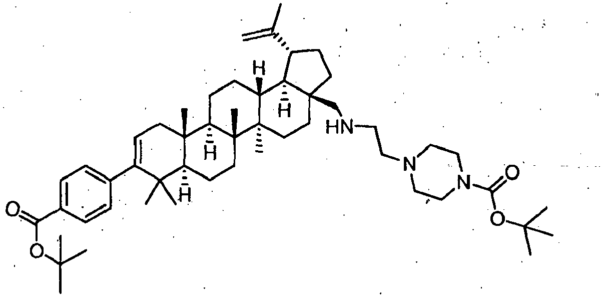

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (0.019 ml, 0.334 mmol) and 4-*N*-(2-Aminoethyl)-1-*N*-Boc-piperazine (0.077 g, 0.334 mmol). The mixture was stirred at rt for 2 h then to.the mixture was added sodium triacetoxyborohydride (0.177 g, 0.835 mmol). The mixture was stirred for 3 days at rt then was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure. The crude product was used in the next step without further purification. LCMS: m/e 812.3 (M+H)⁺, 3.30 min (method 6).

### EXAMPLE 8. Preparation of 4-((1R,3aS,5aR,5bR,7aR,11aS,11bR,13aR,13bR)-5a,5b,8,8,11a-pentamethyl-3a-((2-(piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

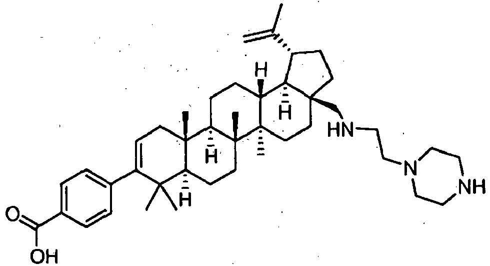

To a solution of *tert*-butyl 4-(2-(((1R,3aS,5aR,5bR,7aR,11aS,11bR,13aR,13bR)-9-(4-(tert-butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)ethyl)piperazine-1-carboxylate (136 mg, 0.167 mmol) in DCM (1 ml) was added TFA (0.4 ml, 5.19 mmol). The mixture was stirred at rt for 5 h then was concentrated under reduced pressure. The residue was dissolved in dioxane and MeOH and was purified by prep. HPLC. The fractions containing the expected product were combined and concentrated under reduced pressure. HPLC showed some impurities were still present, so the reaction was repurified by prep. HPLC. The fractions were concentrated under reduced pressure to give the title compound as a white solid (18 mg, 0.027 mmol, 16.43 % yield). LCMS: m/e 656.6 (M+H)⁺, 2.24 min (method 6). ¹H NMR (500 MHz, *Acetic*) δ ppm 8.03 (d, *J*=8.24 Hz, 2 H),7.30 (d, *J*=8.24 Hz, 2 H), 5.37 (d, *J*=4.58 Hz, 1 H), 4.79 (s, 1 H), 4.68 (s, 1 H), 3.50 - 3.67 (m, 6 H), 3.40 (d, *J*=12.82 Hz, 1 H), 3.22 - 3.29 (m, 6 H), 2.99 (d, *J*=13.12 Hz, 1 H), 2.49 - 2.58 (m, 1 H), 1.76 (s, 3 H), 1.19 (s, 3 H), 1.16-2.23 (m, 22 H), 1.10 (s, 3 H), 1.08 (s, 3 H), 1.02 (s, 3 H), 1.00 (s, 3 H).

### Preparation of tert-butyl 4-((1R,3aS,5aR,5bR,7aR,11aS,11bR,13aR,13bR)-5a,5b,8,8,11a-pentamethyl-3a-((2-(piperidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

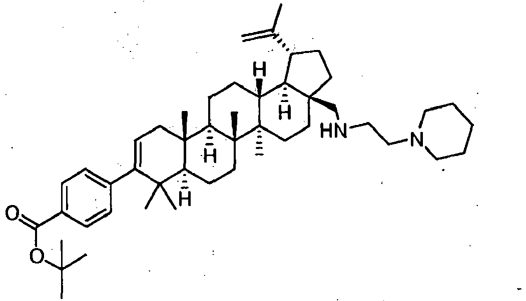

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (0.019 ml, 0.334 mmol) and *N*-(2-aminoethyl)piperidine (0.048 ml, 0.334 mmol). The mixture was stirred at rt for 2 h then sodium triacetoxyborohydride (0.177 g, 0.835 mmol) was added. The mixture was stirred at rt for three days then was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure. The crude product was used in the next step with no additional purification. LCMS: m/e 711.2 (M+H)⁺, 3.32 min (method 6).

### EXAMPLE 9. Preparation of 4-((1R,3aS,5aR,5bR,7aR,11aS,11bR,13aR,13bR)-5a,5b,8,8,11a-pentamethyl-3a-((2-(piperidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

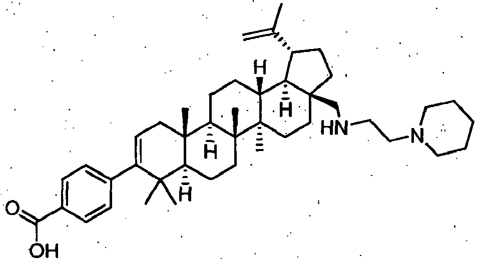

To a solution of *tert*-butyl 4-((1R,3aS,5aR,5bR,7aR,11aS,11bR,13aR,13bR)-5a,5b,8,8,11a-pentamethyl-3a-((2-(piperidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (119 mg, 0.167 mmol) in DCM (1 ml) was added TFA (0.4 ml, 5.19 mmol). The mixture was stirred at rt for 6 h then was concentrated under reduced pressure. The residue was dissolved in dioxane and MeOH and was purified by prep HPLC. The fractions containing the expected product were combined and concentrated under reduced pressure. The residue was dissolved in dioxane and methanol and the mixture was heated with a heat gun to reflux. Water was slowly added until the mixture was slightly cloudy. The mixture was allowed to cool to rt and then was refrigerated overnight. The solids that formed were collected by filtration and were washed with water to afford the title compound as a light-yellow solid (57 mg, 0.087 mmol, 52.1 % yield). LCMS: m/e 655.6 (M+H)⁺, 2.28 min (method 6). 1H NMR (400 MHz, *Acetic Acid d4*) δ ppm 7.99 (d, *J*=8.28 Hz, 2 H), 7.25 (d, *J*=8.28 Hz, 2 H), 5.32 (d, *J*=4.52 Hz, 1 H), 4.75 (s, 1 H), 4.63 (s, 1 H), 3.72 (s, 2 H), 3.56 - 3.66 (m, 2 H), 3.33 (d, *J*=12.80 Hz, 1 H), 2.98 (d, *J*=12.80 Hz, 1 H), 2.48 (br. s., 1 H), 1.71 (s, 3 H), 1.14 (s, 3 H), 1.09 - 2.22 (m, 32 H), 1.05 (s, 3 H), 1.03 (s, 3 H), 0.97 (s, 3 H), 0.96 (s, 3 H).

### Preparation of tert-butyl 4-((1R,3aS,5aR,5bR,7aR,11aS,11bR,13aR,13bR)-3a-((2-(dimethylamino)-2-(pyridin-3-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

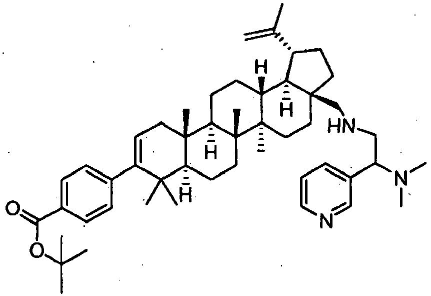

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmol) in DCE (2 ml) was added acetic acid (0.019 ml, 0.334 mmol) and (2-Amino-1-(3-pyridyl)ethyl)dimethylamine (0.055 g, 0.334 mmol). The mixture was stirred at rt for 2 h then sodium triacetoxyborohydride (0.177 g, 0.835 mmol) was added and it was stirred at rt for three days. The mixture was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure. The crude product was used in the next step with no additional purification. LCMS: m/e 748.3 (M+H)⁺, 3.47 min (method 6).

### EXAMPLE 10. Preparation of4-((1R,3aS,5aR,5bR,7aR,11aS,11bR,13aR,13bR)-3a-((2-(dimethylamino)-2-(pyridin-3-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

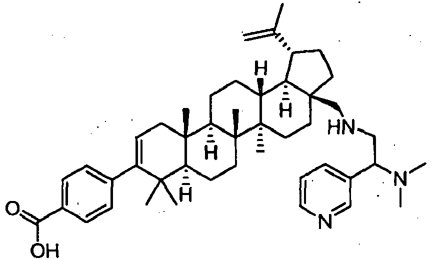

To a solution of *tert*-butyl 4-((1R,3aS,5aR,5bR,7aR,11aS,11bR,13aR,13bR)-3a-((2-(dimethylamino)-2-(pyridin-3-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]cbrysen-9-yl)benzoate (125 mg, 0.167 mmol) in DCM (1 ml) was added TFA (0.4 ml, 5.19 mmol). The mixture was stirred at rt for 4.5 h then was concentrated under reduced pressure. The residue was dissolved in dioxane and MeOH and was purified by prep HPLC. The fractions containing the expected product were combined and concentrated under reduced pressure. Impurities were still present so the residue was purified again by prep HPLC. The fractions containing the expected product were combined and concentrated under reduced pressure to give the title compound as an off-white solid (16 mg, 13.8%). LCMS: m/e 692.5 (M+H)⁺, 2.30 min (method 6). ¹H NMR (500 MHz, *Acetic Acid*) δ ppm 8.83-8.98 (m, 2 H), 8.27 - 8.42 (m, 1 H), 7.99 - 8.11 (m, 2 H), 7.71 - 7.84 (m, 1 H), 7.27-7.35 (m, 2 H), 5.33 - 5.42 (m, 1 H), 5.22 - 5.30 (m, 1 H), 4.79 (d, *J*=14.34 Hz, 1 H), 4.67 (d, *J*=12.82 Hz, 1 H), 4.21 - 4.38 (m, 2 H), 3.34 - 3.52 (m, 1 H), 2.97 - 3.15 (m, 1 H), 2.83 (br. s., 3 H), 2.82 (br. s., 3 H), 2.43 - 2.58 (m, 1 H), 0.88 - 2.23 (m, 40 H).

### Preparation of tert-butyl 4-((1R,3S,5aR,5bR,7aR,11aS,11bR,13aR,13bR)-5a,5b,8,8,11a-pentamethyl-3a-((2-(4-methylpiperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

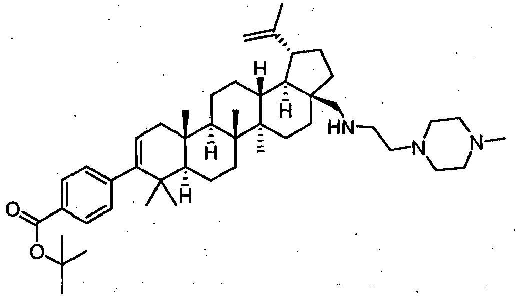

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.1 g, 0.167 mmo) in DCE (2 ml) was added acetic acid (0.019 ml, 0.334 mmol) and 2-(4-methyl-piperazin-1-yl)-ethylamine (0.048 g, 0.334 mmol). The mixture was stirred at rt for 2 h, then sodium triacetoxyborohydride (0.177 g, 0.835 mmol) was added and it was stirred at rt for 3 days. The mixture was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure. The crude product was used in the next with no additional purification. LCMS: m/e 726.5 (M+H)⁺, 3.07 min (method 6).

### EXAMPLE 11. Preparation of 4-((1R,3aS,5aR,5bR,7aR,11aS,1bR,13aR,13bR)-5a,5b,8,8,11a-pentamethyl-3a-((2-(4-methylpiperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

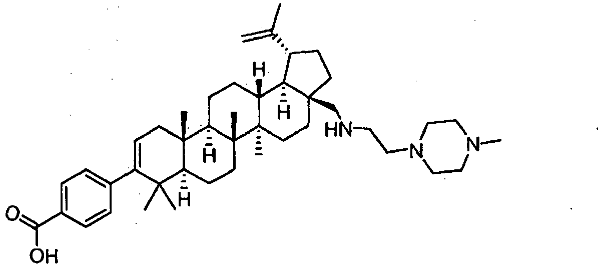

To a solution of *tert*-butyl 4-((1R,3aS,5aR,5bR,7aR,11aS,11bR,13aR,13bR)-5a,5b,8,8,11a-pentamethyl-3a-((2-(4-methylpiperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (0.121 g, 0.167 mmol) in DCM (1 ml) was added TFA (0.4 ml, 5.19 mmol). The mixture was stirred at rt for 5.5 h, then was concentrated under reduced pressure. The residue was dissolved in dioxane and MeOH and was purified twice by prep. HPLC to afford the title compound as a white solid (0.044 g, 0.066 mmol, 39.3 % yield). LCMS: m/e 670.6 (M+H)⁺, 2.23 min (method 6). ¹H NMR (500 MHz, *Acetic acids*) δ ppm 8.03 (d, *J*=8.24 Hz, 2 H), 7.30 (d, *J*=8.55 Hz, 2 H), 5.37 (d, J=4.58 Hz, 1 H), 4.79 (s, 1 H), 4.68 (s, 1 H), 3.43 - 3.66 (m, 6 H), 3.40 (d, *J*=13.43 Hz, 1 H), 3.17 - 3.31 (m, 6 H), 2.98 (d, *J*=12.82 Hz, 1 H), 2.92 (s, 3 H), 2.47 - 2.57 (m, 1 H), 1.75 (s, 3 H), 1.18 (s, 3 H), 1.15 - 2.23 (m, 22 H), 1.10 (s, 3 H), 1.08 (s, 3 H), 1.02 (s, 3 H), 1.00 (s, 3 H).

### EXAMPLE 12. Preparation of 4-((1R,3aS,5aR,5bR,7aR,11aS,11bR,13aR,13bR)-3a-((2-carboxy-N-(2-(dimethylamino)ethyl)acetamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

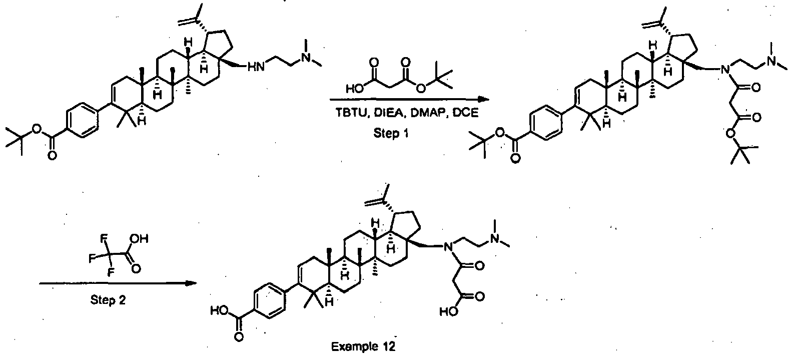

### Step 1: Preparation of tert-butyl 4-((1R,3aS,5aR,5bR,7aR,11aS,11bR,13aR,13bR)-3a-((3-tert-butoxy-N-(2-(dimethylamino)ethyl)-3-oxopropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

To a solution of *tert*-butyl 4-((1R,3aS,5aR,5bR,7aR,11aS,11bR,13aR,13bR)-3a-((2-(dimethylamino)ethylamino)methyl)-5a,5b,8,8,11a-pentamethyl-1-(prop-1-en-2-yl)-2,3,3a,4,5,5a,5b,6,7,7a,8,11,11a, 11b,12,13,13a,13b-octadecahydro-1H-cyclopenta[a]chrysen-9-yl)benzoate (27 mg, 0.040 mmol) in DCE (2 ml) was added Hunig's base (0.021 ml, 0.121 mmol), DMAP (1 mg, 8.19 µmol), Mono-*tert*-butyl malonate (0.012 ml, 0.080 mmol), and *O*-benzotriazol-1-yl-*N*,*N*,*N',N'*-tetra-methyluronium tetrafluoroborate (19.38 mg, 0.060 mmol). The mixture was stirred at rt. After 5 h of stirring, the mixture was loaded directly onto a silica gel column and ((1R,3aS,5aR,5bR,7aR,11aS,11bR,13aR,13bR)-3a-((3-tert-butoxy-*N*-(2-(dimethylainino)ethyl)-3-oxopropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate as a white foam (31.8 mg, 0.039 mmol, 97 % yield). LCMS: m/e 813.4 (M-H)⁻, 3.46 min (method 6).

### Step 2: Carboxylic acids deprotection.

To a solution of *tert*-butyl 4-((1R,3aS,5aR,5bR,7aR,11aS,11bR,13aR,13bR)-3a-((3-*tert*-butoxy-*N*-(2-(dimethylamino)ethyl)-3-oxopropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (31 mg, 0.038 mmol) in dichloromethane (1 ml) was added TFA (0.25 ml, 3.24 mmol). The mixture was stirred at rt for 3 h then was concentrated under reduced pressure. The residue was purified by prep HPLC. The fractions containing the expected product were combined and concentrated under reduced pressure to afford 4-((1R,3aS,5aR,5bR,7aR,11aS,11bR,13aR,13bR)-3a-((2-carboxy-*N*-(2-(dimethylamino)ethyl)acetamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid as a white solid (15 mg, 0.021 mmol, 56.1 % yield). LCMS: m/e 701.5 (M+H)⁺, 2.11 min (method 6). ¹H NMR (500 MHz, *Acetic acid-d₃ acid-d*) δ ppm 8.04 (d, *J*=8.24 Hz, 2 H), 7.31 (d, *J*=8.24 Hz, 2 H), 5.38 (d, *J*=5.19 Hz, 1 H), 4.85 (d, *J*=16.17 Hz), H), 4.69 (d, *J*=15.56 Hz, 1 H), 3.86 - 4.13 (m, 2 H), 2.97 - 3.82 (m, 6 H), 2.62 - 2.73 (m, 1 H), 2.13 - 2.24 (m, 2 H), 1.06 - 1.92 (m, 38 H), 1.03 (s, 3 H), 1.01 (s, 3 H).

### EXAMPLE 13. Preparation of 4-((1R,3aS,5aR,5bR,7aR,11aS,11bR,13aR,13bR)-3a-((3-carboxy-N-(2-(dimethylamino)ethyl)propanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

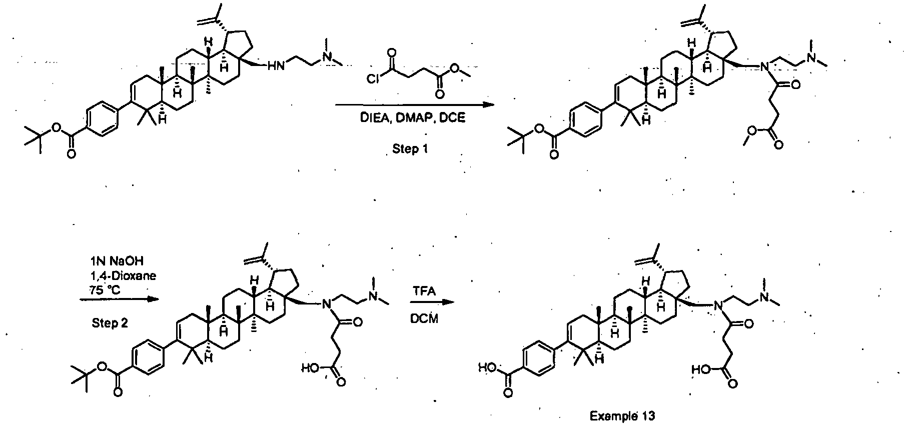

### Step 1. Preparation of tert-butyl 4-((1R,3aS,5aR,5bR,7aR,11aS,11bR,13aR,13bR)-3a-((N-(2-(dimethylamino)ethyl)-4-methoxy-4-oxobutanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate.

To a solution of *tert*-butyl 4-((1R,3aS,5aR,5bR,7aR,11aS,11bR,13aR,13bR)-3a-((2-(dimethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (48 mg, 0.072 mmol) in DCE (2 ml) was added Hunig's base (0.037 ml, 0.215 mmol), 3-carbomethoxypropionyl chloride (21.54 mg, 0.143 mmol), and DMAP (1 mg, 8.19 µmol). The mixture was stirred at rt for 5 h then was loaded directly onto a silica gel column and was purified using a 0-5% MeOH in dichloromethane gradient to afford *tert*-butyl 4-((1R,3aS,5aR,5bR,7aR,11aS,11bR,13aR,13bR)-3a-((*N*-(2-(dimethylamino)ethyl)-4-methoxy-4-oxobutanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate as a white foam (53.8 mg, 0.051 mmol, 71.8 % yield). LCMS: m/e 785.6 (M+H)⁺, 3.21 min (method 6).

### Step 2. Preparation of 4-((((1R,3aS,5aR,5bR,7aR,11aS,11bR,13aR,13bR)-9-(4-(tert-butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)(2-(dimethylamino)ethyl)amino)-4-oxobutanoic acid.

To a solution of *tert*-butyl 4-((1R,3aS,5aR,5bR,7aR,11aS,11bR,13aR,13bR)-3a-((N-(2-(dimethylamino)ethyl)-4-methoxy-4-oxobutanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (53 mg, 0.068 mmol) in 1,4-dioxane (1 ml) was added NaOH (0.338 ml, 0.338 mmol). The mixture was heated to 75 °C for 3 h. The mixture was cooled to rt and was quenched with IN HCl (3 ml) and extracted with dichloromethane (3 x 7 ml). The combined organic layers were dried with Na₂SO₄, were filtered, and concentrated under reduced pressure. The crude,product was used in the next step with no additional purification. LCMS: m/e 771.6 (M+H)⁺, 2.70 min (method 6).

### Step 3. Deprotection of the benzoic acid.

To a solution of 4-((((1R,3aS,5aR,5bR,7aR,11aS,11bR,13aR,13bR)-9-(4-(tert-butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)(2-(dimethylamino)ethyl)amino)-4-oxobutanoic acid (47 mg, 0.061 mmol) in DCM (1 ml) was added TFA (0.25 ml, 3.24 mmol). The mixture was stirred at rt. After 1.75 h, The mixture was concentrated under reduced pressure, was diluted with dioxane and MeOH and was purified by prep HPLC to afford 4-((1R,3aS,5aR,5bR,7aR,11aS,11bR,13aR,13bR)-3a-((3-carboxy-*N*-(2-(dimethylamino)ethyl)propanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid as a white solid (25 mg, 0.035 mmol, 57.4 % yield. LCMS: m/e 715.4 (M+H)⁺, 2.16 min (method 6).

### General procedure for the preparation of C28 amines

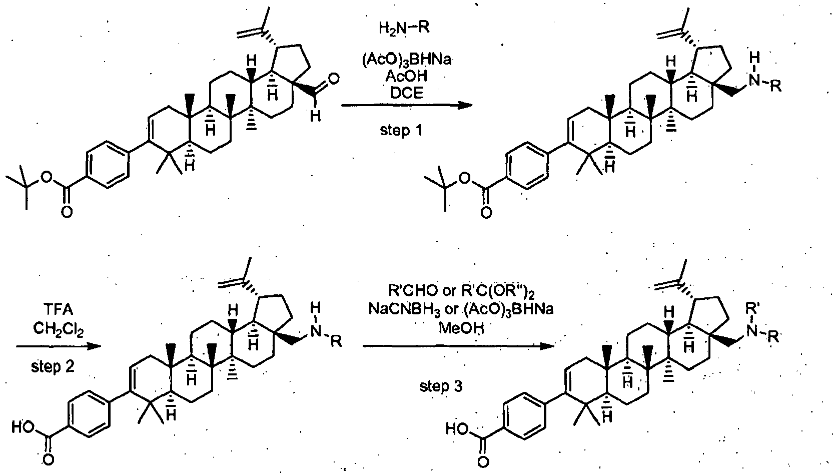

### Step 1: Preparation of C28 amines

A suspension of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1 eq.), the corresponding amine (2 eq.) and acetic acid (2 - 5 eq.) in DCE (2 ml) was stirred at rt for 30 min. Sodium triacetoxyborohydride (5 eq.) was added. The resulting mixture was stirred at rt for 18 - 72 h: The reaction mixture was diluted with 5 ml of saturated sodium carbonate and extracted with DCM (3 x 10 ml). The combined organic layers were dried over sodium sulfate, filtered and concentrated *in vacuo.* The crude product was purified by Biotage flash chromatography or was used directly in the next step without further purification.

### Step 2: Preparation of benzoic acids by hydrolysis of corresponding tert-butylesters

To a solution of the corresponding C28 amine from Step 1 in DCM (2 ml) was added TFA (0.5 ml). The mixture was stirred at rt for 1 - 2 h. The reaction mixture was concentrated *in vacuo.* The crude product was purified by prep. HPLC to afford the desired benzoic acid.

### Step 3: Reductive amination to form tertiary amines

To a solution of the material from Step 2 (1 eq.) in methanol (2 ml) was added the corresponding aldehyde or ketal (2 eq.) followed by acetic acid (1 eq). The resulting solution was stirred at rt for 10 min. Sodium triacetoxyhydroborate (3 eq.) was added and the resulting suspension was stirred at rt for 2 - 48 h. The reaction mixture was quenched by sodium bicarbonate solution and extracted with DCM (3 x 10 ml). The organic layer were combined and dried over sodium sulfate. The residue was purified by prep. HPLC to afford the desired product.

### EXAMPLE 14. Preparation of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

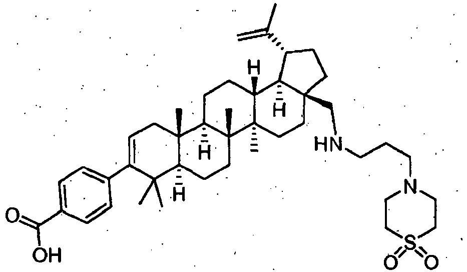

The title compound was prepared following the general procedures described above for the C28 amine formation and hydrolysis using 4-(3-aminopropyl) thiomorpholine 1,1-dioxide as the reactant amine. The product was isolated as a white solid (56 mg, 57.5 %). LCMS: m/e 719.5 (MH⁺), 2.60 min (method 1). ¹H NMR (400 MHz, *MeOD)* δ ppm 7.90 (2 H, d, *J*=8.6 Hz), 7.20 (2 H, d, *J*=8.6 Hz), 5.20 - 5.34 (1 H, m), 4.73 (1 H, s), 4.62 (1 H, s), 3.44 - 3.58 (4 H_{,} m), 3.39 (4 H, d, *J*=4.8 Hz), 3.12 - 3.26 (3 H, m), 3.08 (2 H, t, *J*=7.2 Hz), 2.78 - 2.91 (1 H, m), 2.39 - 2.58 (1 H, m), 2.07 - 2.19 (3 H, m), 1.97 - 2.08 (1 H, m), 1.64 - 1.88'(10 H, m), 1.39 - 1.63 (8 H, m), 1.18 -1.38 (5 H, m), 1.15 (3 H, s), 1.05 (3 H, s), 1.02 (3 H, s), 0.95 (3 H, s), 0.87 - 0.94 (3 H, s).

### EXAMPLE 15. Preparation of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

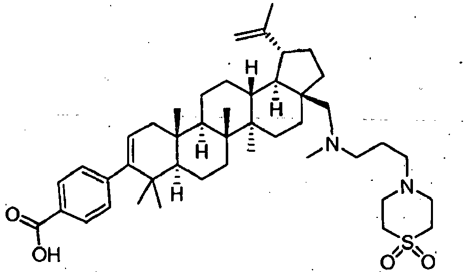

The title compound was prepared following the general procedures described above for the C28 amine formation, hydrolysis and tertiary amine formation using 4-(3-aminopropyl) thiomorpholine 1,1-dioxide as the reactant amine and formaldehyde as reactant aldehyde. The product was isolated as a white solid (10 mg, 46.6 %). LCMS: m/e 733.6 (MH⁺), 2.56 min (method 3). ¹H NMR (400 MHz, *MeOD*) δ ppm 7.94 (m, *J*=8.5 Hz, 2 H), 7.24 (m, *J*=8.5 Hz, 2 H), 5.26 - 5.41 (m, 1 H), 4.80 (d, *J*=1.5 Hz, 1 H), 4.69 (s, 1 H), 3.23 - 3.46 (m, 12 H), 3.03 (s, 3 H), 2.91 (t, *J*=6.9 Hz, 2 H), 2.56 (br. s., 1 H), 2.08 - 2.31 (m, 2 H), 1.98 - 2.08 (m, 1 H), 1.70 - 1.95 (m, 10 H), 1.47 - 1.70 (m, 9 H), 1.43 (d, *J*=10.0 Hz, 1 H), 1.28 - 1.41 (m, 3 H), 1.15 - 1.23 (m, 4 H), 1.12 (s, 3 H), 1.05 (s, 3 H), 0.99 (s, 3 H), 0.97 (s, 3 H).

### EXAMPLE 16. Preparation of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a11b,12,13,13a,13b-octadecahydro-1H-cyclopenta[a]chrysen-9-yl)benzoic acid.

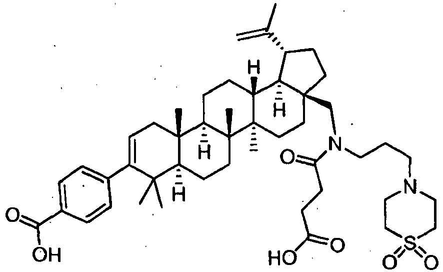

To a solution of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid (10 mg, 0,014 mmole) in CH₂Cl₂ (0.5 ml) was added dihydrofuran-2,5-dione (4.18 mg, 0.042 mmol) followed by DMAP (1.953 mg, 0.014 mmol) and DIPEA (2.429 µL, 0.014 mmol). The mixture was stirred at rt for 18 hours. The solvent was removed *in vacuo* and the resulting residue was purified by prep. HPLC to afford the title compound as a white solid (10 mg, 83 %). LCMS: m/e 819.3 (MH⁺), 2.45 min (method 3). ¹H NMR (400 MHz, *MeOD*) 8 ppm 7.94 (m, *J*=8.3 Hz, 2 H), 7.24 (m, *J*=8.5 Hz, 2 H), 5.33 (d, *J*=6.3 Hz, 1 H), 4.75 (s, 1H), 4.63 (s, 1H), 3.82 (br. s., 2 H), 3.48 - 3.73 (m, 6 H), 3.44 (br. s., 3 H), 3.20 3.31 (m, 2 H), 3.03 - 3.20 (m, 1 H), 2.56 - 2.80 (m, 5 H), 2.08 (br. s., 4 H), 1.75 (d, *J*=11.3 Hz, 8 H), 1.57 (d, *J*=2.0 Hz, 5 H), 1.48 (br. s., 2 H), 1.24 - 1.45 (m, 5 H), 1.22 (s, 4 H), 1.11 - 1.16 (m, 2 H), 1.02 - 1.11 (m, 6 H), 0.88 - 1.02 (m, 6 H).

### EXAMPLE 17. Preparation of 4-((1R,3aS,5aR,5bR,7aR,11aS,11bR,13aR,13bR)-5a,5b,8,8,11a-pentamethyl-1-(prop-1-en-2-yl)-3a-((3-(-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

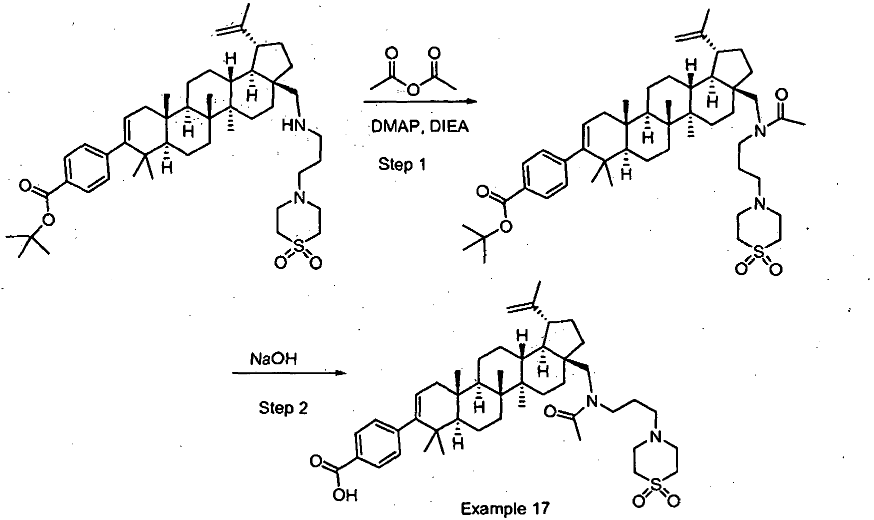

### Step 1. N-Acetylation

To a solution of *tert*-butyl 4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoate (50 mg, 0,065 mmole) in CH₂Cl₂ (5 ml) was added acetic anhydride (6.58 mg, 0.065 mmol) followed by DMAP (9.06 mg, 0.065 mmol)) and DIPEA (11 µL, 0.065 mmol). The mixture was stirred for 18 hours at room temperature. The solvent was removed *in vacuo* and the resulting residue was used as it without further purification. LCMS: m/e 817.3 (MH⁺), 2.75 min (method 3).

### Step 2. Saponification of the benzoate ester

To a solution of material from Step 1 (6 mg, 7.34 µmol) in dioxane (1 ml) and MeOH (5 ml) sodium hydroxide (5.87 mg, 0.147 mmol) (powder) was added, followed by 5 drops of water. The resulting solution was stirred at 70 °C for 12 h. The solvent was removed *in vacuo* and the resulting residue was purified by prep. HPLC. The product was isolated as a white solid (6 mg, 100 %). LCMS: m/e 761.3 (MH⁺), 2.51 min (method 3). ¹H NMR (400 MHz, *MeOD*) δ ppm 7.94 (m, *J*=8.3 Hz, 2 H), 7.26 (m, 2 H), 5.22 - 5.44 (m, 1 H), 4.76 (s, 1H), 4.63 (s, 1H), 3.82 (br. s., 2 H), 3.59 - 3.68 (m, 3 H), 3.51 - 3.59 (m, 2 H), 3.40 - 3.51 (m, 4 H), 3.23 - 3.31 (m, 2 H), 3.02 - 3.16 (m, 1 H), 2.57 - 2.73 (m, 1 H), 2.12 - 2.22 (m, 4 H), 1.97 - 2.12 (m, 3 H), 1.85 (d, *J*=12.3 Hz, 2 H), 1.65 - 1.80 (m, 6 H), 1.58 (d, *J*=16.6 Hz, 4 H), 1.45 - 1.54 (m, 3 H), 1.26 - 1.45 (m, 4 H), 1.21 (s, 4 H), 1.11 - 1.19 (m, 3 H), 1.01 - 1.11 (m, 6 H), 0.92 -1.01 (m, 6 H).

### EXAMPLE 18. Preparation of 4-((1R,3aS,5aR,5bR,7aR,11aS,11bR,13aR,13bR)-5a,5b,8,8,11a-pentamethyl-3a-((3-(methyl(phenyl)amino)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

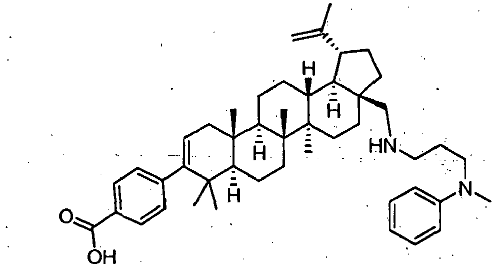

The title compound was prepared following the general procedures described above for the C28 amine formation and hydrolysis using *N*1-methyl-*N*1-phenylpropane-1,3-diamine as the reactant amine. The product was isolated as a white solid (7 mg, 14.4 %). LCMS: m/e 691.7 (MH⁺), 2.71 min (method 1). ¹H NMR (500 MHz, *CHLOROFORM-d*) δ ppm 7.97 (2 H, d, *J*=7.9 Hz), 7.16 - 7.27 (4 H, m), 6.65 - 6.84 (3 H, m), 5.23 - 5.37 (1 H, m), 4.70 (1 H, br. s.), 4.61 (1 H, br. s.), 3.30 - 3.48 (2 H, m), 2.95 - 3.03 (4 H, m), 2.93 (3 H, s), 2.41 - 2.53 (1 H, m), 2.00 - 2.14 (2 H, m), 1.89 - 2.00 (4 H, m), 1.73 - 1.89 (7 H, m), 1.64 - 1.73 (2 H, m), 1.60 (2 H, br. s.), 1.52-1.58 (2 H, m), 1.46 - 1.52 (2 H, m), 1.44 (2 H, d, *J*=10.1 Hz), 1.18 - 1.33 (2 H, m), 1.03 - 1.12 (3 H, m), 1.02 (2 H, br. s.), 0.98 - 1.01 (6 H, m), 0.87 - 0.98 (6 H, m).

### EXAMPLE 19. Preparation of 4-((1R,3aS,5aR,5bR,7aR,11aS,11bR,13aR,13bR)-5a,5b,8,8,11a-pentamethyl-3a-((3-(methylamino)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

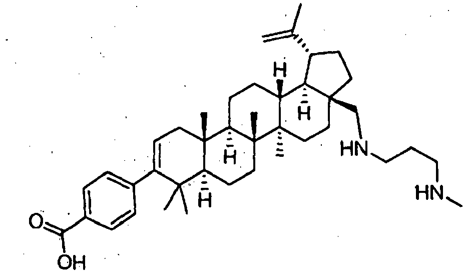

The title compound was prepared following the general procedures described above for the C28 amine formation and hydrolysis using *tert*-butyl 3-aminopropyl (methyl) carbamate as the reactant amine. The product was isolated as a white solid (26 mg, 51.6 %). LCMS: m/e 615.5 (MH⁺), 2.50 min (method 1). ¹H NMR (500 MHz, *Acetic Acid-d*) δ ppm 8.03 (2 H, d, *J*=8.2 Hz), 7.29 (2 H, d, *J*=8.2 Hz), 5.36 (1 H, d, *J*=4.6 Hz), 4.79 (1 H, s), 4.67 (1 H, s), 3.36 - 3.43 (1 H, m), 3.27 - 3.36 (2 H, m), 3.21 (2 H, t, *J*=7.5 Hz), 2.94 (1 H, d, *J*=12.8 Hz), 2.78 (3 H, s), 2.48 - 2.56 (1 H, m), 2.24 - 2.38 (2 H, m), 2.13 - 2.24 (2 H, m), 2.00 - 2.13 (5 H, m), 1.85 - 2.00 (3 H, m), 1.68 - 1.85 (6 H, m), 1.58 - 1.68 (2 H, m), 1.41 - 1.58 (3 H, m), 1.23 - 1.41 (2 H, m), 1.12 - 1.23 (5 H, m), 1.09 (3 H, s), 1.07 (3 H, s), 1.01 (3 H, s), 1.00 (3 H, s).

### EXAMPLE 20. Preparation of 4-((1R,3aS,5aR,5bR,7aR,11aS,11bR,13aR,13bR)-3a-((2-(1H-imidazol-1-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

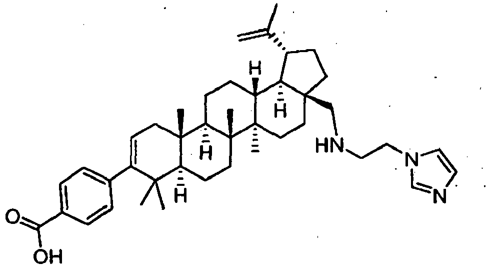

The title compound was prepared following the general procedures described above for the C28 amine formation and hydrolysis using 2-(1H-imidazol-1-yl)ethanamine as the reactant amine. The product was isolated as a white solid (15 mg, 51.7 %). LCMS: m/e 638.6 (MH⁺), 2.49 min (method 1). ¹H NMR (500 MHz, *MeOD*) 8 ppm 9.03 (1 H, s), 7.94 (2 H, d, *J*=8.2 Hz), 7.73 (1 H, d, *J*=1.5 Hz), 7.65 (1 H, d, *J*=1.5 Hz), 7.24 (2 H, d, *J*=8.2 Hz), 5.25 - 5.36 (1 H, m), 4.77 (1 H, s), 4.74 (2 H, t, *J*=6.7 Hz), 4.66 (1 H, s), 3.63 - 3.79 (2 H, m), 3.36 (1H, m), 2.95 (1 H, d, *J*=12.8 Hz), 2.52 (1 H, dt, *J*=10.8, 5.5 Hz), 2.12 - 2.27 (1 H, m), 1.98 - 2.12 (1 H, m), 1.83 - 1.98 (2 H, m), 1.78-1.83 (2 H, m), 1.70 - 1.78 (6 H, m), 1.45 - 1.64 (6 H, m), 1.35 (2 H, dd, *J*=11.3, 8.2 Hz), 1.26 - 1.32 (2 H, m), 1.22 (1 H, d, *J*=2.7 Hz), 1.18 (5 H, s), 1.09 (3 H, s), 1.05 (3 H, s), 0.99 (3 H, s), 0.97 (3 H, s).

### EXAMPLE 21. Preparation of 4-((1R,3aS,5aR,5bR,7aR,11aS,11bR,13aR,13bR)-3a-((2-(diethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

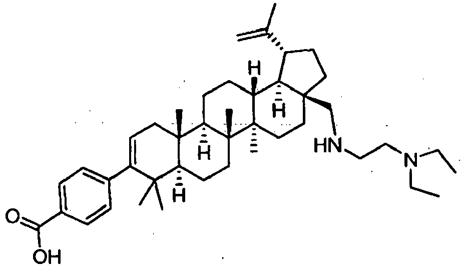

The title compound was prepared following the general procedures described above for the C28 amine formation and hydrolysis using *N*1,*N*1-diethylethane-1,2-diamine as the reactant amine. The product was isolated as a white solid (27 mg, 86.0 %). LCMS: m/e 643.6 (MH⁺), 2.52 min (method 1). ¹H NMR (500 MHz, *MeOD*) δ ppm 7.89 (2 H, m, *J*=7.6 Hz), 7.16 (2 H, m, *J*=7.9 Hz), 5.30 (1 H, d, *J*=4.6 Hz), 4.75 (1 H, br. s.), 4.63 (1 H, br. s.), 3.04 - 3.23 (5 H, m), 2.88 - 3.02 (4 H, m), 2.69 (1 H, d, *J*=11.9 Hz), 2.51 (1 H, d, *J*=5.5 Hz), 2.15 (1 H, dd, *J*=17.1, 6.1 Hz), 2.09 (1 H, br. s.), 1.93 - 2.00 (6 H, m), 1.83 - 1.93 (2 H, m), 1.79 (2 H, br. s.), 1.73 (4 H, br. s.), 1.42-1.59 (5 H, m), 1.26 (2 H, br. s.), 1.22 (6 H, t, *J*=7.2 Hz), 1.16 (5 H, br. s.), 1.06 (3 H, br. s.), 1.04 (3 H, br. s.), 0.88 - 1.01 (6 H, m).

### EXAMPLE 22. Preparation of 4-((1R,3aS,5aR,5bR,7aR,11aS,11bR,13aR,13bR)-5a,5b,8,8,11a-pentamethyl-3a-((3-(methylamino)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

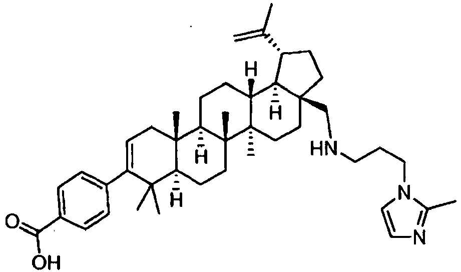

The title compound was prepared following the general procedures described above for the C28 amine formation and hydrolysis using 3-(2-methyl-1H-imidazol-1-yl) propan-1-amine as the reactant amine. The product was isolated as a white solid (40 mg, 66.5 %). LCMS: m/e 666.5 (MH⁺), 2.48 min (method 1). ¹H NMR (400 MHz, *MeOD*) 8 ppm 7.90 (2 H, d, *J*=8.6 Hz), 7.52 (1 H, d, *J*=2.3 Hz), 7.44 (1 H, d, *J*=2.0 Hz), 7.20 (2 H, d, *J*=8.3 Hz), 5.27 (1 H, d, *J*=4.8 Hz), 4.72 (1 H, s), 4.61 (1 H, s), 4.25 (2 H, t, *J*=7.2 Hz), 3.11 - 3.26 (3 H, m), 2.79 - 2.92 (1 H, m), 2.65 (3 H, s), 2.39 - 2.58 (1 H, m), 2.30 (2 H, dq, *J*=7.9, 7.7 Hz), 2.12 (1 H, dd, *J*=17.2, 6.4 Hz), 1.93 - 2.08 (1 H, m), 1.76 - 1.93 (2 H, m), 1.63 - 1.76 (8 H, m), 1.57 (1 H, br. s.), 1.54 (1 H, d, *J*=7.6 Hz), 1.38 - 1.52 (6 H, m), 1.29 - 1.38 (1 H, m), 1.22 - 1.29 (2 H, m), 1.19 (1 H, br. s.), 1.07 - 1.16 (4 H, m), 1.04 (3 H, s), 1.02 (3 H, s), 0.96 (3 H, s), 0.90 (3 H, s).

### EXAMPLE 23. Preparation of 4-((1R,3aS,5aR,5bR,7aR,11aS,11bR,13aR,13bR)-5a,5b,8,8,11a-pentamethyl-3a-((3-(3-oxopiperazin-1-yl)propylamino)methyl)-1-(prop-1-en-2-yl)-2,3,3a,4,5,5a,5b,6,7,7a,8,11,11a,11b,12,13,13a,13b-octadecahydro-1 H-cyclopenta[a]chrysen-9-yl)benzoic acid.

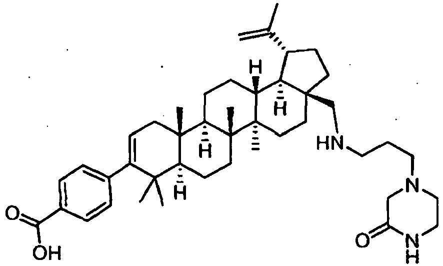

The title compound was prepared following the general procedures described above for the C28 amine formation and hydrolysis using 4-(3-aminopropyl)piperazin-2-one as the reactant amine. The product was isolated as a white solid (55 mg, 80 %). LCMS: m/e 684.5 (MH⁺), 2.53 min (method 1). ¹H NMR (400 MHz, *MeOD*) δ ppm 7.91 (2 H, d, *J*=3.3 Hz), 7.21 (2 H, d, *J*=2.8 Hz), 5.28 (1 H, br. s.), 4.74 (1 H, br. s.), 4.63 (1 H, br. s.), 3.93 (2 H, br. s.), 3.52 - 3.69 (4 H, m), 3.3 (2h, m), 3.04 - 3.27 (3 H, m), 2.78 - 2.97 (1 H, m), 2.41 - 2.63 (1 H, m), 2.21 - 2.38 (2 H, m), 1.97 - 2.21 (2 H, m), 1.8 (2H, m), 1.71 (8 H, br. s.), 1.51 (8 H, br. s.), 1.25 (2H, m), 1.15 (5 H, br. s.), 1.03 (7 H, d, *J*=13.8 Hz), 0.85 - 0.99 (6 H, m).

### EXAMPLE 24. Preparation of 4-((1R,3aS,5aR,5bR,7aR,11aS,1bR,13aR,13bR)-3a-((2-(bis(2-hydroxyethyl)amino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

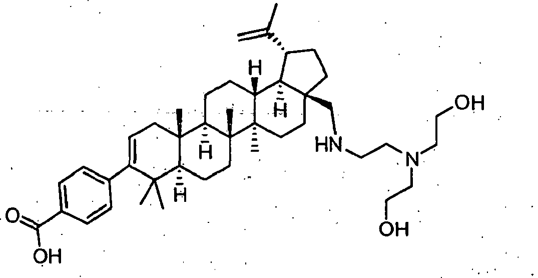

The title compound was prepared following the general procedures described above for the C28 amine formation and hydrolysis using 2,2'-(2-aminoethylazanediyl)diethanol as the reactant amine. The product was isolated as a white solid (19 mg, 40.7 %). LCMS: m/e 675.6 (MH⁺), 2.51 min (Method 1). ¹H NMR (400 MHz, *MeOD*) 8 ppm 7.90 (d, *J*=8.3 Hz, 2 H), 7.20 (d, *J*=8.3 Hz, 2 H), 5.18 - 5.37 (m, 1 H), 4.74 (s, 1 H), 4.62 (s, 1 H), 3.79 - 3.95 (m, 4 H), 3.45 - 3.66 (m, 4 H), 3.20-3.35 (m, 5H), 2.78 - 3.00 (m, 1 H), 2.39 - 2.59 (m, 1 H), 2.13 (dd, *J*=17.0, 6.4 Hz, 1 H), 2,04 (d, *J*=8.6 Hz, 1 H), 1.78 - 1.92 (m, 2 H), 1.63 - 1.78 (m, 8 H), 1.55 (d, *J*=6.5 Hz, 2 H), 1.40 - 1.53 (m, 6 H), 1.22 - 1.40 (m, 4 H), 1.09 - -1.19 (m, 4 H), 1.05 (s, 3 H), 1.02 (s, 3 H), 0.95 (s, 3 H), 0.93 (s, 3 H).

### EXAMPLE 25. Preparation of 4-((1R,3aS,5aR,5bR,7aR,11aS,11bR,13aR,13bR)-3a-((2-(1H-imidazol-4-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

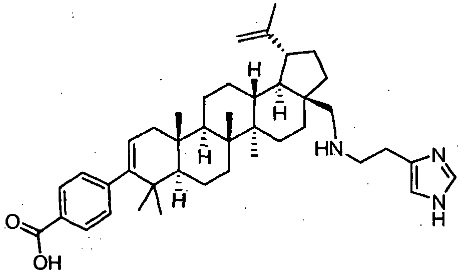

The title compound was prepared following the general procedures described above for the C28 amine formation and hydrolysis using 2-(1H-imidazol-4-yl) ethanamine as the reactant amine. The product was isolated as a white solid (40 mg, 63.4 %). LCMS: m/e 638.5 (MH⁺), 2.48 min (method 1). ¹H NMR (400 MHz, *MeOD*) δ ppm 8.86 (d, *J*=1.3 Hz, 1 H), 7.90 (d, *J*=8.3 Hz, 2 H), 7.45 (s, 1 H), 7.20 (d, *J*=8.3 Hz, 2 H), 5.16 - 5.33 (m, 1 H), 4.74 (s, 1 H), 4.63 (s, 1 H), 3.36 - 3.53 (m, 2 H), 3.16 - 3.26 (m, 3 H), 2.90 (d, *J*=12.8 Hz, 1 H), 2.40 - 2.59 (m, 1 H), 2.08 - 2.20 (m, 1 H), 1.98.-2.08 (m, 1 H), 1.79 - 1.91 (m, 2 H), 1.62 - 1.79 (m, 7 H), 1.53 - 1.62 (m, 3 H), 1.41-1.53 (m, 5 H), 1.23 - 1.41 (m, 4 H), 1.09 - 1.23 (m, 5 H), 1.06 (s, 3 H), 1.02 (s, 3 H), 0.95 (s, 3 H), 0.93 (s, 3 H).

### EXAMPLE 26. Preparation of 4-((1R,3aS,5aR,5bR,7aR,11aS,11aR,13aR,13bR)-3a-((2-((2-hydroxyethyl)(methyl)amino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

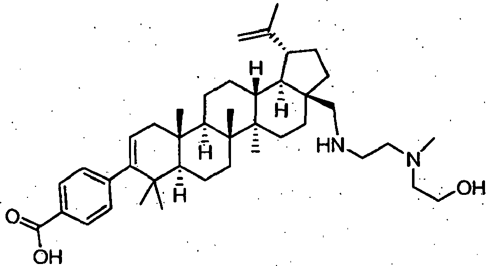

The title compound was prepared following the general procedures described above for the C28 amine formation and hydrolysis using 2-((2-aminoethyl)(methyl)amino)ethanol as the reactant amine. The product was isolated as a white solid (21 mg, 45.7 %). LCMS: m/e 645.5 (MH⁺), 2.49 min (method 1). ¹H NMR (400 MHz, *MeOD*) δ ppm 7.90 (m, *J*=8.3 Hz, 2 H), 7.20 (m, *J*=8.3 Hz, 2 H), 5.21 - 5.34 (m, 1 H), 4.74 (s, 1 H), 4.63 (s, 1 H), 3.82 - 3.97 (m, 2 H), 3.50 - 3.75 (m, 4 H), 3.30 - 3.43 (m, 3 H), 2.98 (s, 3 H), 2.93 (d, *J*=12.8 Hz, 1 H), 2.49 (td, *J*=10.6, 5.8 Hz, 1 H), 2.08 - 2.20 (m, 2 H), 1.79 - 1.93 (m, 2 H), 1.64 - 1.79 (m, 8 H), 1.40-1.64 (m, 8 H), 1.23 - 1.40 (m, 4 H), 1.13 - 1.23 (m, 4 H), 1.05 (s, 3 H), 1.02 (s, 3 H), 0.95 (s, 3 H), 0.93 (s, 3 H).

### EXAMPLE 27. Preparation of 4-((1R,3aS,5aR,5bR,7aR,11aS,11bR,13aR,13bR)-3a-((4-tert-butoxy-4-oxobutylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

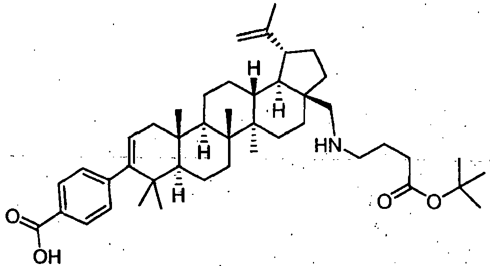

The title compound was prepared following the general procedures described above for the C28 amine formation and hydrolysis using *tert*-butyl 4-aminobutanoate hydrochloride as the reactant amine. The product was isolated as a white solid (2 mg, 3.62 %). LCMS: m/e 689.5 (MH⁺), 2.96 min (method 1). ¹H NMR (400 MHz, *CHLOROFORM-d*) δ ppm 7.90 (m, *J*=8.3 Hz, 2 H), 7.19 (m, *J*=8.1 Hz, 2 H), 5.29 (d, *J*=4.3 Hz, 1 H), 4.65 (s, 1 H), 3.21 (s, 2 H), 2.85 (d, *J*=4.5 Hz, 1 H), 2.53 - 2,78 (m, 1 H), 2.28 - 2.45 (m, 1 H), 2.02 - 2.19 (m, 2 H), 1.80 - 2.02 (m, 2 H), 1.72 (s, 8 H), 1.61 (s, 9 H), 1.46 - 1.54 (m, 4 H), 1.43 (d, *J*=7.1 Hz, 5 H), 1.18 - 1.36 (m, 6 H), 1.07-1.18 (m, 6 H), 1.03 (s, 3 H), 1.00 (s, 3 H), 0.76 - 0.97 (m, 6 H).

### EXAMPLE 28. Preparation of 4-((1R,3aS,5aR,5bR,7aR,11aS,11bR,13aR,13bR)-3a-((3-carboxypropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

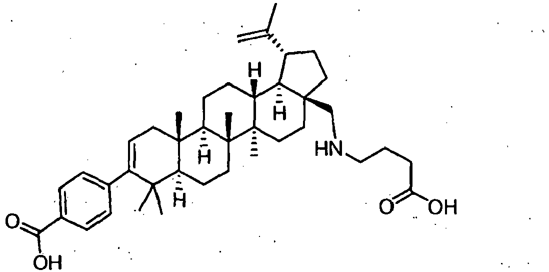

The title compound was prepared following the general procedures described above for the C28 amine formation and hydrolysis using *tert*-butyl 4-aminobutanoate hydrochloride as the reactant amine. The product was isolated as a white solid (2 mg, 3.62 %). LCMS: m/e 630.5 (MH⁺), 2.59 min (method 1). ¹H NMR (400 MHz, *acetonitrile-d₃)* 8 ppm 7.93 (d, *J*=8.3 Hz, 2 H), 7.23 (d, *J*=8.3 Hz, 2 H), 5.25 - 5.36 (m, 1 H), 4.77 (s, 1 H), 4.66 (s, 1 H), 3.21 - 3.49 (m, 1 H), 3.16 (t, *J*=7.7 Hz, 2 H), 2.87 (d, *J*=12.8 Hz, 1 H), 2.43 - 2.60 (m, 3 H), 2.12 - 2.26 (m, 1 H), 1.92 - 2.12 (m, 3 H), 1.66 - 1.90 (m, 8 H), 1.59 (d, *J*=6.0 Hz, 2 H), 1.46 - 1.57 (m, 6 H), 1.33 - 1.34 (m, 2 H), 1.28 (d, *J*=15.4 Hz, 4 H), 1.19 (s, 4 H), 1.09 (s, 3 H), 1.05 (s, 3 H), 0.98 (s, 3 H), 0.96 (s, 3 H).

### EXAMPLE 29. Preparation of 4-((1R,3aS,5aR,5bR,7aR,11aS,11bR,13aR,13bR)-5a,5b,8,8,11a-pentamethyl-3a-((2-oxopyrrolidin-1-yl)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

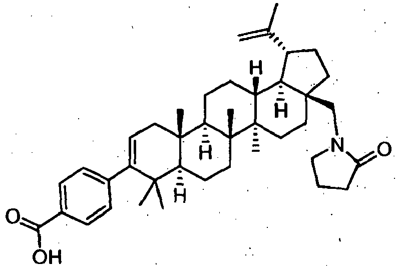

The title compound was prepared following the general procedures described above for the C28 amine formation and hydrolysis using *tert*-butyl 4-aminobutanoate hydrochloride as the reactant amine. The product was isolated as a white solid (3 mg, 6.28 %). LCMS: m/e 612.4 (MH⁺), 3.18 min (method 1). ¹H NMR (400 MHz, *MeOD*) δ ppm 7.88 (m, *J*=8.3 Hz, 2 H), 7.17 (m, *J*=8.3 Hz, 2 H), 5.14 - 5.31 (m, 1 H), 4.69 (s, 1 H), 4.56 (s, 1 H), 3.36 - 3.58 (m, 3 H), 3.13 (d, *J*=1.4.4 Hz, 1 H), 2.46 - 2.61 (m, 1 H), 2.34 (t, *J*=8.2 Hz, 2 H), 2.00 - 2.19 (m, 4 H), 1.97 (s, 1 H), 1.58 - 1.75 (m, 8 H), 1.47 (br. s., 7 H), 1.30 (d, *J*=7.1 Hz, 3 H), 1.24 (s, 4 H), 1.14 (s, 3 H), 1.00 (s, 3 H), 0.98 (s, 3 H), 0.92 (s, 3 H), 0.91 (s, 3 H).

### EXAMPLE 30. Preparation of 4-((1R,3aS,5aR,5bR,7aR,11aS,11bR,13aR,13bR)-3a-((2-methoxy-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

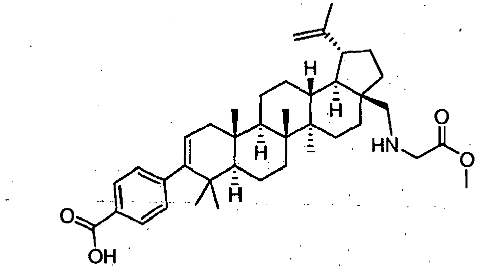

The title compound was prepared following the general procedures described above for the C28 amine formation and hydrolysis using methyl 2-aminoacetate hydrochloride as the reactant amine. The product was isolated as a white solid (27 mg, 45.9 %). LCMS: m/e 616.5 (MH⁺), 2.14 min (method 3). ¹H NMR (400 MHz, *MeOD*) δ ppm 7.90 (d, *J*=8.3 Hz, 2 H), 7.20 (d, *J*=8.3 Hz, 2 H), 5.20 - 5.33 (m, 1 H), 4.72 (s, 1 H), 4.62 (s, 1 H), 4.05 (d, *J*=3.8 Hz, 2 H), 3.86 (s, 3 H), 3.33 - 3.45 (m, 1 H), 2.89 (d, *J*=12.6Hz, 1 H), 2.45 (td, *J*=10.8, 5.7 Hz, 1 H), 1.93 - 2.18 (m, 2 H), 1.77 - 1.93 (m, 3 H), 1.63 - 1. 77 (m, 7 H), 1.39 - 1.63 (m, 8 H), 1.18 - 1.39 (m, 5 H), 1.15 (s, 3 H), 1.06 (s, 3 H), 1.00 (s, 3 H), 0.95 (s, 3 H), 0.93 (s, 3 H).

### EXAMPLE 31. Preparation of 4-((1R,3aS,5aR,5bR,7aR,11aS,11bR,13aR,13bR)-3a-((carboxymethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

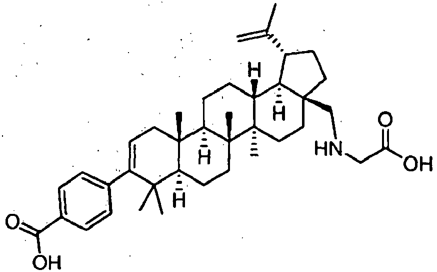

The title compound was prepared following the procedures described below: To the solution of 4-((1R,3aS,5aR,5bR,7aR,11aS,11bR,13aR,13bR)-3a-((2-methoxy-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (50 mg, 0.081 mmol) in dioxane (2 ml) sodium hydroxide (0.162 ml, 0.162 mmol) was added. The resulting mixture was stirred at 70 °C for 2 h. The solvent was evaporated and the residue was purified by prep. HPLC to afford the title compound as a white solid (18 mg, 35 %). LCMS: m/e 602.4 (MH⁺), 2.32 min (method 3). ¹H NMR (400 MHz, *MeOD*) δ ppm 7.90 (d, *J*=8.3 Hz, 2 H), 7.20 (d, *J*=8.3 Hz, 2 H), 5.18 - 5.40 (m, 1 H), 4.73 (s, 1 H), 4.62 (br. s., 1 H), 3.96 (d, *J*=5.0 Hz, 2 H), 2.78 - 2.94 (m, 1 H), 2.37 - 2.60 (m, 1 H), 2.10 (s, 2 H), 1.78 - 1.92 (m, 3 H), 1.62 - 1.78 (m, 6 H), 1.38 - 1.61 (m, 8 H), 1.16 - 1.38 (m, 6 H), 1.11 - 1.16 (m, 4 H), 1.07 (s, 3 H), 1.01 (s, 3 H), 0.95 (s, 3 H), 0.93 (s, 3 H).

### EXAMPLE 32. Preparation of 4-((1R,3aS,5aR,5bR,7aR,11aS,11bR,13aR,13bR)-3a-((2-methoxy-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

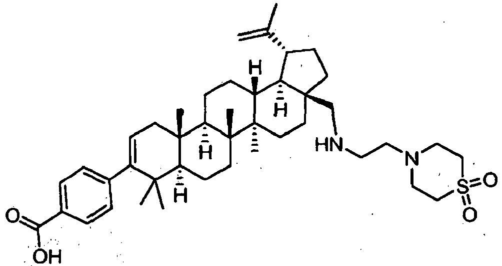

The title compound was prepared following the general procedures described above for the C28 amine formation and hydrolysis using methyl 2-aminoacetate hydrochloride as the reactant amine. The product was isolated as a white solid (34 mg, 58.1 %). LCMS: m/e 704.5 (MH⁺), 2.27 min (method 3). ¹H NMR (400 MHz, *MeOD*) δ ppm 7.90 (d, *J*=8.3 Hz, 2 H), 7.20 (d, *J*=8.1 Hz, 2 H), 5.19 - 5.36 (m, 1 H), 4.74 (s, 1 H), 4.63 (s, 1 H), 3.18 - 3.35 (m, 5 H), 3.12 (br. s., 6 H), 2.89 - 3.01 (m, 2 H), 2.86 (d, *J*=13.8 Hz, 1 H), 2.41 - 2.61 (m, 1 H), 2.12 (dd, *J*=17.1, 6.3 Hz, 1 H), 1.93 - 2.07 (m, 1 H), 1.64 - 1.88 (m, 10 H), 1.41 - 1.62 (m, 8 H), 1.18 - 1.41 (m, 5 H), 1.15 (s, 3 H), 1.06 (s, 3 H), 1.02 (s, 3 H), 0.95 (s, 3 H), 0.93 (s, 3 H).

### EXAMPLE 33. Preparation of 4-((1R,3aS,5aR,5bR,7aR,11aS,11bR,13aR,13bR)-3a-((2-methoxy-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

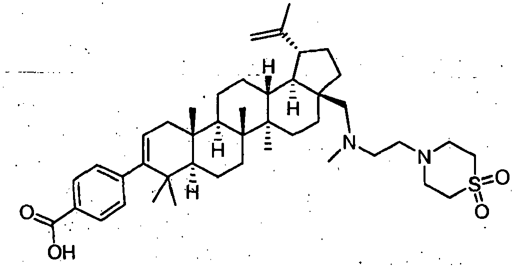

The title compound was prepared following the general procedures described above for the C28 amine formation, hydrolysis and tertiary amine formation using 4-(3-aminoethyl) thiomorpholine 1,1-dioxide as the reactant amine and formaldehyde as reactant aldehyde. The product was isolated as a white solid (10 mg, 46.6 %). LCMS: m/e 719.7 (MH⁺), 2.56 min (method 3). ¹H NMR (400 MHz, *MeOD*) δ ppm 7.94 (m, 2 H), 7.25 (m, 2 H), 5.24 - 5.40 (m, 1 H), 4.80 (d, *J*=1.5 Hz, 1 H), 4.69 (s, 1 H), 3.37 - 3.56 (m, 3 H), 3.16 (d, *J*=8.3 Hz, 9 H), 3.05 - 3.11 (m, 3 H), 2.86 - 3.05 (m, 2 H), 2.56 (td, *J*=11.1, 5.4 Hz, 1 H), 2.17 (dd, *J*=17.1, 6.3 Hz, 1 H), 2.01 (br. s., 2 H), 1.69 - 1.93 (m, 9 H), 1.44 - 1.69 (m, 9 H), 1.25 - 1.42 (m, 3 H), 1.13 - 1.25 (m, 4 H), 1.12 (s, 3 H), 1.05 (s, 3 H), 0.99 (s, 3 H), 0.98 (s, 3 H).

### EXAMPLE 34. Preparation of 4-((1R,3aS,5aR,5bR,7aR,11aS,11bR,13aR,13bR)-5a,5b,8,8,11a-pentamethyl-3a-((2-(2-oxopyrrolidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

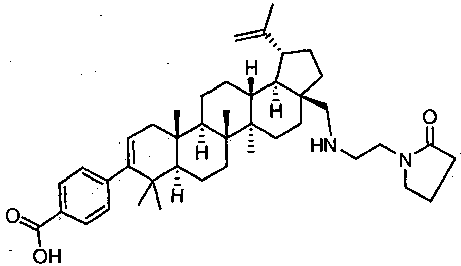

The title compound was prepared following the general procedures described above for the C28 amine formation and hydrolysis using 1-(2-aminoethyl)pyrrolidin-2-one as the reactant amine. The product was isolated as a white solid (34 mg, 63.8 %). LCMS: m/e 655.5 (MH⁺), 2.33 min (method 3). ¹H NMR (400 MHz, *MeOD*) 8 ppm 7.90 (m, *J*=8.3 Hz, 2 H), 7.20 (m, *J*=8.3 Hz, 2 H), 5.22 - 5.34 (m, 1 H), 4.74 (d, *J*=1.8 Hz, 1 H), 4.62 (s, 1 H), 3.57 - 3.71 (m, 2 H), 3.53 (t, *J*=7.2 Hz, 2 H), 3.27 - 3.35 (m, 2 H), 3.25 (d, *J*=12.8 Hz, 1 H), 2.92 (d, *J*=13.1 Hz, 1 H), 2.50 (td, *J*=10.6, 5.7 Hz, 1 H), 2.42 (t, *J*=8.2 Hz, 2 H), 1.97 - 2.18 (m, 4 H), 1.62 - 1.87 (m, 10 H), 1.41 - 1.62 (m, 8 H), 1.20 - 1.41 (m, 4 H), 1.13 - 1.20 (m, 4 H), 1.06 (s, 3 H), 1.02 (s, 3 H), 0.95 (s, 3 H), 0.93 (s, 3 H).

### EXAMPLE 35. Preparation of 4-((1R,3aS,5aR,5bR,7aR,11aS,11bR,13aR,13bR)-5a,5b,8,8,11a-pentamethyl-1-(prop-1-en-2-yl)-3a-((4-sulfamoylbenzylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

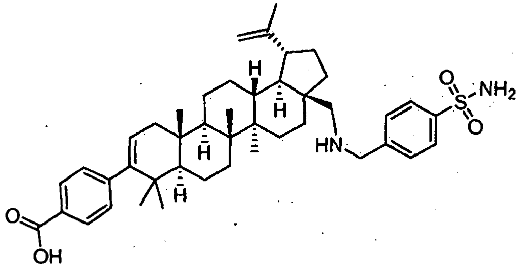

The title compound was prepared following the general procedures described above for the C28 amine formation and hydrolysis using 4-(aminomethyl)benzenesulfonamide as the reactant amine. The product was isolated as a white solid (14 mg, 26.1 %). LCMS: m/e 713.4 (MH⁺), 2.30 min (method 3). ¹H NMR (400 MHz, *MeOD*) δ ppm 7.97 - 8.08 (m, 2 H), 7.90 (m, *J*=8.3 Hz, 2 H), 7.74 (m, *J*=8.3 Hz, 2 H), 7.19 (d, *J*=8.3 Hz, 2 H), 5.15 - 5.35 (m, 1 H), 4.71 (s, 1 H), 4.60 (s, 1 H), 4.41 - 4.54 (m, 1 H), 4.29 (d, *J*=13.3 Hz, 1 H), 2.95 - 3.12 (m, 1 H), 2.83 (d, *J*=12.8 Hz, 1 H), 2.41 (td, *J*=11.1, 5.4 Hz, 1 H), 2.09 (dd, *J*=17.1, 6.3 Hz, 1 H), 1.78-1.99 (m, 1 H), 1.57 - 1.77 (m, 8 H), 1.30 - 1.54 (m, 9 H), 1.18 - 1.29 (m, 3 H), 1.03 - 1.16 (m, 2 H), 1.01 (br. s.; 1 H), 0.98 (s, 6 H), 0.94 (s, 3 H), 0.91 (s, 3 H), 0.78 (s, 3 H).

### EXAMPLE 36. Preparation of 4-((1R,3aS,5aR,5bR,7aR,11aS,11bR,13aR,13bR)-5a,5b,8,8,11a-pentamethyl-1-(prop-1-en-2-yl)-3a-((4-sulfamoylphenethylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

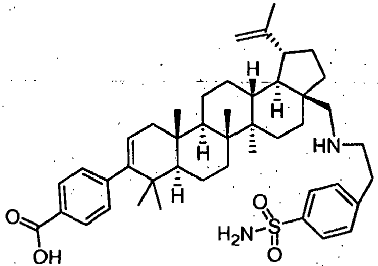

The title compound was prepared following the general procedures described above for the C28 amine formation and hydrolysis using 4-(2-aminoethyl)benzenesulfonamide as the reactant amine. The product was isolated as a white solid (18 mg, 33.5 %). LCMS: m/e 727.4 (MH⁺), 2.30 min (method 3). ¹H, NMR (400 MHz, *MeOD*) 8 ppm 7.82 - 7.94 (m, 4 H), 7.48 (d, *J*=8.3 Hz, 2 H), 7.20 (d, *J*=8.3 Hz, 2 H), 5.28 (d, *J*=4.8 Hz, 1 H), 4.74 (s, 1 H), 4.63 (s, 1 H), 3.31 - 3.39 (m, 2 H), 3.27 - 3.29 (m, 1 H), 3.09 - 3.19 (m, 2 H), 2.82 - 2.92 (m, 1 H), 2.49 (td, *J*=10.6, 5.9 Hz, 1 H), 2.09 - 2.20 (m, 1 H), 1.97 - 2.09 (m, 1 H), 1.78 - 1.89 (m, 2 H), 1.63 - 1.78 (m, 8 H), 1.40 - 1.62 (m, 8 H), 1.18 - 1.40 (m, 5 H), 1.15 (s, 3 H), 1.06 (s, 3 H), 1.02 (s, 3 H), 0.95 (s, 3 H), 0.90 (s, 3 H).

### EXAMPLE 37. Preparation of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

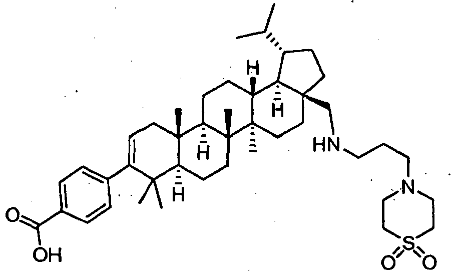

To a solution of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid (50 mg, 0.070 mmol) in MeOH (15 ml) and acetic acid (5.00 ml) was added palladium on carbon (15 mg, 0.141 mmol). The reaction was conducted in a Parr shaker at 40 psi at rt for 16 h. A 30% conversion was observed. The solvent was removed *in vacuo.* The residue was purified by prep. HPLC to afford the title compound as a white solid (10 mg, 19.0 %). LCMS: m/e 721.2 (MH⁺), 2.39 min (method 3). ¹H NMR (400 MHz, *MeOD*) δ ppm 7.95 (m, *J*=8.3 Hz, 2 H), 7.25 (m, *J*=8.3 Hz, 2 H), 5.34 (dd, *J*=6.1, 1.6 Hz, 1 H), 3.10 - 3.29 (m, 11 H), 2.78 - 2.92 (m, 3 H), 2.20 (dd, *J*=17.2, 6.4 Hz, 1 H), 1.93 - 2.12 (m, 3 H), 1.78 (d, *J*=12.0 Hz, 2 H), 1.75 (d, *J*=7.8 Hz, 4 H), 1.43 - 1.67 (m, 10 H), 1.25 - 1.41 (m, 4 H), 1.18 - 1.25 (m, 3 H), 1.11 (br. s., 2 H), 1.08 (d, *J*=3.0 Hz, 6 H), 1.00 (s, 3 H), 0.98 (s, 3 H), 0.93 (d, *J*=6.8 Hz, 3 H), 0.86 (d, *J*=6.8 Hz, 3 H).

### EXAMPLE 38. Preparation of 4-((1R,3aS,5aR,5bR,7aR,11aS,11bR,13aR,13bR)-3a-((4-methoxy-4-oxobutylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

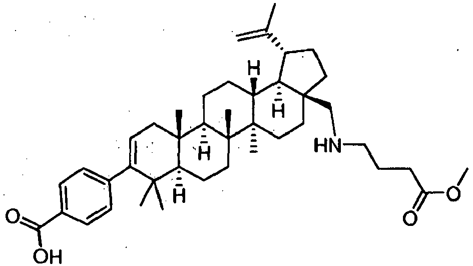

The title compound was prepared following the general procedures described above for the C28 amine formation and hydrolysis using methyl 4-aminobutanoate hydrochloride as the reactant amine. The product was isolated as a white solid (7 mg, 48.2 %). LCMS: m/e 644.4 (MH⁺), 2.21 min (method 3). ¹H NMR (500 MHz, *MeOD*) δ ppm 7.82 - 8.02 (m, 2 H), 7.19 - 7.30 (m, 2 H), 5.22 - 5.39 (m, 1 H), 4.78 (s, 1 H), 4.67 (br. s., 1 H), 3.72 (s, 3 H), 3.27 (d, *J*=13.1 Hz, 1 H), 3.08 - 3.22 (m, 2 H), 2.88 (d, *J*=12.8 Hz, 1 H), 2.43 - 2.62 (m, 3 H), 2.12 - 2.31 (m, 1 H), 1.96 - 2.12 (m, 3 H), 1.69 - 1.88 (m, 10 H), 1.47 - 1.67 (m, 8 H), 1.22 - 1.39 (m, 4 H), 1.14 - 1.22 (m, 4 H), 1.11 (s, 3 H), 1.06 (s, 3 H), 0.99 (s, 3 H), 0.97 (s, 3 H).

### EXAMPLE 39. Preparation of 4-((1R,3aS,5aR,5bR,7aR,11aS,11bR,13aR,13bR)-3a-((2-(dimethylamino)-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

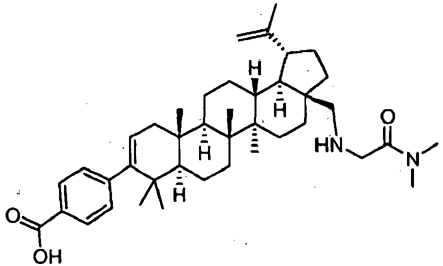

The title compound was prepared following the general procedures described above for the C28 amine formation and hydrolysis using 2-amino-*N*,*N*-dimethylacetamide hydrochloride as the reactant amine. The product was isolated as a white solid (34 mg, 87.9 %). LCMS: m/e 629.4 (MH⁺), 2.37 min (method 3). ¹H NMR (400 MHz, *MeOD*) 8 ppm 7.94 (m, *J*=8.5 Hz, 2 H), 7.24 (m, *J*=8.3 Hz, 2 H), 5.26 - 5.38 (m, 1 H), 4.75 (d, *J*=1.5 Hz, 1 H), 4.65 (s, 1 H), 4.00 - 4.21 (m, 2 H), 3.25 - 3.40 (m, 1 H), 2.98 - 3.12 (m, 6 H), 2.86 (d, *J*=12.5 Hz, 1 H), 2.47 (td, *J*=10.9, 5.6 Hz, 1 H), 2.04 - 2.26 (m, 2 H), 1.85 - 2.04 (m, 3 H), 1.67 - 1.85 (m, 6 H), 1.43 - 1.67 (m, 8 H), 1.19 - 1.42 (m, 5 H), 1.13 - 1.19 (m, 4 H), 1.08 (s, 3 H), 1.05 (s, 3 H), 1.00 (s, 3 H), 0.93 (s, 3 H).

### EXAMPLE 40. Preparation of 4-((1R,3aS,5aR,5bR,7aR,11aS,11bR,13aR,13bR)-3a-(((3-(1,1-dioxido-2-isothiazolidinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

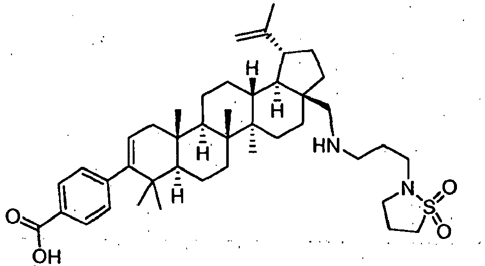

The title compound was prepared following the general procedures described above for the C28 amine formation and hydrolysis using 2-amino-*N*,*N*-dimethylacetamide hydrochloride as the reactant amine. The product was isolated as a white solid (34 mg, 77.9 %). LCMS: m/e 705.8 (MH⁺), 2.34 min (method 3). ¹H NMR (400 MHz, *MeOD*) δ ppm 7.94 (m, *J*=8.3 Hz, 2 H), 7.24 (m, *J*=8.3 Hz, 2 H), 5.25 - 5.42 (m, 1 H), 4.78 (d, *J*=1.5 Hz, 1 H), 4.66 (s, 1 H), 3.31 - 3.39 (m, 1 H), 3.13 - 3.30 (m, 8 H), 2.89 (d, - *J*=13.1 Hz, 1 H), 2.45 - 2.65 (m, 1 H), 2.30 - 2.45 (m, 2 H), 1.99 - 2.21 (m, 4 H), 1.67 - 1.91 (m, 10 H), 1.45 - 1.67 (m, 8 H), 1.24 - 1.45 (m, 4 H), 1.12 - 1.24 (m, 4 H), 1.09 (s, 3 H), 1.04 (s, 3 H), 0.99 (s, 3 H), 0.97 (s, 3 H).

### EXAMPLE 41. Preparation of 4-((1R,3aS,5aR,5bR,7aR,11aS,11bR,13aR,13bR)-5a,5b,8,8,11a-pentamethyl-3a-((2-(methylsulfonyl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

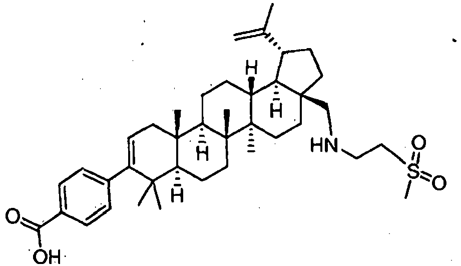

The title compound was prepared following the general procedures described above for the C28 amine formation and hydrolysis using 2-(methylsulfonyl)ethanamine hydrochloride as the reactant amine. The product was isolated as a white solid (22 mg, 47.8 %). LCMS: m/e 650.3 (MH⁺), 2.09 min (method 3). ¹H NMR (400 MHz, *CHLOROFORM-d*) δ ppm 8.01 (m, *J*=8.3 Hz, 2 H), 7.25 (m, *J*=8.3 Hz, 2 H), 5.32 (d, *J*=4.5 Hz, 1 H), 4.74 (s, 1 H), 4.66 (s, 1 H), 3.64 - 3.88 (m, 4 H), 3.34 (br. s., 1 H), 3.06 - 3.22 (m, 3 H), 2.84 (d, *J*=11.8 Hz, 1 H), 2.32 - 2.45 (m, 1 H), 2.12 (dd, *J*=17.2, 6.4 Hz, 1 H), 2.05 (m, 1 H), 1.79 (br. s., 2 H), 1.61 - 1.76 (m, 8 H), 1.56 (d, *J*=8.3 Hz, 3 H), 1.39 - 1.51 (m, 4 H), 1.15 - 1.39 (m, 5 H), 1.07 - 1.15 (m, 4 H), 1.03 (s, 3 H), 1.00 (s, 3 H), 0.96 (d, *J*=3.3 Hz, 6 H).

### EXAMPLE 42. Preparation of 4-((1R,3aS,5aR,5bR,7aR,11aS,11bR,13aR,13bR)-3a-((2,2-diethoxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

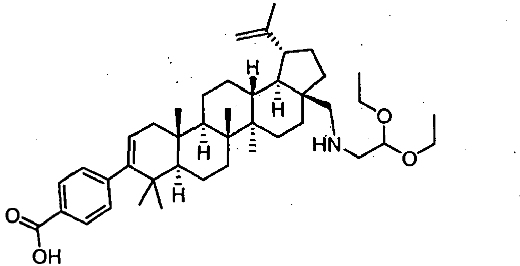

The title compound was prepared following the general procedures described above for the C28 amine formation and hydrolysis using 2, 2-diethoxyethanamine as the reactant amine. The product was isolated as a white solid (5 mg, 8.4 %). LCMS: m/e 660.4 (MH⁺), 2.39 min (method 3). ¹H NMR (400 MHz, *MeOD*) δ ppm 7.94 (m, *J*=8.3 Hz, 2 H), 7.24 (m, *J*=8.5 Hz, 2 H), 5.27 - 5.41 (m, 1 H), 4.92 - 4.98 (m, 1 H), 4.74 - 4.81 (m, 1 H), 4.67 (d, *J*=1.8 Hz, 1 H), 3.79 - 3.95 (m, *J*=9.5, 7.1, 7.1, 7.1, 7.1 Hz, 2 H), 3.63 - 3.79 (m, *J*=9.6, 7.0, 7.0, 7.0, 2.9 Hz, 2 H), 3.48 (d, *J*=13.8 Hz, 1 H), 3.22 - 3.31 (m, 2 H), 2.91 - 3.08 (m, 1 H), 2.47 - 2.55 (m, 1 H), 2.18 (dd, *J*=17.1, 6.3 Hz, 1 H), 2.05(m, 1 H), 1.69 - 1.93 (m, 9 H), 1.62 (br. s., 2 H), 1.46 - 1.61 (m, 6 H), 1.24 - 1.44 (m, 10 H), 1.13 - 1.24 (m, 5 H), 1.11 (s, 3 H), 1.06 (s, 3 H), 0:99 (s, 3 H), 0.98 (s, 3 H).

### EXAMPLE 43. Preparation of 4-((1R,3aS,5aR,5bR,7aR,11aS,11bR,13aR,13bR)-5a,5b,8,8,11a-pentamethyl-3a-((2-(piperazin-1-ylsulfonyl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

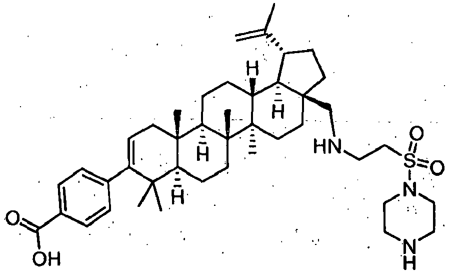

The title compound was prepared following the general procedures described above for the C28 amine formation and hydrolysis using *tert*-butyl 4-(2-aminoethylsulfonyl)piperazine-1-carboxylate hydrochloride as the reactant amine. The product was isolated as a white solid (47 mg, 91.0 %). LCMS: m/e 720.3 (MH⁺), 2.31 min (method 3). ¹H NMR (400 MHz, *MeOD*) δ ppm 7.94 (m, 2H), 7.24 (m, 2 H), 5.20 - 5.41 (m, 1 H), 4.78 (d, *J*=1.8 Hz, 1 H), 4.66 (s, 1 H), 3.64 - 3.74 (m, 6 H), 3.56 - 3.64 (m, 2 H), 3.35 - 3.41 (m, 4 H), 3.31 - 3.35 (m, 1 H), 2.96 (d, *J*=13.1 Hz, 1 H), 2.47 - 2.61 (m, 1 H), 2.00 - 2.24 (m, 2 H), 1.69 - 1.92 (m, 10 H), 1.60 (br. s., 2 H), 1.43 - 1.59 (m, 6 H), 1.26 - 1.43 (m, 3 H), 1.17 - 1.25 (m, 4 H), 1.14 (d, *J*=2.8 Hz, 1 H), 1.09 (s, 3 H), 1.04 (s, 3 H), 0.99 (s, 3 H), 0.95 (s, 3 H).

### EXAMPLE 44. Preparation of 4-((1R,3aS,5aR,5bR,7aR,11aS,11bR,13aR,13bR)-5a,5b,8,8,11a-pentamethyl-3a-((methyl(2-(4-methylpiperazin-1-ylsulfonyl)ethyl)amino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

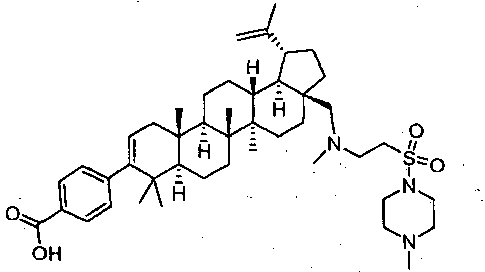

The title compound was prepared following the general procedures described above for the C28 amine formation, hydrolysis and tertiary amine formation using *tert*-butyl 4-(2-aminoethylsulfonyl)piperazine-1-carboxylate hydrochloride as the reactant amine and formaldehyde as the reactant aldehyde. The product was isolated as a white solid (17 mg, 33.1 %). LCMS: m/e 748.5 (MH⁺), 2.37 min (method 3). ¹H NMR (400 MHz, *MeOD*) 8 ppm 7.94 (m, *J*=8.3 Hz, 2 H), 7.24 (m, *J*=8.5 Hz, 2 H), 5.23 - 5.40 (m, 1 H), 4.79 (d, *J*=1.3 Hz, 1 H), 4.68 (s, 1 H), 4.00 (br. s., 1 H), 3.77 - 3.95 (m, 4 H), 3.70 (t, *J*=7.4 Hz, 4 H), 3.38 - 3.58 (m, 3 H), 3.26 - 3.37 (m, 1 H), 3.13 - 3.25 (m, 1 H), 3.07 (s, 3 H), 2.98 (s, 3 H), 2.56 (dt, *J*=10.9, 5.5 Hz, 1 H), 2.00 - 2.25 (m, 2 H), 1.79 - 2.00 (m, 3 H), 1.69 - 1.79 (m, 5 H), 1.46 - .1.69 (m, 9 H), 1.42 (d, *J*=10.8 Hz, 1 H), 1.24 - 1.40 (m, 4 H), 1.21 (s, 3 H), 1.14 - 1.19 (m, 1 H), 1.11 (s, 3 H), 1.05 (s, 3 H), 1.01 (s, 3 H), 0.97 (s, 3 H).

### EXAMPLE 45. Preparation of 4-((1R,3aS,5aR,5bR,7aR,11aS,11bR,13aR,13bR)-5a,5b,8,8,11a-pentamethyl-1-(prop-1-en-2-yl)-3a-((2-sulfamoylethylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

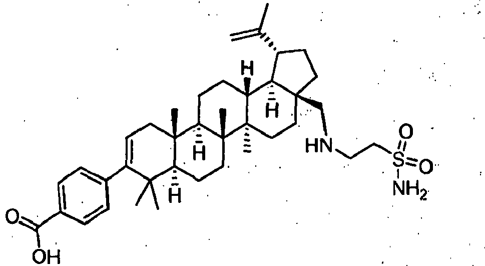

The title compound was prepared following the general procedures described above for the C28 amine formation and hydrolysis using 2-aminoethanesulfonamide as the reactant amine. The product was isolated as a white solid (23 mg, 59.3 %). LCMS: m/e 651.3 (MH⁺), 2.35 min (method 3). ¹H NMR (400 MHz, *MeOD*) δ ppm 7.94 (m, *J*=8.5 Hz, 2 H), 7.24 (m, *J*=8.5 Hz, 2 H), 5.18 - 5.40 (m, 1 H), 4.78 (d, *J*=1.8 Hz, 1 H), 4.67 (s, 1 H), 3.57 - 3.65 (m, 2 H), 3.42 (m, 1H), 3.33 (dq, *J*=3.2, 1.5 Hz, 2 H), 2.97 (d, *J*=12.8 Hz, 1 H), 2.54 (td, *J*=10.7, 5.6 Hz, 1 H), 1.98 - 2.22 (m, 2 H), 1.67-1.92 (m, 10 H), 1.57 (dd, *J*=19.8, 7.5 Hz, 5 H), 1.50 (d, *J*=13.8 Hz, 3 H), 1.24 - 1.45 (m, 4 H), 1.18 - 1.24 (m, 4 H), 1.10 (s, 3 H), 1.04 (s, 3 H), 0.99 (s, 3 H), 0.97 (s, 3 H).

### EXAMPLE 46. Preparation of 4-((1R,3aS,5aR,5bR,7aR,11aS,11bR,13aR,13bR)-3a-((4-(methoxycarbonyl)phenethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

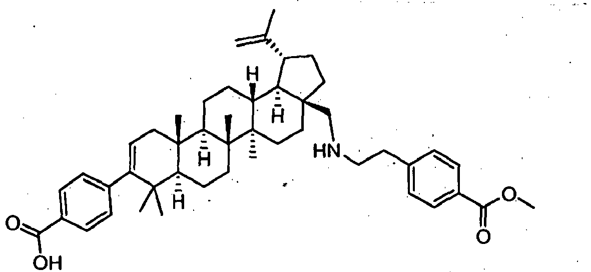

The title compound was prepared following the general procedures described above for the C28 amine formation and hydrolysis using methyl 4-(2-aminoethyl) benzoate as the reactant amine. The product was isolated as a white solid (35 mg, 90.0 %). LCMS: m/e 706.3 (MH⁺), 2.34 min (method 3).

### EXAMPLE 47. Preparation 4-((1R,3aS,5aR,5bR,7aR,11aS,11bR,13aR,13bR)-3a-((4-carboxyphenethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

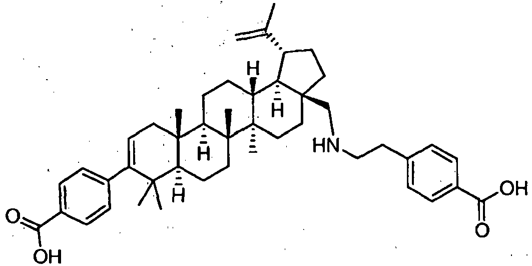

The title compound was prepared following the procedures described below: To the solution of 4-((1R,3aS,5aR,5bR,7aR,11aS,11bR,13aR,13bR)-3a-((4-(methoxycarbonyl)phenethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (5 mg) in dioxane (2 ml) and MeOH (2 ml), 3 mg of lithium hydroxide was added followed by 0.5 ml of water. The resulting clear solution was stirred at 50° C for 12 h. The solvent was removed *in vacuo* and yellow solid was obtained. The crude material was purified by prep. HPLC to afford the title compound as a white solid (1.1 mg, 22.0 %). LCMS: m/e 692.5 (MH⁺), 2.28 min (method 3). ¹H NMR (400 MHz, *MeOD*) δ ppm 6.50 (d, *J*=8.3 Hz, 2 H), 6.38 (d, *J*=8.3 Hz, 2 H), 5.91 (d, *J*=8.3 Hz, 2 H), 5.68 (d, *J*=8.3 Hz, 2 H), 3.75 - 3.81 (m, 1 H), 3.22 (s, 1H), 3.11 (s, 1H), 1.79 - 1.91 (m, 2 H), 1.66 - 1.75 (m, 1 H), 1.51 - 1.66 (m, 2 H), 1.33 (s, 1 H), 0.84 - 1.02 (m, 1 H), 0.44 - 0.68 (m, 2 H), 0.10 - 0.33 (m, 8 H), 0.12 - 0.10 (m, 6 H), 0.34 - 0.12 (m, 6 H), 0.42 - 0.34 (m, 6 H), 0.46 (s, 3 H), 0.49 (s, 3 H), 0.57 (s, 3 H), 0.59 (s, 3 H).

### EXAMPLE 48. Preparation of 4-((1R,3aS,5aR,5bR,7aR,11aS,11bR,13aR,13bR)-3a-((2-(4,4-dilluoropiperidin-1-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

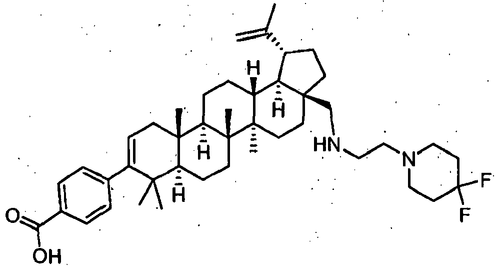

The title compound was prepared following the general procedures described above for the C28 amine formation and hydrolysis using 2-(4,4-difluoropiperidin-1-yl)ethanamine as the reactant amine. The product was isolated as a white solid (45 mg, 62.5 %). LCMS: m/e 691.6 (MH⁺), 2.54 min (method 3). ¹H NMR (400 MHz, *MeOD*) 8 ppm 7.94 (m, 2 H), 7.24 (m, 2 H), 5.31 (d, *J*=4.5 Hz, 1 H), 4.72 - 4.82 (m, 1 H), 4.66 (s, 1 H), 3.49 - 3.67 (m, 2 H), 3.36 - 3.49 (m, 2 H), 3.31 (d, *J*=5.0 Hz, 5 H), 2.89 - 3.02 (m, 1 H), 2.52 (dt, *J=*10.6, 5.4 Hz, 1 H), 2.23 - 2.41 (m, 4 H), 2.01 - 2.23 (m, 2 H), 1.83 - 1.95 (m, 2 H), 1.67 - 1.83 (m, 8 H), 1.43 - 1.67 (m, 8 H), 1.27 - 1.43 (m, 3 H), 1.24 (br. s., 1 H), 1.11 - 1.21 (m, 4 H), 1.09 (s, 3 H), 1.05 (s, 3 H), 0.99 (s, 3 H), 0.94 (s, 3 H).

### EXAMPLE 49. Preparation of 4-((1R,3aS,5aR,5bR,7aR,11aS,11bR,13aR,13bR)-3a-(((S)-1,4-dimethoxy-1,4-dioxobutan-2-ylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

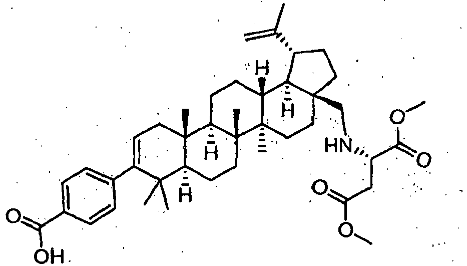

The title compound was prepared following the general procedures described above for the C28 amine formation and hydrolysis using (S)-dimethyl 2-aminosuccinate as the reactant amine. The product was isolated as a white solid (6 mg, 15.7 %). LCMS: m/e 688.6 (MH⁺), 2.73 min (method 3). ¹H NMR (400 MHz, *MeOD*) 8 ppm 7.96 (m, 2 H), 7.24 (m, *J*=8.5 Hz, 2 H), 5.22 - 5.36 (m, 1 H), 4.78 (s, 1 H), 4.67 (s, 1 H), 4.62 (dd, *J*=8.4, 4.6 Hz, 1 H), 3.92 (s, 3 H), 3.83 (s, 3 H), 3.44 (m, 1H), 3.22 (m, 1H), 2.93 - 3.16 (m, 2 H), 2.38 - 2.62 (m, 1 H), 2.11 - 2.26 (m, 2 H), 2.07 (br. s., 1 H), 1.88-2.03 (m, 2 H), 1.67 - 1.88 (m, 6 H), 1.45 - 1.67 (m, 8 H), 1.25 - 1.45 (m, 4 H), 1.15 - 1.25 (m, 3 H), 1.09 - 1.15 (m, 2 H), 1.06 (s, 3 H), 1.04 (s, 3 H), 1.00 (s, 3 H), 0.98 (s, 3 H).

### EXAMPLE 50. Preparation of 4-((1R,3aS,5aR,5bR,7aR,11aS,11bR,13aR,13bR)-3a-((1-carboxycyclopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

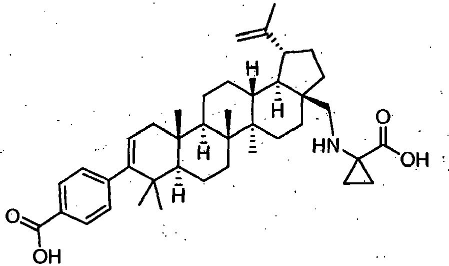

The title compound was prepared following the general procedures described above for the C28 amine formation and hydrolysis using methyl 1-aminocyclopropanecarboxylate hydrochloride as the reactant amine. The product was isolated as a white solid (10 mg, 50 %). LCMS: m/e 628.6 (MH⁺), 2.28 min (method 3). ¹H NMR (400 MHz, *ACETONITRILE-d₃₎* δ ppm 7.91 (m, *J*=7.8 Hz, 2 H), 7.24 (m, *J*=8.0 Hz, 2 H), 5.28 (d, *J=5.0* Hz, 1 H), 4.72 (s, 1 H), 4.62 (br. s., 1 H), 3.43 (d, *J*=12.5 Hz, 1 H), 2.94 (d, *J*=12.5 Hz, 1 H), 2.47 (br. s., 1 H), 2.12 (dd, *J*=17.2, 6.7 Hz, 1 H), 2.00 - 2.08 (m, 1 H), 1.92 (d, *J*=2.5 Hz, 1 H), 1.85 (br. s., 2 H), 1.62 - 1.77 (m, 8 H), 1.57 (br. s., 5 H), 1.44 (br. s., 6 H), 1.33 (br. s., 1 H), 1.28 (br. s., 3 H), 1.08 - 1.19 (m, 4 H), 1.04 (s, 3 H), 1.01 (s, 3 H), 0.86 - 0.97 (m, 6 H).

### EXAMPLE 51. Preparation of4-((1R,3aS,5aR,5bR,7aR,11aS,11bR,13aR,13bR)-3a-((1-(methoxycarbonyl)cyclopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

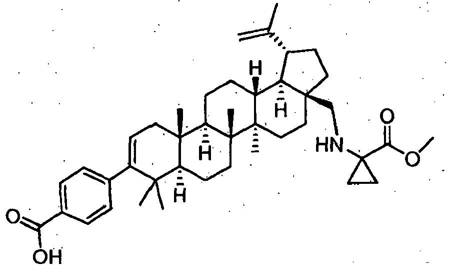

The title compound was prepared following the general procedures described above for the C28 amine formation and hydrolysis using methyl 1-aminocyclopropanecarboxylate hydrochloride as the reactant amine. The product was isolated as a white solid (12mg, 41.3 %). LCMS: m/e 642.6 (MH⁺), 2.64 min (method 3). ¹H NMR (400 MHz, *MeOD)* δ ppm 7.94 (m, 2 H), 7.25 (m, 2 H), 5.32 (d, *J*=4.5 Hz, 1 H), 4.77 (s, 1 H), 4.66 (s, 1 H), 3.76 - 3.87 (m, 3 H), 3.53 (d, *J*=12.5 Hz, 1 H), 3.05 (d, *J*=12.8 Hz, 1 H), 2.44 - 2.62 (m, 1 H), 2.02 - 2.26 (m, 2 H), 1.87 - 2.01 (m, 3 H), 1.59 - 1.80 (m, 13 H), 1.48 - 1.57 (m, 6 H), 1.27 - 1.44 (m, 3 H), 1.13 - 1.27 (m, 5 H), 1.10 (s, 3 H), 1.06 (s, 3 H), 1.01 (s, 3 H), 0.95 (s, 3 H).

### EXAMPLE 52. Preparation of 4-((1R,3aS,5aR,5bR,7aR,11aS,11bR,13aR,13bR)-3a-((1-((diethylamino)methyl)cyclopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

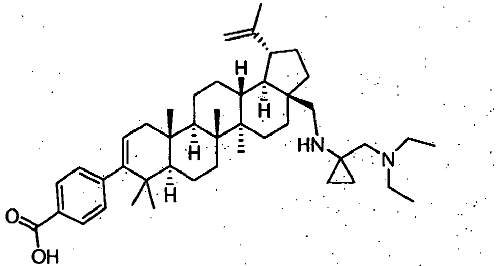

The title compound was prepared following the general procedures described above for the C28 amine formation and hydrolysis using 1-((diethylamino)methyl)cyclopropane amine dihydrochloride as the reactant amine. The product was isolated as a white solid (15 mg, 25.0 %). LCMS: m/e 669.6 (MH⁺), 2.70 min (method 3). ¹H NMR (400 MHz, *MeOD)* δ ppm 7.95 (m, 2 H), 7.24 (m, 2 H), 5.31 (d, *J*=4.5 Hz, 1 H), 4.79 (d, *J=1.5* Hz, 1 H), 4.67 (s, 1 H), 3.83 - 4.00 (m, 1 H), 3.30 - 3.54 (m, 6 H), 3.02 (d, *J*=12.5 Hz, 1 H), 2.48 - 2.70 (m, 1 H), 2.16 (dd, *J*=17.1, 6.5 Hz, 1 H), 1.91 - 2.10 (m, 1 H), 1.64 - 1.88 (m, 10 H), 1.45 - 1.64 (m, 8 H), 1.36 - 1.45 (m, 7 H), 1.21 - 1.36 (m, 6 H), 1.11 - 1.21 (m, 5 H), 1.08 (s, 3 H), 1.05 (s, 3 H), 0.99 (s, 3 H), 0.97 (s, 3 H).

### EXAMPLE 53. Preparation of 4-((1R,3aS,5aR,5bR,7aR,11aS,11bR,13aR,13bR)-3a-((furan-3-ylmethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

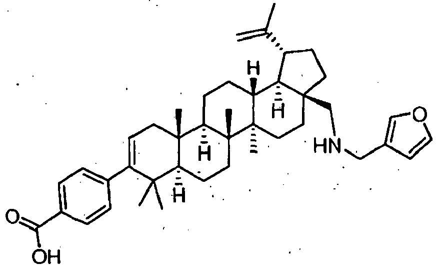

The title compound was prepared following the general procedures described above for the C28 amine formation and hydrolysis using furan-3-ylmethanamine as the reactant amine. The product was isolated as a white solid (60 mg, 41.0 %). LCMS: m/e 624.6 (MH⁺) 2.61 min (method 3). ¹H NMR (400 MHz, *MeOD)* δ ppm 6.28 - 6.44 (m, 2 H), 6.17 - 6.28 (m, 1 H), 6.03 - 6.16 (m, 1 H), 5.53 - 5.73 (m, 2 H), 5.06 (d, *J*=1.3 Hz, 1 H), 3.57 - 3.80 (m, 1 H), 3.09 - 3.20 (m, 1 H), 3.03 (s, 1 H), 2.47 - 2.71 (m, 2 H), 1.57 (d, *J*=12.8 Hz, 1 H), 1.12 - 1.32 (m, 1 H), 0.85 (td, *J*=11.1, 5.6 Hz, 1 H), 0.52 (dd, *J*=17*.*1, 6.3 Hz, 1 H), 0.20 - 0.42 (m, 1 H), 0.04 - 0.19 (m, 8 H), 0.25 - 0.00 (m, 8 H), 0.39 - 0.25 (m, 4 H), 0.56 - 0.39 (m, 3 H), 0.58 (s, 6 H), 0.63 (s, 3 H), 0.64 (s, 3 H), 0.66 (s, 3 H).

### EXAMPLE 54. Preparation of 4-((1R,3aS,5aR,5bR,7aR,11aS,11bR,13aR,13bR)-3a-(((4-(1,1-dioxido-4-thiomorpholinyl)-4-oxobutyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

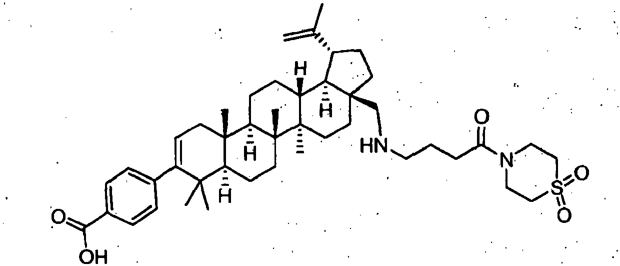

The title compound was prepared following the general procedures described above for the C28 amine formation and hydrolysis using thiomorpholine amide as the reactant amine. The product was isolated as a white solid (12 mg, 41.0 %). LCMS: m/e 747.5 (MH⁺), 2.36 min (method 3). ¹H NMR (400 MHz, *MeOD)* 8 ppm 7.97 (m, 2 H), 7.25 (m, 2 H), 5.27 - 5.34 (m, 1 H), 4.78 (d, *J*=1.5 Hz, 1 H), 4.67 (s, 1 H), 4.05 - 4.19 (m, 2 H), 3.92 - 4.05 (m, 2 H), 3.20 - 3.30 (m, 3 H), 3.08 - 3.20 (m, 4 H), 2.82 - 2.97 (m, 1 H), 2.75 (t, *J*=6.4 Hz, 2 H), 2.53 (td, *J*=10.7, 5.4 Hz, 1 H), 2.17 (dd, *J*=17.1, 6.3 Hz, 1 H), 1.99 - 2.13 (m, 3 H), 1.83 - 1.95 (m, 2 H), 1.67 - 1.83 (m, 8 H), 1.44 - 1.67 (m, 8 H), 1.25 - 1.44 (m, 4 H), 1.13 - 1.25 (m, 4 H), 1.10 (s, 3 H), 1.07 (s, 3 H), 1.00 (s, 3 H), 0.97 (s, 3 H).

### EXAMPLE 55. 4-((1R,3aS,5aR,5bR,7aR,11aS,11bR,13bR,13bR)-3a-((3-(1H-imidazol-1-yl)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

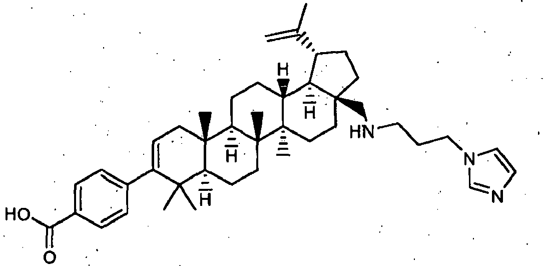

The title compound was prepared in 15 % yield following the general procedures described above for the C28 amine formation and hydrolysis using 3-(1H-imidazol-1-yl)propan-1-amine as the reactant amine. MS: m/e 652.6 (MH+), 1.63 min (method 1). ¹H NMR (400 MHz, *CHLOROFORM-d*) δ ppm 0.86 (s, 3 H) 0.91 (s, 3 H) 0.99 (s, 3 H) 1.00 (s., 3 H) 1.10 (s, 3 H) 1.69 (s, 3 H) 0.88 - 2.60 (m, 24H) 2.29 (d, *J*=11.58 Hz, 1 H) 2.61 - 2.78 (m, 3 H) 2.82 (d, *J*=11.58 Hz, 1 H) 4.02 - 4.10 (m, 1 H) 4.12 - 4.21 (m, 1 H) 4.60 (s, 1 H) 4.70 (s, 1 H) 5.30 (d, *J*=4.53 Hz, 1 H) 6.94 (s, 1 H) 7.15 (s, 1 H) 7.19 (d, *J*=8.31 Hz, 2 H) 7.63 (s, 1 H) 7.99 (d, *J*=8.31 Hz, 2 H).

### EXAMPLE 56. 4-((1R,3aS,5aR,5bR,7aR,11aS,11bR,13aR,13bR)-5a,5b,8,8,11a-pentamethyl-3a-((3-(4-methylpiperazin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

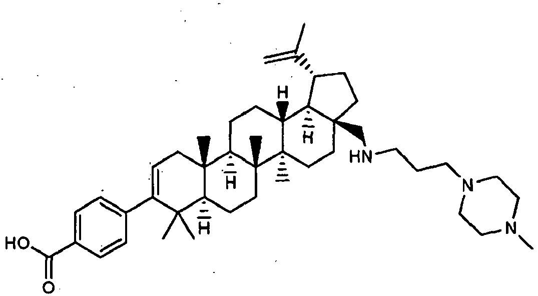

The title compound was prepared in 69 % yield following the general procedures described above for the C28 amine formation and hydrolysis using 3-(4-methylpiperazin-1-yl)propan-1-amine as the reactant amine. MS: m/e 684.6 (MH+), 1.64 min (method 1). ¹H NMR (400 MHz, *CHLOROFORM-d)* δ ppm 0.91 (s, 3 H) 0.93 (s, 3 H) 0.97 (s, 3 H) 1.00 (s, 3 H) 1.08 (s, 3 H) 1.69 (s, 3 H) 2.38 (s, 3 H) 0.85 - 2.13 (m, 31 H) 2.55 - 2.66 (m, 4 H) 3.05 - 3.25 (m, 4 H) 4.62 (s, 1 H) 4.71 (s, 1 H) 5.30 (d, *J*=4.78 Hz, 1 H) 7.17 (d, *J*=8.31 Hz, 2 H) 7.95 (d, *J*=8.06 Hz, 2 H).

### EXAMPLE 57. 4-((1R,3aS,5aR,5bR,7aR,11aS,11bR,13aR,13bR)-3a-((2-(diisopropylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

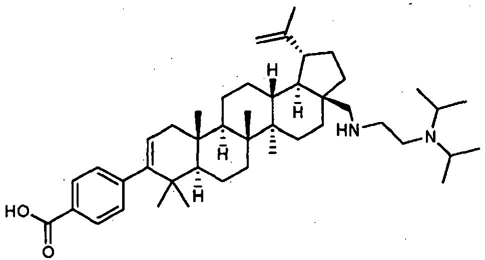

The title compound was prepared in 17 % yield following the general procedures described above for the C28 amine formation and hydrolysis using *N*1,*N*1-diisopropylethane-1,2-diamine as the reactant amine. MS: m/e 671.7 (MH+), 1.65 min (method 1). ¹H NMR (400 MHz, *CHLOROFORM-d)* 8 ppm 0.94 (s, 3 H) 0.94 (s, 3 H) 0.97 (s, 3 H) 1.00 (s, 3 H) 1.07 (s, 3 H) 1.69 (s, 3 H) 0.85 - 2.15 (m, 32 H) 2.09 (dd, *J*=15.99, 5.67 Hz, 1 H) 2.30 - 2.39 (m, 1 H) 2.76 (d, *J*=12.09 Hz, 1 H) 3.25 (d, *J=*12.09 Hz, 1 H) 3.58 - 3.70 (m, 6 H) 3.70 - 3.79 (m, 1 H) 4.62 (s, 1 H) 4.69 (s, 1 H) 5.29 (d, *J*=4.53 Hz, 1 H) 7.23, (d, *J*=8.31 Hz, 2 H) 7.98 (d, *J*=8.31 Hz, 2 H).

### EXAMPLE 58. 4-((1R,3aS,5aR,5bR,7aR,11aS,11bR,13aR,13bR)-5a,5b,8,8,11a-pentamethyl-3a-((3-(2-oxopyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

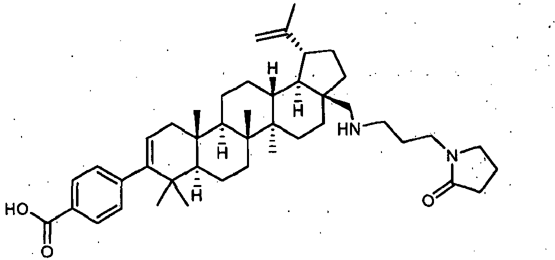

The title compound was prepared in 55 % yield following the general procedures described above for the C28 amine formation and hydrolysis using 1-(3-aminopropyl)pyrrolidin-2-one as the reactant amine. MS: m/e 669.6 (MH+), 1.77 min (method 1). ¹H NMR(400 MHz, *CHLOROFORM-d)* 8 ppm 0.94 (s, 6 H) 0.98 (s, 3 H) 1.01 (s, 3 H) 1.08 (s, 3 H) 1.70 (s, 3 H) 0.77 - 1.79 (m, 17 H) 1.97 - 2.18 (m, 8 H) 2.38 (td, *J*=10.14, 5.67 Hz, 1 H) 2.49 (t, *J*=8.18 Hz, 2 H) 2.70 (t, *J*=10.45 Hz, 1 H) 2.97-3.08(m, 1 H) 3.08 - 3.17 (m, 1 H) 3.22 (t, *J*=10.58 Hz, 1 H) 3.36 - 3.54 (m, 3 H) 3.48 (t, *J*=7.05 Hz, 2 H) 4.62 (s, 1 H) 4.70 (s, 1 H) 5.30 (d, *J*=4.78 Hz, 1 H) 7.23 (d, *J*=8.06 Hz, 2 H) 7.99 (d, *J*=8.06 Hz, 2 H).

### EXAMPLE 59. 4-((1R,3aS,5aR,5bR,7aR,11aS,11bR,13aR,13bR)-5a,5b,8,8,11a-pentamethyl-3a-((2-(2-methyl-1H-imidazol-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

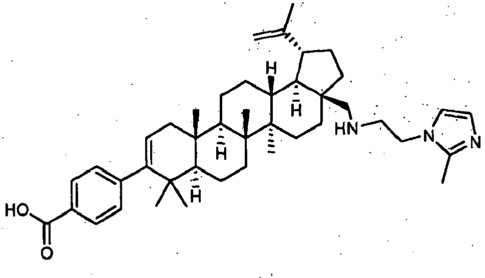

The title compound was prepared in 52 % yield following the general procedures described above for the C28 amine formation and hydrolysis using 2-(2-methyl-1H-imidazol-1-yl)ethanamine as the reactant amine. MS: m/e 652.6 (MH+), 1.67 min (method 1). ¹H NMR (400 MHz, *MeOD)* δ ppm 0.95 (s., 3 H) 0.96 (s, 3 H) 1.03 (s, 3 H) 1.08 (s, 3 H) 1.17 (s, 3 H) 1.73 (s., 3 H) 0.90 - 1.93 (m, 21 H) 2.00 - 2.10 (m, 1 H) 2.14 (dd, *J*=17.12, 6.55 Hz, 1 H) 2.46 - 2.55 (m, 1 H) 2.69 (s, 3 H) 2.93 (d, *J=*12.34 Hz, 1 H) 3.53 - 3.69 (m, 2 H) 4.54 (t, *J*=7.30 Hz, 2 H) 4.65 (s., 1 H) 4.75 (s., 1 H) 5.27 - 5.33 (m, 1 H) 7.21 (d, *J*=8.31 Hz, 2 H) 7.49 - 7.52 (m, 1 H) 7.54 (d, *J*=2.01 Hz, 1 H) 7.91 (d, *J*=8.31 Hz, 2 H).

### EXAMPLE 60. 4-((1R,3aS,5aR,5bR,7aR,11aS,11bR,13aR,13bR)-3a-((3-(bis(2-hydroxyethyl)amino)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

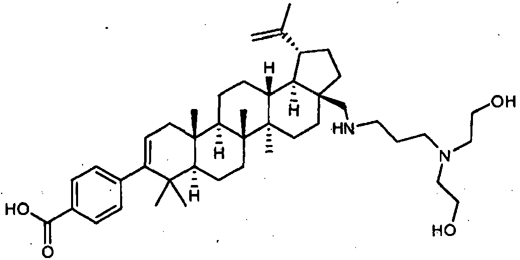

The title compound was prepared in 76 % yield following the general procedures described above for the C28 amine formation and hydrolysis using 2,2'-(3-aminopropylazanediyl)diethanol as the reactant amine. MS: m/e 689.6 (MH+), 1.63 min (method 1). ¹H NMR (400 MHz, *MeOD)* δ ppm 0.95 (s, 3 H) 0.96 (s, 3 H) 1.04 (s, 3 H) 1.08 (s, 3 H) 1.17 (s, 3 H) 1.73 (s, 1 H) 0.85 - 1.89 (m. 29 H) 2.00 - 2.11 (m, 1 H) 2.15 (dd, *J*=17.12, 6.04 Hz, 1 H) 2.19 - 2.29 (m, 2 H) 2.46 - 2.56 (m, 1 H) 2.87 (d, *J*=12.34 Hz, 1 H) 3.15 - 3.29 (m, 4 H) 3.90 (t, *J*=4.78 Hz, 4 H) 4.65 (s, 1 H) 4.76 (s, 1 H) 5.28 - 5.33 (m, 1 H) 7.22 (d, *J*=8.31 Hz, 2 H) 7.92 (d, *J*=8.31 Hz, 2 H).

### EXAMPLE 61. 4-((1R,3aS,5aR,5bR,7aR,11aS,11bR,13aR,13bR)-3a-((3-(2-hydroxyethylamino)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

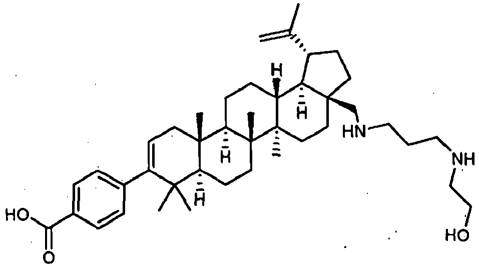

The title compound was prepared in 35 % yield following the general procedures described above for the C28 amine formation and hydrolysis using 2-(3-aminopropylamino)ethanol as the reactant amine. MS: m/e 645.6 (MH+), 1.65 min (method 1). ¹H NMR (400 MHz, *MeOD)* δ ppm 0.94 (s, 3 H) 0.96 (s, 3 H) 1.03 (s, 3 H) 1.07 (s, 3 H) 1.16 (s, 3 H) 1.72 (s, 3 H) 0.85 - 1.89 (m, 21 H) 1.98 - 2.26 (m, 4 H) 2.45 - 2.55 (m, 1 H) 2.85 (d, *J*=12.84 Hz, 1 H) 3.11 - 3.28 (m, 8 H) 3.78 - 3.84 (m, 2 H) 4.64 (s, 1 H) 4.75 (s, 1 H) 5.29 (d, *J*=4.53 Hz, 1 H) 7.21 (d, *J*=8.31 Hz, 2 H) 7.91 (d, *J*=8.31 Hz, 2 H).

### EXAMPLE 62. 4-((1R,3aS,5aR,5bR,7aR,11aS,11bR,13aR,13bR)-3a-((2-(2-hydroxyethylamino)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

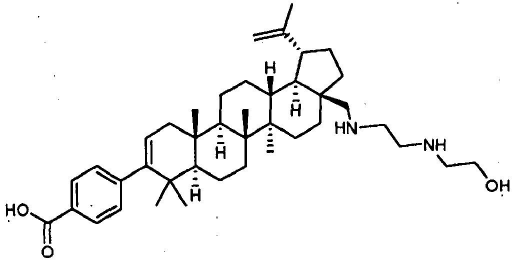

The title compound was prepared in 83 % yield following the general procedures described above for the C28 amine formation and hydrolysis using 2-(2-aminoethylamino)ethanol as the reactant amine. MS: m/e 631.5 (MH+), 1.65 min (method 1). ¹H NMR (400 MHz, *MeOD)* 8 ppm 0.95 (s, 3 H) 0.96 (s, 3 H) 1.04 (s, 3 H) 1.07 (s, 3 H) 1.17 (s, 3 H) 1.73 (s, 3 H) 0.85 - 1.93 (m, 22 H) 2.00 - 2.11 (m, 1 H) 2.15 (dd, *J=*17.12, 6.30 Hz, 1 H) 2.47 - 2.56 (m, 1 H) 2.93 (d, *J*=12.84 Hz, 1 H) 3.22 (dd, *J*=5.79, 4.53 Hz, 2 H) 3.27 - 3.29 (m, 1 H) 3.46 - 3.56 (m, 4 H) 3.83 (dd, *J*=5.67, 4.41 Hz, 2 H) 4.64 (br. s., 1 H) 4.76 (s, 1 H) 5.27 - 5.33 (m, 1 H) 7.21 (d, *J*=8.31 Hz, 2 H) 7.91 (d, *J*=8.31 Hz, 2 H).

### EXAMPLE 63. 4-((1R,3aS,5aR,5bR,7aR,11aS,11bR,13aR,13bR)-5a,5b,8,8,11a-pentamethyl-3 a-((2-morpholinoethylamino)methyl))-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

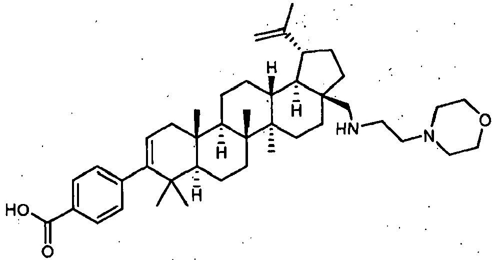

The title compound was prepared in 92 % yield following the general procedures described above for the C28 amine formation and hydrolysis using 2-, morpholinoethanamine as the reactant amine. MS: m/e 657.6 (MH+), 1.70 min (method 1). ¹H NMR (400 MHz, *MeOD)* 8 ppm 0.94 (s, 3 H) 0.96 (s, 3 H) 1.03 (s, 3 H) 1.07 (s, 3 H) 1.16 (s, 3 H) 1.72 (s, 3 H) 0.83 - 1.90 (m, 21 H) 1.97 - 2.09 (m, 1 H) 2.14 (dd, *J*=17.12, 6.30 Hz, 1 H) 2.46 - 2.55 (m, 1 H) 2.82 - 2.95 (m, 5 H) 3.02 - 3.11 (m, 2 H) 3.39 (t, *J*=6.42 Hz, 2 H) 3.81 (t, *J*=4.03 Hz, 4 H) 4.64 (s, 1 H) 4.74 - 4.77 (m, 1 H) 5.27 - 5.31 (m, 1 H) 7.21 (d, *J*=8.31 Hz, 2 H) 7.91 (d, *J=*8*.*31 Hz, 2 H).

### EXAMPLE 64. 4-((1R,3aS,5aR,5bR,7aR,11aS,11bR,13aR,13bR)-3a-((2-carboxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

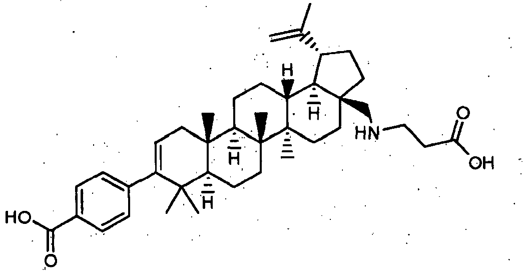

The title compound was prepared in 74 % yield following the general procedures described above for the C28 amine formation and hydrolysis using *tert*-butyl 3-aminopropanoate hydrochloride as the reactant amine. MS: m/e 616.4 (MH+), 1.69 min (method 1). ¹H NMR (400 MHz, *MeOD)* 8 ppm 0.95 (s, 3 H) 0.97 (s, 3 H) 1.04 (s, 3 H) 1.08 (s, 3 H) 1.18 (s, 3 H) 1.73 (s, 3 H) 0.92 - 1.86 (m, 24H) 1.99 - 2.10 (m, 1 H) 2.15 (dd, *J*=17.12,6.30 Hz, 1 H) 2.47 - 2.56 (m, 1 H) 2.76 (t, *J*=6.42 Hz, 2 H) 2.88 (d, *J*=12.34 Hz, 1 H) 4.64 (s, 1 H) 4.76 (s, 1 H) 5.28 - 5.32 (m, 1 H) 7.22 (d, *J*=8.56 Hz, 2 H) 7.91 (d, *J*=8.31 Hz, 2 H).

### EXAMPLE 65. 4-((1R,3aS,5aR,5bR,7aR,11aS,11bR,13aR,13bR)-3a-((3-bromopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

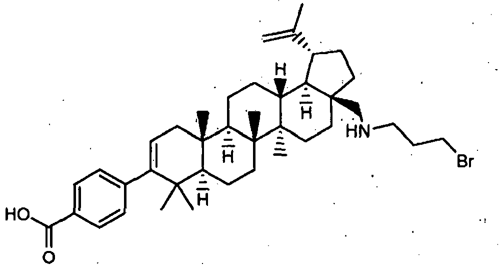

The title compound was prepared in 23 % yield following the general procedures described above for the C28 amine formation and hydrolysis using 3-bromopropan-1-amine hydrobromide as the reactant amine. MS: m/e 664 (MH+), 1.58 min (method 2). ¹H NMR (400 MHz, *MeOD)* δ ppm 0.95 (s, 3 H) 0.97 (s, 3 H) 1.04 (s, 3 H) 1.08 (s, 3 H) 1.18 (s, 3 H) 1.73 (s, 3 H) 0.85 - 1.85 (m, 19 H) 1.99 - 2.10 (m, 1 H) 2.15 (dd, *J=*17.37*,* 6.55 Hz, 1 H) 2.26 - 2.36 (m, 2 H) 2.47 - 2.56 (m, 1 H) 2.88 (d, *J*=12.34 Hz, 1 H) 3.20 - 3.28 (m, 4 H) 3.56 (t, *J*=6.30 Hz, 2 H) 4.64 (s, 1 H) 4.76 (s, 1 H) 5.28 - 5.32 (m, 1 H) 7.22 (d, *J*=8.31 Hz, 2 H) 7.92 (d, *J*=8.56 Hz, 2 H).

### EXAMPLE 66. 4-((1R,3aS,5aR,5bR,7aR,11aS,11bR,13aR,13bR)-5a,5b,8,8,11a-pentamethyl-1-(prop-1-en-2-yl)-3 a-(((1-(pyrrolidin-1-ylmethyl)cyclopropyl)methylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

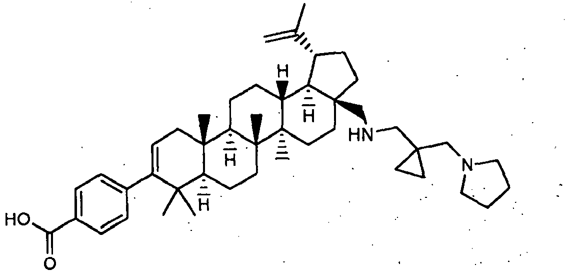

The title compound was prepared in 50 % yield following the general procedures described above for the C28 amine formation and hydrolysis using (1-(pyrrolidin-1-ylmethyl)cyclopropyl)methanamine as the reactant amine. MS: m/e 681.3 (MH+), 1.51 min (method 2). ¹H NMR (400 MHz, *MeOD)* 8 ppm 0.95 (s, 3 H) 0.96 (s, 3 H) 1.03 (s, 3 H) 1.07 (s, 3 H) 1.15 (s, 3 H) 1.72 (s, 3 H) 0.84 - 1.97 (m, 27 H) 2.00 - 2.19 (m, 6 H) 2.48 (td, *J*= 10.95, 5.79 Hz, 1 H) 2.89 (d, *J*=13.09 Hz, 1 H) 3.21 - 3.45 (m, 6 H) 4.64 (s, 1 H) 4.75 (s, 1 H) 5.27 - 5.32 (m, 1 H) 7.22 (d, *J*=8.31 Hz, 2 H) 7.91 (d, *J*=8.31 Hz, 2 H).

### EXAMPLE 67. 4-((1R,3aS,5aR,5bR,7aR,11aS,11bR,13aR,13bR)-3a-((3-aminopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

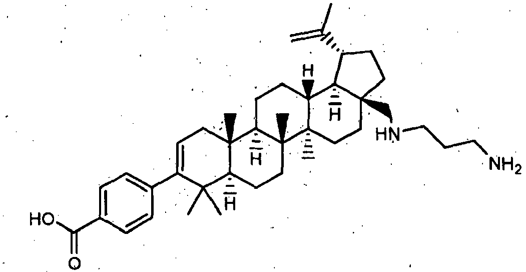

The title compound was prepared in 46 % yield following the general procedures described above for the C28 amine formation and hydrolysis using *tert-*butyl 3-aminopropylcarbamate as the reactant amine. MS: m/e 601.4 (MH+), 1.48 min (method 2). ¹H NMR (400 MHz, *MeOD)* 8 ppm 0.94 (s, 3 H) 0.96 (s, 3 H) 1.03 (s, 3 H) 1.06 (s, 3 H) 1.16 (s, 3 H) 1.72 (s, 3 H) 0.85 - 1.89 (m, 22 H) 1.99 - 2.20 (m, 4 H) 2.50 (td, *J*=10.45, 5.79 Hz, 1 H) 2.85 (d, *J*=13.09 Hz, 1 H) 3.05 (t, *J*=7.55 Hz, 2 H) 3.13 - 3.28 (m, 3 H) 4.63 (s, 1 H) 4.73 - 4.76 (m, 1 H) 5.27 - 5.31 (m, 1 H) 7.21 (d, *J=*8.31 Hz, 2 H) 7.91 (d, *J*=8.31 Hz, 2 H).

### EXAMPLE 68. 4-((1R,3aS,5aR,5bR,7aR,11aS,11bR,13aR,13bR)-5a,5b,8,8,11a-pentamethyl-1-(prop-1-en-2-yl)-3a-((3-(pyrrolidin-1-yl)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

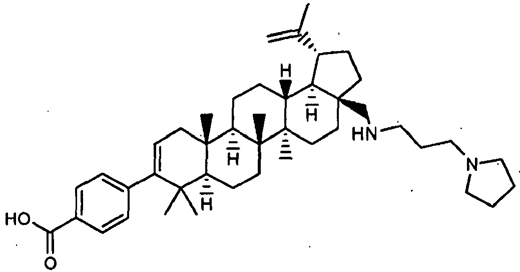

The title compound was prepared in 52 % yield following the general procedures described above for the C28 amine formation and hydrolysis using 3-(pyrrolidin-1-yl)propan-1-amine as the reactant amine. MS: m/e 655.4 (MH+), 1.49 min (method 2). ¹H NMR (400 MHz, *MeOD)* 8 ppm 0.94 (s, 3 H) 0.96 (s, 3 H) 1.03 (s, 3 H) 1.07 (s, 3 H) 1.16 (s, 3 H) 1.72 (s, 3 H) 0.85 - 1.89 (m, 25 H) 1.99 - 2.27 (m, 8 H) 2.50 (td, *J*=10.32, 5.54 Hz, 1 H) 2.87 (d, *J*=13.09 Hz, 1 H) 3.19 (dt, *J*=12.53, 3.56 Hz, 2 H) 3.22 - 3.30 (m, 2 H) 4.64 (s, 1 H) 4.75 (s, 1 H) 5.27 - 5.31 (m, 1 H) 7.21 (d, *J*=8.31 Hz, 2 H) 7.92 (d, *J=*8.31 Hz, 2 H).

### EXAMPLE 69. 4-((1R,3aS,5aR,5bR,7aR,11aS,11bR,13aR,13bR)-3a-((3-hydroxypropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

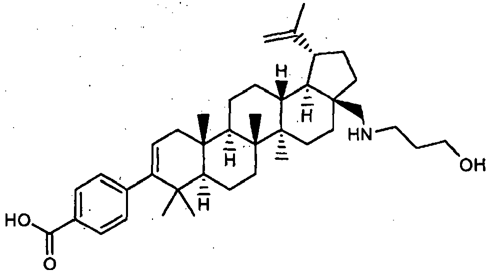

The title compound was prepared in 31 % yield following the general procedures described above for the C28 amine formation and hydrolysis using 3-aminopropan-1-ol as the reactant amine. MS: m/e 602.4 (MH+), 1.52 min (method 2). ¹H NMR (500 MHz, *MeOD)* δ ppm 0.95 (s, 3 H) 0.96 (s, 3 H) 1.04 (s, 3 H) 1.08 (s, 3 H) 1.18 (s, 3 H) 1.73 (s, 3 H) 0.87 - 1.81 (m, 21 H) 1.91 - 1.99 (m, 2 H) 1.99 - 2.09 (m, 1 H) 2.15 (dd, *J=*17.24, 6.26 Hz, 1 H) 2.47 - 2.55 (m, 1 H) 2.86 (d, *J*=12.51 Hz, 1 H) 3.21 - 3.27 (m, 3 H) 3.74 - 3.79 (m, 2 H) 4.64 (s, 1 H) 4.76 (s, 1 H) 5.27 - 5.32 (m, 1 H) 7.22 (d, *J*=8.24 Hz, 2 H) 7.91 (d, *J*=8.24 Hz, 2 H).

### EXAMPLE 70. 4-((1R,3aS,5aR,5bR,7aR,11aS,11bR,13aR,13bR)-5a,5b,8,8,11a-pentamethyl-3a-((3-oxo-3-(pyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

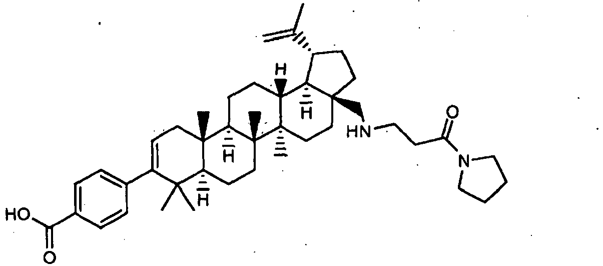

The title compound was prepared in 43 % yield following the general procedures described above for the C28 amine formation and hydrolysis using 3-amine-1-(pyrrolidin-1-yl)propan-1-one hydrochloride as the reactant amine. MS: m/e 669.4 (MH+), 1.58 min (method 2). ¹H NMR (500 MHz, *MeOD)* δ ppm 0.95 (s, 3 H) 0.96 (s, 3 H) 1.04 (s, 3 H) 1.08 (s, 3 H) 1.17 (s, 3 H) 1.73 (s, 3 H) 0.87 - 1.86 (m, 20 H) 1.88 - 1.95 (m, 2 H) 1.97 - 2.11 (m, 3 H) 2.15 (dd, *J*=17.09, 6.41 Hz, 1 H) 2.48 - 2.56 (m, 1 H) 2.81 (t, *J*=5*.*80 Hz, 2 H) 2.88 (d, *J*=13.12 Hz, 1 H) 3.25 (d, *J*=13.43 Hz, 1 H) 3.32 - 3.42 (m, 2 H) 3.42 - 3.52 (m, 4 H) 4.64 (s, 1 H) 4.76 (s, 1 H) 5.27 - 5.32 (m, 1 H) 7.22 (d, *J*=7.93 Hz, 2 H) 7.91 (d, *J*=8.24 Hz, 2 H).

### EXAMPLE 71. 4-((1R,3aS,5aR,5bR,7aR,11aS,11bR,13aR,13bR)-3a-((3-(2,5-dioxopyrrolidin-1-yl)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

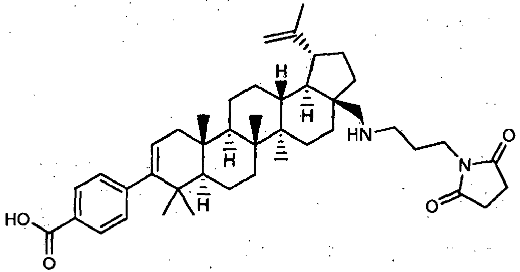

The title compound was prepared in 25 % yield following the general procedures described above for the C28 amine formation and hydrolysis using 1-(3-aminopropyl)pyrrolidine-2,5-dione hydrochloride as the reactant amine. MS: m/e 683.4 (MH+), 1.56 min (method 2). ¹H NMR (500 MHz, *MeOD)* δ ppm 0.95 (s, 3 H) 0.97 (s, 3 H) 1.04 (s, 3 H) 1.08 (s, 3 H) 1.17 (s, 3 H) 1.73 (s, 3 H) 0.85 - 2.19 (m, 26 H) 2.45 - 2.53 (m, 1 H) 2.71 - 2.75 (m, 2 H) 2.79 - 2.85 (m, 1 H) 3.09 (q, *J*=6.56 Hz, 2 H) 3.18 - 3.24 (m, 1 H) 3.63 (q, *J*=6.51 Hz, 2 H) 4.64 (s, 1 H) 4.75 (s, 1 H) 5.27 - 5.31 (m, 1 H) 7.22 (d, *J*=8.24 Hz, 2 H) 7.91 (d, *J*=8.24 Hz, 2 H).

### EXAMPLE 72. 4-((1R,3aS,5aR,5bR,7aR,11aS,11bR,13aR,13bR)-5a,5b,8,8,11a-pentamethyl-3 a-((3-(piperidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

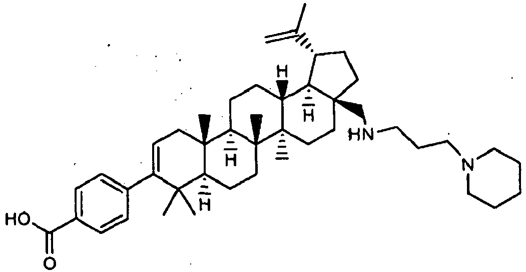

The title compound was prepared in 49 % yield following the general procedures described above for the C28 amine formation and hydrolysis using 3-(piperidin-1-yl)propan-1-amine as the reactant amine. MS: m/e 683.4 (MH+), 1.50 min (method 2). ¹H NMR (500 MHz, *MeOD)* δ ppm 0.95 (s, 3 H) 0.96 (s, 3 H) 1.03 (s, 3 H) 1.08 (s, 3 H) 1.17 (s, 3 H) 1.73 (s, 3 H) 0.81 - 2.11 (m, 27 H) 2.15 (dd, *J*=17.24, 6.26 Hz, 1 H) 2.22 (dt, *J*=16.25, 8.20 Hz, 2 H) 2.51 (td, *J*=10.45, 6.26 Hz, 1 H) 2.84 - 3.00 (m, 2 H) 3.13 - 3.28 (m, 6 H) 3.50 - 3.61 (m, 2 H) 4.65 (s, 1 H) 4.75 (s, 1 H) 5.30 (d, *J*=6.10 Hz, 1 H) 7.21 (dd, *J*=8.24, 1.53 Hz, 2 H) 7.91 (dd, *J*=8.24, 1.53 Hz, 2 H).

### EXAMPLE 73. 4-((1R,3aS,5aR,5bR,7aR,11aS,11bR,13aR,13bR)-3a-((((2E)-4-(1,1-dioxido-4-thiomorpholinyl)-2-buten-1-yl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

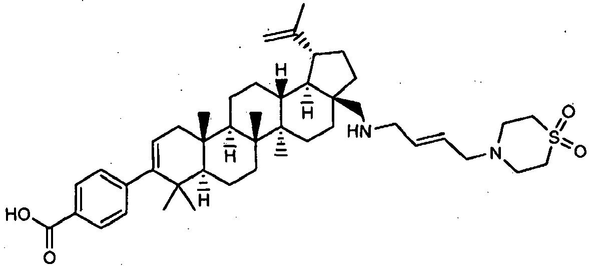

The title compound was prepared in 11 % yield following the general procedures described above for the C28 amine formation and hydrolysis using (1,1-dioxido-4-thiomorpholinyl)-2-buten-1-amine as the reactant amine. MS: m/e 731.2 (MH+), 1.54 min (method 2). ¹H NMR (400 MHz, *MeOD)* 8 ppm 0.95 (s, 3 H) 0.97 (s, 3 H) 1.04 . (s, 3 H) 1.08 (s, 3 H) 1.16 (s, 3 H) 1.72 (s, 3 H) 0.85 - 1.82 (m, 19 H) 1.95 - 2.09 (m, 1 H) 2.15 (dd, *J=*17.07, 6.53 Hz, 1 H) 2.49 (td, *J*=10.67, 5.52 Hz, 1 H) 2.81 (d, *J*=13.05 Hz, 1 H) 3.05 - 3.12 (m, 4 H) 3.12 - 3.18 (m, 4 H) 3.19 - 3.26 (m, 2 H) 3.33 - 3.37 (m, 2 H) 3.69 - 3.82 (m, 2 H) 4.65 (s, 1 H) 4.76 (d, *J*=1.51 Hz, 1 H) 5.30 (dd, *J*=6.27, 1.51 Hz, 1 H) 5.85 (ddd, *J*=15.43, 7.15, 7.03 Hz, 1 H) 6.11 (dt, *J*=15.25, 6.31 Hz, 1 H) 7.21 (d, *J*=8.53 Hz, 2 H) 7.91 (d, *J*=8.28 Hz, 2 H).

### EXAMPLE 74. 4-((1R,3aS,5aR,5bR,7aR,11aS,11bR,13aR,13bR)-3a-((3-(2-(hydroxymethyl)pyrrolidin-1-yl)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

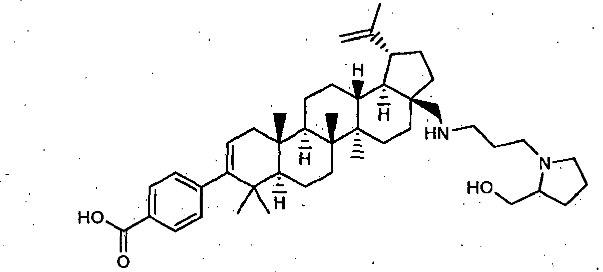

The title compound was prepared in 39 % yield following the general procedures described above for the C28 amine formation and hydrolysis using (1-(3-aminopropyl)pyrrolidin-2-yl)methanol as the reactant amine. MS: m/e 685.3 (MH+), 1.47 min (method 2). ¹H NMR (400 MHz, *MeOD)* δ ppm 0.95 (s, 3 H) 0.96 (s, 3 H) 1.04 (s, 3 H) 1.08.(s, 3 H) 1.17 (s, 3 H) 1.73 (s, 3 H) 0.86 - 2.32 (m, 30 H) 2.50 (td, *J*=10.85, 5.65 Hz, 1 H) 2.87 (d, *J*=13.55 Hz, 1 H) 3.15 - 3.23 (m, 4 H) 3.49 - 3.76 (m, 4 H) 3.90 (dd, *J*=12.05, 3.51 Hz, 1 H) 4.65 (s, 1 H) 4.75 (d, *J*=1.51 Hz, 1 H) 5.30 (dd, *J*=6.02, 1.51 Hz, 1 H) 7.21 (d, *J*=8.28 Hz, 2 H) 7.91 (d, *J*=8.53 Hz, 2 H).

### EXAMPLE 75. 4-((1R,3aS,5aR,5bR,7aR,11aS,11bR,13aR,13bR)-3a-((3-(dimethylamino)-3 -oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

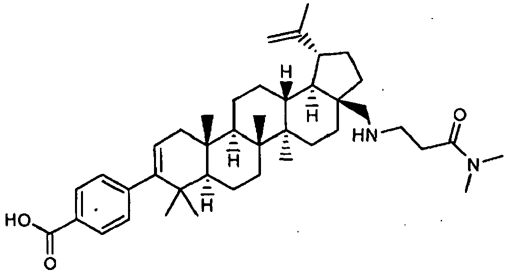

The title compound was prepared in 39 % yield following the general procedures described above for the C28 amine formation and hydrolysis using 3-amino-*N*,*N-*dimethylpropanamide as the reactant amine. MS: m/e 643.3 (MH+), 1.53 min (method 2). ¹H NMR (400 MHz, *MeOD)* δ ppm 0.96 (s, 3 H) 0.98 (s, 3 H) 1.05 (s, 3 H) 1.10 (s, 3 H) 1.18 (s, 3 H) 1.74 (s, 3 H) 0.88 - 1.87 (m, 20 H) 2.01 - 2.11 (m, 1 H) 2.16 (dd, *J*=17.07, 6.53 Hz, 1 H) 2.53 (td, *J*=10.67, 5.52 Hz, 1 H) 2.85 - 2.92 (m, 3 H) 2.99 (s, 3 H) 3.08 (s, 3 H) 3.24 - 3.29 (m, 1 H) 3.34 - 3.39 (m, 2 H) 4.65 - 4.67 (m, 1 H) 4.77 (d, *J*=1.76 Hz, 1 H) 5.31 (dd, *J*=6.27, 1.76 Hz, 1 H) 7.23 (d, *J*=8.53 Hz, 2 H) 7.93 (d, *J*=8.28 Hz, 2 H).

### EXAMPLE 76. 4-((1R,3aS,5aR,5bR,7aR,11aS,11bR,13aR,13bR)-3a-(((4-(1,1-dioxido-4-thiomorpholinyl)butyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

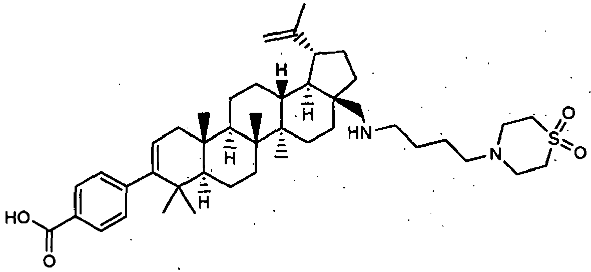

The title compound was prepared in 11 % yield following the general procedures described above for the C28 amine formation and hydrolysis using 4-(1,1-dioxido-4-thiomorpholinyl)butyl)amine as the reactant amine. MS: m/e 733.2 (MH+), 1.49 min (method 2). ¹H NMR (400 MHz, *MeOD)* δ ppm 0.95 (s, 3 H) 0.97 (s, 3 H) 1.04 (s, 3 H) 1.08 (s, 3 H) 1.18 (s, 3 H) 1.73 (s, 3 H) 0.86 - 1.91 (m, 27 H) 1.98 - 2.09 (m, 1 H) 2.15 (dd, *J*=17.19, 6.40 Hz, 1 H) 2.51 (td, *J*=10.35, 5.40 Hz, 1 H) 2.81 - 2.87 (m, 1 H) 2.94 (t, *J*=7.15 Hz, 2 H) 3.08 - 3.16 (m, 2 H) 3.18 - 3.27 (m, 2 H) 3.36 - 3.44 (m, 4 H) 4.65 (s, 1 H) 4.76 (d, *J*=1.51 Hz, 1 H) 5.30 (dd, *J*=6.02, 1.51 Hz, 1 H) 7.21 (d,*J*=8.28 Hz, 2 H) 7.91 (d, *J*=8.53 Hz, 2H).

### EXAMPLE 77. 4-((1R,3aS,5aR,5bR,7aR,11aS,11bR,13aR,13bR)-5a,5b,8,8,11a-pentamethyl-3a-((2-(2-oxoimidazolidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

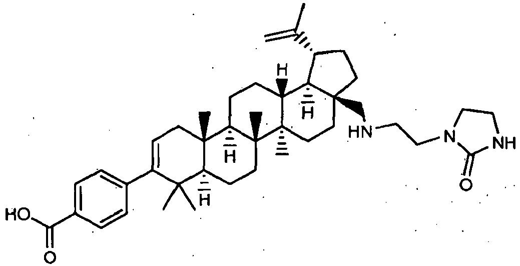

The title compound was prepared in 9 % yield following general procedures described above for the C28 amine formation and hydrolysis using 1-(2-aminoethyl)imidazolidin-2-one as the reactant amine. MS: m/e 656.3 (MH+), 1.56 min (method 2). ¹H NMR (400 MHz, *MeOD).* 8 ppm 0.95 (s, 3 H) 0.96 (s, 3 H) 1.04 (s, 3 H) 1.08 (s, 3 H) 1.17 (s, 3 H) 1.72 (s, 3 H) 0.87 - 1.87 (m, 20 H) 1.99 - 2.11 (m, 1 H) 2.15 (dd, *J*=17.07, 6.02 Hz, 1 H) 2.51 (td, *J*=10.79, 6.02 Hz, 1 H) 2.92 (d, *J*=13.55 Hz, 1 H) 3.21 - 3.33,(m, 3 H) 3.42 - 3.59 (m, 6 H) 4.63 - 4.65 (m, 1 H) 4.76 (d, *J*=1.76 Hz, 1 H) 5.30 (dd, *J*=6.15, 1.63 Hz, 1 H) 7.21 (d, *J*=8.28 Hz, 2 H) 7.91 (d, *J*=8.28 Hz, 2 H).

### EXAMPLE 78. 4-((1R,3aS,5aR,5bR,7aR,11aS,11bR,13aR,13bR)-5a,5b,8,8,11a-pentamethyl-3a-((2-(2-oxopiperidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

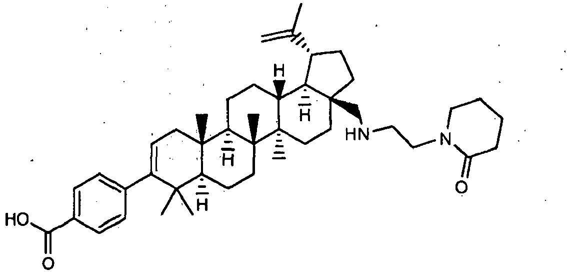

The title compound was prepared in 28 % yield following the general procedures described above for the C28 amine formation and hydrolysis using 1-(2-aminoethyl)piperidin-2-one hydrobromide as the reactant amine. MS: m/e 669.6 (MH+), 2.13 min (method 2). ¹H NMR (400 MHz, *MeOD)* δ ppm 0.95 (s, 3 H) 0.96 (s, 3 H) 1.04 (s, 3 H) 1.08 (s, 3 H) 1.17 (s, 3 H) 1.73 (s, 3 H) 0.85 - 1.92 (26 H) 2.00 - 2.11 (m, 1 H) 2.15 (dd, *J*=17.19, 6.40 Hz, 1 H) 2.40 (t, *J*=6.40 Hz, 2 H) 2.51 (td, *J*=10.60, 4.89 Hz, 1 H) 2.91,(d, *J*=12.80 Hz, 1 H) 3.25 (d, *J*=12.80 Hz, 1 H) 3.44 (t, *J*=5.77 Hz, 2 H) 3.60 - 3.74 (m, 2 H) 4.64 (s, 1 H) 4.76 (d, *J*=1.76 Hz, 1 H) 5.30 (dd, *J*=6.15, 1.63 Hz, 1 H) 7.21 (d, *J*=8.28 Hz, 2 H) 7.91 (d, *J*=8.53 Hz, 2 H).

### EXAMPLE 79. 4-((1R,3aS,5aR,5bR,7aR,11aS,11bR,13aR,13bR)-5a,5b,8,8,11a-pentamethyl-3a-((2-methyl-3-(2-oxopyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

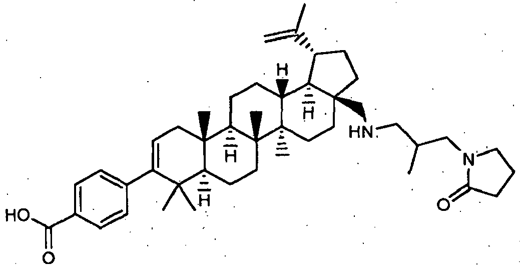

The title compound was prepared in 76 % yield following the general procedures described above for the C28 amine formation and hydrolysis using 1-(3-amine-2-methylpropyl)pyrrolidin-2-one as the reactant amine. MS: m/e 683.6 (MH+), 1.62 min (method 2). ¹H NMR (400 MHz, *MeOD)* δ ppm 0.95 (s, 3 H) 0.96 (s, 3 H) 1.04 (s, 3 H) 1.08 (s, 3 H) 1.10 (d, *J*=7.03 Hz, 3 H) 1.17 (s, 3 H) 1.73 (s, 3 H) 0.91 1.99 (m, 21 H) 2.01 - 2.19 (m, 4 H) 2.28 - 2.55 (m, 4 H) 2.76 - 2.91 (m, I H) 3.04 (dd, *J*=12.80, 5.27 Hz, 1 H) 3.14 (dt, *J*=15.00, 3.80 Hz, 1 H) 3.18 - 3.27 (m, 1 H) 3.50 - 3.58 (m, 1 H) 3.58 - 3.67 (m, 2 H) 4.64 (s, 1 H) 4.75 (s, 1 H) 5.30 (dd, *J*=6.15, 1.63 Hz, 1 H) 7.21 (d, *J*=8.28 Hz, 2 H) 7.91 (d, *J*=8.53 Hz, 2 H).

### EXAMPLE 80. 4-((1R,3aS,5aR,5bR,7aR,11aS,11bR,13aR,13bR)-3a-((3-(2,6-dioxopiperidin-1-yl)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

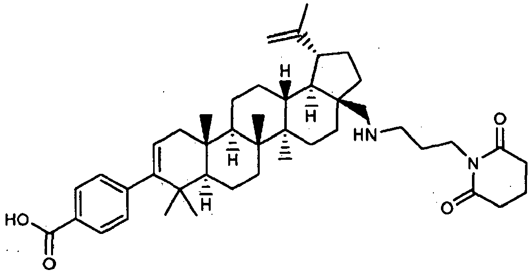

The title compound was prepared in 60 % yield following the general procedures described above for the C28 amine formation and hydrolysis using 1-(3-aminopropyl)piperidine-2,6-dione hydrochloride as the reactant amine. MS: m/e 697.5 (MH+), 1.63 min (method 2). ¹H NMR (400 MHz, *MeOD)* δ ppm 0.95 (s, 3 H) 0.97 (s, 3 H) 1.04 (s, 3 H) 1.08 (s, 3 H) 1.18 (s, 3 H) 1.73 (s, 3 H) 0.92 - 1.91 (m, 20 H) 1.92 - 2.03 (m, 4 H) 2.03 - 2.11 (m, 1 H) 2.15 (dd, *J*=17.07, 6.78 Hz, 1 H) 2.45 - 2.54 (m, 1 H) 2.70 (t, *J*=6.53 Hz, 4 H) 2.83 (d, *J*=13.30 Hz, 1 H) 3.05 (t, *J*=6.78 Hz, 2 H) 3.20 (d, *J*=13.30 Hz, 1 H) 3.89 (t, *J*=6.65 Hz, 2 H) 4.62 - 4.66 (m, 1 H) 4.75 (d, *J*=1.76 Hz, 1 H) 5.30 (dd, *J*=6.15, 1.38 Hz, 1 H) 7.22 (d, *J*=8.53 Hz, 2 H) 7.91 (d, *J*=8.53 Hz, 2 H).

### EXAMPLE 81. 4-((1R,3aS,5aR,5bR,7aR,11aS,11bR,13aR,13bR)-3a-((2-hydroxy-3-(2-oxopyrrolidin-1-yl)propylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

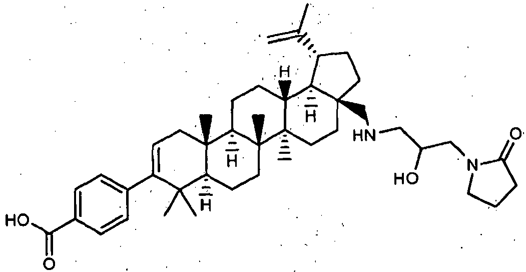

The title compound was prepared in 22 % yield following the general procedures described above for the C28 amine formation and hydrolysis using 1-(3-amino-2-hydroxypropyl)pyrrolidin-2-one as the reactant amine. MS: m/e 685.5 (MH+), 1.72 min (method 2). ¹H NMR (400 MHz, *MeOD)* δ ppm 0.95 (s, 3 H) 0.96 (s, 3 H) 1.04 (s, 3 H) 1.08 (s, 3 H) 1.18 (s, 3 H) 1.73 (s, 3 H) 0.82 - 1.91 (m, 20 H) 2.02 - 2.19 (m, 4 H) 2.38 - 2.46 (m, 2 H) 2.46 - 2.56 (m, 1 H) 2.89 (d, *J*=13.05 Hz, 1 H) 3.01 - 3.19 (m, 2H) 3.32 - 3.50 (m, 4H) 3.53 - 3.70(m, 2H)4.18 - 4.26(m, *J*=9.41, 5.02, 4.71, 4.71 Hz, 1 H) 4.64 (s, 1 H) 4.75 (d, *J*=1.76 Hz, 1 H) 5.29 (dd, *J*=6.02, 1.51 Hz, 1 H) 7.22 (d, *J*=8.53 Hz, 2 H) 7.91 (d, *J=*8.28 Hz, 2 H).

### EXAMPLE 82.4-((1R,3aS,5aR,5bR,7aR,11aS,11bR,13aR,13bR)-5a,5b,8,8,11a-pentamethyl-3a-((1-(piperidin-1-ylmethyl)cyclopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

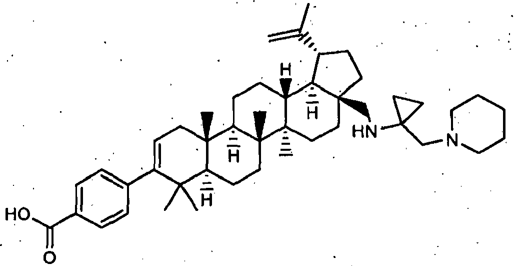

The title compound was prepared in 87 % yield following the general procedures described above for the C28 amine formation and hydrolysis using 1-(piperidin-1-ylmethyl)cyclopropanamine hydrochloride as the reactant amine. MS: m/e 681.7 (MH+), 1.76 min (method 2). ¹H NMR (400 MHz, *MeOD)* δ ppm 0.95 (s, 3 H) 0.97 (s, 3 H) 1.04 (s, 3 H) 1.07 (s, 3 H) 1.17 (s, 3 H) 1.72 (s, 3 H) 0.87 - 1.90 (m, 38 H) 1.93 - 2.06 (m, 1 H) 2.15 (dd, *J*=17.19, 6.40 Hz, 1 H) 2.56 (td, *J*=11.23, 5.90 Hz, 1 H) 4.63 (s, 1 H) 4.75 (s, 1 H) 5.30 (dd, *J*=6.02, 1.51 Hz, 1 H) 7.22 (d, *J*=8.28 Hz, 2 H) 7.92 (d, *J*=8.53 Hz, 2 H).

### EXAMPLE 83. Preparation of 4-((1R,3aS,5aR,5bR,7aR,11aS,11bR,13aR,13bR)-3a-((dimethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

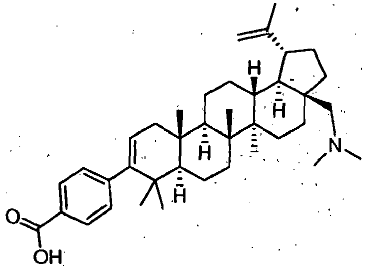

The title compound was prepared following the procedure described above in Step 3 using formaldehyde as the reactant aldehyde and 4-((1R,3aS,5aR,5bR,7aR,11aS,11bR,13aR,13bR)-3a-((3-aminopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (example 67) as the starting material amine. The product was isolated as a white solid (17 mg, 34.4 %). LCMS: m/e 572.5(MH⁺), 2.21 min (method 3). ¹H NMR (400 MHz, *MeOD)* δ ppm 6.37 (d, *J*=8.5 Hz, 2 H), 5.67 (d, *J*=8.3 Hz, 2 H), 3.75 (dd, *J=*6.1, 1.6 Hz, 1 H), 3.22 (d, *J*=1.5 Hz, 1 H); 3.11 (s, 1 H), 1.79 - 1.93 (m, 1 H), 1.54 (d, *J*=13.8 Hz, 1 H), 1.36 - 1.48 (m, 6 H), 0.97 (td, *J*=11.0, 5.4 Hz, 1 H), 0.60 (dd, *J*=17.2, 6.4 Hz, 1 H), 0.42 - 0.55 (m, 1 H), 0.31 - 0.38 (m, 1 H), 0.10 - 0.30 (m, 8 H), 0.11 - 0.10 (m, 8 H), 0.32 - 0.11 (m, 5 H); 0.44 - 0.32 (m, 4 H), 0.47 (s, 3 H), 0.52 (s, 3 H), 0.56 (s, 3 H), 0.60 (s, 3 H).

### EXAMPLE 84. Preparation of 4-((1R,3aS,5aR,5bR,7aR,11aS,11bR,13aR,13bR)-3a-((cyclopropyl(3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

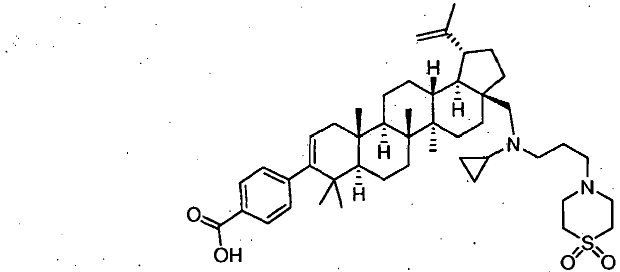

The title compound was prepared following the general procedures described above for the C28 amine formation, hydrolysis and tertiary amine formation using 4-(3-aminopropyl) thiomorpholine 1,1-dioxide as the reactant amine and (1-ethoxycyclopropoxy)trimethylsilane as reactant aldehyde equivalent. The product was isolated as a white solid (5 mg, 22.5 %). LCMS: m/e 759.6 (MH⁺), 2.56 min (method 3). ¹H NMR (400 MHz, *MeOD)* δ ppm 6.38 (d, *J*=8.3 Hz, 2 H), 5.68 (d, *J*=8.3 Hz,-2 H), 3.77 (d, *J*=4.5 Hz, 1 H), 3.25 (s, 1H), 3.13 (s, 1H), 1.71 - 1.76 (m, 2 H), 1.56 - 1.71 (m, 8 H), 1.47 (s, 2 H), 1.24 (br. s., 2 H), 1.01 (br. s., 1 H), 0.79 (br. s., 1 H), 0.61 (d, *J*=4.8 Hz, 2 H), 0.49 (m, 1H), 0.20 (s, 9 H), 0.06 (br. s., 7 H), 0.06 (br. s., 4 H), 0.29 - 0.13 (m, 4 H), 0.38 - 0.29 (m, 4 H), 0.46 - 0.38 (m, 5 H), 0.54 - 0.46 (m, 4 H); 0.57 (s, 3 H), 0.58 (s, 3 H).

### General procedures for preparation of additional C28 amines. Examples 85-86.

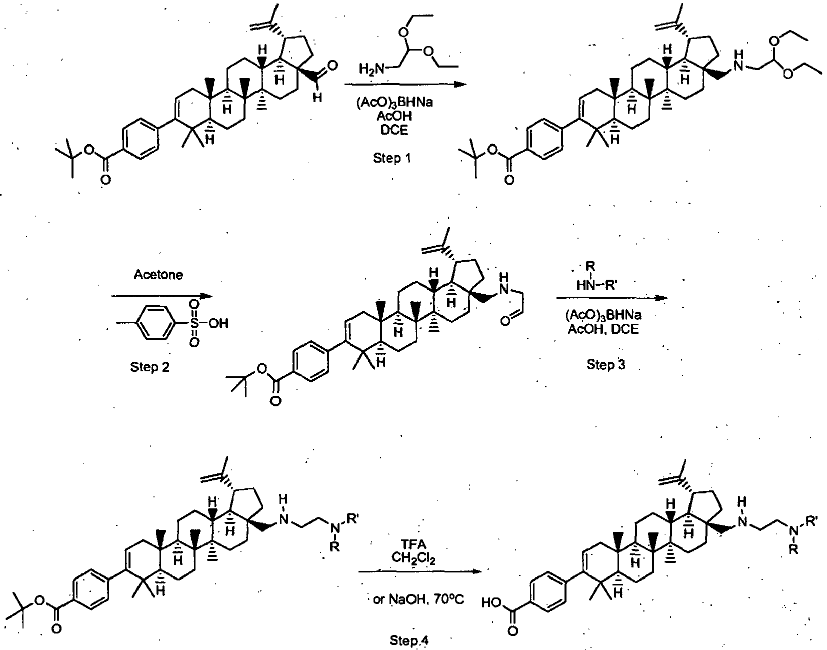

### Step 1. Reductive amination

A suspension oftert-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1 eq.), 2, 2-diethoxyethanamine (2 eq.) and acetic acid (2 - 5 eq.) in DCE (2 ml) was stirred at rt for 30 min. Sodium triacetoxyborohydride (5 eq.) was added. The resulting mixture was stirred at rt for 18 - 72 h. The reaction mixture was diluted with 5 ml of saturated sodium carbonate and extracted with DCM (3 x 10 ml). The combined organic layers were dried over sodium sulfate, filtered and concentrated *in vacuo.* The crude product was purified by Biotage flash chromatography to afford the desired product was isolated as a white solid (88 mg, 15 %). LCMS: m/e 716.6.4 (MH⁺), 2.96 min (method 3).

### Step 2. Conversion of the acetal to aldehyde

To a solution of *te*rt-butyl 4-((1R,3aS,5aR,5bR,7aR,11aS,11bR,13aR,13bR)-3a-((2,2-diethoxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate from Step 1 in acetone (10 ml) was added 2 eq. of 4-methylbenzenesulfonic acid. The mixture was stirred and refluxed for 14 h. The solvent was removed *in vacuo* and the resulting residue was re-dissolved in methylene chloride and washed with sodium bicarbonate solution. The organic layer was separated and dried over sodium sulfate. The yellow solid obtained was used in the next step without further purification. LCMS: m/e 674.6 (M+CH₃OH⁺), 2.78 min (method 3).

### Step 3. Reductive amination

A suspension of *tert*-butyl 4-((1R,3aS,5aR,5bR,7aR,11aS,11bR,13aR,13bR)-5a,5b,8,8,11a-pentamethyl-3a-((2-oxoethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1 eq.), the corresponding amine (2 eq.) and acetic acid (2 - 5 eq.) in DCE (2 ml) was stirred at rt for 30 min. Sodium triacetoxyborohydride (5 eq.) was added. The resulting mixture was stirred at rt for 18 - 72 h. The reaction mixture was diluted with 5 ml of saturated sodium carbonate and extracted with DCM (3 x 10 ml). The combined organic layers were dried over sodium sulfate, filtered and concentrated *in vacuo.* The crude product was purified by Biotage flash chromatography or was used directly in the next step without further purification.

### Step 4.

(a) Acidic hydrolysis - To a solution of the material from Step 3 in DCM (4 - 5 ml) was added TFA (0.4 - 0.5 ml). The mixture was stirred at rt for 2 - 6 h. The solvent was evaporated under vacuum. The resulting crude product was purified by prep. HPLC to give the desired benzoic acids.
(b) Basic hydrolysis - To a solution of the material from Step 3 in dioxane (2 ml) and methanol (2 ml) was added NaOH (75 mg, 1.875 mmol) and H₂O (0.5 ml). The resulting solution was stirred at 70 °C for 5 - 10 h. The solvent was evaporated under vacuum and the resulting crude product was purified by prep. HPLC to give the desired benzoic acids.

### EXAMPLE 85. Preparation of 4-((1R,3aS,5aR,5bR,7aR,11aS,11bR,13aR,13bR)-5a,5b,8,8,11a-pentamethyl-3a-((2-(methyl(2-(methylsulfonyl)ethyl)amino)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

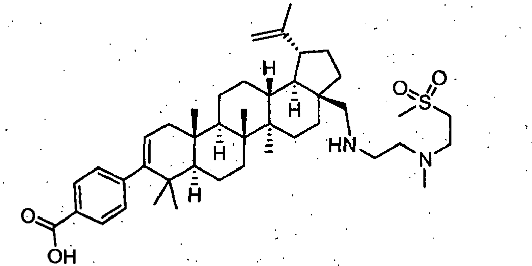

The title compound was prepared following the general procedures described above using *N*-methyl-2-(methylsulfonyl)ethanamine as the reactant amine. The product was isolated as a white solid (10 mg, 18.4 %). LCMS: m/e 707.7 (MH⁺), 2.55 min (method 3).

### EXAMPLE 86. Preparation of 4-((1R,3aS,5aR,5bR,7aR,11aS,11bR,13aR,13bR)-5a,5b,8,8,11a-pentamethyl-1-(prop-1-en-2-yl)-3a-((2-thiomorpholinoethylamino)methyl)-2,3,3a,4,5,5a,5b,6,7,7a,8,11,11a,11b,12,13,13a,13b-octadecahydro-1H-cyclopenta[a]chrysen-9-yl)benzoic acid.

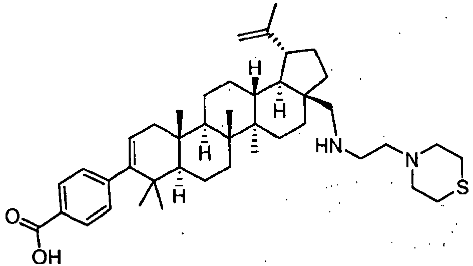

The title compound was prepared following the general procedures described above using thiomorpholine as the reactant amine. The product was isolated as a white solid (6 mg, 20.0 %). LCMS: m/e 673.6 (MH⁺), 2.56 min (method 3). ¹H NMR (400 MHz, *MeOD)* δ ppm 7.94 (m, *J*=8.5 Hz, 2 H), 7.24 (m, *J*=8.3 Hz, 2 H), 5.33 (d, *J*=4.3 Hz, 1 H), 4.79 (d, *J*=2.0 Hz, 1 H), 4.68 (s, 1 H), 3.42 - 3.55 (m, 2 H), 3.33 (m, 2H), 3.15 (ddd, *J*=3.4, 1.6, 1.5 Hz, 5 H), 2.84 - 3.01 (m, 5 H), 2.53 (br. s., 1 H), 2.18 (dd, *J*=17.2, 6.4 Hz, 2 H), 1.67 - 1.92 (m, 8 H), 1.47 - 1.67 (m, 8 H), 1.38 (br. s., 2 H), 1.31 (d, *J*=3.5 Hz, 3 H), 1.14 - 1.26 (m, 5 H), 1.11 (s, 3 H), 1.07 (s, 3 H), 0.99 (s, 3 H), 0.98 (s, 3 H).

### General procedure for the preparation of C28 amines with fluorinated benzoic acids. Examples 87-95.

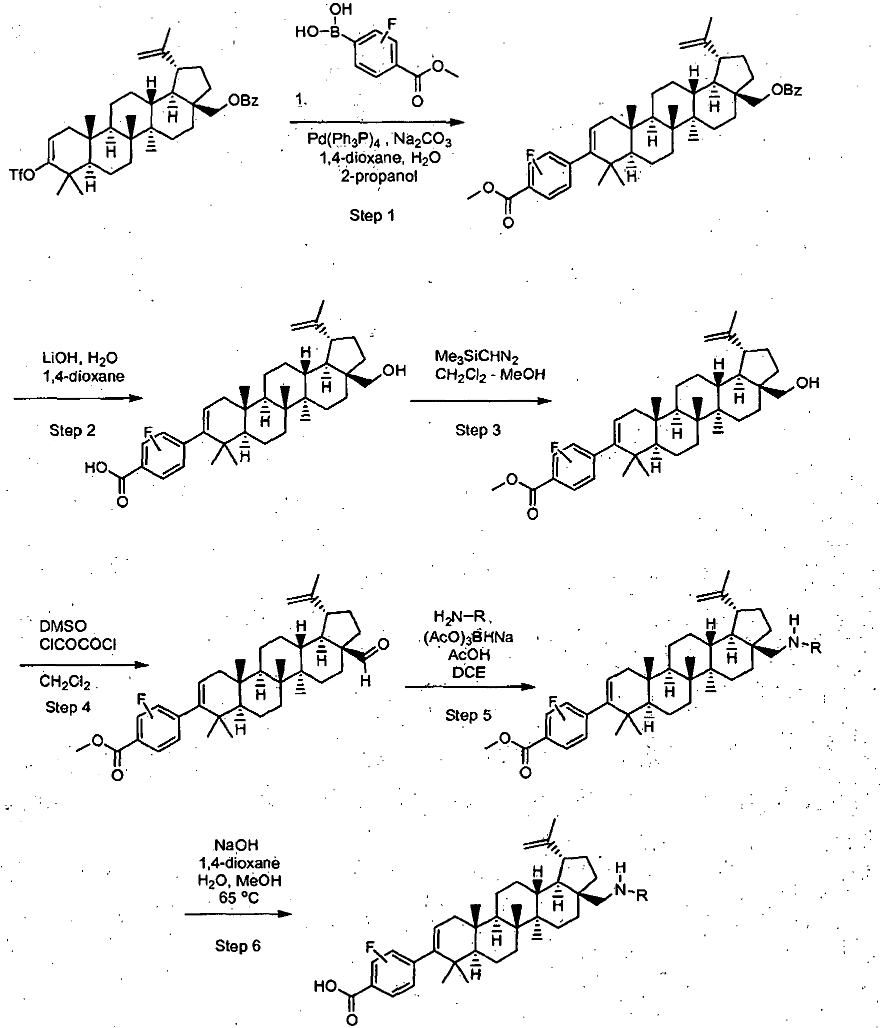

### Step 1: Suzuki coupling

To a solution of ((1R,3aS,5aR,5bR,7aR,11aR,11bR,13aR,13bR)-5a,5b,8,8,11a-pentamethyl-1-(prop-1-en-2-yl)-9-(trifluoromethylsulfonyloxy)-2,3,3a,4,5,5a,5b,6,7,7a,8,11,11a,11b,12,13,13a,13b-octadecahydro-1H-cyclopenta[a]chrysen-3a-yl)methyl benzoate (675 mg, 1 mmol) in 1,4-dioxane (5 ml) was added 2-propanol (5 ml), H₂O (2 ml), Na₂CO₃ (317 mg, 3 mmol), the corresponding boronic acid (296 mg, 1.5 mmol) and Pd(Ph₃P)₄ (34.6 mg, 0.030 mmol). The mixture was refluxed under nitrogen for 4 h. The reaction mixture was diluted with H₂O (10 ml) and extracted with EtOAc (3 x 10 ml). The combined organic layers were dried over Na₂SO₄, filtered and concentrated *in vacuo.* The crude product was purified by Biotage (Thomson 25 g silica gel column; 9:1 Hex/EtOAc). The fractions containing the expected product were combined and concentrated *in vacuo* to give the corresponding fluorobenzoic methyl esters.

### Preparation of methyl 4-((1R,3aS,5aR,5bR,7aR,11aS,11bR,13aR,13bR)-3a-(benzoyloxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)-3-fluorobenzoate

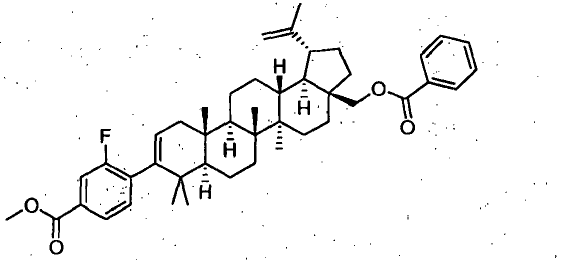

The title compound was prepared in 62 % yield following the procedure described for the Suzuki coupling, using 2-fluoro-4-(methoxycarbonyl)phenylboronic acid as the reactant. ¹H NMR (400 MHz, *CHLOROFORM-d)* 8 0.90 (3 H, s), 0.95 (3 H, s), 1.02 (3 H, s), 1.05 (3 H, s), 1.14 (3 H, s), 1.73 (3 H, s), 0.84 - 1.87 (18 H, m), 1.91 - 2.10 (3 H, m), 2.14 (1 H, dd, *J*=17.3, 6.5 Hz), 2.56 (1 H, td, *J*=11.0, 5.8 Hz), 3.93 (3 H, s), 4.14 (1 H, d, *J*=10.3 Hz), 4.56 (1 H, d, *J*=9.8 Hz), 4.61 - 4.65 (1 H, m), 4.75 (1 H, d, *J*=2.0 Hz), 5.36 (1 H, dd, *J*=6.3, 1.8 Hz), 7.17 (1 H, t, *J*=7.5 Hz), 7.43 - 7.49 (2 H, m), 7.55 - 7.60 (1 H, m), 7.69 (1 H, dd, *J*=9.7, 1.6 Hz), 7.74 (1 H, dd, *J*=7.9, 1.6 Hz), 8.07 (2 H, dd, *J*=8.4, 1.4 Hz).

### Preparation of methyl 4-((1R,3aS,5aR,5bR,7aR,11aS,11bR,13aR,13bR)-3a-(benzoyloxymethyl)-5a,5b,8,8,11a-pentamethyl-1-(prop-1-en-2,-yl)-2,3,3a,4,5,5a,5b,6,7,7a,8,11,11a,11b,12,13,13a,13b-octadecahydro-1H-cyclopenta[a]chrysen-9-yl)-2-fluorobenzoate

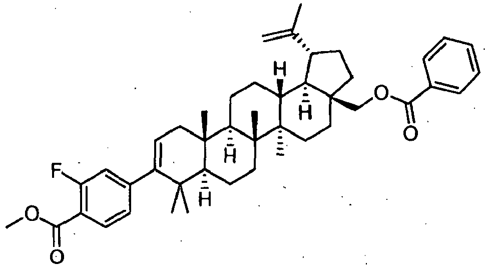

The title compound was prepared in 66 % yield following the procedure described for the Suzuki coupling, using 3-fluoro-4-(methoxycarbonyl)phenylboronic acid as the reactant. ¹H NMR (400 MHz, *CHLOROFORM-d)* δ 0.94 (3 H, s), 0.95 (3 H, s), 0.98 (3 H, s), 1.05 (3 H, s), 1.13 (3 H, s), 1.73 (3 H, s), 0.87 - 1.87 (m, 18 H), 1.91 - 2.08 (3 H, m), 2.12 (1 H, dd, *J*=17.2, 6.4 Hz), 2.56 (1 H, td, *J*=11.0, 5.8 Hz), 3.93 (3 H, s), 4.13 (1 H, d, *J*=10.0 Hz), 4.55 (1 H, d, *J*=10.0 Hz), 4.61 - 4.65 (1 H, m), 4.74 (1 H, d, *J*=2.0 Hz), 5.33 (1 H, dd, *J*=6.3, 1.8 Hz), 6.93 (1 H, dd, *J*=11.8, 1.5 Hz), 6.98 (1 H, dd, *J*=8.0, 1.5 Hz), 7.43 - 7.49 (2 H, m), 7.54 - 7.60 (1 H, m), 7.82 (1 H, t, *J*=7.9 Hz), 8.07 (2 H, dd, *J*=8.4, 1.4 Hz).

### Step 2: Deprotection of C28 alcohols

To a solution of the fluorobenzoic methyl esters from Step 1 in 1,4-dioxane (15 ml) and H₂O (2 ml) was added lithium hydroxide hydrate (3.0 eq). The resulting mixture stirred at 75 °C for 48 h. LCMS showed the reaction was incomplete. The mixture was partitioned between H₂O (50 ml) and DCM (50 ml) and neutralized with IN HCl. The organic layer was washed with H₂O (2 x 50 ml), dried over Na₂SO₄, filtered and concentrated *in vacuo.* The solid was re-dissolved into 1,4-dioxane (15 ml) and MeOH (15 ml). Lithium hydroxide hydrate (3 eq) and H₂O (2 ml) were added. The resulting mixture was stirred at 75 °C for 24 h. LC/MS showed the reaction was complete and that the methyl ester had also been cleaved. The mixture was partitioned between H₂O (50 ml) and DCM (50 ml) and neutralized with IN HCl. The organic layer was washed with H₂O (2 x 50ml), dried over Na₂SO₄, filtered and concentrated *in vacuo* to give the crude C28 alcohols as solids which were used in the next step without further purification.

### Preparation of methyl 3-fluoro-4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

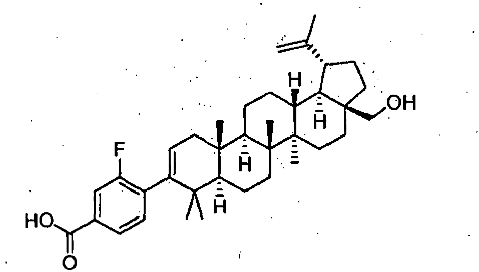

The title compound was prepared following the C28 alcohol deprotection procedure described above. ¹H NMR (400 MHz, *CHLOROFORM-d*) δ 0.91 (3 H, d, *J*=1.3 Hz), 0.96 (3 H, s), 1.02 (3 H, s), 1.03 (3 H, s), 1.11 (3 H, s), 1.71 (3 H, s), 0.82 - 1.79 (m, 18 H), 1.84 - 2.03 (3 H, m), 2.13 (1 H, dd, *J*=17.3, 6.5 Hz), 2.42 (1 H, td, *J*=10.9, 6.1 Hz), 3.37 (1 H, d, *J=*11.0 Hz), 3.84 (1 H, d, *J*=10.5 Hz), 4.60 (1 H, dd, *J*=2.1, 1.4 Hz); 4.71 (1 H, d, *J*=2.0 Hz), 5.37 (1 H, dd, *J=*6.3, 1.8 Hz), 7.21 (1 H, t, *J*=7.4 Hz), 7.73 (1 H, dd, *J*=9.5, 1.5 Hz), 7.79 (1 H, dd, *J*=7.9, 1.6 Hz); MS *m*/*z* 561.6 (M-H)⁻, 2.36 min (Method 6).

### Preparation of methyl 2-fluoro-4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

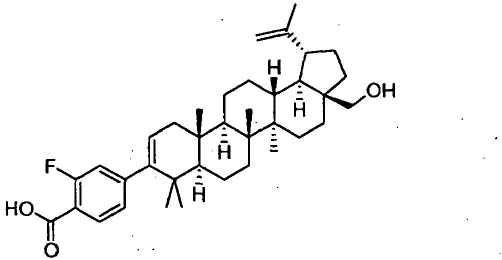

The title compound was prepared following the C28 alcohol deprotection procedure described above. ¹H NMR (400 MHz, *CHLOROFORM-d*) δ 0.95 (3 H, s), 0.96 (3 H, s), 0.98 (3 H, s), 1.03 (3 H, s), 1.10 (3 H, s), 1.71 (3 H, s), 0.83 - 1.79 (m, 18 H), 1.84 - 2.06 (3 H, m), 2.12 (1 H, dd, *J*=17.4, 6.4 Hz), 2.42 (1 H, td, *J*=10.7, 6.1 Hz), 3.37 (1 H, d, *J*=10.8 Hz), 3.84 (1 H, d, *J=*10.5 Hz), 4.60 (1 H, s), 4.71 (1 H, d, *J=*1.5 Hz), 5.34 (1 H, dd, *J*=6.1, 1.6 Hz), 6.97 (1 H, dd, *J*=11.9, 1.1 Hz), 7.02 (1 H, dd, *J*=8.2, 1.4 Hz), 7.91 (1 H, t, *J*=7.9 Hz); MS *mlz* 561.6. (M-H)⁻, 2.33 min (Method 6).

### Step 3: Preparation of methyl esters of the fluorobenzoic acids

To a suspension of the material from Step 2 in DCM (40 ml) and MeOH (12 ml) was added trimethylsilyldiazomethane (2 M in hexane) (4.8 eq.). The resulting mixture was stirred at rt under nitrogen for 4 days. The reaction mixture was concentrated *in vacuo* to give crude products as solid which were used in the next step without further purification.

### Preparation of methyl 3-fluoro-4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

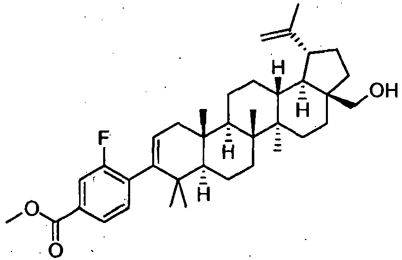

The title compound was prepared following the procedure described above for the methyl ester formation. ¹H NMR (400 MHz, *CHLOROFORM-d*) δ 0.89 (3 H, d, *J*=1.0 Hz), 0.95 (3 H, s), 1.01 (3 H, s), 1.02 (3 H, s), 1.10 (3 H, s), 1.70 (3 H, s), 0.81 - 1.78 (m, 18 H), 1.84 - 2.05 (3 H, m), 2.12 (1 H, dd, *J*=17.1, 6.3 Hz), 2.42 (1 H, td, *J*=11.0, 5.9 Hz), 3.36 (1 H, dd, *J*=10.4, 5.6 Hz), 3.84 (1 H, dd, *J=*9.8, 5.3 Hz), 3.93 (3 H, s), 4.60 (1 H, dd, *J*=2.3, 1.3 Hz), 4.70 (1 H, d, *J*=2.0 Hz), 5.36 (1 H, dd, *J*=6.3, 1.8 Hz), 7.17 (1 H, t, *J*=7.5 Hz), 7.68 (1 H, dd, *J*=9.5, 1.5 Hz), 7.74 (1 H, dd, *J*=7.9, 1.6 Hz).

### Preparation of methyl 2-fluoro-4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

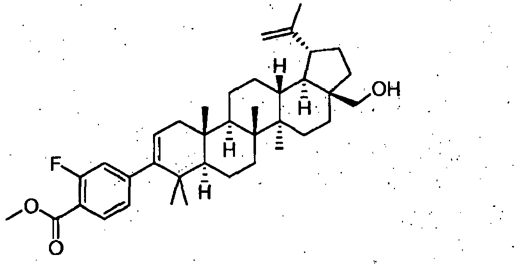

The title compound was prepared following the procedure described above for the methyl ester formation. ¹H NMR (400 MHz, *CHLOROFORM-d*) δ 0.94 (3 H, s), 0.95 (3 H, s), 0.97 (3 H, s), 1.02 (3 H, s), 1.09 (3 H, s), 1.70 (3 H, s), 0.86 - 1.78 (m, 18 H), 1.84 - 2.02 (3 H, m), 2.11 (1 H, dd, *J*=17.3, 6.3 Hz), 2.42 (1 H, td, *J*=10.5, 5.5 Hz), 3.36 (1 H, dd, *J*=10.3, 6.0 Hz), 3.83 (1 H, dd, *J*=10.4, 6.1 Hz), 3.93 (3 H, s), 4.60 (1 H, dd, *J*=2.3, 1.5 Hz), 4.70 (1 H, d, *J*=2.3 Hz), 5.32 (1 H, dd, *J*=6.1, 1.9 Hz), 6.93 (1 H, dd, *J=*11.9, 1.4 Hz), 6.98 (1 H, dd, *J=*8.0, 1.5 Hz), 7.82 (1 H, t, *J*=7.9 Hz).

### Step 4: Preparation of C28 aldehydes

To a solution of oxalyl chloride (1.2 eq) in DCM (5 ml) at -70 °C was added drop wise a solution of DMSO (1.5 eq) in DCM (5 ml) under nitrogen. The mixture was warmed to -50 °C. A solution of the crude product from Step 3 in DCM (2 ml) was added drop wise. After stirring for 15 min at -50 °C, Et₃N (3 eq) was added drop wise and the mixture was warmed to rt. The reaction mixture was diluted with DCM (50 ml) and washed with H₂O (2 x 50 ml) followed by brine (50 ml), dried over Na₂SO₄, filtered and concentrated *in vacuo.* The crude product was purified by Biotage (Thomson 25 g silica gel column; 9:1 Hex/EtOAc). The fractions containing the expected product were combined and concentrated *in vacuo* to give the corresponding aldehyde.

### Preparation of methyl 3-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

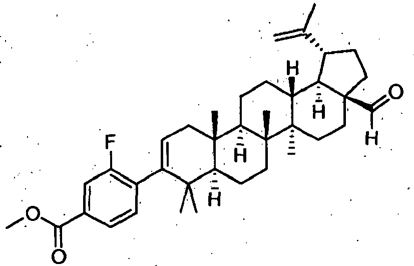

The title compound was prepared in 80 % yield in 3 steps (steps 2 - 4) following the procedure described above for the C28 aldehyde formation. ¹H NMR (400 MHz, *CHLOROFORM-d*) δ 0.89 (3 H, d, *J*=1.3 Hz), 0.94 (3 H, s), 0.99 (3 H, s), 1.00 (3 H, s), 1.02 (3 H, s), 1.72 (3 H, s), 1.96 - 0.81 (m, 19 H), 2.04 - 2.17 (3 H, m), 2.90 (1 H, td, *J=*11.1, 5.9 Hz), 3.92 (3 H, s), 4.65 (1 H, dd, *J*=2.1, 1.4 Hz), 4.78 (1 H, d, *J*=2.0 Hz), 5.36 (1 H, dd, *J*=6.3, 1.8 Hz), 7.16 (1 H, t, *J*=7.5 Hz), 7.68 (1 H, dd, *J*=9.5, 1.5 Hz), 7.74 (1 H, dd, *J*=7.9, 1.6 Hz), 9.70 (1 H, d, *J*=1.5 Hz). ¹⁹F NMR (376 MHz, *MeOD*) δ ppm -115.50 (1 F, s)); MS *m*/*z* 575.5 (M+H)⁺, 2.93 min (Method 2).

### Preparation of methyl 2-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

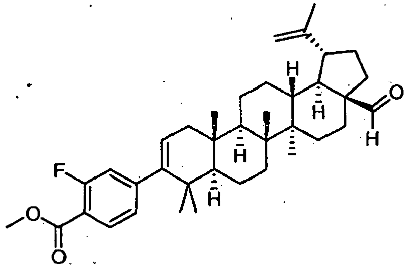

The title compound was prepared in 84 % yield in 3 steps (steps 2 - 4) following the procedure described above for the C28 aldehyde formation. ¹H NMR (400 MHz, *CHLOROFORM-d*) δ ppm 0.93 (3 H, s), 0.94 (3 H, s), 0.96 (3 H, s), 0.98 (3 H, s), 1.01 (3 H, s), 1.72 (3 H, s), 1.96 - 0.80 (m, 19 H), 2.04 - 2.16 (3 H, m), 2.90 (1 H, td, *J=*11.1, 5.9 Hz), 3.93 (3 H, s), 4.65 (1 H, dd, *J*=2.0, 1.5 Hz), 4.78 (1 H, d, *J*=2.0 Hz), 5.32 (1 H, dd, *J*=6.3, 2.0 Hz), 6.93 (1 H, dd, *J*=11.9, 1.4 Hz), 6.97 (1 H, dd, *J*=8.0, 1.5 Hz), 7.82 (1 H, t, *J*=7.8 Hz), 9.70 (1 H, d, *J=*1.5 Hz). ¹⁹F NMR (376 MHz, *MeOD)* δ ppm -114.87 (1 F, s): MS *m*/*z* 575.5 (M+H)⁺, 2.78 min (Method 2).

### Step 5: Preparation of C28 amines

A suspension of the corresponding aldehyde, an amine (2 eq.) and acetic acid (2 - 5 eq.) in DCE (2 - 5 ml) was stirred at rt for 0.5 - 1 h. Sodium triacetoxyborohydride (5 eq.) was added. The resulting mixture was stirred at rt for 18 - 72 h. The reaction mixture was diluted with 5 ml of saturated sodium carbonate and extracted with DCM (3 x 10 ml). The combined organic layers were dried over sodium sulfate, filtered and concentrated *in vacuo.* The crude product was purified by Biotage flash chromatography or was used directly in the next step without further purification.

### Step 6: Preparation of benzoic acids

To a solution of C28 amine from Step 5 in 1,4-dioxane (1 ml) and methanol (0.5 ml) was added IN sodium hydroxide (0.5 - 1 ml). The mixture was stirred at 65 °C for 2 - 5 h. The crude reaction mixture was purified by prep. HPLC to afford the desired benzoic acids.

### EXAMPLE 87. 3-fluoro-4-((1R,3aS,5aR,5bR,7aR,11aS,11bR,13aR,13bR)-5a,5b,8,8,11a-pentamethyl-3a-((3-(2-oxopyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

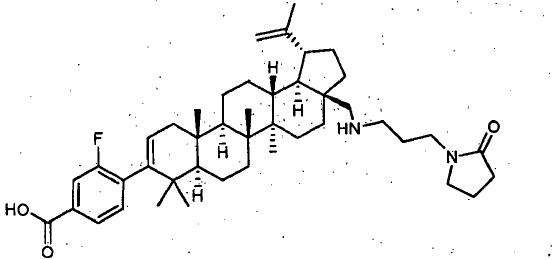

The title compound was prepared in 79 % yield following steps 5 and 6 described above , using methyl 3-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 1-(3-aminopropyl)pyrrolidin-2-one as the reactant amine. MS: m/e 687.5 (MH⁺), 1.71 min (method 2). ¹H NMR (400 MHz, *MeOD*) δ ppm 0.91 (s, 3 H) 0.96 (s, 3 H) 1.05 (s, 3 H) 1.08 (s, 3 H) 1.17 (s, 3 H) 1.73 (s, 3 H) 0.85 - 1.91 (m, 20 H) 1.96 - 2.20 (m, 6 H) 2.44 (t, *J*=8.03 Hz, 2 H) 2.47 - 2.56 (m, 1 H) 2.83 (d, *J*=13.05 Hz, 1 H) 3.07 (t, *J*=7.03 Hz, 2 H) 3.21 (d, *J*=13.05 Hz, 1 H) 3.43 (t, *J*=6.40 Hz, 2 H) 3.52 (t, *J*=7.15 Hz, 2 H) 4.62 - 4.66 (m, 1 H) 4.75 (d, *J*=1.51 Hz, 1 H) 5.35 (dd, *J*=6.02, 1.51 Hz, 1 H) 7.22 (t, *J*=7.65 Hz, 1 H) 7.64 (dd, *J*=9.79, 1.51 Hz, 1 H) 7.75 (dd, *J=*7.91, 1.63 Hz, 1 H). ¹⁹F NMR (376 MHz, *MeOD*) δ ppm-114.92 (s, 1 F).

### EXAMPLE 88. 2-fluoro-4-((1R,3aS,5aR,5bR,7aR,11aS,11bR,13aR,13bR)-5a,5b,8,8,11a-pentamethyl-3a-((3-(2-oxopyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1 H-cyclopenta[a]chrysen-9-yl)benzoic acid.

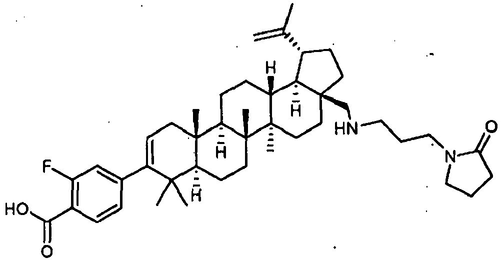

The title compound was prepared in 82 % yield following steps 5 and 6 described above, using methyl 2-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 1-(3-aminopropyl)pyrrolidin-2-one as the reactant amine. MS: m/e 687.5 (MH⁺), 1.71 min (method 2). ¹H NMR (400 MHz, *MeOD*) δ ppm 0.96 (s, 3 H) 0.98 (s, 3 H) 1.02 (s, 3 H) 1.08 (s, 3 H) 1.17 (s, 3 H) 1.73 (s, 3 H) 0.90 - 1.91 (m, 20 H) 1.96 - 2.20 (m, 6 H) 2.44 (t, *J*=8.03 Hz, 2 H) 2.47 - 2.55 (m, 1 H) 2.83 (d, *J*=13.05 Hz, 1 H) 3.06 (t, *J*=7.03 Hz, 2 H) 3.20 (d, *J*=13.55 Hz, 1 H) 3.43 (t, *J*=6.53 Hz, 2 H) 3.51 (t, *J*=7.15 Hz, 2 H) 4.62 - 4.66 (m, 1 H) 4.75 (d, *J*=1.76 Hz, 1 H) 5.35 (dd, *J*=6.15, 1.88 Hz, 1 H) 6.94 (dd, *J*=11.80, 1.25 Hz, 1 H) 7.02 (dd, *J*=8.03, 1.51 Hz, 1 H) 7.83 (t, *J*=7.91 Hz, 1 H). ¹⁹F NMR (376 MHz, *MeOD*) δ ppm-112.76 (s, 1 F).

### EXAMPLE 89. 3-fluoro-4-((1R,3aS,5aR,5bR,7aR,11aS,11bR,13aR,13bR)-5a,5b,8,8,11a-pentamethyl-3a-((3-oxo-3-(pyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

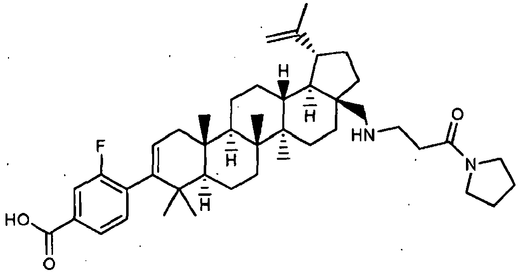

The title compound was prepared in 72 % yield following steps 5 and 6, using methyl 3-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 3-amino-1-(pyrrolidin-1-yl)propan-1-one hydrochloride as the reactant amine. MS: m/e 687.5 (MH⁺), 1.76 min (method 2). ¹H NMR (400 MHz, *MeOD*) δ ppm 0.91 (s, 3 H) 0.96 (s, 3 H) 1.05 (s, 3 H) 1.09 (s, 3 H) 1.17 (s, 3 H) 1.73 (s, 3 H) 0.85 - 1.85 (m, 20 H) 1.87 - 1.96 (m, 2 H) 2.01 (dt, *J*=13.36, 6.74 Hz, 2 H) 2.04 - 2.12 (m, 1 H) 2.16 (dd, *J=*17.07, 6.53 Hz, 1 H) 2.48 - 2.57 (m, 1 H) 2.81 (t, *J*=6.02 Hz, 2 H) 2.88 (d, *J*=12.80 Hz, 1 H) 3.33 - 3.43 (m, 3 H) 3.43 - 3.52 (m, 4 H) 4.65 (s, 1 H) 4.76 (d, *J*=1.25 Hz, 1 H) 5.35 (dd, *J*=6.02, 1.51 Hz, 1 H) 7.22 (t, *J*=7.5 Hz, 1 H) 7.64 (dd, *J*=9.79, 1.25 Hz, 1 H) 7.75 (dd, *J*=8.03, 1.51 Hz, 1 H). ¹⁹F NMR (376 MHz, *MeOD*) δ ppm -113.36 (s, 1 F).

### EXAMPLE 90. 2-fluoro-4-((1R,3aS,5aR,5bR,7aR,11aS,11bR,13aR,13bR)-5a,5b,8,8,11a-pentamethyl-3a-((3-oxo-3-(pyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

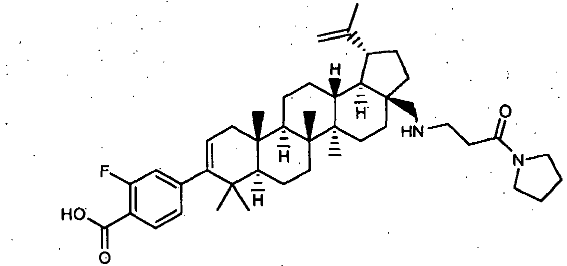

The title compound was prepared in 67 % yield following steps 5 and 6, using methyl 2-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 3-amino-1-(pyrrolidin-1-yl)propan-1-one hydrochloride as the reactant amine. MS: m/e 687.5 (MH⁺), 1.74 min (method 2). ¹H NMR (400 MHz, *MeOD*) δ ppm 0.96 (s, 3 H) 0.98 (s, 3 H) 1.02 (s, 3 H) 1.08 (s, 3 H) 1.16 (s, 3 H) 1.73 (s, 3 H) 0.90 - 1.86 (m, 20 H) 1.88 - 1.96 (m, *J*=6.78, 6.78, 6.65, 6.40 Hz, 2 H) 1.97 - 2.10 (m, 1 H) 2.01 (dt, *J*=13.74, 6.81 Hz, 2 H) 2.16 (dd, *J*=17.19, 6.40 Hz, 1 H) 2.47 - 2.57 (m, 1 H) 2.81 (t, *J*=6.02 Hz, 2 H) 2.88 (d, *J*=12.55 Hz, 1 H) 3.33 - 3.42 (m, 3 H) 3.42 - 3.52 (m, 4 H) 4.65 (s, 1 H) 4.76 (d, *J*=1.76 Hz, 1 H) 5.35 (dd, *J*=5.77, 1.00 Hz, 1 H) 6.94 (d, *J*=11.80 Hz, 1 H) 7.02 (dd, *J*=8.03, 1.25 Hz, 1 H) 7.83 (t, *J*=7.91 Hz, 1 H). ¹⁹F NMR (376 MHz, *MeOD*) δ ppm-112.76 (s, 1 F).

### EXAMPLE 91. 2-fluoro-4-((1R,3aS,5aR,5bR,7aR,11aS,11bR,13aR,13bR)-5a,5b,8,8,11a-pentamethyl-3a-((2-(2-oxopyrrolidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

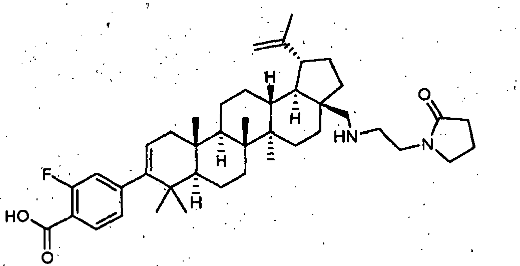

The title compound was prepared in 60 % yield following steps 5 and 6, using methyl 2-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 1-(2-aminoethyl)pyrrolidin-2-one oxalate as the reactant amine. MS: m/e 673.6 (MH⁺), 1.72 min (method 2). ¹H NMR (400 MHz, *MeOD*) δ ppm 0.96 (s, 3 H) 0.98 (s, 3 H) 1.02 (s, 3 H) 1.08 (s, 3 H) 1.17 (s, 3 H) 1.73 (s, 3 H) 2.01 - 2.21 (m, 4 H) 2.44 (t, *J*=8.16 Hz, 2 H) 2.51 (td, *J*=10.79, 5.52 Hz, 1 H) 2.94 (d, *J*=13.05 Hz, 1 H) 3.30 (dt, *J*=3.26, 1.63 Hz, 3 H) 3.55 (t, *J*=7.15 Hz, 2 H) 3.58 - 3.70 (m, 2 H) 4.62 - 4.67 (m, 1 H) 4.76 (d, *J*=1.76 Hz, 1 H) 5.35 (dd, *J*=6.27, 1.76 Hz, 1 H) 6.94 (dd, *J*=11.92, 1.38 Hz, 1 H) 7.02 (dd, *J*=8.03, 1.51 Hz, 1 H) 7.83 (t, *J*=7.91 Hz, 1 H). ¹⁹F NMR (376 MHz, *MeOD*) δ ppm -112.75 (s, 1 F).

### EXAMPLE 92. Preparation of 4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)-3-fluorobenzoic acid.

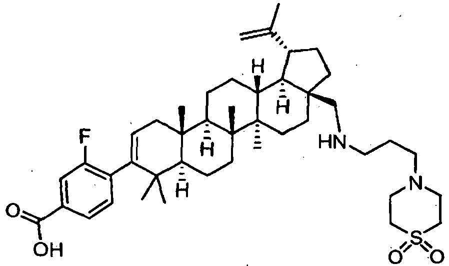

The title compound was prepared following steps 5 and 6 using methyl 3-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 4-(3-aminopropyl) thiomorpholine 1,1-dioxide as the reactant amine. The product was isolated as a white solid (70 mg, 68.0 %). LCMS: m/e 737.5 (MH⁺), 2.59 min (method 3). ¹H NMR (400 MHz, *MeOD*) δ ppm 7.77 (d, *J*=7.8 Hz, 1 H), 7.67 (d, *J*=9.8 Hz, 1 H), 7.19 - 7.31 (m, 1 H), 5.38 (d, *J*=4.8 Hz, 1 H), 4.78 (br. s., 1 H), 4.67 (br. s., 1 H), 3.41 (br. s., 4 H), 3.30 - 3.38 (m, 4 H), 3.27 (d, *J=*12.5 Hz, 1 H), 3.21 (br. s., 2 H), 3.00 (t, *J*=6.4 Hz, 2 H), 2.90 (br. s., 1 H), 2.53 (br. s., 1 H), 2.10 (br. s., 4 H), 1.84 (br. s., 3 H), 1.75 (br. s., 7 H), 1.54 (br. s., 8 H), 1.36 (br. s., 3 H), 1.20 (br. s., 5 H), 1.11 (br. s., 3 H), 1.07 (br. s., 3 H), 0.98 (br. s., 3 H), 0.94 (br. s., 3 H). ¹⁹F NMR (376 MHz, *MeOD*) δ ppm - 113.31 (s, 1 F).

### EXAMPLE 93. Preparation of 4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)-2-fluorobenzoic acid.

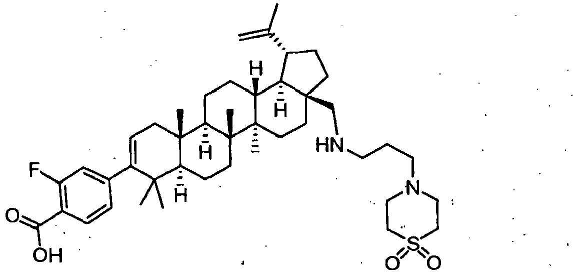

The title compound was prepared following steps 5 and 6 using methyl 2-fluoro-4-((1R,3aS,5aR,5bR,7aR,11aS,11bR,13aR,13bR)-3a-forrnyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 4-(3-aminopropyl) thiomorpholine 1,1-dioxide as the reactant amine. The product was isolated as a white solid (50 mg, 55.0 %). LCMS: m/e 737.5 (MH⁺), 2.53 min (method 3). ¹H NMR (400 MHz, *MeOD*) δ ppm 7.85 (d, *J*=3.8 Hz, 1 H), 7.01 - 7.14 (m, 1 H), 6.98 (br. s., 1 H), 5.37 (br. s., 1 H), 4.78 (br. s., 1 H), 4.67 (br. s., 1 H), 3.56 (br. s., 4 H), 3.43 (br. s., 4 H), 3.21 (m, 3H), 3.12 (br. s., 2 H), 2.90 (br. s., 1 H), 2.53 (br. s., 1 H), 2.16 (br. s., 4 H), 1.75 (br. s., 10 H), 1.53 (br. s., 8 H), 1.31 (br. s., 3 H), 1.19 (br. s., 5 H), 1.10 (br. s., 3 H), 1.05 (br. s., 3 H), 0.99 (d, *J*=8.5 Hz, 6 H). ¹⁹F NMR (376 MHz, *MeOD*) δ ppm -112.6 (s, 1 F).

### EXAMPLE 94. Preparation of 4-((1R,3aS,5aR,5bR,7aR,11aS,11bR,13aR,13bR)-3a-(((2-(1,1-dioxido-4-thiomorpholinyl)ethyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)-3-fluorobenzoic acid.

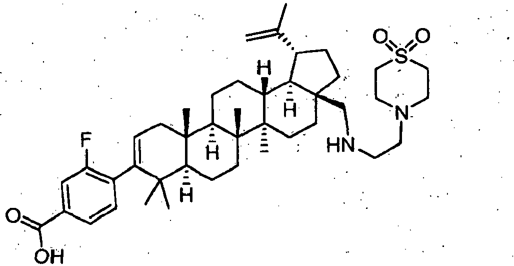

The title compound was prepared following steps 5 and 6 using methyl 3-fluoro-4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 4-(3-aminoethyl) thiomorpholine 1,1-dioxide as the reactant amine. The product was isolated as a white solid (70 mg, 66.4 %). LCMS: m/e 723.5 (MH⁺), 2.57 min (method 3). ¹H NMR (400 MHz, *MeOD*) δ ppm 7.77 (dd, *J*=7.9, 1.6 Hz, 1 H), 7.67 (dd, *J*=9.8, 1.5 Hz, 1 H), 7.24 (t, *J*=7.5 Hz, 1 H), 5.36 (d, *J*=4.8 Hz, 1 H), 4.78 (d, *J*=1.5 Hz, 1 H), 4.66 (s, 1 H), 3.23 - 3.45 (m, 3 H), 3.16 (d, *J*=2.5 Hz, 8 H), 2.94 - 3.05 (m, 2 H), 2.91 (d, *J=*13.1 Hz, 1 H), 2.55 (td, *J*=10.5, 5.8 Hz, 1 H), 2.17 (dd, *J*=17.1, 6.5 Hz, 1 H), 1.96 - 2.13 (m, 1 H), 1.68 - 1.93 (m, 10 H), 1.43 - 1.66 (m, 8 H), 1.28 - 1.43 (m, 3 H), 1.13 - 1.25 (m, 5 H), 1.09 (s, 3 H), 1.06 (s, 3 H), 0.98 (s, 3 H), 0.93 (s, 3 H). ¹⁹F NMR (376 MHz, *MeOD*) 8 ppm -113.10 (s, 1 F).

### EXAMPLE 95. Preparation of 4-((1R,3aS,5aR,5bR,7aR,11aS,11bR,13aR,13bR)-3a-(((2-(1,1-dioxido-4-thiomorpholinyl)ethyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)-2-fluorobenzoic acid.

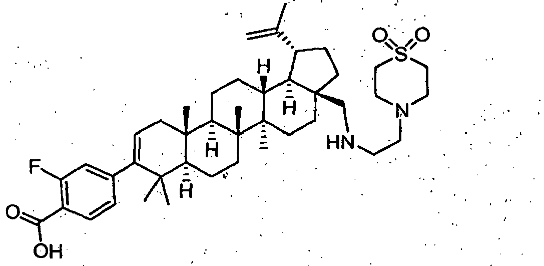

The title compound was prepared following steps 5 and 6 using methyl 2-fluoro-4-((1R,3aS,5aR,5bR,7aR,11aS,1bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate and 4-(3-aminoethyl) thiomorpholine 1,1-dioxide as the reactant amine. The product was isolated as a white solid (53 mg, 70.7 %). LCMS: m/e 723.5 (MH⁺), 2.56 min (method 3). ¹H NMR (400 MHz, *MeOD*) δ ppm 7.74 - 7.96 (m, 1 H), 6.99 - 7.16 (m, 1 H), 6.78 - 6.99 (m, 1 H), 5.25 - 5.40 (m, 1 H), 4.77 (d, *J*=1.5 Hz, 1 H), 4.58 - 4.70 (m, 1 H), 3.24 - 3.40 (m, 3 H), 3.06 - 3.24 (m, 8 H), 2.93 - 3.05 (m, 2 H), 2.81 - 2.93 (m, 1 H), 2.53 (td, *J=*10.5, 5.5 Hz, 1 H), 2.17 (dd, *J*=17.2, 6.4 Hz, 1 H), 1.94 - 2.12 (m, 1 H), 1.67 - 1.92 (m, 10 H), 1.42 - 1.66 (m, 8 H), 1.21 - 1.42 (m, 5 H), 1.18 (s, 3 H), 1.09 (s, 3 H), 1.04 (s, 3 H), 0.92 - 1.02 (m, 6 H). ¹⁹F NMR (376 MHz, *MeOD*) δ ppm -112.49 (s, 1 F), -112.51 (s, 1 F), -112.54 (s, 1 F).

### General procedure for preparation of C28 amines with amide end cap (examples 96-130):

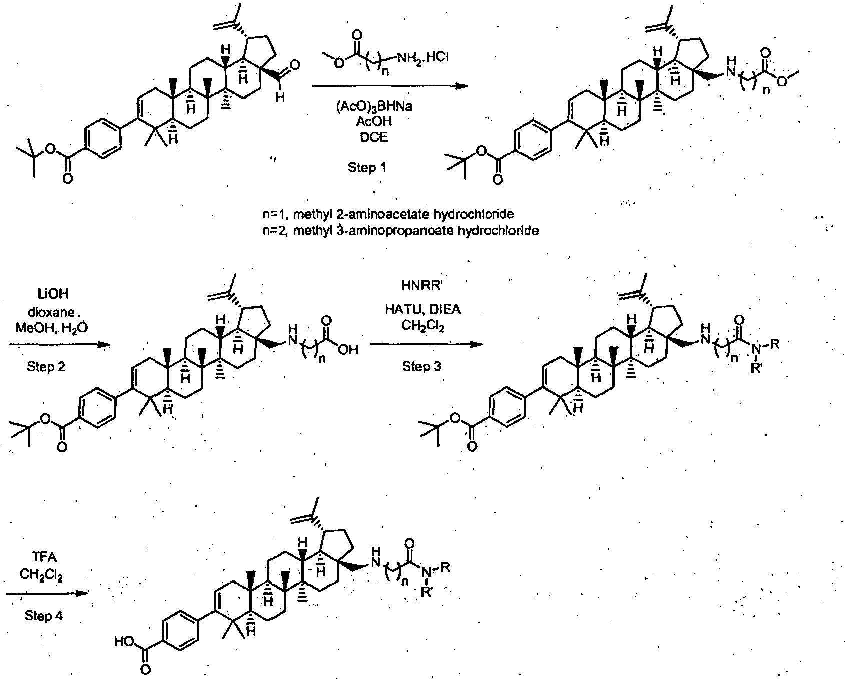

### Step 1: Preparation of methyl aminoacetate or methyl 3-aminopropanoate

To a solution of the aldehyde (200 mg, 0.334 mmol) in DCE was added potassium carbonate (185 mg, 1.336 mmol). After stirring at rt for 30 min, acetic acid (80 mg, 1.336 mmol) and methyl 2-aminoacetate hydrochloride or methyl 3-aminopropanoate hydrochloride (4 eq. 1.336 mmol) were added. The mixture was stirred at rt for 10 min, then sodium triacetoxyhydroborate (566 mg, 2.67 mmol) was added. The mixture was stirred rt for 48 h. The mixture was diluted with 7 ml of sat. NaHCO₃ and was extracted with DCM (3 x 7 ml). The combined organic layers were dried with sodium sulfate. The drying agent was removed by filtration and the filtrate was concentrated under reduced pressure to give the crude product which was used in the next step with no additional purification.

### Step 2: Preparation of aminoacetic acid or 3-aminopropanoic acid

To a solution of the material from Step 1 in dioxane (1 ml) and methanol (5 ml) was added lithium hydroxide (5 eq.) followed by H₂O (0.5 ml). The clear solution was stirred at rt for 12 h. The solvent was evaporated and the resulting yellow solid was re-dissolved in dichloromethane and the pH was adjusted to ∼4 by adding HCl. The mixture was extracted with DCM (3 x 10 ml). The combined organic layers were dried with sodium sulfate. The drying agent was removed by filtration and the filtrate was concentrated under reduced pressure to give the crude product which was used in the next step with no additional purification.

### Step 3: Preparation of aminoacetic amides or 3-aminopropanoic amides

To the solution of material from Step 2 (1 eq.) in DMC at 0 °C was added the corresponding amine (1.5 eq.), HATU (2 eq.) followed by DIPEA (3 eq.). The resulting suspension was stirred at rt for 18 h. LC/MS was consistent with the expected product. The solvent was removed *in vacuo* to give the crude product which was used in the next step with no additional purification.

### Step 4: Preparation of benzoic acids

To the solution of the material from Step 3 in DCM (4 - 5 ml) was added TFA (0.4 - 0.5 ml,). The mixture was stirred at rt for 2 - 16 h. The solvent was removed under reduced pressure. The resulting crude product was purified by prep. HPLC to afford the desired benzoic acids.

### EXAMPLE 96. 4-((1R,3aS,5aR,5bR,7aR,11aS,11bR,13aR,13bR)-5a,5b,8,8,11a-pentamethyl-3a-((3-oxo-3-(piperidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

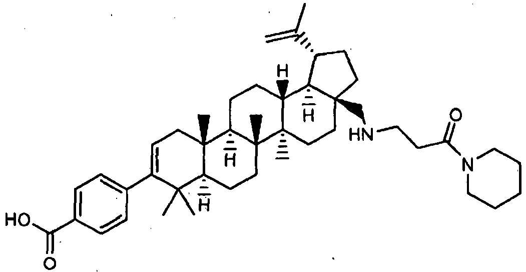

The title compound was prepared in 53 % yield following the general procedure described above for the preparation of C28 amines with amide end cap using piperidine as the reactant amine. MS: m/e 683.5 (MH⁺), 1.63 min (method 2). ¹H NMR (400 MHz, *MeOD*) δ ppm 0.95 (s, 3 H) 0.97 (s, 3 H) 1.04 (s, 3 H) 1.08 (s, 3 H) 1.17 (s, 3 H) 1.73 (s, 3 H) 0.87 - 1.85 (m, 26 H) 1.99 - 2.10 (m, 1 H) 2.15 (dd, *J=*16.94*,* 6.40 Hz, 1 H) 2.52 (td, *J=*10.48, 4.89 Hz, 1 H) 2.83 - 2.90 (m, 3 H) 3.26 (d, *J*=13.80 Hz, 1 H) 3.32 - 3.38 (m, 2 H) 3.44 - 3.50 (m, 2 H) 3.54 - 3.60 (m, 2 H) 4.65 (s, 1 H) 4.76 (d, *J*=1.76 Hz, 1 H) 5.30 (dd, *J*=5.90, 1.13 Hz, 1 H) 7.21 (d, *J*=8.28 Hz, 2 H) 7.91 (d, *J*=8.28 Hz, 2 H).

### EXAMPLE 97. 4-((1R,3aS,5aR,5bR,7aR,11aS,11bR,13aR,13bR)-3a-((3-(4-(N,N-dimethylsulfamoyl)piperazin-1-yl)-3-oxopropylamino)methyl)-5a,5b,8,8,11 a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

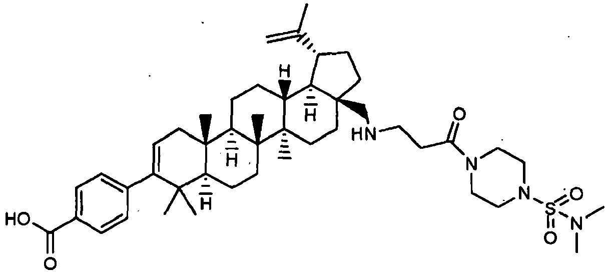

The title compound was prepared in 81 % yield following the general procedure described above for the preparation of C28 amines with amide end cap using *N*,*N-*dimethylpiperazine-1-sulfonamide as the reactant amine. MS: m/e 791.5 (MH⁺), 1.74 min (method 2). ¹H NMR (400 MHz, *MeOD*) δ ppm 0.95 (s, 3 H) 0.96 (s, 3 H) 1.04 (s, 3 H) 1.08 (s, 3 H) 1.17 (s, 3 H) 1.73 (s, 3 H) 0.89 - 1.86 (m, 20 H) 1.99 - 2.10 (m, 1 H) 2.15 (dd, *J*=17.32, 6.53 Hz, 1 H) 2.52 (td, *J*=10.60, 6.15 Hz, 1 H) 2.84 (s, 6 H) 2.85 - 2.93 (m,3H) 3.19 - 3.28 (m,4 H) 3.33 - 3.44 (m, 3 H) 3.56 - 3.62 (m, 2 H) 3.63 - 3.76 (m, 2 H) 4.65 (s, 1 H) 4.76 (d, *J=*1.51 Hz, 1 H) 5.30 (dd, *J*=6.15, 1.63 Hz, 1 H) 7.22 (d, *J*=8.53 Hz, 2 H) 7.92 (d, *J*=8.53 Hz, 2 H).

### EXAMPLE 98. 4-((1R,3aS,5aR,5bR,7aR,11aS,11bR,13aR,13bR)-3a-((3-(4-(ethoxycarbonyl)piperidin-1-yl)-3-oxopropylamino)methyl)-5a,5b,8,8,11 a-pentamethyl-1-(prop-1-en-2-yl)-2,3,3a,4,5,5a,5b,6,7,7a,8,11,11a,1b,12,13,13a,13b-octadecahydro-1H-cyclopenta[a]chrysen-9-yl)benzoic acid.

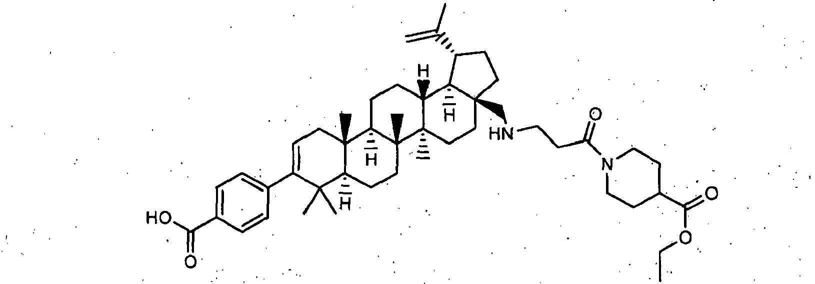

The title compound was prepared in 35 % yield following the general procedure described above for the preparation of C28 amines with amide end cap using ethyl piperidine-4-carboxylate as the reactant amine. MS: m/e 755.5 (MH⁺), 1.62 min (method 2). ¹H NMR (400 MHz, *MeOD*) δ ppm 0.95 (s, 3 H) 0.97 (s, 3 H) 1.04 (s, 3 H) 1.08 (s, 3 H) 1.17 (d, *J*=1.25 Hz, 3 H) 1.24 (t, *J*=7.03 Hz, 3 H) 1.73 (s, 3 H) 0.87-1.85 (m, 22 H) 1.91 - 2.10 (m, 3 H) 2.15 (dd, *J*=16.94, 6.40 Hz, 1 H) 2.46 - 2.56 (m, 1 H) 2.60 - 2.72 (m, 1 H) 2.81 - 2.95 (m, 4 H) 3.16 - 3.43 (m, 4 H) 3.82 - 3.90 (m, 1 H) 4.14 (q, *J*=7.11 Hz, 2 H) 4.35 - 4.43 (m, 1 H) 4.65 (s, 1 H) 4.76 (d, *J*=1.51 Hz, 1 H) 5.30 (dd, *J*=6.02, 1.51 Hz, 1 H) 7.21 (d, *J*=8.28 Hz, 2 H) 7.91 (d, *J*=8.53 Hz, 2 H).

### EXAMPLE 99. 4-((1R,3aS,5aR,5bR,7aR,11aS,11bR,13aR,13bR)-3a-((3-(3-(dimethylamino)-3-oxopropylamino)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

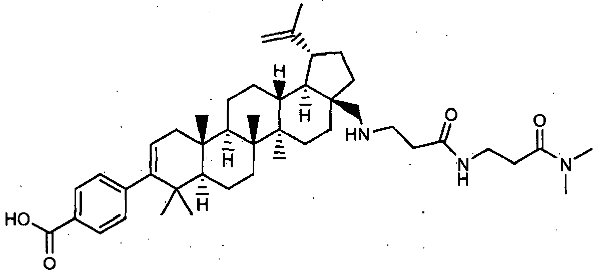

The title compound was prepared in 71 of yield following the general procedure described above for the preparation of C28 amines with amide end cap using 3-amino-*N*,*N-*dimethylpropanamide hydrochloride as the reactant amine. MS: m/e 714.6 (MH⁺), 1.67 min (method 2). ¹H NMR (400 MHz, *MeOD*) δ ppm 0.95 (s, 3 H) 0.96 (s, 3 H) 1.04 (s, 3 H) 1.08 (s, 3 H) 1.17 (s, 3 H) 1.73 (s, 3 H) 0.89 - 1.87 (m, 20 H). 1.98 - 2.10 (m, 1 H) 2.15 (dd, *J*=17.19, 6.40 Hz, 1 H) 2.52 (td, *J*=10.98, 5.65 Hz, 1 H) 2.61 (t, *J*=6.53 Hz, 2 H) 2.67 (t, *J*=6.27 Hz, 2 H) 2.87 (d, *J*=13.05 Hz, 1 H) 2.93 (s, 3 H) 3.04 (s, 3 H) 3.26 - 3.37 (m, 3 H) 3.47 (t, *J*=6.53 Hz, 2 H) 4.64 (s, 1 H) 4.76 (d, *J*=1.76 Hz, 1 H) 5.30 (dd, *J=*6.15, 1.63 Hz, 1 H) 7.22 (d, *J*=8.28 Hz, 2 H) 7.92 (d, *J*=8.53 Hz, 2 H).

### EXAMPLE 100. (R)-1-(3-(((1R,3aS,5aR,5bR,7aR,11aS,11bR,13aR,13bR)-9-(4-carboxyphenyl)-5a,5b,8,8, 11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)propanoyl)pyrrolidine-2-carboxylic acid:

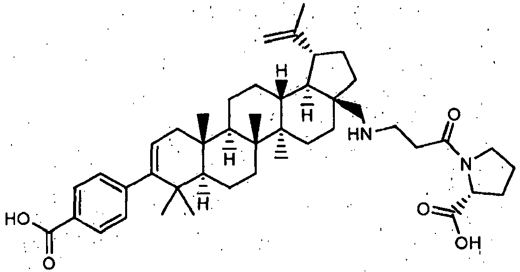

The title compound was prepared in 45 % yield following the general procedure described above for the preparation of C28 amines with amide end cap using (R)-*tert-*butyl pyrrolidine-2-carboxylate as the reactant amine. MS: m/e 713.5 (MH⁺), 1.59 min (method 2). ¹H NMR (400 MHz, *MeOD*) δ ppm 0.95 (s, 3 H) 0.97 (s, 3 H) 1.04 (s, 3 H) 1.08 (s, 3 H) 1.17 (s, 3 H) 1.73 (s, 3 H) 0.87 - 1.86 (m, 20 H) 1.99 - 2.11 (m, 4 H) 2.15 (dd, *J*=17.07, 6.02 Hz, 1 H) 2.24 - 2.32 (m, 1 H) 2.47 - 2.56 (m, 1 H) 2.84-2.92 (m, 3 H) 3.23 - 3.28 (m, 1 H) 3.38 (t, *J*=5.90 Hz, 2 H) 3.51 - 3.69 (m, 2 H) 4.48 (dd, *J*=8.41, 3.14 Hz, 1 H) 4.64 (s, 1 H) 4.76 (d, *J*=1.25 Hz, 1 H) 5.30 (dd, *J*=6.02, 1.76 Hz, 1 H) 7.21 (d, *J*=8.28 Hz, 2 H) 7.91 (d, *J*=8.28 Hz, 2 H).

### EXAMPLE 101. 4-((1R,3aS,5aR,5bR,7aR,11aS,11bR,13aR,13bR)-3a-((3-((S)-2-(methoxycarbonyl)pyrrolidin-1-yl)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

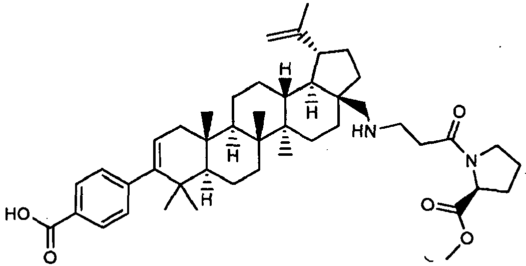

The title compound was prepared in 43 % yield following the general procedure described above for the preparation of C28 amines with amide end cap using (S)-methyl pyrrolidine-2-carboxylate hydrochloride as the reactant amine. MS: m/e 727.5 (MH⁺), 1.60 min (method 2). ¹H NMR (400 MHz, *MeOD*) δ ppm 0.95 (s, 3 H) 0.96 (s, 3 H) 1.04 (s, 3 H) 1.08 (s, 3 H) 1.18 (s, 3 H) 1.73 (s, 3 H) 0.87 - 1.84 (m, 22 H) 1.98 - 2.10 (m, 4 H) 2.15 (dd, *J*=17.07, 6.27 Hz, 1 H) 2.22 - 2.31 (m, 1 H) 2.84 - 2.91 (m, 3 H) 3.38 (ddd, *J*=6.78, 5.14, 4.89 Hz, 2 H) 3.53 - 3.69 (m, 2 H) 3.73 (s, 3 H) 4.49 (dd, *J*=8.66, 3.39 Hz, 1 H) 4.64 (s, 1 H) 4.76 (d, *J*=1.76 Hz, 1 H) 5.30 (dd, *J*=6.15, 1.63 Hz, 1 H) 7.21 (d, *J*=8.53 Hz, 2 H) 7.91 (d, *J*=8.28 Hz, 2 H).

### EXAMPLE 102. (S)-1-(3-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)propanoyl)pyrrolidine-2-carboxylic acid.

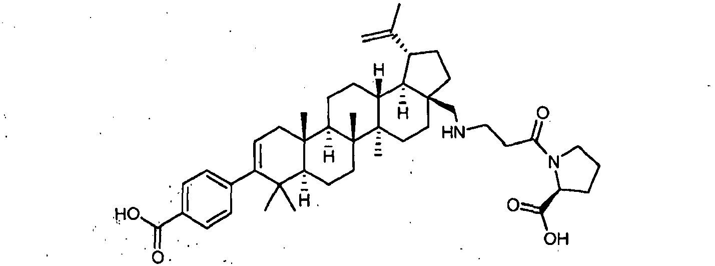

To a solution of 4-((1R,3aS,5aR,5bR,7aR,11aS,11bR,13aR,13bR)-3a-((3-((S)-2-(methoxycarbonyl)pyrrolidin-1-yl)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (10 mg, 0.014 mmol) in dioxane (1 ml) and methanol (2 ml) was added 1N sodium hydroxide (0.08 ml, 0.08 mmol) and H₂O (0.5 ml). The resulting mixture was stirred at rt for 45 h. The reaction mixture was neutralized with IN HCl and concentrated *in vacuo.* The residue was purified by prep. HPLC to afford the title compound (8.5 mg, 85%). MS: m/e 713.5 (MH⁺), 1.68 min (method 2). ¹H NMR (400 MHz, *MeOD*) δ ppm 0.95 (s, 3 H) 0.97 (s, 3 H) 1.04 (s, 3 H) 1.08 (s, 3 H) 1.18 (s, 3 H) 1.73 (s, 3 H) 0.87 - 1.86 (m, 20 H) 1.99 - 2.10 (m, 4 H) 2.15 (dd, *J*=17.32, 6.53 Hz, 1 H) 2.24 - 2.33 (m, 1 H) 2.52 (td, *J*=9.60, 4.14 Hz, 1 H) 2.83 - 2.92 (m, 3 H) 3.24 - 3.28 (m, 1 H) 3.38 (t, *J*=6.15 Hz, 2 H) 3.52 - 3.68 (m, 2 H) 4.48 (dd, *J*=8.41, 3.39 Hz, 1 H) 4.64 (s, 1 H) 4.76 (d, *J*=1.76 Hz, 1 H) 5.30 (dd, J=6.15, 1.63 Hz, 1 H) 7.21 (d, *J*=8.53 Hz, 2 H) 7.91 (d, *J*=8.28 Hz, 2 H).

### EXAMPLE 103, 4-((1R,3aS,5aR,5bR,7aR,11aS,11bR,13aR,13bR)-3a-((3-(3,3-difluoropyrrolidin-1-yl)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

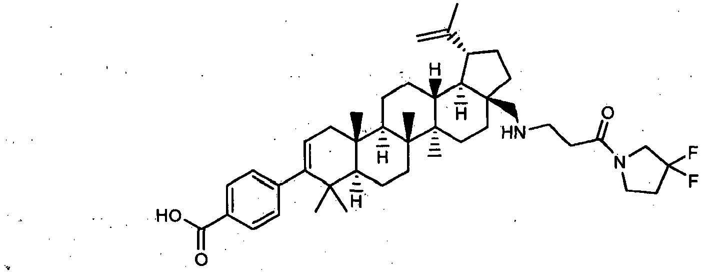

The title compound was prepared in 76 % yield following the general procedure described above for the preparation of C28 amines with amide end cap using 3,3-difluoropyrrolidine hydrochloride as the reactant amine. MS: m/e 705.5 (MH⁺), 1.69 min (method 2). ¹H NMR (400 MHz, *MeOD*) δ ppm 0.95 (s, 3 H) 0.96 (s, 3 H) 1.04 (s, 3 H) 1.08 (s, 3 H) 1.17 (s, 3 H) 1.73 (s, 3 H) 0.86 - 1.86 (m, 22 H) 1.99 - 2.10 (m, 1 H) 2.15 (dd, *J*=17.32, 6.53 Hz, 1 H) 2.37 - 2.58 (m, 3 H) 2.79 (t, *J*=6.02 Hz, 1 H) 2.84 (t, *J*=6.02 Hz, 1 H) 2.89 (d, *J*=13.05 Hz, 1 H) 3.36 - 3.41 (m, 1 H) 3.70 (t, *J*=7.65 Hz, 1 H) 3.77 (d, *J*=7.78 Hz, 1 H) 3.80 (t, 1 H) 3.92 (t, *J*=12.55 Hz, 1 H) 4.65 (s, 1 H) 4.76 (d, *J*=1.76 Hz, 1 H) 5.30 (dd, J=6.15, 1.63 Hz, 1 H) 7.22 (d, *J*=8.53 Hz, 2 H) 7.92 (d, *J*=8.53 Hz, 2 H). 19F NMR (376 MHz, *MeOD*) δ ppm -104.01 (d, *J*=74.56 Hz, 1 F) -103.19 (d, *J*=26.01 Hz, 1 F).

### EXAMPLE 104. 1-(3-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)propanoyl)piperidine-4-carboxylic acid.

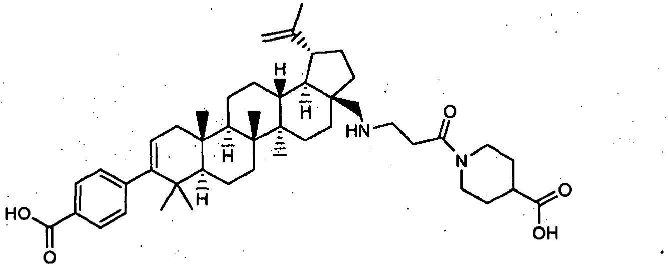

The title compound was prepared in 51% yield following the general procedure described above for the preparation of C28 amines with amide end cap using ethyl piperidine-4-carboxylate as the reactant amine, followed by basic hydrolysis of the ester group as described below:

To a solution of 4-((1R,3aS,5aR,5bR,7aR,11aS,11bR,13aR,13bR)-3a-((3-(4-(ethoxycarbonyl)piperidin-1-yl)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (16 mg, 0.021 mmol) in dioxane (1 ml) and methanol (2 ml) was added IN sodium hydroxide (0.3 ml, 0.1 1 mmol) and H₂O (0.5 ml). The resulting mixture was stirred at rt for 10 days. The reaction mixture was neutralized with IN HCl and concentrated *in vacuo.* The crude product was purified by prep. HPLC to afford the title compound (8 mg). MS: m/e 727.6 (MH⁺), 1.69 min (method 2). ¹H NMR (400 MHz, *MeOD*) δ ppm 0.95 (s, 3 H) 0.97 (s, 3 H) 1.04 (s, 3 H) 1.08 (s, 3 H) 1.17 (d, *J*=2.76 Hz, 3 H) 1.73 (s, 3 H) 0.87 - 1. 86 (m, 23 H) 1.92 - 2.10 (m, 3 H) 2.15 (dd, *J*=17.32, 6.53 Hz, 1 H) 2.47 - 2.56 (m, 1 H) 2.57 - 2.67 (m, *J*=7.34, 7.12, 7.12, 3.76 Hz, 1 H) 2.83 - 2.94 (m, 4 H) 3.16 - 3.41 (m, 4 H) 3.82 - 3.91 (m, 1 H) 4.35 - 4.44 (m, 1 H) 4.64 (s, 1 H) 4.76 (d, *J*=1.51 Hz, 1 H) 5.30 (d, *J*=5.27 Hz, 1 H) 7.22 (d, *J*=8.28 Hz, 2 H) 7.91 (d, *J*=8.28 Hz, 2 H).

### EXAMPLE 105. 4-((1R,3aS,5aR,5bR,7aR,11aS,11bR,13aR,13bR)-5a,5b,8,8,11a-pentamethyl-3a-((3-(4-methylpiperazin-1-yl)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

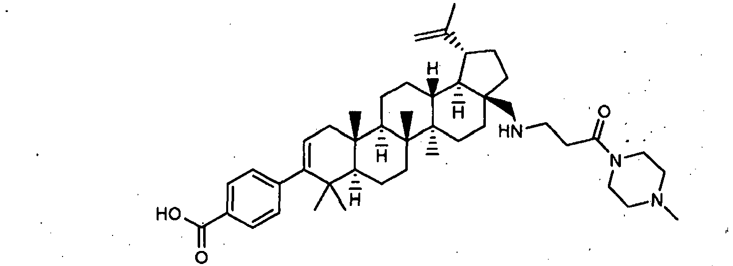

The title compound was prepared in 54 % yield following the general procedure described above for the preparation of C28 amines with amide end cap using 1-methylpiperazine as the reactant amine. MS: m/e 698.5 (MH⁺), 1.64 min (method 2). ¹H NMR (400 MHz, *MeOD*) δ ppm 0.95 (s, 3 H) 0.96 (s, 3 H) 1.04 (s, 3 H) 1.08 (s, 3 H) 1.17 (s, 3 H) 1.73 (s, 3 H) 0.84 - 1.88 (m, 22 H) 1.99 - 2.22 (m, 2 H) 2.46 - 2.58 (m, 1 H) 2.85 - 3.02 (m, 6 H) 3.15 - 3.51 (m, 11 H) 4.65 (s, 1 H) 4.76 (d, *J*=1.00 Hz, 1 H) 5.30 (d, *J*=3.76 Hz, 1 H) 7.21 (d, *J*=8.03 Hz, 2 H) 7.92 (d, *J*=8.28 Hz, 2 H).

### EXAMPLE 106.4-((1R,3aS,5aR,5bR,7aR,11aS,11bR,13aR,13bR)-5a,5b,8,8,11a-pentamethyl-3a-((3-oxo-3-(3,3,4,4-tetrafluoropyrrolidin-1-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

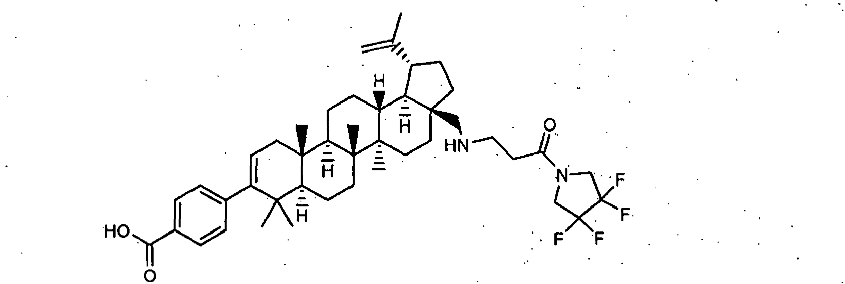

The title compound was prepared in 62 % yield following the general procedure described above for the preparation of C28 amines with amide end cap using 3,3,4,4-tetrafluoropyrrolidine hydrochloride as the reactant amine. MS: m/e 741.5 (MH⁺), 1.75 min (method 2). ¹H NMR (400 MHz, *MeOD*) 8 ppm 0.95 (s, 3 H) 0.97 (s, 3 H) 1.04 (s, 3 H) 1.08 (s, 3 H) 1.17 (s, 3 H) 1.73 (s, 3 H) 0.85 - 1.85 (m, 20 H) 1.99 - 2.10 (m, 1 H) 2.15 (dd, *J*=17.07, 6.53 Hz, 1 H) 2.52 (td, *J=*10.35, 5.40 Hz, 1 H) 2.83 (t, *J*=6.02 Hz, 2 H) 2.90 (d, *J*=12.80 Hz, 1 H) 3.25 - 3.27 (m, 1 H) 3.40 (td, *J*=5.96, 2.13 Hz, 2 H) 4.05 (t, *J*=13.93 Hz, 2 H) 4.21 (t, *J*=13.43 Hz, 2 H) 4.65 (s, 1 H) 4.76 (d, *J*=1.76 Hz, 1 H) 5.30 (dd, *J*=6.15, 1.63 Hz, 1 H) 7.22 (d, *J=*8.53 Hz, 2 H) 7.91 (d, *J*=8.28 Hz, 2 H). 19F NMR (376 MHz, *MeOD*) δ ppm -125.84 (dd, *J=*8.67, 3.47 Hz, 2 F) -125.07 (d, *J*=6.94 Hz, 2 F).

### EXAMPLE 107. 4-((1R,3aS,5aR,5bR,7aR,11aS,11bR,13aR,13bR)-5a,5b,8,8,11a-pentamethyl-3a-((3-morpholino-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

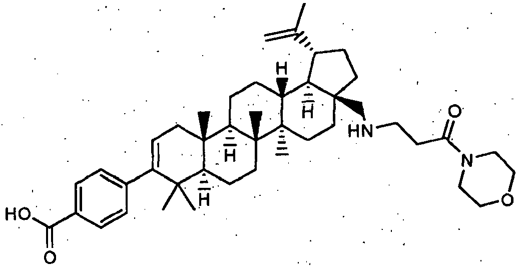

The title compound was prepared in 54 % yield following the general procedure described above for the preparation of C28 amines with amide end cap using morpholine as the reactant amine. MS: m/e 685.6 (MH⁺), 1.74 min (method 2). ¹H NMR (400 MHz, *MeOD*) δ ppm 0.95 (s, 3 H) 0.97 (s, 3 H) 1.04 (s, 3 H) 1.08 (s, 3 H) 1.18 (s, 3 H) 1.73 (s, 3 H) 0.87 - 1.86 (m, 20 H) 1.99 - 2.10 (m, 1 H) 2.15 (dd, *J*=17.57, 6.53 Hz, 1 H) 2.48 - 2.56 (m, 1 H) 2.84 - 2.91 (m, 3 H) 3.23 - 3.26 (m, 1 H) 3.34 - 3.41 (m, 2 H) 3.49 - 3.54 (m, 2 H) 3.58 - 3.63 (m, 2 H) 3.68 (ddd, *J*=10.10, 4.96, 4.77 Hz, 4 H) 4.65 (s, 1 H) 4.76 (d, *J*=1.51 Hz, 1 H) 5.30 (dd, *J*=6.27, 1.76 Hz, 1 H) 7.22 (d, *J=*8.28 Hz, 2 H) 7.92 (d, *J*=8.28 Hz, 2 H).

### EXAMPLE 108. Preparation of 4-((1R,3aS,5aR,5bR,7aR,11aS,11bR,13aR,13bR)-3a-(((2-(1,11-dioxido-4-thiomorpholinyl)-2-oxoethyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

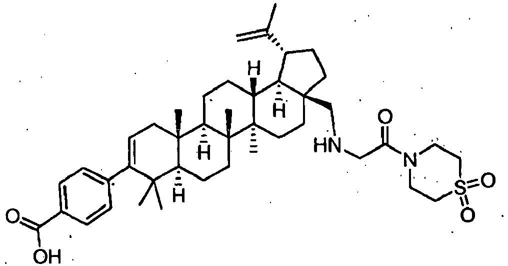

The title compound was prepared following the general procedures described above the general procedure described above for the preparation of C28 amines with amide end cap using methyl 2-aminoacetate hydrochloride and thiomorpholine 1,1-dioxide as the reactant amines. The product was isolated as a white solid (6.5 mg, 33.3 %). LCMS: m/e 719.2 (MH⁺), 2.32 min (method 3). ¹H NMR (400 MHz, *MeOD*) δ ppm 7.94 (m, *J*=8.3 Hz, 2 H), 7.24 (m, *J*=8.5 Hz, 2 H), 5.32 (dd, *J*=6.1, 1.6 Hz, 1 H), 4.76 (d, *J=*1.5 Hz, 1 H), 4.66 (s, 1 H), 4.22 - 4.35 (m, 2 H), 4.18 (br. s., 2 H), 3.92 (br. s., 2 H), 3.35 - 3.41 (m, 1 H), 3.25 - 3.30 (m, 2 H), 3.20 (d, *J*=9.0 Hz, 2 H), 2.86 - 2.94 (m, 1 H), 2.48 (d, *J*=5.5 Hz, 1 H), 2.09 - 2.20 (m, 2 H), 1.90 - 2.04 (m, 3 H), 1.69 - 1.90 (m, 8 H), 1.58 - 1.66 (m, 3 H), 1.47 - 1.58 (m, 5 H), 1.25 - 1.41 (m, 3 H), 1.13 - 1.25 (m, 4 H), 1.11 (s, 3 H), 1.05 (s, 3 H), 0.92 - 1.02 (m, 6 H).

### EXAMPLE 109. Preparation of 4-((1R,3aS,5aR,5bR,7aR,11aS,11bR,13aR,13bR)-5a,5b,8,8,11a-pentamethyl-3a-((2-oxo-2-(pyrrolidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

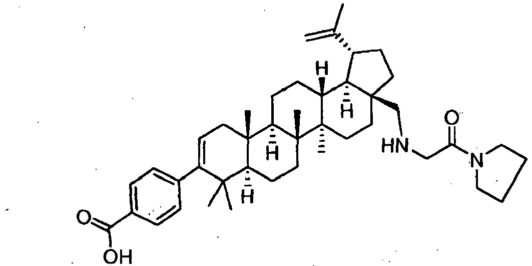

The title compound was prepared following the general procedure described above for the preparation of C28 amines with amide end cap using methyl 2-aminoacetate hydrochloride and pyrrolidine as the reactant amines. The product was isolated as a white solid (12.9 mg, 53.2 %). LCMS: m/e 655.3 (MH⁺), 2.13 min (method 3). ¹H NMR (400 MHz, *MeOD*) δ ppm 7.94 (m, *J*=8.5 Hz, 2 H), 7.24 (m, *J*=8.5 Hz, 2 H), 5.32 (dd, *J*=6.1, 1.6 Hz, 1 H), 4.76 (d, *J*=1.8 Hz, 1 H), 4.66 (s, 1 H), 3.99 - 4.13 (m, 2 H), 3.52 (dt, *J*=13.6, 6.8 Hz, 4 H), 3.38 (m, 1 H), 2.81 - 2.99 (m, 1 H), 2.47 (dt, *J*=11.0, 5.5 Hz, 1 H), 2.12 - 2.27 (m, 2 H), 2.02 - 2.12 (m, 2 H), 1.84 - 2.02 (m, *J*=6.7, 6.7, 6.5, 6.3 Hz, 5 H), 1.77 - 1.84 (m, 1 H), 1.67 - 1.77 (m, 6 H), 1.44 - 1.67 (m, 8 H), 1.26 - 1.39 (m, 3 H), 1.19 - 1.26 (m, 1 H), 1.14 - 1.19 (m, 4 H), 1.08 - 1.14 (m, 3 H), 1.04 (s, 3 H), 0.99 (s, 3 H), 0.95 (s, 3 H).

### EXAMPLE 110. Preparation of 4-((1R,3aS,5aR,5bR,7aR,11aS,11bR,13aR,13bR)-5a,5b,8,8,11a-pentamethyl-3a-((2-Oxo-2-(piperidin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

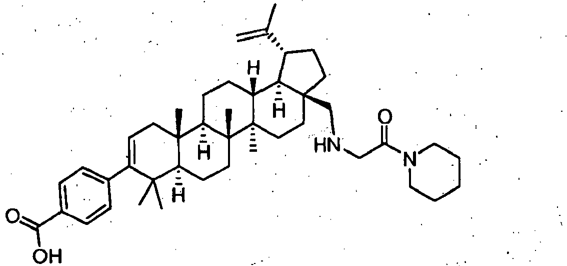

The title compound was prepared following the general procedure described above for the preparation of C28 amines with amide end cap using methyl 2-aminoacetate hydrochloride and piperidine as the reactant amines. The product was isolated as a white solid (10.0 mg, 51.5 %). LCMS: m/e 669.4 (MH⁺), 2.16 min (method 3). ¹H NMR (400 MHz, *MeOD*) δ ppm 7.94 (m, *J*=8.5 Hz, 2 H), 7.24 (m, *J*=8.5 Hz, 2 H), 5.32 (dd, *J*=6.1, 1.6 Hz, 1 H), 4.76 (d, *J*=1.8 Hz, 1 H), 4.66 (s, 1 H), 4.13 (s, 2 H), 3.56 - 3.72 (m, 2 H), 3.35 - 3.46 (m, 2 H), 3.3 (m, 1 H), 2.86 (d, *J*=12.5 Hz, 1 H), 2.48 (dt, *J=*11.1, 5.6 Hz, 1 H), 2.03 - 2.22 (m, 2 H), 1.84 - 2.03 (m, 3 H), 1.66 - 1.78 (m, 10 H), 1.48 - 1.65 (m, 10 H), 1.29 - 1.45 (m, 3 H), 1.24 (d, *J*=10.5 Hz, 2 H), 1.14 - 1.20 (m, 4 H), 1.11 (s, 3 H), 1.05 (s, 3 H), 0.99 (s, 3 H), 0.98 (s, 3 H).

### EXAMPLE 111. Preparation of 4-((1R,3aS,5aR,5bR,7aR,11aS,11bR,13aR,13bR)-3a-((2-(4-(ethoxycarbonyl)piperidin-1-yl)-2-oxoethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

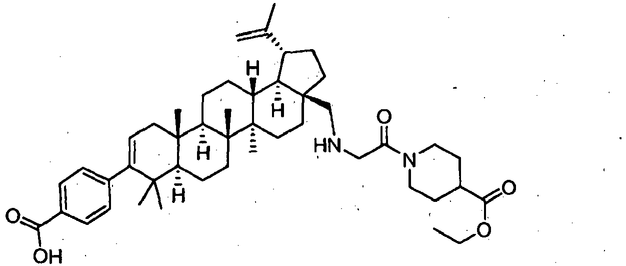

The title compound was prepared following the general procedure described above for the preparation of C28 amines with amide end cap using methyl 2-aminoacetate hydrochloride and ethyl piperidine-4-carboxylate as the reactant amines. The product was isolated as a white solid (10.0 mg, 68.5 %). LCMS: m/e 741.6 (MH⁺), 2.34 min (method 3). ¹H NMR (400 MHz, *MeOD*) δ ppm 7.94 (m, *J*=8.3 Hz, 2 H), 7.27 (m, 2 H), 5.26 - 5.40 (m, 1 H), 4.76 (d, *J*=1.8 Hz, 1 H), 4.66 (s, 1 H), 4.30 - 4.50 (m, 1 H), 4.11 - 4.24 (m, 4 H), 3.64 - 3.82 (m, 1 H), 3.18 - 3.30 (m, 2 H), 2.92 - 3.10 (m, 1 H), 2.87 (dd, *J*=12.4, 4.6 Hz, 1 H), 2.60 - 2.78 (m, 1 H), 2.43 - 2.55 (m, 1 H), 2.08 - 2.25 (m, 2 H), 1.85 - 2.08 (m, 6 H), 1.67 - 1.85 (m, 6 H), 1.46 - 1.67 (m, 8 H), 1.20 - 1.42 (m, 8 H), 1.13 - 1.19 (m, 3 H), 1.11 (s, 3 H), 1.04 (s, 3 H), 0.99 (s, 3 H), 0.96 (s, 3 H).

### EXAMPLE 112. Preparation of 1-(2-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecaliydro-1H-cyclopenta[a]chrysen-3a-yl)methylamino)acetyl)piperidine-4-carboxylic acid.

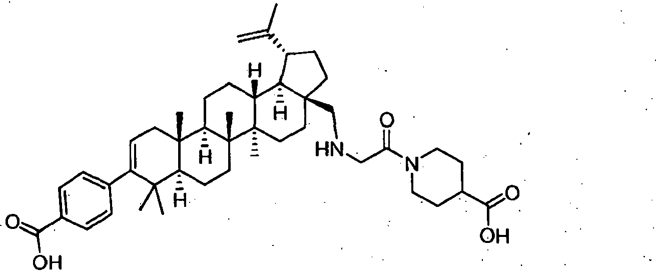

The title compound was prepared following the procedure described below:

To the solution of 4-((1R,3aS,5aR,5bR,7aR,11aS,11bR,13aR,13bR)-3a-((2-(4-(ethoxycarbonyl)piperidin-1-yl)-2-oxoethylamino)methyl)-5a,5b,8,8,11a-pentamethyl-1-(prop-1-en-2-yl)-2,3,3a,4,5,Sa,5b,6,7,7a,8,11,11 a,11b,12,13,13a,13b-octadecahydro-1H-cyclopenta[a]chrysen-9-yl)benzoic acid (12 mg, 0.016 mmol) in MeOH (3.00 ml) and dioxane (3 ml), sodium hydroxide (10 mg, 0.250 mmol) was added followed by 0.5 ml of water. The resulting suspension was stirred at 25 °C for 4 h. The solvent was removed *in vacuo,* the product was isolated by prep. HPLC as a white solid (5 mg, 41.4 %). LCMS: m/e 713.5 (MH⁺), 2.37 min (method 3). ¹H NMR (400 MHz, *MeOD)* δ ppm 7.95 (m, 2 H), 7.27 (m, 2 H), 5.32 (d, *J*=4.5 Hz, 1 H), 4.76 (d, *J*=1.8 Hz, 1 H), 4.66 (s, 1 H), 4.29 - 4.46 (m, 1 H), 4.09 - 4.25 (m, 2 H), 3.64 3.85 (m, 1 H), 3.17 - 3.32 (m, 2 H), 3.05 (m, 1H), 2.87 (dd, *J*=12.8, 4.8 Hz, 1 H), 2.67 (tt, *J*=10.8, 4.0 Hz, 1 H), 2.49 (dt, *J*=10.9, 5.5 Hz, 1 H), 2.07 - 2.29 (m, 2 H), 1.85-2.07 (m, 4 H), 1.67 - 1.85 (m, 8 H), 1.43 - 1.67 (m, 9 H), 1.36 (br. s., 2 H), 1.32 (d, *J=*6.5 Hz, 2 H), 1.24 (d, *J*=14.8 Hz, 1 H), 1.13 - 1.20 (m, 4 H), 1.11 (s, 3 H), 1.04 (s, 3 H), 0.99 (s, 3 H), 0.98 (s, 3 H).

### EXAMPLE 113. Preparation of 4-((1R,3aS,5aR,5bR,7aR,llaS,libR,13aR,13bR)-3a-((2-(4-(N,N-dimethylsulfamoyl)piperazin-l-yl)-2-oxoethylamino)methyl)-5a, 5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

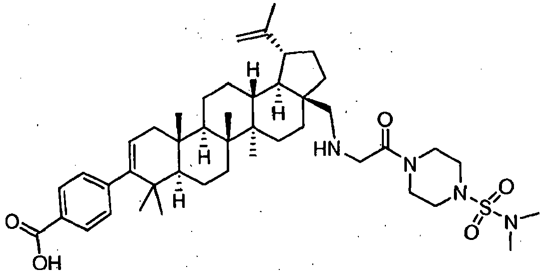

The title compound was prepared the general procedure described above for the preparation of C28 amines with amide end cap using methyl 2-aminoacetate hydrochloride and *N,N*-diniethylpiperazine-1-sulfonamide as the reactant amines. The product was isolated as a white solid (9.0 mg, 61.1 %). LCMS: m/e 777.6 (MH⁺), 2.39 min (method 3). ¹HNMR (400 MHz, *MeOD)* 8 ppm 7.96 (m, 2 H), 7.24 (m, *J*=8.5 Hz, 2 H), 5.28 - 5.38 (m, 1 H), 4.76 (d, *J*=1.5 Hz, 1 H), 4.66 (s, 1 H), 4.19 (s, 2 H), 3.75 (t, J=5.0 Hz, 2 H), 3.46 - 3.58 (m, 2 H), 3.33 - 3.38 (m, 3 H), 3.26 - 3.31 (m, 2 H), 2.79 - 2.95 (m, 7 H), 2.48 (td, *J*=10.8*,* 5.8,Hz, 1 H), 2.03 - 2.24 (m, 2 H), 1.90 - 2.03 (m, 3 H), 1.77 - 1.86 (m, 1 H), 1.67 - 1.77 (m, 6 H), 1.44 - 1.67 (m, 8 H), 1.34 (d, *J*=12.0 Hz, 3 H), 1.20 - 1.28 (m, 1 H), 1.13 - 1.20 (m, 4 H), 1.11 (s, 3 H), 1.05 (s, 3 H), 0.99 (s, 3 H), 0.98 (s, 3 H).

### EXAMPLE 114. Preparation of (R)-1-(2-(((1R,3aS,5aR,5bR,7aR,11aS,11bR,13aR,13bR)-9-(4-carboxyphenyl)-5a,5b,8,8,11a-pentamethyl-1-(prop-1-en-2yl)-2,3,3a,4,5,5a,5b,6,7,7a,8,11,11a,11b,12,13,13a,13b-octadecahydro-1H-cyclopenta[a]chrysen-3a-yl)methylamino)acetyl)pyrrolidine-2-carboxylic acid.

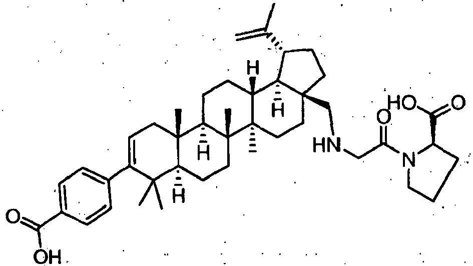

The title compound was prepared following the general procedure described above for the preparation of C28 amines with amide end cap using methyl 2-aminoacetate hydrochloride and (R)-*tert*-butyl pyrrolidine-2-carboxylate as the reactant amines. The product was isolated as a white solid (9.0 mg, 69.6 %). LCMS: m/e 699.3 (MH⁺), 2.34 min (method 3): ¹H NMR (400 MHz, *MeOD)* δ ppm 7.88 - 8.01 (m, 2 H), 7.17-7.31 (m, 2 H), 5.32 (d, *J*=4.8 Hz, 1 H), 4.75 (d, *J*=1.8 Hz, 1 H), 4.61 - 4.69 (m, 1 H), 4.56 (m, 1 H), 4.02 - 4.24 (m, 2 H), 3.64 - 3.73 (m, 1 H), 3.51 - 3.64 (m, 1 H), 3.34 - 3.43 (m, 1 H), 2.75 - 2.97 (m, 1 H), 2.43 - 2.56 (m, 1 H), 2.24 - 2.43 (m, 1 H), 2.01-2.22 (m, 5 H), 1.86 - 2.01 (m, 3 H), 1.67 - 1.81 (m, 6 H), 1.44 - 1.67 (m, 8 H), 1.27-1.44 (m, 4 H), 1.13 - 1.27 (m, 5 H), 1.10 (s, 3 H), 1.04 (s, 3 H), 0.99 (s, 3 H), 0.96 (s, 3 H).

### EXAMPLE 115. Preparation of 4-((1R,3aS,5aR,5bR,7aR,11aS,11bR,13aR)13bR)-3a-((2-(4,4-difluoropiperidin-1-yl)-2-oxoethylamino)methyl)-5a,5b,8,8,11 a-pentamethyl-1-(prop-1-en-2-yl)-2,3,3a,4,5,5a,5b,6,7,7a,8,11,11 a,11 b,12,13,13a,13b-octadecahydro-1H-cyclopenta[a]chrysen-9-yl)benzoic acid.

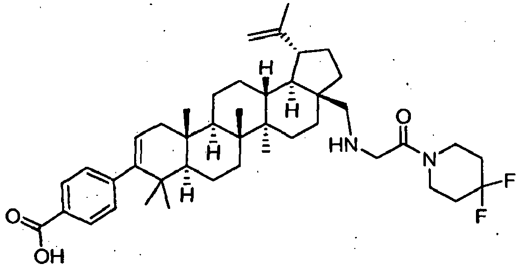

The title compound was prepared the general procedure described above for the preparation of C28 amines with amide end cap using methyl 2-aminoacetate hydrochloride and 4, 4-difluoropiperidine as the reactant amines. The product was isolated as a white solid (28 mg, 86.0 %). LCMS: m/e 705.5 (MH⁺), 2.36 min (method 3). ¹H NMR (400 MHz, *MeOD)* δ ppm 7.94 (m, *J*=8.3 Hz, 2 H), 7.24 (m, *J*=8.5 Hz, 2 H), 5.32 (dd, *J*=6.3, 1.8 Hz, 1 H), 4.76 (d, *J*=1.5 Hz, 1 H), 4.66 (s, 1 H), 4.22 (s, 2 H), 3.73 - 3.88 (m, 2 H), 3.50 - 3.67 (m, 2 H), 3.32 - 3.41 (m, 1 H), 2.88 (d, *J*=12.5 Hz, 1 H), 2.47 (td, *J*=10.9, 5.5 Hz, 1 H), 2.02 - 2.19 (m, 5 H), 1.93 - 2.00 (m, 2 H), 1.68 - 1.85 (m, 7 H), 1.44 - 1.68 (m, 9 H), 1.20 - 1.42 (m, 5 H), 1.13 - 1.19 (m, 4 H), 1.09 (s, 3 H), 1.05 (s, 3 H), 1.00 (s, 3 H), 0.95 (s, 3 H). ¹⁹F NMR (376 MHz, *MeOD)* δ ppm -99.61 (p, J=13.5 Hz, 2F).

### EXAMPLE 116. Preparation of 4-((1R,3aS,5aR,5bR,7aR,11aS,11bR,13aR,13bR)-3a-(((1-((1,1-dioxido-4-thiomorpholinyl)carbonyl)cyclopropyl)amino)methyl)-1-isopropenyl-5a,5b,8,8,11 a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

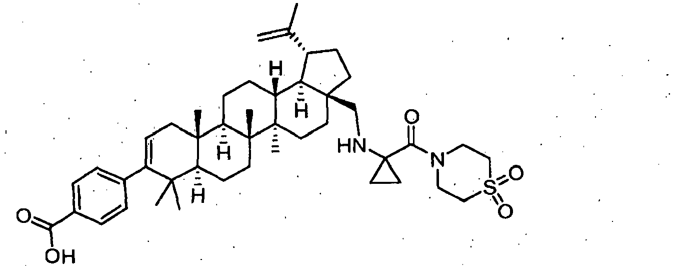

The title compound was prepared following the general procedure described above for the preparation of C28 amines with amide end cap using methyl 1-aminocyclopropanecarboxylate hydrochloride and thiomorpholine-1,1-dioxide as the reactant amines. The product was isolated as a white solid (3mg, 37.5 %). LCMS: m/e 745.6 (MH⁺), 2.76 min (method 3). ¹H NMR (400 MHz, *MeOD)* δ ppm 6.38 (d, *J*=8.3 Hz, 2 H), 5.68 (d, *J*=8.5 Hz, 2 H), 3.76 (dd, *J*=6.3, 1.8 Hz, 1 H), 3.20 (d, *J*=2.0 Hz, 1 H), 3.08 (s, 1 H), 2.63 (br. s., 4H), 1.59 1.69 (m, 4 H), 1.45 - 1.58 (m, 1 H), 1.11-1.27 (m, 1 H), 0.87 - 1.07 (m, 1 H), 0.61 (d, *J*=11.0 Hz, 1 H), 0.29 - 0.52 (m, 2 H), 0.08 - 0.25 (m, 9 H), 0.11 - 0.08 (m, 6 H), 0.28 - 0.11 (m, 6 H), 0.30 (br. s., 2 H), 0.45 - 0.33 (m, 6 H), 0.48 (s, 3 H), 0.50 (s, 3 H), 0.57 (s, 3 H), 0.59 (s, 3 H).

### EXAMPLE 117. Preparation of 4-((1R,3aS,5aR,5bR,7aR,11aS,11bR,13aR,13bR)-3a-((3-(4,4-difluoropiperidin-1-yl)-3-oxopropylathino)methyl)-5a,5b,9,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

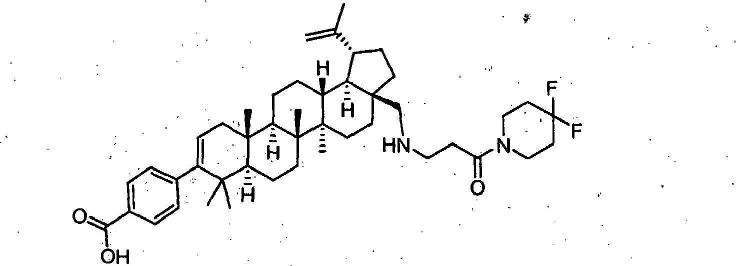

The title compound was prepared following the general procedure described above for the preparation of C28 amines with amide end cap using 3-aminopropanoate hydrochloride and 4, 4-difluoropiperidine as the reactant amines. The product was isolated as a white solid (25 mg, 74.0 %). LCMS: m/e 719.5 (MH⁺), 2.63 min (method 3). ¹H NMR (400 MHz, *MeOD)* δ ppm 7.95 (m, *J*=8.5 Hz, 2 H), 7.23 (m, 2 H), 5.26 - 5.38 (m, 1 H), 4.78 (d, *J*=1.8 Hz, 1 H), 4.67 (s, 1 H), 3.70 - 3.85 (m, 2 H), 3.59 - 3.70 (m, 2 H), 3.35 - 3.45 (m, 2 H), 3.30 (d, *J*=13.3 Hz, 1 H), 2.84 - 3.01 (m, 3 H), 2.55 (d, *J*=5.8 Hz, 1 H), 1.95 - 2.20 (m, 6 H), 1.68 - 1.92 (m, 10 H), 1.45 - 1.68 (m, 8 H), 1.38 (d, *J*=3.5 Hz, 1 H), 1.32 (br. s., 2 H), 1.13 - 1.27 (m, 5 H), 1.10 (s, 3 H), 1.06 (s, 3 H), 0.99 (s, 3 H), 0.97 (s, 3 H). ¹⁹F NMR (376 MHz, *MeOD*) δ ppm -99.53 (p, J=13.4 Hz, 2F).

### EXAMPLE 118. Preparation of 4-((1R,3aS,5aR,5bR,7aR,11aS,11bR,13aR,13bR)-3a-(((3-(1,1-dioxido-4-thiomorpholinyl)-3-oxopropyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

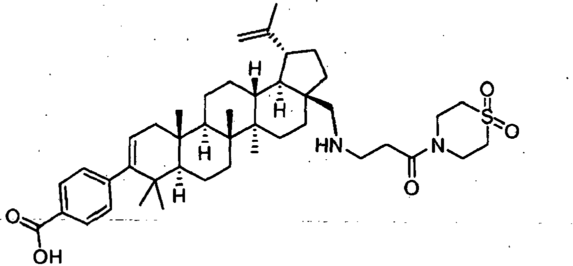

The title compound was prepared following the general procedure described above for the preparation of C28 amines with amide end cap using 3-aminopropanoate hydrochloride and thiomorpholine 1, 1-dioxide as the reactant amines. The product was isolated as a white solid (25 mg, 72.6 %). LCMS: m/e 733.5 (MH⁺), 2.55 min (method 3). ¹H NMR (400 MHz, *MeOD)* δ ppm 7.97 (m, 2 H), 7.25 (m, 2 H), 5.26 - 5.39 (m, 1 H), 4.78 (d, *J=*1.8 Hz, 1 H), 4.67 (s, 1 H), 4.05 - 4.19 (m, 2 H), 3.92 - 4.05 (m, 2 H), 3.39 - 3.48 (m, 2 H), 3.28 - 3.32 (m, 1 H), 3.20 - 3.27 (m, 2 H), 3.12 - 3.20 (m, 2 H), 2.99 (t, *J*=5.8 Hz, 2 H), 2.93 (d, *J*=12.8 Hz, 1 H), 2.54 (dt, *J*=10.6, 5.4 Hz, 1 H), 2.00 - 2.24 (m, 2 H), 1.68 - 1.93 (m, 10 H), 1.44 - 1.68 (m, 8 H), 1.24 - 1.44 (m, 4 H), 1.13 - 1.24 (m, 4 H), 1.11 (s, 3 H), 1.04 (s, 3 H), 0.99 (s, 3 H), 0.97 (s, 3 H).

### EXAMPLE 119. Preparation of 4-((1R,3aS,5aR,5bR,7aR,11aS,11bR,13aR,13bR)-5a,5b,8,8,11a-pentamethyl-3a-((3-oxo-3-(3-(trifluoromethyl)-5,6-dihydro-[1,2,4]triazolo[4,3-a]pyrazin-7(8H)-yl)propylamino)methyl)-1-(prop-1-en-2-yl)-2,3,3a,4,5,5a,5b,6,7,7a,8,11,11a,11b,12,13,13a, 13b-octadecahydro-1H-cyclopenta[a]chrysen-9-yl)benzoic acid.

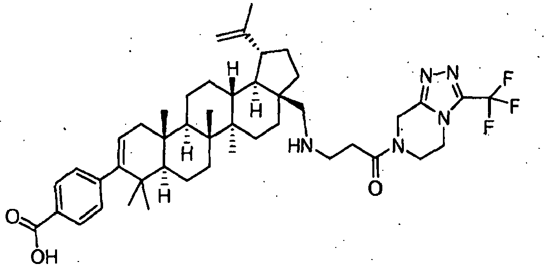

The title compound was prepared following the general procedure described above for the preparation of C28 amines with amide end cap using 3-aminopropanoate hydrochloride and 3-(trifluoromethyl)-5,6,7,8-tetrahydro-[1,2,4]triazolo[4,3-a]pyrazine as the reactant amines. The product was isolated as a white solid (20 mg, 35.7%). LCMS: m/e 790.5 (MH⁺), 2.48 min (method 3). ¹H NMR (400 MHz, *MeOD)* 8 ppm 7.94 (m, *J*=8.5 Hz, 2 H), 7.23 (m, 2 H), 5.32 (d, *J*=6.0 Hz, 1 H), 4.94 - 5.19 (m, 2 H), 4.78 (s, 1 H), 4.67 (s, 1 H), 4.32 - 4:44 (m, 1 H), 4.28 (br. s., 1 H), 3.96 - 4.19 (m, 2 H), 3.40 - 3.59 (m, 2 H), 3.26 - 3.33 (m, 1 H), 2.99 - 3.18 (m, 2 H), 2.92 (d, *J*=12.8 Hz, 1 H), 2.54 (br. s., 1 H), 2.34 (br. s., 1 H), 2.14 (br. s., 3 H), 1.52 (br. s., 12 H), 1.38 (br. s., 2 H), 1.31 (br. s., 3 H), 1.14 - 1.18 (m, 2 H), 0.94 - 1.12 (m, 17 H).

### EXAMPLE 120. Preparation of 4-((1R,3aS,5aR,5bR,7aR,11aS,11bR,13aR,13bR)-3a-(((3-(1,1-dioxido-1,3-thiazolidin-3-yl)-3-oxopropyl)amino)methyl)-1-isopropenyl-5a,5b,8,8,11a-pentamethyl-2,3,3a,4,5,5a,5b,6,7,7a,8,11,11a,11b,12;13,13a,13b-octadecahydro-1H-cyclopenta[a]chrysen-9-yl)benzoic acid.

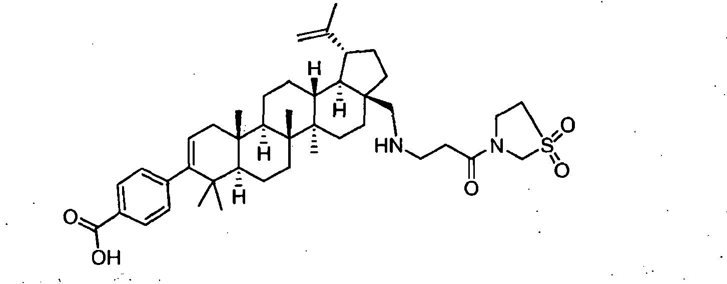

The title compound was prepared following the general procedure described above for the preparation of C28 amines with amide end cap using 3-aminopropanoate hydrochloride and sulfone as the reactant amines. The product was isolated as a white solid (20 mg, 35.7 %). LCMS: m/e 719.5 (MH⁺), 2.56 min (method 3). ¹H NMR (400 MHz, *MeOD)* 8 ppm 7.94 (d, *J*=8.5 Hz, 2 H), 7.24 (d, *J*=8.5 Hz, 2 H), 5.24 - 5.43 (m, 1 H), 4.8 (s, 1H), 4.61 - 4.72 (m, 1 H), 4.02 - 4.20 (m, 2 H), 3.46 - 3.63 (m, 1 H), 3.43 (t, *J*=7.3 Hz, 2 H), 3.35 - 3.41 (m, 2 H), 3.22 - 3.31 (m, 2 H), 2.95 (br. s., 1 H), 2.88 (s, 1 H), 2.73 (m, 1H), 2.44 - 2.64 (m, 1 H), 2.05 - 2.22 (m, 2 H), 1.67 - 1.90 (m, 8 H), 1.44 - 1.67 (m, 8 H), 1.39 (br. s., 5 H), 1.16 - 1.27 (m, 5 H), 1.11 (s, 3 H), 1.07 (s, 3 H), 0.99 (s, 3 H), 0.98 (s, 3 H).

### EXAMPLE 121. Preparation of 4-((1R,3aS,5aR,5bR,7aR,11aS,11bR,13aR,13bR)-5a,5b,8,8,11a-pentamethyl-3a-((3-((1R,5S)-8-methyl-3,8-diazabicyclo[3.2.1]octan-3-yl)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

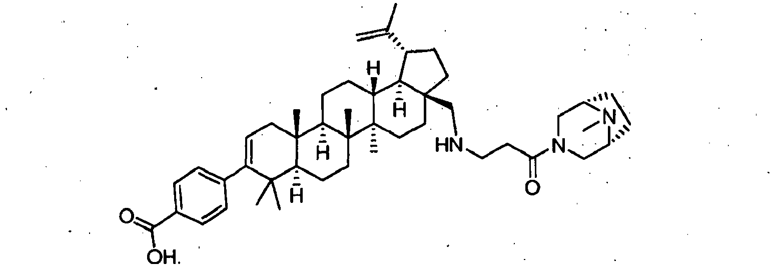

The title compound was prepared following the general procedure described above for the preparation of C28 amines with amide end cap using 3-aminopropanoate hydrochloride and (1R,5S)-8-methyl-3,8-diazabicyclo[3.2.1]octane as the reactant amines. The product was isolated as a white solid (7 mg, 71.7 %). LCMS: m/e 724.5 (MH⁺), 2.41 min (method 3). ¹H NMR (400 MHz, *MeOD)* δ ppm 7.92 (m, 2 H), 7.23 (m, 2 H), 5.25 - 5.41 (m, 1 H), 4.79 (d, *J*=1.8 Hz, 1 H), 4.67 (s, 1 H), 4.47 - 4.61 (m, 1 H), 4.03 - 4.09 (m, 2 H), 4.00 (d, *J*=1.8 Hz, 1 H), 3.68 (d, *J*=14.1 Hz, 1 H), 3.39 - 3.48 (m, 2 H), 3.16 - 3.26 (m, 1 H), 2.98 - 3.13 (m, 1 H), 2.78 - 2.98 (m, 4 H), 2.50 - 2.61 (m, 1 H), 2.25 - 2.47 (m, 2 H), 2.13 - 2.23 (m, 1 H), 1.99 - 2.13 (m, 2 H), 1.72 - 1.91 (m, 10 H), 1.50 - 1.68 (m, 8 H), 1.32 - 1.38 (m, 3 H), 1.28 - 1.32 (m, 2 H), 1.24 - 1.28 (m, 2 H), 1.14 - 1.24 (m, 4 H), 1.11 (s, 3 H), 1.05 (s, 3 H), 0.99 (s, 3 H), 0.98 (s, 3 H).

### EXAMPLE 122. Preparation of 4-((1R,3aS,5aR,5bR,7aR,11aS,11bR,13aR,13bR)-5a,5b,8,8,11a-pentamethyl-3a-((3-oxo-3-thiomorpholinopropylamino)methyl)-1-(prop-1-en-2-yl)-2,3,3a,4,5,5a,5b,6,7,7a,8,11,11 a,11b,12,13,13a,13b-octadecahydro-1H-cyclopenta[a]chrysen-9-yl)benzoic acid.

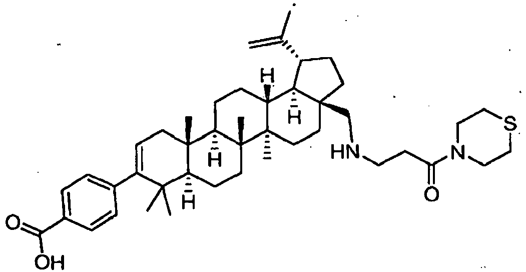

The title compound was prepared following the general procedure described above for the preparation of C28 amines with amide end cap using 3-aminopropanoate hydrochloride and thiomorpholine as the reactant amines. The product was isolated as a white solid (6 mg, 61.6 %). LCMS: m/e 701.5 (MH⁺), 2.67 min (method 3). ¹H NMR (400 MHz, *MeOD)* 8 ppm 7.81 - 8.00 (m, 2 H), 7.18 - 7.31 (m, 2 H), 5.24 - 5.47 (m, 1 H), 4.79 (d, *J*=1.5 Hz, 1 H), 4.67 (s, 1 H), 3.85 - 3.99 (m, 2 H), 3.80 (ddd, *J*=4.8, 2.9, 2.6 Hz, 2 H), 3.36 - 3.49 (m, 2 H), 3.25 - 3.32 (m, 1 H), 2.83 - 2.92 (m, 3 H), 2.61 - 2.79 (m, 4 H), 2.47 - 2.61 (m, 1 H), 1.99 - 2.23 (m, 2 H), 1.67 - 1.92 (m, 10 H), 1.47 - 1.67 (m, 8 H), 1.24 - 1.45 (m, 4 H), 1.20 (s, 4 H), 1.11 (s, 3 H), 1.05 (s, 3 H), 0.99 (s, 3 H), 0.96 (s, 3 H).

### EXAMPLE 123. Preparation of 4-((1R,3aS,5aR,5bR,7aR,11aS,11bR,13aR,13bR)-5a,5b,8,8,11a-pentamethyl-3a-((3-(2-methyl-1-oxo-2,8-diazaspiro[4.5]decan-8-yl)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

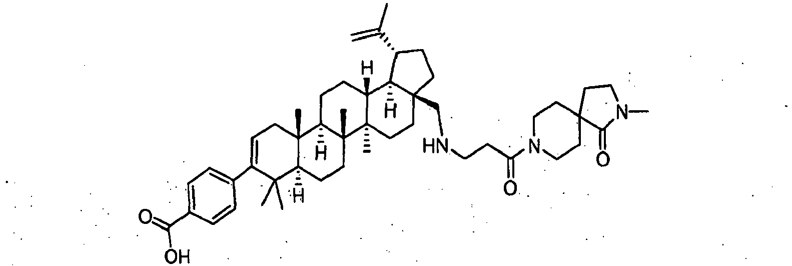

The title compound was prepared following the general procedure described above for the preparation of C28 amines with amide end cap using 3-aminopropanoate hydrochloride and 2-methyl-2,8-diazaspiro[4.5]decan-1-one as the reactant amines. The product was isolated as a white solid (7.6 mg, 77 %). LCMS: m/e 766.6 (MH⁺), 2.63 min (method 3). ¹H NMR (400 MHz, *MeOD*) δ ppm 7.94 (m, *J*=8.3 Hz, 2 H), 7.24 (m, *J*=8.5 Hz, 2 H), 5.33 (d, *J*=4.5 Hz, 1 H), 4.79 (s, 1 H), 4.67 (s, 1 H), 4.38 (d, *J*=13.6 Hz, 1 H), 3.90 (d, *J*=14.3 Hz, 1 H), 3.45 (t, *J*=6.9 Hz, 2 H), 3.34 - 3.42 (m, 2 H), 3.33 (m, 1H), 3.28 (dd, *J*=5.4, 2.1 Hz, 1 H), 3.02 - 3.15 (m, 1 H), 2.91 - 2.96 (m, 3 H), 2.80 - 2.88 (m, 3 H), 2.55 (br. s., 1 H), 2.01 - 2.24 (m, 4 H), 1.67 - 1.92 (m, 12 H), 1.46 - 1.67 (m, 10 H), 1.39 (br. s., 1 H), 1.23 - 1.36 (m, 3 H), 1.14 - 1.23 (m, 4 H), 1.11 (s, 3 H), 1.02 - 1.09 (s, 3 H), 1.00 (s, 3 H), 0.98 (s, 3 H).

### EXAMPLE 124. Preparation of 4-((1R,3aS,5aR,5bR,7aR,11aS,11bR,13aR,13bR)-5a,5b,8,8,11a-pentamethyl-3a-((3-(4-(2-(methylsulfonyl)ethyl)piperazin-l-yl)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]ehrysen-9-yl)benzoic acid.

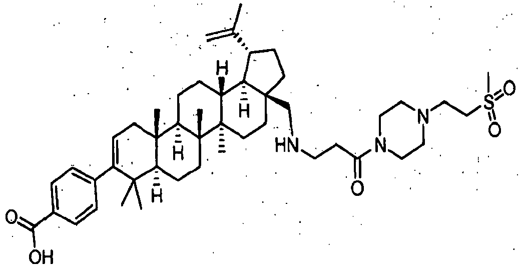

The title compound was prepared following the general procedure described above for the preparation of C28 amines with amide end cap using 3-aminopropanoate hydrochloride and 1-(2-(methylsulfonyl)ethyl)piperazine as the reactant amines. The product was isolated as a white solid (7.0 mg, 71.2 %). LCMS: m/e 790.6 (MH⁺), 2.51 min (method 3). ¹H NMR (400 MHz, *MeOD*) δ ppm 7.94 (m, 2 H), 7.26 (m, 2 H), 5.24 - 5.42 (m, 1 H), 4.79 (d, *J*=1.8 Hz, 1 H), 4.67 (s, 1 H), 4.00 (s, 2 H), 3.83 (d, *J*=5.3 Hz, 2 H), 3.63 - 3.75 (m, 2 H), 3.53 - 3.63 (m, 2 H), 3.38 - 3.47 (m, 2 H), 3.35-3.38 (m, 2 H), 3.28 (dd, *J*=3.9,2.1 Hz, 3 H), 3.12 (s, 3 H), 2.88 - 3.00 (m, 3 H), 2.54 (br. s., 1 H), 2.12 - 2.27 (m, 1 H), 2.09 (d, *J*=2.8 Hz, 1 H), 1.67 - 1.91 (m, 10 H), 1.47 - 1.67 (m, 8 H), 1.39 (br. s., 1 H), 1.28 - 1.36 (m, 2 H), 1.25 (br. s., 1 H), 1.14 - 1.21 (m, 4 H), 1.11 (s, 3 H), 1.05 (s, 3 H), 1.01 (s, 3 H), 0.96 (s, 3 H).

### EXAMPLE 125. Preparation of 4-((1R,3aS,5aR,5bR,7aR,11aS,11bR,13aR,13bR)-5a,5b,8,8,11a-pentamethyl-3a-((3-(methyl(2-(methylsulfonyl)ethyl)amino)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

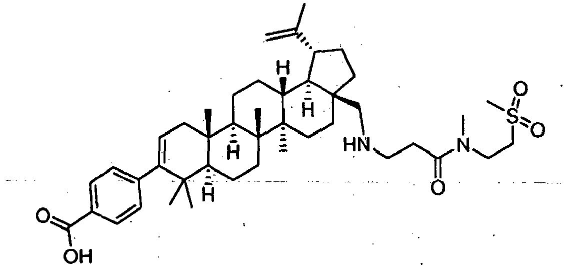

The title compound was prepared following the general procedure described above for the preparation of C28 amines with amide end cap using 3-aminopropanoate hydrochloride and *N*-methyl-2-(methylsulfonyl)ethanamine as the reactant amines. The product was isolated as a white solid (5.0 mg, 50.0 %). LCMS: m/e 735.5 (MH⁺), 2.24 min (method 3). ¹H NMR (400 MHz, *MeOD)* δ ppm 7.94 (m, *J*=8.5 Hz, 2 H), 7.24 (m, *J*=8.3 Hz, 2 H), 5.33 (dd, *J*=6.3, 1.8 Hz, 1 H), 4.79 (s, 1 H), 4.67 (d, *J=1.5* Hz, 1 H), 3.85 - 4.06 (m, 2 H), 3.35 - 3.48 (m, 4 H), 3.25 - 3.32 (m, 1 H), 3.13 (s, 3 H), 3.07 (s, 3 H), 2.82 - 2.96 (m, 3 H), 2.55 (br. s., 1 H), 2.13 - 2.27 (m, 1 H), 2.11 (d, *J*=12.3 Hz, 1 H), 1.66 - 1.91 (m, 8 H), 1.57 - 1.66 (m, 2 H), 1.52 (d, *J*=13.8 Hz, 3 H), 1.29 - 1.47 (m, 4 H), 1.20 (s, 3 H), 1.11 (s, 3 H), 1.06 (s, 3 H), 0.99 (s, 3 H), 0.95 (s, 3 H).

### EXAMPLE 126. Preparation of 4-((1R,3aS,5aR,5bR,7aR,11aS,11bR,13aR,13bR)-5a,5b,8,8,11a-pentamethyl-3a-((3-oxo-3-(1,4-dioxa-8-azaspiro[4.5]decan-8-yl)propylamino)methyl)-I-(prop-1-en-2-yl)-2,3,3a,4,5,5a,5b,6,7,7a,8,11,11a, 11b,12,13,13a,13b-octadecahydro-1H-cyclopenta[a]chrysen-9-yl)benzoic acid.

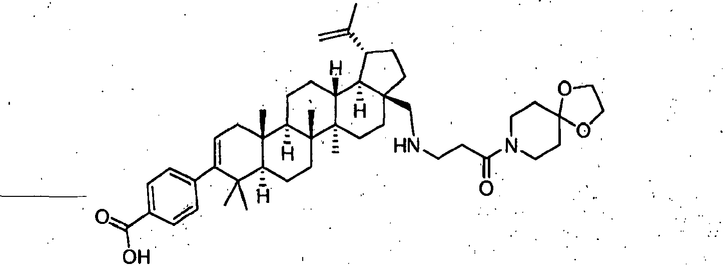

The title compound was prepared following the general procedure described above for the preparation of C28 amines with amide end cap using 3-aminopropanoate hydrochloride and 1,4-dioxa-8-azaspiro[4.5]decane as the reactant amines. The product was isolated as a white solid (3 mg, 30 %). LCMS: m/e 741.6 (MH⁺), 2.65 min (method 3). ¹H NMR (400 MHz, *MeOD)* δ ppm 7.94 (m, *J*=8.3 Hz, 2 H), 7.24 (m, *J*=8.5 Hz, 2 H), 5.33 (dd, *J*=6.3, 1.8 Hz, 1 H), 4.79 (d, *J*=1.5 Hz, 1 H), 4.67 (s, 1 H), 3.89 - 4.03 (m, 4 H), 3.65 - 3.81 (m, 2 H), 3.61 (dd, *J*=7.3, 4.5 Hz, 2 H), 3.36-3.43 (m, 2 H), 3.22 - 3.31 (m, 1 H), 2.85 - 3.00 (m, 3 H), 2.54 (br. s., 1 H), 2.18 (dd, *J*=16.9, 6.4 Hz, 1 H), 2.08 (br. s., 1 H), 1.67 - 1.91 (m, 11 H), 1.48 - 1.67 (m, 8 H), 1.24 - 1.48 (m, 6 H), 1.13 - 1.24 (m, 5 H), 1.11 (s, 3 H), 1.02 - 1.09 (m, 3 H), 1.00 (s, 3 H), 0.98 (s, 3 H).

### EXAMPLE 127. Preparation of 4-((1R,3aS,5aR,5bR,7aR,11aS,11bR,13aR,13bR)-5a,5b,8,8,11a-pentamethyl-3a-((3-(methylamino)-3-oxopropylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

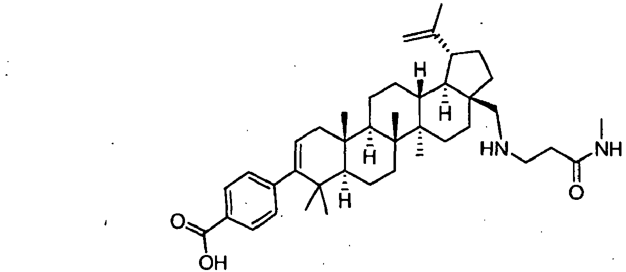

The title compound was prepared following the general procedure described above for the preparation of C28 amines with amide end cap using 3-aminopropanoate hydrochloride and methylamine as the reactant amines. The product was isolated as a white solid (5.0 mg, 50.0 %). LCMS: m/e 629.6 (MH⁺), 2.58 min (Method 3).

### EXAMPLE 128. Preparation of 4-((1R,3aS,5aR,5bR, 7aR,11aS,11bR,13aR,13bR)-5a,5b,8,8,11a-pentamethyl-3a-((3-oxo-3-(4-oxopiperidin-l-yl)propylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

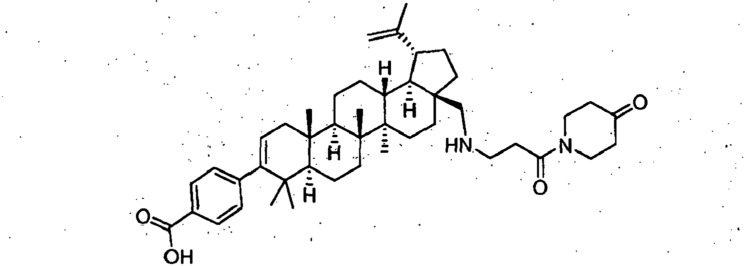

The title compound was prepared following the general procedure described above for the preparation of C28 amines with amide end cap using 3-aminopropanoate hydrochloride and piperidin-4-one,as the reactant amines. The product was isolated as a white solid (2 mg, 21.6 %). LCMS: m/e 697.6 (MH⁺), 2.61 min (method 3). ¹H NMR (400 MHz, *MeOD)* 8 ppm 7.94 (m, *J*=8.3 Hz, 2 H), 7.24 (m, *J*=8.3 Hz, 2 H), 5.25 - 5.39 (m, 1 H), 4.79 (s, 1 H), 4.67 (s, 1 H), 3.50 - 3.63 (m, 2 H), 3.35 - 3.47 (m, 6 H), 3.28 (dt, *J*=3.3, 1.6 Hz, 2 H), 3.13 - 3.25 (m, 1 H), 2.81 - 2.97 (m, 3 H), 2.44-2.64 (m, 1 H), 2.18 (dd, *J*=17.2, 6.4 Hz, 2 H), 1.67 - 1.91 (m, 8 H), 1.60 (br. s., 3 H), 1.46 - 1.57 (m, 4 H), 1.24 - 1.46 (m, 6 H), 1.15 - 1.24 (m, 5 H), 1.11 (s, 3 H), 1.01 - 1.09 (m, 3 H), 1.00 (s, 3 H), 0.98 (s, 3 H).

### EXAMPLE 129. Preparation of 4-((1R,3aS,5aR,5bR,7aR,11aS,11bR,13aR,13bR)-3a-((3-(1,3-dihydroxy-2-methylpropan-2-ylamino)-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

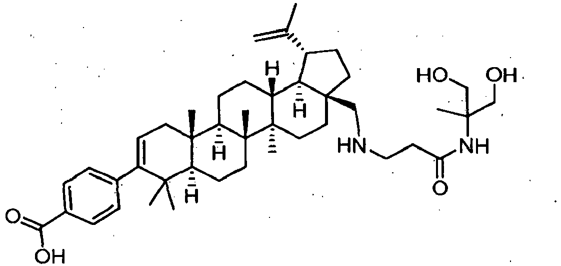

The title compound was prepared following the general procedure described above for the preparation of C28 amines with amide end cap using 3-aminopropanoate hydrochloride and *N*,3-dimethyloxetan-3-amine as the reactant amines. The product was isolated as a white solid (1 mg, 11.6 %). LCMS: m/e 703.7 (MH⁺), 2.47 min (method 3).

### EXAMPLE 130. Preparation of 4-((1R,3aS,5aR,5bR,7aR,11aS,11bR,13aR,13bR)= 5a,5b,8,8,11a-pentamethyl-3a-((2-oxo-2-(piperidin-1-yl)ethylamino)methyl)-1-(prop-1-en-2-yl)-2,3,3a,4,5)5a,5b,6,7,7a,8,11,11a,11b,12,13,13a,13b-octadecahydro-1H-cyclopenta[a]chrysen-9-yl)benzoic acid.

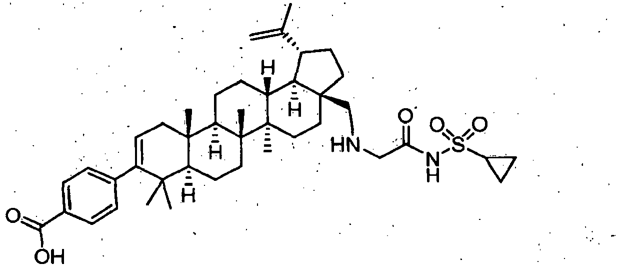

The title compound was prepared following the general procedure described above for the preparation of C28 amines with amide end cap using methyl 2-aminoacetate hydrochloride and piperidine as the reactant amines. The product was isolated as a white solid (10.0 mg, 51.5 %). LCMS: m/e 669.4 (MH⁺), 2.16 min (method 3). ¹H NMR (400 MHz, *MeOD)* δ ppm 7.94 (m, *J*=8.5 Hz, 2 H), 7.24 (m, *J*=8.5 Hz, 2 H), 5.32 (dd, *J*=6.1, 1.6 Hz, 1 H), 4.76 (d, *J*=1.8 Hz, 1 H), 4.66 (s, 1 H), 4.13 (s, 2 H), 3.56 - 3.72 (m, 2 H), 3.35 - 3.46 (m, 2 H), 3.3 (m, 1 H), 2.86 (d, *J*=12.5 Hz, 1 H), 2.48 (dt, *J*=11.1, 5.6 Hz, 1 H), 2.03 - 2.22 (m, 2 H), 1.84 - 2.03 (m, 3 H), 1.66 - 1.78 (m, 10 H), 1.48 - 1.65 (m, 10 H), 1.29 - 1.45 (m, 3 H), 1.24 (d, *J*=10.5 Hz, 2 H), 1.14 - 1.20 (m, 4 H), 1.11 (s, 3 H), 1.05 (s, 3 H), 0.99 (s, 3 H), 0.98 (s, 3 H).

### EXAMPLE 131. Preparation of 4-((1R,3aS,5aR,5bR,7aR,11aS,11bR,13aR,13bR)-3a-((3-carboxy-1-(3,3-difluoropyrrolidin-1-yl)-1-oxopropan-2-ylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

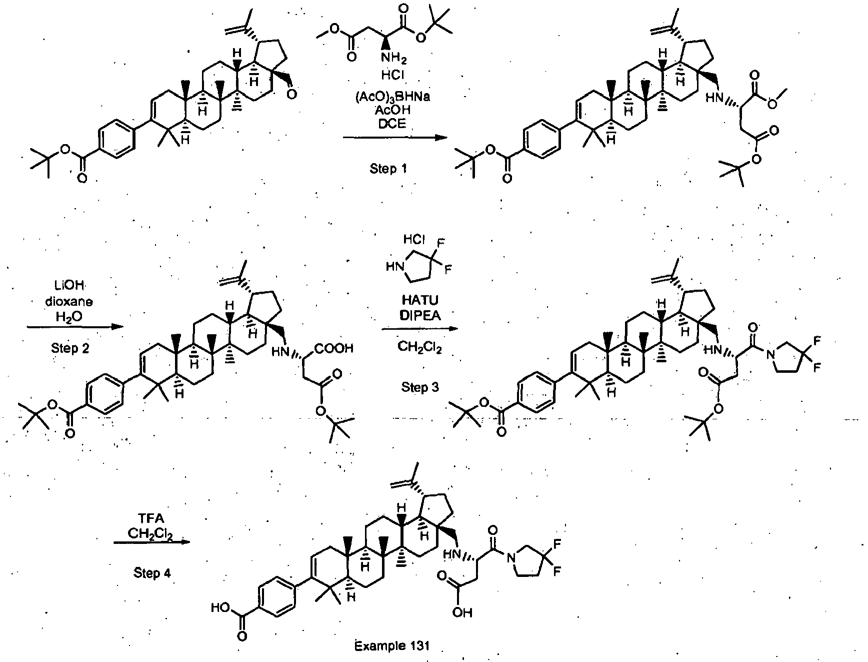

### Step 1: Preparation of the C28 amine

To a solution of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (100 mg, 0.167 mmol)) in DCE was added (S)-1-*tert*-butyl 4-methyl 2-aminosuccinate hydrochloride (120 mg, 0.501 mmol) and acetic acid (10.03 mg, 0.167 mmol). The mixture was stirred at rt for 10 min. Then sodium triacetoxyhydroborate (106 mg, 0.501 mmol) was added and it was stirred at rt for 48 h. The mixture was diluted with 7 ml of a sat. aqueous solution of sodium carbonate and was extracted with DCM (3 x 7 ml). The combined organic layers were dried with sodium sulfate. The drying agent was removed by filtration and the filtrate was concentrated under reduced pressure. The crude product was used in the next step with no additional purification. MS: m/e 786.6 (MH⁺), 3.03 min (method 3). ¹H NMR (400 MHz, *CHLOROFORM-d*) δ ppm 0.92 (s, 3 H) 0.92 (s, 3 H) 0.98 (s, 3 H) 0.99 (s, 3 H) 1.09 (s, 3 H) 1.48 (s, 9 H) 1.58 (s, 9 H) 1.68 (s, 3 H) 0.84 - 1.78 (m, 20 H) 1.86 - 2.01 (m, 2 H) 2.09 (dd, *J*=17.19, 6.40 Hz, 1 H) 2.33 (d, *J*=11.29 Hz, 1 H) 2.40 (td, *J*=11.04, 5.52 Hz, 1 H) 2.53 - 2.63 (m, 2 H) 2.67 - 2.75 (m, 1 H) 3.53 (dd, *J*=7.28, 6.27 Hz, 1 H) 3.68 (s, 3 H) 4.57 (s, 1 H) 4.68 (d, *J*=2.01 Hz, 1 H) 5.27 (dd, *J*=6.02, 1.51 Hz, 1 H) 7.16 (d, *J*=8.28 Hz, 2 H) 7.88 (d, *J*=8.28 Hz, 2 H).

### Step 2: Hydrolysis of the methyl ester

To a solution of crude material (0.112 g, 0.142 mmol) from Step 1 in dioxane (1.5 ml) was added lithium hydroxide (6.82 mg, 0.285 mmol). The reaction was heated up to 63 °C for 4 h. Solvent was evaporated and the crude product was purified by prep. HPLC (YMC Combiprep ODS 30x50 mm S5, MeOH / H₂O /TFA). MS: m/e 772.6 (MH⁺), 3.02 min (method 3). The fractions containing the desired product were combined and concentrated under reduce pressure. The residue was taken to the next step without further purification.

### Step 3: Preparation of the amide end cap

To a mixture of material (30 mg, 0.039 mmol) from Step 2, 3,3-difluoropyrrolidine hydrochloride (6.69 mg, 0.047 mmol) in DCM (1 ml) was added DIPEA (0.034 ml, 0.194 mmol) followed by HATU (22.16 mg, 0.058 mmol). The resulting solution was stirred at rt for 1 h. The reaction mixture was concentrated *in vacuo* to give crude product which was taken to the next step without further purification. MS: m/e 861.6 (MH⁺), 2.07 min (method 2).

### Step 4: Preparation of the di-acid.

To a solution of crude material from Step 3 in DCM (2 ml) was added TFA (0.5 ml, 6.49 mmol). The mixture was stirred at rt for 2 h and then was concentrated *in vacuo.* The crude product was purified by prep. HPLC (YMC Combiprep ODS 30x50 mm S5, MeOH / H₂O /TFA) to afford 4-((1R,3aS,5aR,5bR,7aR,11aS,11bR,13aR,13bR)-3a-((3-carboxy-1-(3,3-difluoropyrrolidin-1-yl)-1-oxopropan-2-ytamino)methyl)-5a,5b,8,8,11a-pentamethyl-1-(prop-1-en-2-yl)-2,3,3a,4,5,5a,5b,6,7,7a,8,11,11a, 11b,12,13,13a,13b-octadecahydro-1H-cyclopenta[a]chrysen-9-yl)benzoic acid (15 mg, 51 %). MS: m/e 749.6 (MH+), 1.74 min (method 2). ¹H NMR (400 MHz, *MeOD)* δ ppm 0.95 (s, 3 H) 0.9.7 (s, 3 H) 1.04 (s, 3 H) 1.08 (s, 3 H) 1.19 (s, 3 H) 1.72 (s, 3 H) 0.86 - 2.02 (m, 24H) 2.15 (dd, *J*=16.56, 6.27 Hz, 1 H) 2.38 - 2.61 (m, 3 H) 2.71 (t, *J*=11.54 Hz, 1 H) 2.78 - 2.89 (m, 1 H) 2.93 - 3.04 (m, 1 H) 3.75 - 4.03 (m, 3 H) 4.61 - 4.65 (m, 1 H) 4.73 (d, *J*=1.76 Hz, 1 H) 5.30 (dd, *J*=6.40, 1.88 Hz, 1 H) 7.22 (d, *J*=8.28 Hz, 2 H) 7.91 (d, *J*=8.28 Hz, 2 H). ¹⁹F NMR (376 MHz, *MeOD)* δ ppm -105.07 - -101.67 (m, 2 F).

### EXAMPLE 132. Preparation of 4-thiomorpholinebutanoic acid, 4-((1R,3aS,5aR,5bR,7aR,11aS,11bR,13aR,13bR)-3a-((((1S)-1-(carboxymethyl)-2-(1,1-dioxido-4-thiomorpholinyl)-2-oxoethyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

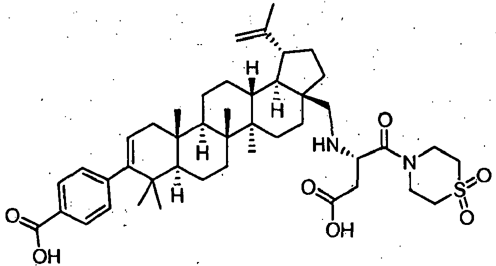

The title compound was prepared following the procedure described above for the preparation of 4-((1R,3aS,5aR,5bR,7aR,11aS,11bR,13aR,13bR)-3a-((3-carboxy-1-(3,3-difluoropyrrolidin-1-yl)-1-oxopropan-2-ylamino)metbyl)-5a,5b,8,8,11a-pentamethyl-1-(prop-1-en-2-yl)-2,3,3a,4,5,5a,5b,6,7,7a,8,11,11 a,11b,12,13,13a,13b-octadecahydro-1H-cyclopenta[a]chrysen-9-yl)benzoic acid (example 131) using (S)-1-*tert*-butyl 4-methyl 2-aminosuccinate hydrochloride and thiomorpholine 1,1-dioxide as the reactant amines in steps 1 and 3 respectively. The product was isolated as a white solid (21 mg, 63.8 %). LCMS: m/e 777.4 (MH⁺), 2.60 min (method 3). ¹H NMR (400 MHz, *MeOD)* 8 ppm 7.78 - 8.01 (m, 2 H), 7.13 - 7.31 (m, 2 H), 5.32 (d, *J*=4.8 Hz, 1 H), 4.77 (d, *J*=1.0 Hz, 1 H), 4.66 (s, 1 H), 4.18 (d, *J*=4.3 Hz, 2 H), 3.94-4.15 (m, 2 H), 3.37 - 3.49 (m, 1 H), 3.19 - 3.28 (m, 4 H), 2.91 - 3.15 (m, 3 H), 2.71 - 2.86 (m, 1 H), 2.48 *(d, J*=5.8 Hz, 1 H), 2.16 (dd, *J=*17.1, 6.3 Hz, 1 H), 1.95 - 2.09 (m, 2 H), 1.90 (d, *J*=14.6 Hz, 1 H), 1.68 - 1.87 (m, 8 H), 1.60 (br. s., 2 H), 1.47 - 1.60 (m, 6 H), 1.36 - 1.44 (m, 1 H), 1.25 - 1.36 (m, 3 H), 1.16 - 1.25 (m, 4 H), 1.10 (s, 3 H), 1.05 (s, 3 H), 0.99 (s, 3 H), 0.96 (s, 3 H).

### General procedure for the preparation of C28 reversed amides: Examples 133 -147.

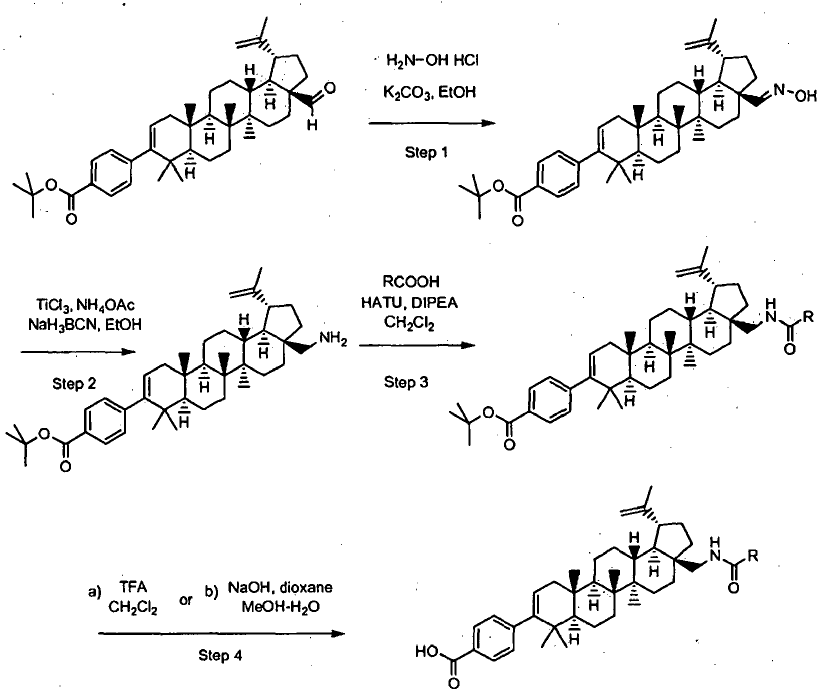

### Step 1: Preparation of the C28 oxime

To a suspension of the *tert*-butyl 4-((1R,3aS,5aR,5bR,7aR,11aS,11bR,13aR,13bR)-3a-formyl-5a,5b,8,8,11a-pentamethyl-l-(prop-l-en-2-yl)-2,3,3a,4,5,5a,5b,6,7,7a,8,11,11a,11b,12,13,13a,13b-octadecahydro-1H-cyclopenta[a]chrysen-9-yl)benzoate (270 mg, 0.451 mmol) in ethanol (20 ml) was added hydroxylamine hydrochloride (433 mg, 6.23 mmol) and potassium carbonate (862 mg, 6.23 mmol). The suspension was stirred at ∼50°C for 2 h. The mixture was diluted with 7 ml of saturated NaHCO₃ solution and was extracted with dichloromethane (3 x 7 ml). The combined organic layers were dried with sodium sulfate. The drying agent was removed by filtration and the filtrate was concentrated under reduced pressure. The crude product (quantitative) was used in the next step with no further purification. ¹H NMR (400 MHz, CHLOROFORM-d) 8 7.90 (2 H, d, *J*=8.3 Hz), 7.59 (1 H, s), 7.19 (2 H, d, *J*=8.3 Hz), 6.96 (1 H, s), 5.29 (1 H, dd, *J*=6.2, 1.6 Hz), 4.75 (1 H, d, *J*=1.8 Hz), 4.64 (1 H, d, *J*=2.0 Hz), 2.56 (1 H, td, *J*=11.1, 5.4 Hz), 2.12 (1 H, dd, *J*=16.9, 6.3 Hz), 1.96 - 2.02 (2 H, m), 1.92 - 1.96 (1 H, m), 1.81 - 1.92 (2 H, m), 1.76 - 1.81 (2 H, m), 1.73 (4 H, s), 1.63 - 1.70 (2 H, m), 1.61 (9 H, s), 1.56 (2 H, br. s.), 1.48 (2 H, br. s.), 1.31 - 1.42 (2 H, m), 1.27 (3 H, s), 1.08 - 1.20 (2 H, m), 1.07 (3 H, s), 1.04 (3 H, s), 1.00 (3 H, s), 0.94 (6 H, s).

### Step 2: Reduction of the C28 oxime

To a clear solution of the crude material from Step 1 (1.205 g, 1.963 mmol) in EtOH (40 ml) was added excess of ammonium acetate (1.059 g, 13.74 mmol) and sodium cyanoborohydride (863 mg, 13.74 mmol). The mixture was stirred in an ice bath until it was cold. To this suspension was added titanium (III) chloride (20% solution, 10 ml, 1.963 mmol). The resulting mixture was blanketed under nitrogen, and stirred at rt for an hour. The dark greenish-blue solution was treated with a solution of sodium hydroxide (10N), 3 ml in 25 ml water, along with 30 ml of methylene chloride. The mixture was stirred vigorously in open air until the aqueous phase became light blue. The suspending titanium residue was removed by a filtration through a short bed of paper cellulose. The clear filtrate was separated, the aqueous phase was extracted with methylene chloride (2 X 25 ml). The organic layers were combined, evaporated to dryness under high vacuum to obtained *tert*-butyl 4-((1R,3aS,5aR,5bR,7aR,11as,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1.3 g, 100%). ¹H NMR (400 MHz, CHLOROFORM-d) 8 0.94 (s, 6 H), 1.00 (s, 3 H), 1.03 (s, 3 H), 1.05 - 1.09 (m, 1 H), 1.10 (s, 3 H), 1.19 - 1.35 (m, 3 H), 1.35 - 1.57 (m, 13 H), 1.61 (s, 9 H), 1.65-1.69 (m, 1 H), 1.72 (s, 3 H), 1.75 - 2.02 (m, 3 H), 2.12 (dd, *J*=17.00, 6.17 Hz, 1 H), 2.36 (d, *J*=13.09 Hz, 1 H), 2.44 (td, *J*=10.89, 5.41 Hz, 1 H), 2.89 (d, *J*=13.09 Hz, 1 H), 3.73 (s, 1 H), 4.61 (s, 1 H), 4.72 (s, 1 H), 5.29 (d, J=4.53 Hz, 1 H), 7.19 (d, *J*=8.06 Hz, 2 H), 7.90 (d, *J*=8.31 Hz, 2 H).

### Step 3: Acylation

To a solution of the material from Step 2 (*tert*-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate) and the corresponding carboxylic acid (2 eq.) in DCM (5 - 8 ml) at 0 °C was added HATU (2-3 eq.) followed by DIPEA (4 eq.). The mixture was stirred at rt for 2 - 18 h. The solvent was evaporated under vacuum and the resulting crude product was purified by Biotage flash chromatography or was used directly in the next step without further purification.

### Step 4: Preparation of the benzoic acids

(a) Acidic hydrolysis - To a solution of the material from Step 3 in DCM (4 - 5 ml) was added TFA (0.4 - 0.5 ml). The mixture was stirred at room temperature for 2 - 6 h. The solvent was evaporated under vacuum. The resulting crude product was purified by prep. HPLC to give the desired benzoic acids.
(b) Basic hydrolysis - To a solution of the material from Step 3 in dioxane (2 ml) and methanol (2 ml) was added NaOH (75 mg; 1.875 mmol) and H₂O (0.5 ml). The resulting solution was stirred at 70°C for 5 - 10 h. The solvent was evaporated under vacuum and the resulting crude product was purified by prep. HPLC to give the desired benzoic acids.

### EXAMPLE 133. Preparation of 4-((1R,3aS,5aR,5bR,7aR,11aS,11bR,13aR,13bR) 5a,5b,8,8,11a-pentamethyl-3a-((2-(2-oxopyrrolidin-1-yl)acetamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

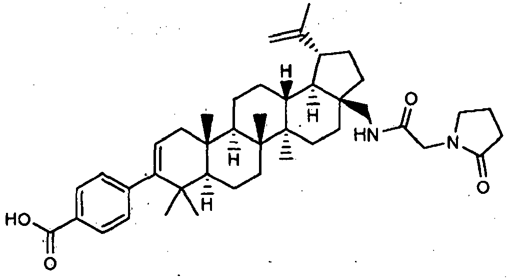

The title compound was prepared in 55 % yield following the general procedure described above for the reversed amides preparation, using 2-(2-oxopyrrolidin-1-yl)acetic acid as the reactant acid and acidic hydrolysis. MS: m/e 725.4 (MH⁺), 1.78 min (method 2). ¹H NMR (500 MHz, *MeOD)* δ ppm 0.94 (s, 3 H) 0.96 (s, 3 H) 1.03 (s, 3 H) 1.04 (s, 3 H) 1.18 (s, 3 H) 1.70 (s, 3 H) 0.89 - 2.18 (m, 24 H) 2.38 - 2.45 (m, 2 H) 2.52 (td, *J*=10.83, 5.80 Hz, 1 H) 2.99 - 3.06 (m, 1 H) 3.45 - 3.51 (m, 2 H) 3.53-3.61 (m, 1 H) 3.97 (d, *J*=2.14 Hz, 2 H) 4.59 (s, 1 H) 4.72 (s, 1 H) 5.27 - 5.32 (m, 1 H) 7.22 (d, *J*=8.24 Hz, 2 H) 7.91 (d, *J*=8.55 Hz, 2 H).

### EXAMPLE 134. Preparation of 4-((1R,3aS,5aR,5bR,7aR,11aS,11bR,13aR,13bR)-3a-((2,6-dioxopiperidine-4-carboxamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

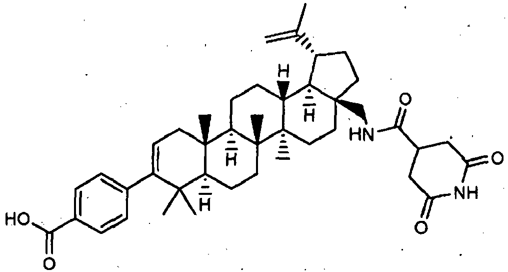

The title compound was prepared in 60 % yield following the general procedure described above for the reversed amides preparation, using 2,6-dioxopiperidine-4-carboxylic acid as the reactant acid and acidic hydrolysis. MS: m/e 683.2 (MH⁺), 1.70 min (method 2). ¹H NMR (500 MHz, *MeOD)* 8 ppm 0.97 (s, 3 H) 0.99 (s, 3 H) 1.06 (s, 3 H) 1.07 (s, 3 H) 1.19 (s, 3 H) 1.73 (s, 3 H) 0.90 - 1.79 (m, 18) 1.81 - 1.96 (m, 2 H) 2.03 - 2.12 (m, 1 H) 2.17 (dd, *J*=16.79, 6.41 Hz, 1 H) 2.54 (td, *J*=11.14, 5.49 Hz, 1 H) 2.73 (d, *J*=6.41 Hz, 4 H) 3.05 (dd, *J*=13.58, 5.95 Hz, 1 H) 3.07 - 3.14 (m, 1 H) 3.55 (dd, *J*=13.28, 5.95 Hz, 1 H) 4.62 (s, 1 H) 4.74 (s, 1 H) 5.32 (d, *J*=5.80 Hz, 1 H) 7.24 (d, *J*=7.32 Hz, 2 H) 7.94 (d, *J*=7.32 Hz, 2 H).

### EXAMPLE 135. Preparation of 4-((1R,3aS,5aR,5bR,7aR,11aS,11bR,13aR,13bR)-3a-(((R)-2-amino-4-(methylsulfonyl)butanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

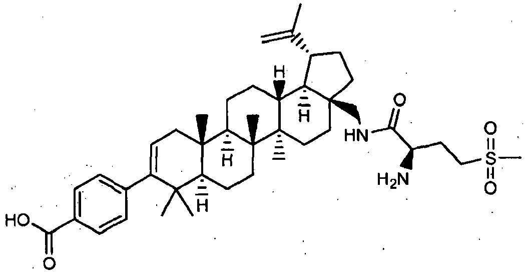

The title compound was prepared in 77 % yield following the general procedure described above for the reversed amides preparation, using (R)-2-(*tert-*butoxycarbonylamino)-4-(methylsulfonyl)butanoic acid as the reactant acid and acidic hydrolysis. MS: m/e 707.2 (MH⁺), 1.54 min (method 2). ¹H NMR (400 MHz, *MeOD)* δ ppm 0.96 (s, 3 H) 0.98 (s, 3 H) 1.05 (s, 3 H) 1.07 (s, 3 H) 1.19 (s, 3 H) 1.73 (s, 3 H) 0.88 - 1.80 (m, 18 H) 1.81 - 1.96 (m, 2 H) 2.04 - 2.12 (m, 1 H) 2.16 (dd, *J*=17.07, 6.27 Hz, 1 H) 2.33 - 2.42 (m, 2 H) 2.54 (td, *J*=11.04, 5.52 Hz, 1 H) 3.04 (s, 3 H) 3.08 (d, *J*=13.80 Hz, 1 H) 3.15 - 3.24 (m, 1 H) 3.68 (d, *J*=13.30 Hz, 1 H) 3.99 (s, 1 H) 4.08 (t, *J*=6.27 Hz, 1 H) 4.62 (dd, *J*=2.13, 1.38 Hz, 1 H) 4.75 (d, *J*=1.76.Hz, 1 H) 5.31 (dd, *J*=6.15*,* 1.63 Hz, 1 H) 7.23 (d, *J*=8.28 Hz, 2 H) 7.93 (d, *J*=8.53 Hz, 2 H).

### EXAMPLE 136. Preparation of 4-((1R,3aS,5aR,5bR,7aR,11aS,11bR,13aR,13bR)-5a,5b,8,8,11a-pentarnethyl-1-(prop-1-en-2-yl)-3a-(((S)-pyrrolidine-2-carboxamido)methyl)-2,3,3a,4,5,5a,5b,6,7,7a,8,11,11a,11b,12,13,13a,13b-octadecahydro-1H-cyclopenta[a]chrysen-9-yl)benzoic acid.

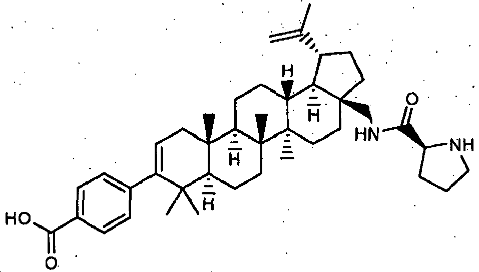

The title compound was prepared in 31 % yield following the general procedure described above for the reversed amides preparation, using (S)-1-(*tert-*butoxycarbonyl)pyrrolidine-2-carboxylic acid as the reactant acid and acidic hydrolysis. MS: m/e 641.7 (MH⁺), 1.77 min (method 2). ¹H NMR (400 MHz, *MeOD*) δ ppm 0.94 (s, 3 H) 0.96 (s, 3 H) 1.03 (s, 3 H) 1.05 (s, 3 H) 1.17 (s, 3 H) 1.71 (s, 3 H) 0.86 - 2.10 (m, 24 H) 2.14 (dd, *J*=17.19, 6.65 Hz, 1 H) 2.39 - 2.47 (m, 1 H) 2.52 (td, *J*=11.17, 5.52 Hz, 1 H) 3.01 (dd, *J*=13.55, 5.02 Hz, 1 H) 3.33 - 3.36 (m, 1 H) 3.38-3.47 (m, 1 H) 3.69 (dd, *J*=13.93, 5.90 Hz, 1 H) 4.23 (dd, *J*=8.03, 7.03 Hz, 1 H) 4.61 (s, 1 H) 4.73 (d, *J*=1.76 Hz, 1 H) 5.30 (dd, *J*=6.02, 1.51 Hz, 1 H) 7.21 (d, *J*=8.28 Hz, 2 H) 7.91 (d, *J*=8.28 Hz, 2 H) 8.13 (t, *J*=6.02 Hz, 1 H).

### EXAMPLE 137. Preparation of 4-((1R,3aS,SaR,5bR,7aR,11aS,11bR,13aR,13bR)-5a,5b,8,8,11a-pentamethyl-1-(prop-1-en-2-yl)-3a-(((R)-pyrrolidine-2-carboxamido)methyl)-2,3,3a,4,5,5a,5b,6,7,7a,8,11,11a,11b,12,13,13a,13b-octadecahydro-1H-cyclopenta[a]chrysen-9-yl)benzoic acid.

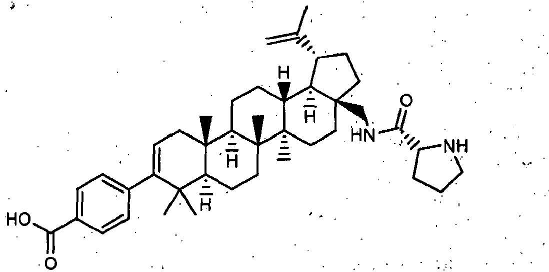

The title compound was prepared in 30 % yield following the general procedure described above for the reversed amides preparation, using (R)-1-(*tert-*butoxycarbonyl)pyrrolidine-2-carboxylic acid as the reactant acid and acidic hydrolysis. MS: m/e 641.7 (MH⁺), 1.78 min (method 2). ¹H NMR (400 MHz, *MeOD)* δ ppm 0.94 (s, 3 H) 0.96 (s, 3 H) 1.03 (s, 3 H) 1.05 (s, 3 H) 1.17 (s, 3 H) 1.71 (s, 3 H) 0.88 - 2.10 (m, 24 H) 2.15 (dd, 1 H) 2.38 - 2.48 (m, 1 H) 2.53 (td, *J*=11.23, 5.14 Hz, 1 H) 3.18 (dd, *J*=12.92, 6.65 Hz, 1 H) 3.31 - 3.33 (m, 1 H) 3.38 - 3.49 (m, 1 H) 3.53 (dd, *J*=12.92, 4.39 Hz, 1 H) 4.23 (dd, *J*=7.78, 6.78 Hz, 1 H) 4.60 (s, 1 H) 4.73 (d, *J*=2.01 Hz, 1 H) 5.30 (dd, *J*=6.27, 1.51 Hz, 1 H) 7.22 (d, *J*=8.53 Hz, 2 H) 7.91 (d, *J*=8.28 Hz, 2 H) 8.13 (t, *J*=6.53 Hz, 1 H).

### EXAMPLE 138. Preparation of 4-((1R,3aS,5aR,5bR,7aR,11aS,11bR,13aR,13bR)-3a-(((S)-1-acetylpyrrolidine-2-carboxamido)methyl)-5a,5b,8,8,11a-pentamethyl-1-(prop-1-en-2-yl)-2,3,3a,4,5,5a,5b,6,7,7a,8,11,11 a,11b,12,13,13a,13b-octadecahydro-1H-cyclopenta[a]chrysen-9-yl)benzoic acid.

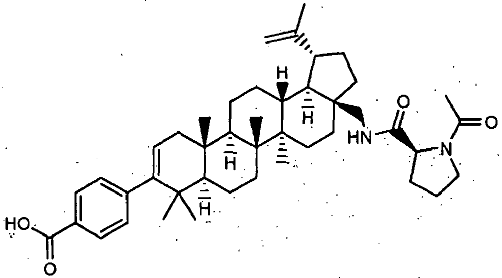

The title compound was prepared in 9 % yield following the general procedure described above for the reversed amides preparation, using (S)-1-acetylpyrrolidine-2-carboxylic acid as the reactant acid and acidic hydrolysis. MS: m/e 683.6 (MH⁺), 2.01 min (method 2). ¹H NMR (400 MHz, *MeOD*) δ ppm 0.94 (s, 3 H) 0.96 (s, 3 H) 1.03 (s, 3 H) 1.04 (s, 3 H) 1.18 (s, 3 H) 1.70 (s, 3 H) 2.08 (s, 3 H) 0.84 - 2.23. (m, 26 H) 2.147 - 2.56 (m, 1 H) 2.99 (dd, *J*=12.67, 4.89 Hz, 1 H) 3.52 - 3.70 (m, 3 H) 4.39 (dd, *J=*8.66, 3.89 Hz, 1 H) 4.59 (s, 1 H) 4.72 (d, *J*=2.26 Hz, 1 H) 5.29 (dd, *J*=5.90, 1.63 Hz, 1 H) 7.22 (d, *J*=8.53 Hz, 2 H) 7.91 (d, *J*=8.53 Hz, 2 H).

### EXAMPLE 139. Preparation of 4-((1R,3aS,5aR,5bR,7aR,11aS,11bR,13aR,13bR)-5a,5b,8,8,11a-pentamethyl-3a-((2-(methylamino)acetamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

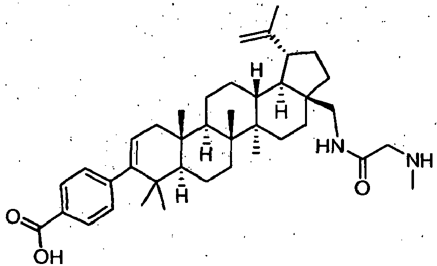

The title compound was prepared following the general procedures described above for the reversed amides preparation, using 2*-*(*tert-*butoxycarbonyl(methyl)amino)acetic acid as the reactant carboxylic acid and acidic hydrolysis. The product was isolated as a white solid (14 mg, 76 %). LCMS: m/e 615.5 (MH⁺), 2.68 min (method 3). ¹H NMR (500 MHz, *MeOD)* δ ppm 7.94 (2 H, d, *J*=8.5 Hz), 7.24 (2 H, d, *J*=8.2 Hz), 5.23 - 5.37 (1 H, m), 4.75 (1 H, br. s.), 4.62 (1 H, d, *J*=1.2 Hz), 3.81 (2 H, s), 3.61 (1 H, d, *J*=13.4 Hz), 3.11 (1 H, d, *J*=113.4 Hz), 2.74 (3H, s), 2.45 - 2.64 (1 H, m), 2.17 (1 H, dd, *J*= 17.2, 6.3 Hz), 2.02 - 2.12 (1H, m), 1. 81 - 1.99 (2 H, m), 1.65 - 1.81 (8 H, m), 1.58 (2 H, dd, *J*=9.3, 2.6 Hz), 1.45 - 1.54 (4 H, m), 1.34 - 1.45 (2 H, m), 1.30 (3 H, s), 1.18 (3 H, s), 1.09 - 1.16 (2 H, m), 1.07 (3 H; s), 1.05(3H,s), 1.00 (3 H, s), 0.97 (3 H, s).

### EXAMPLE 140. Preparation of 4-((1R,3aS,5aR,5bR,7aR,11aS,11bR,13aR,13bR)-3a-(((S)-2-amino-3-(1H-imidazol-4-yl)propanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

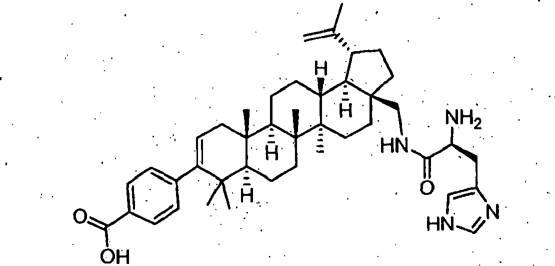

The title compound was prepared following the general procedures described above for the reversed amides preparation, using (S)-2-(*tert*-butoxycarbonylamino)-3-(1H-imidazol-4-yl) propanoic acid as the reactant carboxylic acid and acidic hydrolysis. The product was isolated as a white solid (11 mg, 64.2%). LCMS: m/e 681.5 (MH⁺), 2.59 min (method 3). ¹H NMR (400 MHz, *MeOD)* 8 ppm 8.88 (d, *J*=1.3 Hz, 1 H), 7.90 (d, *J*=8.6 Hz, 2 H), 7.43 (s, 1 H), 7.20 (d, *J*=8.3 Hz, 2 H), 5.19 - 5.36 (m, 1 H), 4.70 (s, 2 H), 4.58 (s, 1 H), 4.25 (t, *J*=7.1 Hz, 1 H), 3.61 (d, *J*=13.3 Hz, 1 H), 3.33 - 3.42 (m, 1 H), 3.26-3.30 (m, 1H), 2.97 (d, *J*=13.8 Hz, 1 H), 2.48 (d, *J*=5.3 Hz, 1 H), 2.13 (dd, *J*=17.1,6.0 Hz, 1 H), 1.89 - 2.08 (m, 1 H), 1.72 - 1.87 (m, 2 H), 1.63 - 1.72 (m, 5 H), 1.52 - 1.63 (m, 4 H), 1.50 (d, *J*=7.3 Hz, 1 H), 1.46 (d, *J*=13.6 Hz, 4 H), 1.37 (br. s., 2 H), 1.31 (d, *J*=3.3 Hz, 1 H), 1.20 - 1.29 (m, 3 H), 1.16 (s, 3 H), 0.96 - 1.07 (m, 6 H), 0.95 (s, 3 H), 0.92 (s, 3 H).

### EXAMPLE 141. Preparation of 4-((1R,3aS,5aR,5bR,7aR,11aS,11bR,13aR,13bR)-5a,5b,8,8,11a-pentamethyl-3a-((3-(piperazin-1-yl)propanamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

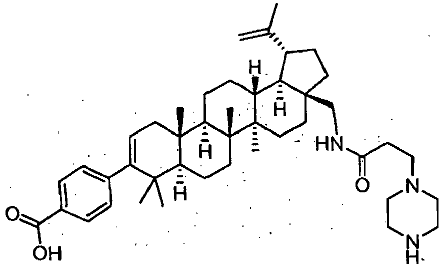

The title compound was prepared following the general procedures described above for the C28 reversed amide preparation and acidic hydrolysis using 3-(4-(*tert-*butoxycarbonyl)piperazin-1-yl) propanoic acid as the reactant carboxylic acid. The product was isolated as a white solid (17 mg, 77.0 %). LCMS: m/e 684.6 (MH⁺), 2.66 min (method 3). ¹H NMR (400 MHz, *MeOD)* 8 ppm 7.90 (d, *J*=8.3 Hz, 2 H), 7.20 (d, *J*=8.3 Hz, 2 H), 5.22 - 5.33 (m, 1 H), 4.70 (s, 1 H), 4.58 (s, 1 H), 3.49 - 3.62 (m, 1 H), 3.38 - 3.49 (m, 4 H), 3.30 - 3.36 (m, 4 H), 3.24 - 3.27 (m, 2 H), 3.02 (d, *J=13.6* Hz, 1 H), 2.68 (t, *J*=6.7 Hz, 2 H), 2.50 (td, *J*=11.1, 5.8 Hz, 1 H), 2.13 (dd, *J*=17.1, 6.5 Hz, 1 H), 2.05 (d, *J*=10.3 Hz, 1 H), 1.86 - 1.96 (m, 1 H), 1.76 - 1.86 (m, 1 H), 1.60 - 1.76 (m, 7 H), 1.55 (br. s., 2 H), 1.45 - 1.52 (m, 4 H), 1.30 - 1.42 (m, 2 H), 1.18 - 1.30 (m, 4 H), 1.14 (s, 3 H), 1.06 - 1.12 (m, 2 H), 1.03 (s, 3 H), 1.02 (s, 3 H), 0.95 (s, 3 H), 0.93 (s, 3 H).

### EXAMPLE 142. Preparation of 4-((1R,3aS,5aR,5bR,7aR,11aS,11bR,13aR,13bR)-3a-((3-(dimethylamino)propanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

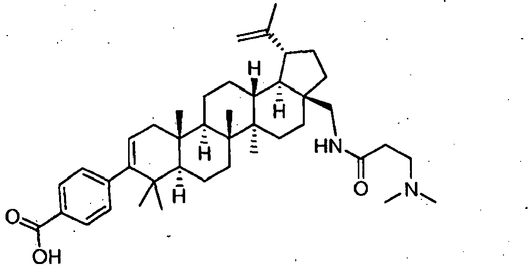

The title compound was prepared following the general procedures described above for the reversed amides preparation, using 3-(dimethylamino)propanoic acid as the reactant carboxylic acid and acidic hydrolysis. The product was isolated as a white solid (12 mg, 24.8 %). LCMS: m/e 643.5 (MH⁺), 2.34 min (method 3). ¹H NMR (400 MHz, *MeOD*) δ ppm 7.80 - 7.99 (m, 2 H), 7.20 (d, *J*=8.3 Hz, 2 H), 5.22 - 5.35 (m, 1 H), 4.70 (s, 1 H), 4.58 (s, 1 H), 3.55 (d, *J*=13.6 Hz, 1 H), 3.37 (t, *J*=6.5 Hz, 2 H), 2.95 - 3.11 (m, 1 H), 2.86 (s, 6 H), 2.68 - 2.81 (m, 2 H), 2.50 (td, *J*=11.1, 5.3 Hz, 1 H), 1.99 - 2.21 (m, 2 H), 1.76 - 1.99 (m, 2 H), 1.60 - 1.76 (m, 7 H), 1.54 (d, *J*=6.8 Hz, 2 H), 1.42 - 1.52 (m, 4 H), 1.31 - 1.42 (m, 2 H), 1.20 - 1.31 (m, 3 H), 1.11 - 1.20 (m, 4 H), 1.06 - 1.11 (m, 2 H), 1.03 (s, 3 H), 1.01 (s, 3 H), 0.95 (s, 3 H), 0.93 (s, 3 H).

### EXAMPLE 143. Preparation of 3-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylcarbamoyl)benzoic acid.

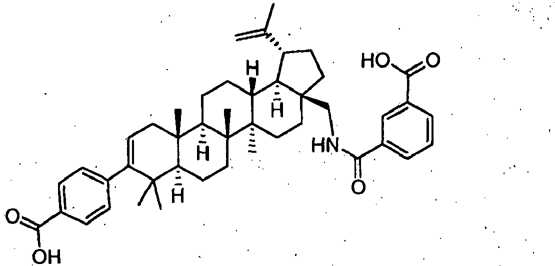

The title compound was prepared following the general procedures described above for the reversed amides preparation using 3-(methoxycarbonyl)benzoic acid as the reactant carboxylic acid and basic hydrolysis. The product was isolated as a white solid (22 mg, 46.0 %). LCMS: m/e 692.4 (MH⁺), 2.68 min (method 3). ¹H NMR (400 MHz, *CHLOROFORM-d)* δ ppm 8.43 (s, 1 H), 8.27 (d, *J*=7.6 Hz, 1 H), 8.12 (d, *J*=7.8 Hz, 1 H), 8.02 (d, *J*=8.3 Hz, 2 H), 7.62 (t, *J*=7.8 Hz, 1 H), 7.24 - 7.33 (m, 2 H), 6.06-6.25 (m, 1 H), 5.26 - 5.41 (m, 1 H), 4.77 (d, *J*=1.5 Hz, 1 H), 4.65 (s, 1 H), 4.00 (s, 3 H), 3.73 - 3.88 (m, 1 H), 3.28 - 3.41 (m, 1 H), 2.55 - 2.65 (m, 1 H), 2.04 - 2.25 (m, 2 H), 1.83 - 2.03 (m, 2 H), 1.64 - 1.82 (m, 6 H), 1.61 (s, 2 H), 1.51 (br. s., 4 H), 1.32 - 1.48 (m, 3 H), 1.23 - 1.32 (m, 2 H), 1.19 (s, 3 H), 1.12 (br. s., 1 H), 1.06 (s, 3 H), 1.02 (s, 3 H), 0.97 (s, 3 H), 0.96 (s, 3 H).

### EXAMPLE 144. Preparation of 4-((1R,3aS,5aR,5bR,7aR,11aS,11bR,13aR,13bR)-5a,5b,8,8,11a-pentamethyl-1-(prop-1-en-2-yl)-3a-((4-sulfamoylbenzamido)methyl)-2,3,3a,4,5,5a,5b,6,7,7a,8,11,11a,11b,12,13,13a,13b-octadecahydro-1H-cyclopenta[a]chrysen-9-yl)benzoic acid.

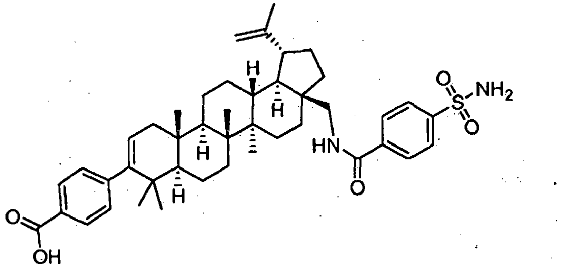

The title compound was prepared following the general procedures described above for the reversed amides preparation using 4-sulfamoylbenzoic acid as the reactant carboxylic acid and basic hydrolysis. The product was isolated as a white solid (35 mg, 53.6 %). LCMS: did not match desired product (method 3). ¹H NMR (400 MHz, *MeOD)* 8 ppm 7.82 - 7.97 (m, 6 H), 7.09 - 7.28 (m, 2 H), 5.29 (d, J=4.5 Hz, 1 H), 4.73 (d, *J*=1.8 Hz, 1 H), 4.60 (s, 1 H), 3.70 - 3.81 (m, 1 H), 3.12 - 3.26 (m, 1 H), 2.58 (td, *J*=11.1, 5.7 Hz, 1 H), 2.14 (dd, *J*=17.2, 6.4 Hz, 2 H), 1.85 - 2.00 (m, 1 H), 1.61-1.85 (m, 8 H), 1.34 - 1.59 (m, 8 H), 1.23 - 1.33 (m, 3 H), 1.21 (s, 3 H), 1.07 - 1.19 (m, 3 H), 1.05 (s, 3 H), 1.03 (s, 3 H), 0.95 (s, 3 H), 0.93 (s, 3 H).

### EXAMPLE 145. Preparation of 4-((1R,3aS,5aR,5bR,7aR,11aS,11bR,13aR,13bR)-3a-((((1,1-dioxido-4-thiomorpholinyl)acetyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

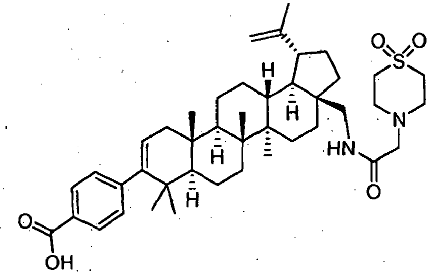

The title compound was prepared following the general procedures described above for the reversed amide preparation, using 4-thiomorpholineacetic acid 1,1-dioxide as the reactant carboxylic acid and basic hydrolysis. The product was isolated as a white solid (14 mg, 35.8 %). LCMS: m/e 719.3 (MH⁺) 2.40 min (method 3). ¹H NMR (500 MHz, *MeOD)* δ ppm 7.86 - 8.01 (m, 2 H), 7.24 (d, *J*=8.2 Hz, 2 H), 5.32 (d, *J*=6.4 Hz, 1 H), 4.75 (s, 1 H), 4.63 (s, 1 H), 3.58 - 3.71 (m, 3 H), 3.42 (d, *J*=4.3 Hz, 4 H), 3.33 - 3.38 (m, 4 H), 3.04 - 3.15 (m, 1 H), 2.55 (td, *J*=11.1, 5.6 Hz, 1 H), 2.13 - 2.23 (m, 2 H), 1.92 - 2.04 (m, 1 H), 1.81 - 1.92 (m, 1 H), 1.64 - 1.81 (m, 8 H), 1.46 - 1.64 (m, 6 H), 1.35 - 1.46 (m, 2 H), 1.24 - 1.35 (m, 2 H), 1.20 (s, 3 H), 1.14 - 1.19 (m, 1 H), 1.11 (d, *J*=10.4 Hz, 2 H), 1.07 (s, 3 H), 1.05 (s, 3 H), 0.99 (s, 3 H), 0.97 (s, 3 H).

### EXAMPLE 146. Preparation of 4-((1R,3aS,5aR,5bR,7aR,11aS,11bR,13aR,13bR)-3a-(((3-(1,1-dioxido-4-thiomorpholinyl)propanoyl)amino)methyl)-1-isopropenyl-5a,5b,8,8,11a-pentamethyl-2,3,3a,4,5,5a,5b,6,7,7a,8,11,11a,11b,12,13,13a,13b-octadecabydro-1H-cyclopent[a]chrysen-9-yl)benzoic acid.

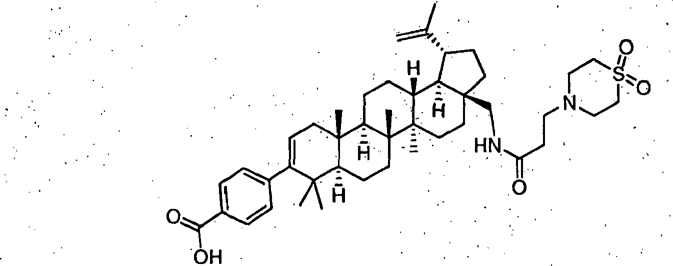

The title compound was prepared following the general procedures described above for the reversed amides preparation, using 3-(1,1-dioxo-λ16,4-thiazinan-4-yl) propanoic acid as the reactant carboxylic acid and basic hydrolysis. The product was isolated as a white solid (14 mg, 35.8 %). LCMS: m/e 733.3 (MH⁺), 2.33 min (method 3). ¹H NMR (500 MHz, *MeOD)* δ ppm 7.94 (d, *J*=8.2 Hz, 2 H), 7.24 (d, *J*=8.2 Hz, 2 H), 5.32 (d, *J*=4.9 Hz, 1 H), 4.74 (d, *J*=1.5 Hz, 1 H), 4.62 (s, 1 H), 3.77 (br. s., 1 H), 3.76 (d, *J*=6.1 Hz, 3 H), 3.58 (d, *J*=13.4 Hz, 1 H), 3.50 - 3.54 (m, 4 H), 3.48 (t, *J*=6.7 Hz, 2 H), 3.07 (d, *J*=13.7 Hz, 1 H), 2.77 (t, *J*=6.7 Hz, 2 H), 2.53 (td, *J*=11.2, 5.6 Hz, 1 H), 2.17 (dd, *J*=17.2, 6.3 Hz, 1 H), 2.03 - 2.13 (m, 1 H), 1.93 (td, *J*=13.6, 4.0 Hz, 1 H), 1.85 (td, *J=*12.2, 3.4 Hz, 1 H), 1.64 - 1.81 (m, 8 H), 1.46 - 1.64 (m, 6 H), 1.34 - 1.46 (m, 2 H), 1.22 - 1.34 (m, 3 H), 1.19 (s, 3 H), 1.10 - 1.12 (m, 2 H), 1.07 (s, 3 H), 1.05 (s, 3 H), 0.99 (s, 3 H), 0.97 (s, 3 H).

### EXAMPLE 147. Preparation of 4-((1R,3aS,SaR,5bR,7aR,11aS,11bR,13aR,13bR)-3a-((((1,1-dioxido-1,2-thiazinan-2-yl)acetyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

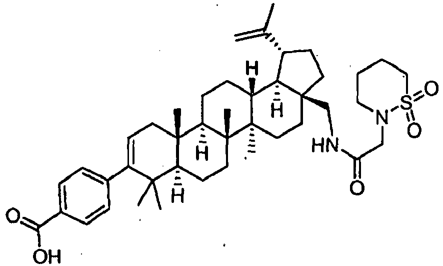

The title compound was prepared following the general procedures described above for the C28 reversed amide preparation using (1,2-dioxo-1,6-[1,2]thiazinan-2-yl) acetic acid as the reactant carboxylic acid and basic hydrolysis. The product was isolated as a white solid (30 mg, 34.8 %). LCMS: m/e 719.6 (MH⁺), 2.99 min (method 3). ¹HNMR (400 MHz, *CHLOROFORM-d)* δ ppm 7.98 (m, *J*=8.3 Hz, 2 H), 7.23 (m, *J*=8.3 Hz, 2 H), 6.60 (t, *J*=6.0 Hz, 1 H), 5.25 - 5.34 (m, 1 H), 4.73 (d, *J*=2.0 Hz, 1 H), 4.62 (s, 1 H), 3.85 - 3.95 (m, 2 H), 3.51 - 3.61 (m, 1 H), 3.37 - 3.51 (m, 2 H), 3.06 - 3.20 (m, 3 H), 2.51 (td, *J*=11.1, 5.6 Hz, 1 H), 2.19 - 2.33 (m, 2 H), 2.07 - 2.18 (m, 1 H), 2.04 (d, *J*=8.8 Hz, 1 H), 1.89 (br. s., 4 H), 1.81 (td, *J*=11.8,4.0 Hz, 2 H), 1.60 - 1.75 (m, 8 H), 1.50 - 1.60 (m, 2 H), 1.37 - 1.50 (m, 4 H), 1.16 - 1.37 (m, 4 H), 1.05 - 1.16 (m, 4 H), 1.02 (s, 3 H), 1.00 (s, 3 H), 0.91 - 0.97 (m, 6 H).

### EXAMPLE 148. Preparation of 4-((1R,3aS,5aR,5bR,7aR,11aS,11bR,13aR,13bR)-3a-((2-(dimethylamino)acetamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

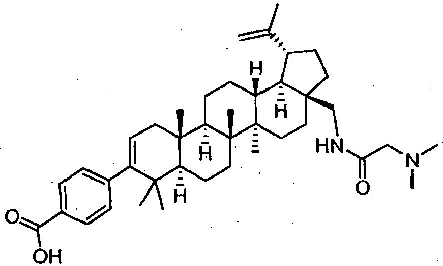

To a solution of 4-((1R,3aS,5aR,5bR,7aR,11aS,11bR,13aR,13bR)-5a,5b,8,8,11a-pentamethyl-3a-((2-(methylamino)acetamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid, example 139, (10 mg, 0.016 mmol) and formaldehyde (0.488 mg, 0.016 mmol) in MeOH (1 ml) was added acetic acid (1.860 µL, 0.033 mmol) and sodium cyanoborohydride (1.022 mg, 0.016 mmol). A clear solution was formed soon after mixing. The mixture was stirred at rt for 2 h, LC/MS showed the mass of the expected product. The mixture was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure to afford the title compound as a white solid (2 mg, 18.6 %). LCMS: m/e 629.4 (MH⁺), 2.72 min (method 3). ¹H NMR (400 MHz, *MeOD)* δ ppm 7.90 (d, *J*=8.6 Hz, 2 H), 7.20 (d, *J*=8.3 Hz, 2 H), 5.19 - 5.42 (m, 1 H), 4.72 (d, *J*=1.8 Hz, 1 H), 4.59 (s, 1 H), 3.93 (s, 2 H), 3.58 (br. s., 1 H), 3.04 - 3.17 (m, 1 H), 2.89 (s, 6 H), 2.46 - 2.60 (m, 1 H), 2.10 - 2.18 (m, 2 H), 1.80 (br. s., 2 H), 1.65 - 1.77 (m, 7 H), 1.63 (s, 1 H), 1.58 (br. s., 2 H), 1.38 - 1.55 (m, 4 H), 1.19 - 1.34 (m, 4 H), 1.13 - 1.19 (m, 4 H), 1.06 - 1.13 (m, 2 H), 1.04 (s, 3 H), 1.02 (s, 3 H), 0.95 (s, 3 H), 0.93 (s, 3 H).

### EXAMPLE 149. Preparation of 4-((1R,3aS,5aR,5bR,7aR,11aS,11bR,13aR,13bR)-3a-((dimethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

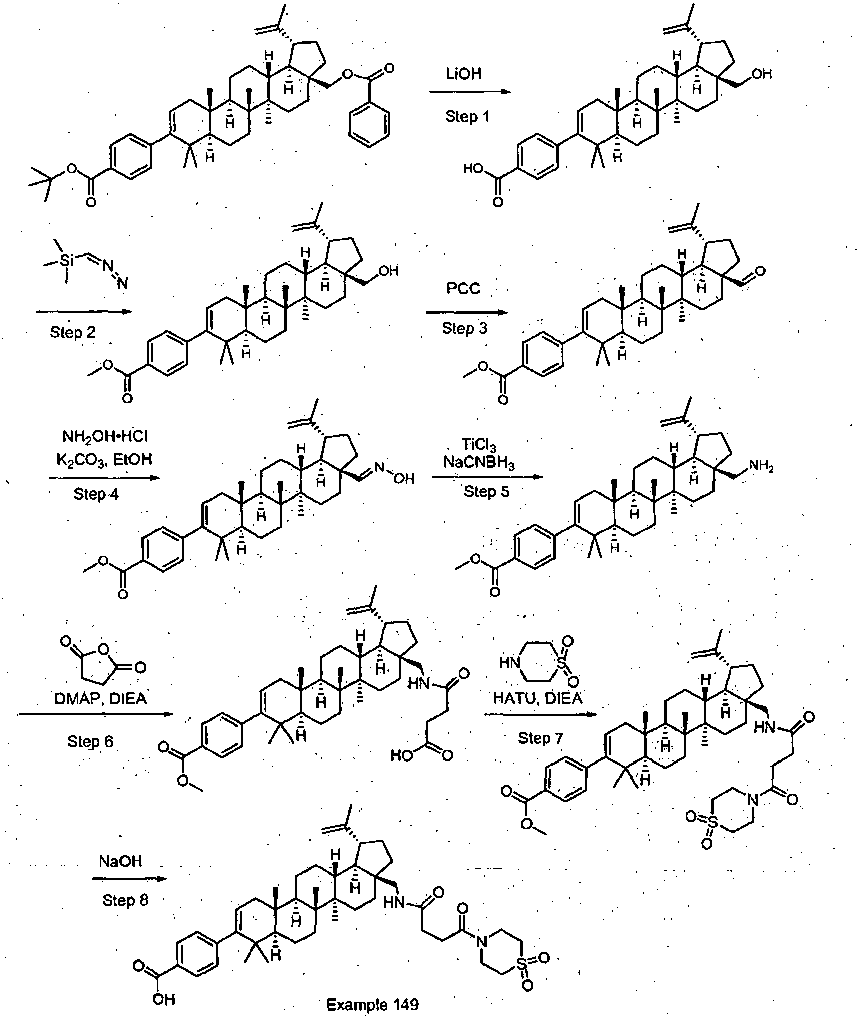

### Step 1. Ester hydrolysis

To a solution of *tert*-butyl 4-((1R,3aS,5aR,5bR,7aR,11aS,11bR,13aR,13bR)-3a-(benzoyloxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (7.2 g, 10.21 mmol) in 1,4-dioxane (75 ml) and water (25 ml) was added lithium hydroxide (1.285 g, 30.6 mmol) and the mixture was stirred at 75 °C. Dioxane (50 ml) was added to dissolve all solids and the stirring was continued for 24 h. The solvent was removed and the residue was redissolved into CH₂Cl₂, the insoluble white solid was collected to afford 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (2.2 g, 39.5%) LCMS: m/e 545.4 (MH⁺), 2.68 min (method 2).

### Step 2. Methyl ester formation

To a solution of 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (4.23 g, 10 mmol) in a mixture of CH₂Cl₂ (50 ml) and MeOH (5 ml) was added (trimethylsilyl)diazomethane (5.00 ml, 10.00 mmol). The solution was stirred at rt for 2 h under nitrogen. LC/MS showed no SM left. The reaction mixture was concentrated to afford methyl 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (4.37 g, 100%). The compound was used in the next step without further purification. LCMS: m/e 559.4 (MH⁺), 3.29 min (method 2). ¹H NMR (400 MHz, *CHLOROFORM-d)* δ ppm 7.95 (m, *J*=8.3 Hz, 2 H), 7.22 (m, *J*=8.6 Hz, 2 H), 5.22 - 5.39 (m, 1 H), 4.72 (d, *J=*2.0 Hz, 1 H), 4.49 - 4.67 (m, 1 H), 3.94 (s, 3 H), 3.87 (s, 1 H), 3.38 (d, *J*=10.6 Hz, 1 H), 2.37 - 2.60 (m, 1 H), 2.05 - 2.21 (m, 1 H), 1.96 (d, *J*=11.1 Hz, 2 H), 1.90 (d, *J*=13.1 Hz, 1 H), 1.61 - 1.82 (m, 7 H), 1.55 (br. s., 8 H), 1.37 - 1.52 (m, 2 H), 1.18 - 1.37 (m, 4 H), 1.08 - 1.18 (m, 3 H), 1.04 (s, 3 H), 1.00 (s, 3 H), 0.95 (s, 6 H).

### Step 3. Oxidation of C28 alcohol to aldehyde

To a solution of methyl 4-((1R,3aS,5aR,5bR,7aR,11aS,11bR,13aR,13bR)-3a-(hydroxymethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1 g, 1.789 mmol) in CH₂Cl₂ (70 ml) was added PCC (1.157 g, 5.37 mmol). The resulting dark brown mixture was stirred at rt for 4 h. TLC analysis showed the reaction was complete. The mixture was filtered through a short bed of Celite and silica gel, washed with excess of CH₂Cl₂. The filtrate was concentrated *in vacuo.* The crude mixture was purified by Biotage on normal phase silica gel. The fractions containing the expected product were combined and concentrated *in vacuo* to afford methyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate as a solid (0.8 g). LCMS: m/e 557.2 (MH⁺), 3.67 min (method 2).

### Step 4. Oxime formation

To a suspension of methyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a, 11b,12,13,13a,13b-octadecahydro-1H-cyclopenta[a]chrysen-9-yl)benzoate (800 mg, 1.437 mmol) in ethanol (60 ml) was added hydroxylamine hydrochloride (1298 mg, 18.68 mmol)) and potassium carbonate (2581 mg, 18.68 mmol). The resulting mixture was stirred at rt for 12 h. The reaction mixture was diluted with 17 ml of sat. NaHCO₃ solution and was extracted with dichloromethane (3 x 20 ml). The combined organic layers were dried over Na₂SO₄. The drying agent was removed by filtration and the filtrate was concentrated under reduced pressure. The crude product was used in the next step with no additional purification. LCMS: m/e 572.3,(MH⁺), 3.26 min (method 2).

### Step 5. Oxime reduction

To the solution of methyl 4-((1R,3aS,5aR,5bR,7aR,11aS,11bR,13aR,13bR)-3a-((hydroxyimino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (800 mg, 1.399 mmol), in ethanol (40 ml) was added excess of ammonium acetate (1078 mg, 13.99 mmol) and sodium cyanoborohydride (879 mg, 13.99 mmol) . The mixture was stirred in an ice bath until cold. To this suspension was added an aqueous solution of titanium(III) chloride (Aldrich Chemicals, 20 % solution used as supplied, 10 ml, 1.963 mmol). The resulting mixture was blanketed with nitrogen, ice bath was removed and stirring continued at rt for an hour. LCMS showed the reaction was complete. At this point, the mixture was dark greenish-blue. A solution of sodium hydroxide, (3 ml, 10N) in 25 ml water was added into the reaction mixture, along with 30 ml of methylene chloride. The mixture was stirred vigorously until the dark blue phase was floating on top of the organic phase. The mixture was filtered through a plug of paper cellulose, the filtrate was clear. The organic layer was collected and the aqueous layer was extracted with CH₂Cl₂ (2 X 20 ml). The combined organic phase was dried over Na₂SO₄. The solvent was removed *in vacuo* to afford methyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate as an off-white solid (∼800 mg). The crude material was used in the next step without further purification. LCMS: m/e 558.5 (MH⁺), 2.53 min (method 2).

### Step 6. Acylation of amine

To a solution of methyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (30 mg, 0.054 mmol) in DCM (2 ml) was added dihydrofuran-2,5-dione (16.15 mg, 0.161 mmol) followed by DMAP (6.57 mg, 0.054 mmol) and DIPEA (9.39 µl, 0.054 mmol). The mixture was stirred at rt for 18 hours. The solvent was removed *in vacuo* and the resulting residue containing 4-(((1R,3as,5aR,5bR,7aR,11aS,11bR,13aR,13bR)-9-(4-(methoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)-4-oxobutanoic acid was used in next step without further purification. LCMS: m/e 658.5 (MH⁺), 3.27 min (method 3).

### Step 7. Amide coupling

To a solution of 4-(((1R,3aS,5aR,5bR,7aR,11aS,11bR,13aR,13bR)-9-(4-(methoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methylamino)-4-oxobutanoic acid (35.4 mg, 0.054 mmol) in DCM (3 ml) at 0 °C was added thiomorpholine-1,1-dioxide (7:27 mg, 0.054 mmol), HATU (40.9 mg, 0.108 mmol) followed by *N-*ethyl-*N-*isopropylpropan-2-amine (20.86 mg, 0.161 mmol) . The resulting solution was stirred at rt for 18 h. Solvent was removed *in vacuo,* the resulting solid was used in the next step without further purification. LCMS: m/e 775.5 (MH⁺), 2.95 min (method 3).

### Step 8. Saponification of benzoate ester

To a solution of the material from Step 7 (40 mg, 52 mmol) in dioxane (1.5 ml) was added sodium hydroxide (0.5 ml, 1 N, 500 mmol) .The resulting solution was stirred at 63 °C for 12 h. The solvent was removed *in vacuo* and the resulting residue was purified by prep. HPLC. The product was isolated as a white solid (26 mg, 62.9 %). LCMS: m/e 761.6 (MH⁺), 2.57 min (method 3). ¹H NMR (400 MHz, *MeOD)* 8 ppm 7.94 (m, *J=8.3* Hz, 2 H), 7.24 (m, *J*=8.5 Hz, 2 H), 5.25 - 5.38 (m, 1 H), 4.74 (d, *J=*2.0 Hz, 1 H), 4.62 (s, 1 H), 3.98 - 4.12 (m, 4 H), 3.56 (s, 1 H), 3.28 (br. s., 2 H), 3.07 - 3.22 (m, 2 H), 3.01 (d, *J*=13.6 Hz, 1 H), 2.69 - 2.83 (m, 2 H), 2.46 - 2.65 (m, 3 H), 2.17 (dd, *J*=17.2, 6.4 Hz, 2 H), 1.84 (d, *J*=12.5 Hz, 2 H), 1.64 - 1.81 (m, 8 H), 1.45 - 1.64 (m, 6 H), 1.39 (br. s., 2 H), 1.22 - 1.33 (m, 4 H), 1.12 - 1.22 (m, 4 H), 1.06 (d, *J*=3.5 Hz, 6 H), 0.87 - 1.04 (m, 6 H).

### EXAMPLE 150. Preparation of 4-((1R,3aS,5aR,5bR,7aR,11aS,11bR,13aR,13bR)-5a,5b,8,8,11a-pentamethyl-3a-(((S)-1-methylpyrrolidine-2-carboxamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

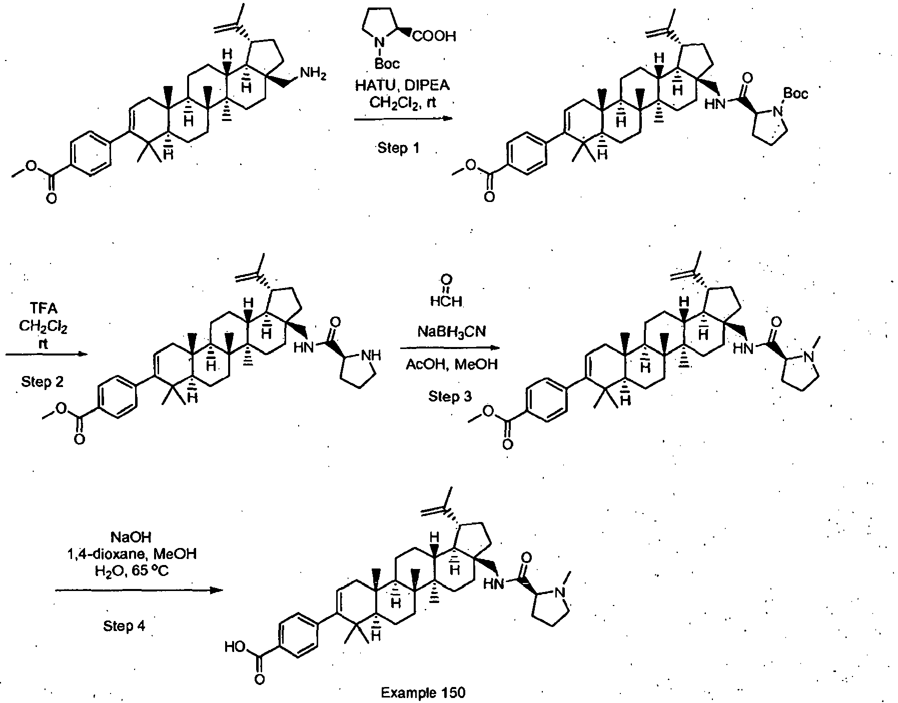

### Step 1: Preparation of the C28 amide

To a mixture of methyl 4-((1R,3aS,5aR,5bR,7aR,11aS,11bR,13aR,13bR)-3a-(aminomethyl)-5a,5b,8,8,11a pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (25 mg, 0.045 mmol), (S)*-*1*-*(*tert-*butoxycarbonyl)pyrrolidine-2-carboxylic acid (11.58 mg, 0.054 mmol) in DCM (1 ml) was added DIPEA (29 mg, 0.224 mmol) followed by HATU (25.6 mg, 0.067 mmol). The resulting solution was stirred at rt for 1 h. The reaction mixture was concentrated *in vacuo* to give crude product without further purification. MS: m/e 755.7 (MH⁺), 2.84 min (method 2).

### Step 2: Deprotection of the amine

To a solution of crude material from Step 1 in DCM (2 ml) was added TFA (0.3 ml, 3.89 mmol). The mixture was stirred at rt for 2 h. The mixture was concentrated *in vacuo* to give crude product which was used in the next step without further purification. MS: m/e 655.6 (MH+), 1.96 min (method 2).

### Step 3: Alkylation of the amine

To a solution of crude material from Step 2 in methanol (2 ml) was added formaldehyde (37% in H₂O (6.9 mg, 0.086 mmol) and acetic acid (4.9 µl, 0.086 mmol). The resulting mixture was stirred at rt for 30 min. Sodium cyanoborohydride (5.4 mg, 0.086 mmol) was added and the mixture was stirred at rt for 3 h. The reaction mixture was concentrated *in vacuo* to give crude product without further purification. MS: m/e 669.7 (MH+), 1.97 min (method 2).

### Step 4: Preparation of the benzoic acid.

To a solution of crude material from Step 3 in 1,4-dioxane (1 ml) and methanol (0.5 ml) was added 1N sodium hydroxide (0.5 ml). The resulting solution was stirred at 65°C for 2 h. The crude product was purified by prep. HPLC (YMC Combiprep ODS 30x50 mm S5) (MeOH / H₂O /TFA) to give 4-((1R,3aS,5aR,5bR,7aR,11aS,11bR,13aR,13bR)-5a,5b,8,8,11a-pentamethyl-3a-(((S-1-methylpyrrolidine-2-carboxamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid as a solid (13 mg, 44%, 4 steps). MS: m/e 655.7 (MH⁺), 1.73 min (method 2). ¹H NMR (400 MHz, *MeOD)* 8 ppm 0.94 (s, 3 H) 0.96 (s, 3 H) 1.03 (s, 3 H) 1.04 (s, 3 H) 1.18 (s, 3 H) 1.71 (s, 3 H) 0.86 - 2.25 (m, 24 H) 2.14 (dd, *J*=17.19, 6.40 Hz, 1 H) 2.47 - 2.63 (m, 2 H) 2.91 (s, 3 H) 3.03 (dd, *J*=13.55, 4.77 Hz, 1 H) 3.20 (dt, *J*=11.23, 8.44 Hz, 1 H) 3.66 - 3.76 (m, 2 H) 4.04 (t, *J*=8.16 Hz, 1 H) 4.61 (s, 1 H) 4.73 (d, *J*=2.01 Hz, 1 H) 5.29 (dd, J=6.15, 1.63 Hz, 1 H) 7.21 (d, *J*=8.53 Hz, 2 H) 7.91 (d, *J*=8.53 Hz, 2 H) 8.23 (t, *J*=6.15 Hz, 1 H).

### EXAMPLE 151. Preparation of 4-((1R,3aS,5aR,5bR,7aR,11aS,11bR,13aR,13bR)-5a,5b,8,8,11a-pentamethyl-3a-(((R)-1-methylpyrrolidine-2-carboxamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

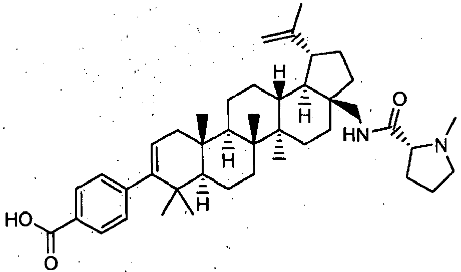

The title compound was prepared in 37 % yield following the procedure described above in the preparation of 4-((1R,3aS,5aR,5bR,7aR,11aS,11bR,13aR,13bR)-5a,5b,8,8,11a-pentamethyl-3a-(((S)-1-methylpyrrolidine-2-carboxamido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid, example 150, using (R)-1-methylpyrrolidine-2-carboxylic acid as the reactant acid. MS: m/e 655.7 (MH⁺), 1.73 min (method 2). ¹H NMR (400 MHz, *MeOD)* δ ppm 0.94 (s, 3 H) 0.96 (s, 3 H) 1.03 (s, 3 H) 1.05 (s, 3 H) 1.17 (s, 3 H) 1.71 (s, 3 H) 0.85 - 2.25 (m, 24 H) 2.14 (dd, *J*=17.07, 6.27 Hz, 1 H) 2.48 - 2.62 (m, 2 H) 2.90 (s, 3 H) 3.15 - 3.25 (m, 2 H) 3.50 - 3.57 (m, 1 H) 3.67 - 3.76 (m, 1 H) 4.04 (t, *J*=8.16 Hz, 1 H) 4.61 (s, 1 H) 4.73 (d, *J*=2.01 Hz, 1 H) 5.29 (dd, *J*=6.15, 1.63 Hz, 1 H) 7.21 (d, *J*=8,28 Hz, 2 H) 7.91 (d, *J*=8.28 Hz, 2 H) 8.24 (t, *J*=5.90 Hz, 1 H). ,

### EXAMPLE 152. Preparation of 4-((1R,3aS,5aR,5bR,7aR,11aS,11bR,13aR,13bR)-3a-((3-(2-(dimethylamino)ethylamino)-2,2-difluoro-3-oxopropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

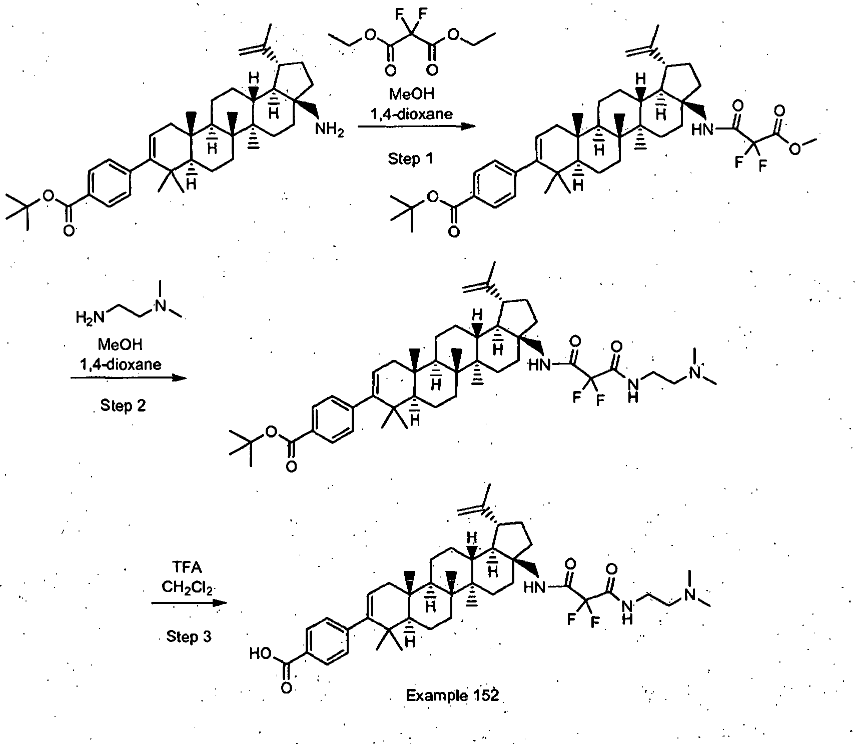

### Step 1: Preparation of the C28 reversed amide

To a solution of *tert*-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (200 mg, 0.333 mmol) in methanol (5 ml) and 1,4-dioxane (5 ml) was added diethyl 2,2-difluoromalonate (654 mg, 3.33 mmol). The resulting solution was stirred at rt for 6 days. The reaction mixture was concentrated *in vacuo.* The crude product was purified by Biotage (Thomson 25 g silica gel column; 4:1 Hex/EtOAc) to give 116 mg (47 %) of product. MS: m/e 736.6 (MH⁺), 2.86 min (method 2). ¹H NMR (400 MHz, *CHLOROFORM-d)* δ ppm 0.93 (s, 6 H) 0.99 (s, 3 H) 1.02 (s, 3 H) 1.13 (s, 3 H) 1.60 (s, 9 H) 1.71 (s, 3 H) 0.85 - 1.83 (20 H) 1.98 - 2.10 (m, 1 H) 2.10 (dd, *J*=17.07, 6.27 Hz, 1 H) 2.49 (td, *J*=11.04, 5.27 Hz, 1 H) 3.16 (dd, *J*=13.55, 6.27 Hz, 1 H) 3.65 (dd, *J*=14.43, 7.15 Hz, 1 H) 3.95 (s, 3 H) 4.61 - 4.65 (m, 1 H) 4.73 (d, *J*=1.76 Hz, 1 H) 5.28 (dd, *J*=6.15, 1.63 Hz, 1 H) 6.28 (t, *J*=7.03 Hz, 1 H) 7.18 (d, *J*=8.53 Hz, 2 H) 7.89 (d, *J*=8.28 Hz, 2 H). ¹⁹F NMR (376 MHz, *CHLOROFORM-d)* δ ppm -112.49 (s, 2 F).

### Step 2: Preparation of the amide end cap

To a solution of the amide from Step 1 (30 mg, 0.041 mmol) in methanol (1 ml) and 1,4-dioxane (1 ml) was added *N*1,*N*1-dimethylethane-1,2-diamine (17.97 mg, 0.204 mmol). The resulting solution was stirred at rt for 3 days. The reaction mixture was concentrated *in vacuo* to give crude product without further purification. MS: m/e 792.7 (MH⁺), 2.06 min (method 2).

### Step 3: Preparation of the benzoic acid

To a solution of crude material from Step 2 (20 mg, 0.025 mmol) in DCM (5 ml) was added TFA (0.5 ml, 6.49 mmol). The mixture was stirred at rt for 2 h. The mixture was concentrated *in vacuo* and the crude product was purified by prep. HPLC (YMC Combiprep ODS 30x50 mm S5, MeOH / H₂O /TFA) to afford 4-((1R,3aS,5aR,5bR,7aR,11as,11bR,13aR,13bR)-3a-((3-(2-(dimethylamino)ethylamino)-2,2-difluoro-3-oxopropanamido)methyl)-5a,5b,8,8,11 a-pentamethyl-1-(prop-1-en-2-yl)-2,3,3 a,4,5,5a,5b,6,7,7a,8,11,11a,11b,12,13,13a,13b-octadecahydro-1H-cyclopenta[a]chrysen-9-yl)benzoic acid (18 mg, 96%). MS: m/e 736.6 (MH⁺), 1.79 min (Method 2). ¹H NMR (400 MHz, *MeOD)* δ ppm 0.94 (s, 3 H) 0.96 (s, 3 H) 1.03 (s, 3 H) 1.05 (s, 3 H) 1.18 (s, 3 H) 1.71 (s, 3 H) 0.84 - 1.88 (m, 20 H) 1.97 - 2.20 (m, 2 H) 2.15 (dd, *J*=17.44, 6.65 Hz, 1 H) 2.53 (td, *J*=10.98, 5.40 Hz, 1 H) 2.96 (s, 6 H) 3.09 (d, *J*=13.05 Hz, 1 H) 3.32 - 3.36 (m, 1 H) 3.61 - 3.71 (m, 3 H) 4.59 - 4.62 (m, 1 H) 4.73 (d, *J*=2.01 Hz, 1 H) 5.30 (dd, *J*=6.15, 1.63 Hz, 1 H) 7.22 (d, *J*=8.53 Hz, 2 H) 7.91 (d, *J*=8.53 Hz, 2 H). ¹⁹F NMR (376 MHz, *MeOD)* 8 ppm - 114.91 (d, *J*=17.34 Hz, 2 F).

### EXAMPLE 153. Preparation of 4-((1R,3aS,5aR,5bR,7aR,11aS,11bR,13aR,13bR)-3a-((2-carboxy-2,2-difluoroacetamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

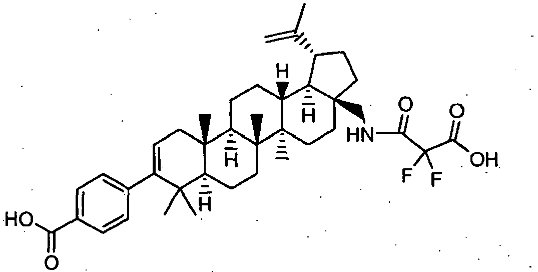

To a solution of the product resulting from Step 1 in example 152, *tert*-butyl 4-((1R,3aS,5aR,5bR,7aR,11aS,11bR,13aR,13bR)-3a-((2,2-difluoro-3-methoxy-3-oxopropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (20 mg, 0.027 mmol) in 1,4-dioxane (1 ml) and methanol (0.5 ml) was added IN sodium hydroxide (0.5 ml, 0.500 mmol). The resulting solution was stirred at 65 °C for 2 h. The crude product was purified by prep. HPLC (YMC Combiprcp ODS 30 x 50 mm S5) (MeOH / H₂O /TFA) to give the title compound as a solid (2 mg, 9 %). MS: m/e 666.5 (MH⁺), 1.96 min (method 2). ¹H NMR (400 MHz, *MeOD)* δ ppm 0.94 (s, 3 H) 0.96 (s, 3 H) 1.03 (s, 3 H) 1.04 (s, 3 H) 1.18 (s, 3 H) 1.71 (s, 3 H) 0.86 - 1.89 (m, 20 H) 1.93 - 2.05 (m, 1 H) 2.14 (dd, *J*=17.07, 6.27 Hz, 1 H) 2.53 (td, *J*=11.29, 5.52 Hz, 1 H) 3.09 (d, *J=13.55* Hz, 1 H) 3.62 (d, *J*=14.05 Hz, 1 H) 4.58 - 4.61 (m, 1 H) 4.73 (d, *J*=1.76. Hz, 1 H) 5.29 (dd, *J*=6.02, 1.51 Hz, 1 H) .7.22 (d, *J*=8.53 Hz, 2 H) 7.91 (d, *J*=8.28 Hz, 2 H). ¹⁹F NMR (376 MHz, *MeOD)* δ ppm -112.65 (s, 2 F).

### General procedures for the preparation of C28 urea derivatives

### Synthetic route 1:

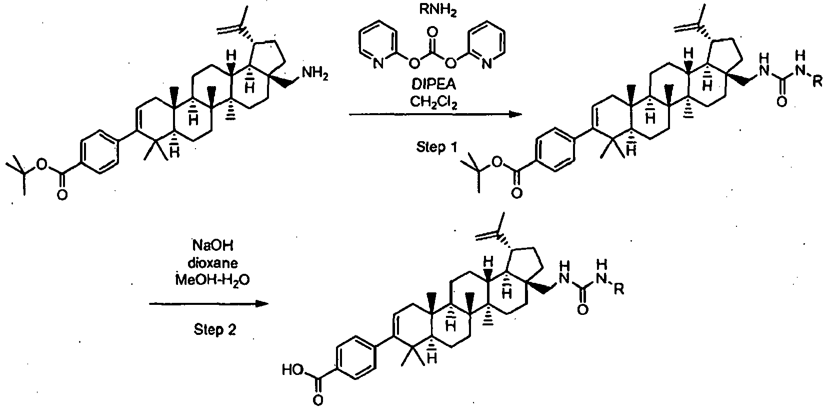

### Step 1: Preparation of ureas

To a solution of *tert-butyl* 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (100 mg, 0.167 mmol) in DCM (5 ml) at 0 °C was added dipyridin-2-yl carbonate (43.2 mg, 0.200 mmol) followed by DIPEA (0.070 ml, 0.400 mmol). The resulting solution was stirred at rt for 2 h. The corresponding amine (1.2 eq.) was added followed by DIPEA (3 eq.). The mixture was stirred for 18 h. The solvent was removed *in vacuo* and the resulting crude product was used without further purification.

### Step 2. Preparation of benzoic acids

To the solution of the urea resulting from Step 1 in dioxane (2.0 ml) and methanol (2.0 ml) was added sodium hydroxide (5 eq.) and H₂O (0.5 ml). The resulting solution was stirred at 70 °C for 5 - 10 h. The solvent was removed *in vacuo* and the crude product was purified by prep. HPLC to give the desired benzoic acids.

### Synthetic route 2:

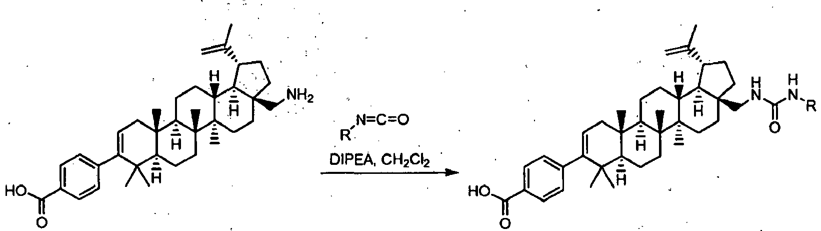

To a solution of 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (1 eq.) and the corresponding isocyanate (2 eq.) in DCM (8 ml) at 0 °C was added DIPEA (3 eq.). The resulting mixture was stirred at rt for 18 h. The solvent was evaporated and the crude product was purified by prep. HPLC to give the desired benzoic acids.

### EXAMPLE 154. 4-((1R,3aS,5aR,5bR,7aR,11aS,11bR,13aR,13bR)-5a,5b,8,8,11a-pentamethyl-3a-((3-(2-(2-oxopyrrolidin-1 -yl)ethyl)ureido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

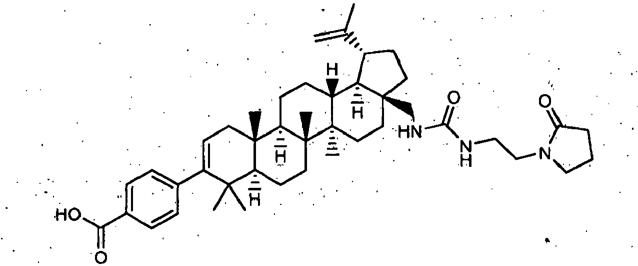

The title compound was prepared in 19 % yield following the synthetic route 1 described above, using 1-(2-aminoethyl)pyrrolidin-2-one oxalate as the reactant amine. MS: m/e 698.3 (MH⁺), 1.77 min (method 2). ¹H NMR (400 MHz, *MeOD)* δ ppm 0.94 (s, 3 H) 0.96 (s, 3 H) 1.03 (s, 3 H) 1.04 (s, 3 H) 1.17 (s, 3 H) 1.70 (s, 3 H) 0.86 - 1.92 (m, 20 H) 2.03 (quin, *J*=7.59 Hz, 3 H) 2.14 (dd, *J*=17.19, 6.40 Hz, 1 H) 2.35 (t, *J*=8.16 Hz, 2 H) 2.51 (td, *J*=10.98, 5.40 Hz, 1 H) 2.93 (d, *J*=13.05 Hz, 1 H) 3.31 - 3.37 (m, 4 H) 3.43 (d, *J*=13.05 Hz, 1 H) 3.51 (t, *J*=7.03 Hz, 2 H) 4.56 - 4.62 (m, 1 H) 4.71 (d, *J*=2.26 Hz, 1 H) 5.29 (dd, *J=*6.27, 1.76 Hz, 1 H) 7.22 (d, *J*=8.53 Hz, 2 H) 7.91 (d, *J*=8.53 Hz, 2 H).

### EXAMPLE 155. 4-((1R,3aS,5aR,5bR,7aR,11aS,11bR,13aR,13bR)-5a,5b,8,8,11a-pentamethyl-3a-((3-(3-(2-oxopyrrolidin-1-yl)propyl)ureido)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

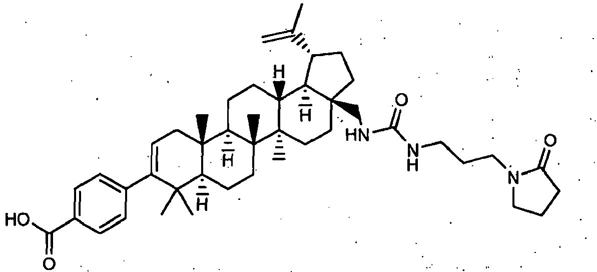

The title compound was prepared in 32 % yield following the synthetic route 1 described above, using 1-(3-aminopropyl)pyrrolidin-2-one as the reactant amine. MS: m/e 712.3 (MH⁺), 1. 85 min (method 2). ¹H NMR (400 MHz, *MeOD)* 8 ppm 0.94 (s, 3 H) 0.96 (s, 3 H) 1.03 (s, 3 H) 1.04 (s, 3 H) 1.18 (s, 3 H) 1.67 - 1.72 (m, 3 H) 0.87-1.93 (m, 22 H) 2.04 (quin, *J*=7.65 Hz, 3 H) 2.14 (dd, *J*=16.94, 6.40 Hz, 1 H) 2.38 (t, *J*=8.03 Hz, 2 H) 2.51 (td, *J*=11.04, 5.02 Hz, 1 H) 2.95 (d, *J=13.55* Hz, 1 H) 3.11 (t, *J=6.65* Hz, 2 H) 3.32 - 3.44 (m, 3 H) 3.46 (t, *J*=7.03 Hz, 2 H) 4.57 - 4.61 (m, 1 H) 4.71 (d, *J*=2.01 Hz, 1 H) 5.29 (dd, *J*=6.27, 1.51 Hz, 1 H) 7.21 (d, *J*=8.28 Hz, 2 H) 7.91 (d, *J*=8.28 Hz, 2 H).

### EXAMPLE 156. Preparation of 4-((1R,3aS,5aR,5bR,7aR,11aS,11bR,13aR,13bR)-3a-((3-(2-ethoxy-2-oxoethyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

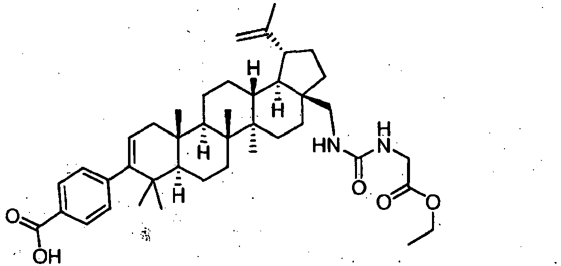

The title compound was prepared following the synthetic route 2 described above using ethyl 2-isocyanatoacetate as the reactant isocyanate, the product was isolated as a white solid (5 mg, 40.4 %). LCMS: m/e 673.5 (MH⁺), 2.90 min (method 3). ¹H NMR (400 MHz, *MeOD)* δ ppm 7.90 (d, *J*=8.3 Hz, 2 H), 7.20 (d, *J*=8.3 Hz, 2 H), 5.15 - 5.36 (m, 1 H), 4.70 (s, 1 H), 4.57 (s, 1 H), 4.16 (q, *J*=7.1 Hz, 2 H), 3.44 (d, *J*=13.6 Hz, 1 H), 3.29 (dt, *J*=3.3, 1.6 Hz, 2 H), 2.95 (d, *J*=13.6 Hz, 1 H), 2.50 (td, *J*=11.1, 5.5 Hz, 1 H), 2.12 (dd, *J*=17.2, 6.4 Hz, 1 H), 1.94 - 2.08 (m, 1 H), 1.76 - 1.94 (m, 3 H), 1.60 - 1.76 (m, 8 H), 1.41 - 1.60 (m, 6 H), 1.28 - 1.41 (m, 2 H), 1.20 - 1.28 (m, 5 H), 1.10 - 1.20 (m, 4 H), 1.05 - 1.10 (m, 1 H), 1.02 (s, 3 H), 1.01 (s, 3 H), 0.94 (s, 3 H), 0.89 (s, 3 H).

### EXAMPLE 157. Preparation of 4-((1R,3aS,5aR,5bR,7aR,11aS,11bR,13aR,13bR)-3a-((3-(carboxymethyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

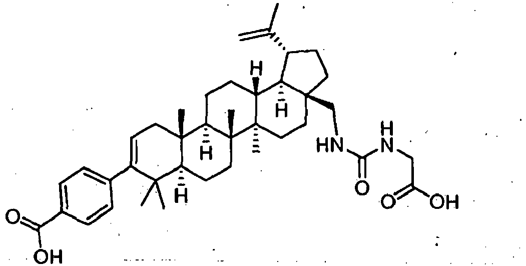

4-((1R,3aS,5aR,5bR,7aR,11aS,11bR,13aR,13bR)-3a-((3-(2-ethoxy-2-oxoethyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (7 mg, 10.40 µmol) was dissolved in dioxane (2 ml). Sodium hydroxide solution, IN (0.021 ml, 0.021 mmol) was added. The mixture was stirred at 70 °C for 2 h. The solvent was evaporated and the residue was purified by prep. HPLC to afford the title compound as a white solid (4 mg, 32.8 %). LCMS: m/e 645.3 (MH⁺), 2.58 min (method 3). ¹H NMR (400 MHz, *MeOD)* δ ppm 7.90 (m, *J*=8.3 Hz, 2 H), 7.20 (m, *J*=8.3 Hz, 2 H), 5.21 - 5.36 (m, 1 H), 4.70 (d, *J*=1.8 Hz, 1 H), 4.57 (s, 1 H), 3.83 (s, 2 H), 3.44 (d, *J*=13.8 Hz, 1 H), 2.95 (d, *J*=13.6 Hz, 1 H), 2.38 - 2.60 (m, 1 H), 2.13 (dd, *J*=17.1, 6.5 Hz, 1 H), 1.93 - 2.08 (m, 1 H), 1.76-1.93 (m, 2 H), 1.60 - 1.76 (m, 8 H), 1.40 - 1.60 (m, 6 H), 1.35 - 1.40 (m, 1 H), 1.29 - 1.35 (m, 1 H), 1.18 - 1.29 (m, 3 H), 1.16 (s, 3 H), 1.05 - 1.13 (m, 2 H), 1.03 (s, 3 H), 1.02 (s, 3 H), 0.97 (s, 3 H), 0.92 (s, 3 H).

### EXAMPLE 158. Preparation of 4-((1R,3aS,5aR,5bR,7aR,11aS,11bR,13aR,13bR)-3a-((3-(2-methoxy-2-oxoethyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadeeahydro-1H-cyclopenta[a]chrysen-9-yl)benzoic acid.

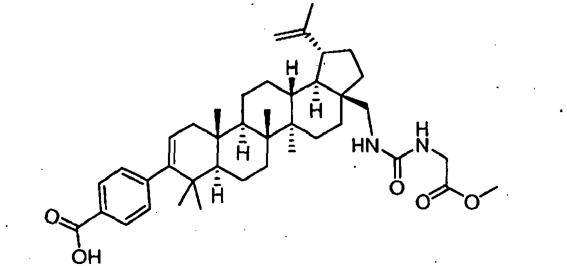

4-((1R,3aS,5aR,5bR,7aR,11aS,11bR,13aR,13bR)-3a-((3-(2-ethoxy-2-oxoethyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (7 mg, 10.40 µmol) was dissolved in dioxane (2 ml) and methanol (1 ml). A solution of sodium hydroxide (IN, 0.021 ml, 0.021 mmol) was added. The mixture was stirred at 70 °C for 2 h. The solvent was removed under reduced pressure and the residue was purified by prep. HPLC to afford the title compound as a white solid (2 mg, 29.2 %). LCMS: m/e 659.4 (MH⁺), 2.84 min (method 3). ¹H NMR (500 MHz, *MeOD)* 8 ppm 7.86 (d, *J*=8.2 Hz, 2 H), 7.14 (d, *J*=7.9 Hz, 2 H), 5.23 - 5.38 (m, 1 H), 4.74 (s, 1 H); 4.62 (s, 1 H), 3.91 (s, 2 H), 3.71 - 3.78 (m, 3 H), 2.95 - 3.10 (m, 1 H), 2.46 - 2.62 (m, 1 H), 2.12 - 2.25 (m, 1 H), 2.05 (s, 1 H), 1.99 - 2.03 (m, 1 H), 1.91 - 1.99 (m, 6 H), 1.89 (br. s., 2 H), 1.76 - 1.83 (m, 2 H), 1.71 - 1.76 (m, 4 H), 1.69 (br. s., 1 H), 1.48 (br. s., 4 H), 1.31 (br. s., 4 H); 1.19 (s, 3 H), 1.07 (br. s., 3 H), 1.05 (s, 3 H), 0.97 (s, 3 H), 0.96 (s, 3 H).

### EXAMPLE 159. Preparation of 4-((1R,3aS,5aR,5bR,7aR,11aS,11bR,13aR,13bR)-5a,5b,8,8,11a-pentamethyl-1-(prop-1-en-2-yl)-3a-((3-pyridin-3-ylureido)methyl)-2,3,3a,4,5,5a,5b,6,7,7a,8,11,11a,11b,12,13,13a,13b-octadecahydro-1H-cyclopenta[a]chrysen-9-yl)benzoic acid.

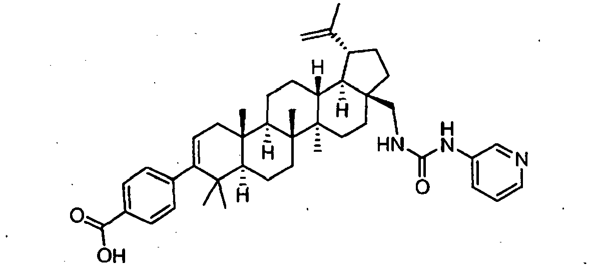

The title compound was prepared following the synthetic route 2 described above, using 3-isocyanatopyridine as the reactant isocyanate. The product was isolated as a white solid (5 mg, 38.9 %). LCMS: m/e 664.5 (MH⁺), 2.80 min (method 3). ¹H NMR (400 MHz, *MeOD)* δ ppm 9.22 (d, *J*=2.5 Hz, 1 H), 8.35 (d, *J*=5.3 Hz, 1 H), 8.13 - 8.28 (m, 1 H), 7.76 - 7.99 (m, 3 H), 7.20 (d, *J*=8.3 Hz, 2 H), 5.23 - 5.37 (m, 1 H), 4.72 (d, *J*=2.0 Hz, 1 H), 4.60 (s, 1 H), 3.56 (s, 1 H), 3.04 (d, *J*=13.3 Hz, 1 H), 2.45 - 2.64 (m, 1 H), 2.10 - 2.20 (m, 2 H), 1.84 (br. s., 2 H), 1.62 - 1.79 (m, 8 H), 1.56 (br. s., 2 H), 1.47 (d, *J*=10.6 Hz, 4 H), 1.39 (d, *J*=13.6 Hz, 1 H), 1.21 - 1.34 (m, 3 H), 1.18 (s, 3 H), 1.10 (d, *J*=11.3 Hz, 3 H), 1.05 (s, 3 H), 1.03 (s, 3 H), 0.95 (s, 3 H), 0.93 (s, 3 H).

### EXAMPLE 160. Preparation of 4-((1R,3aS,5aR,5bR,7aR,11aS,11bR,13aR,13bR)-3a-((3-(4-(methoxycarbonyl)phenyl)ureido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

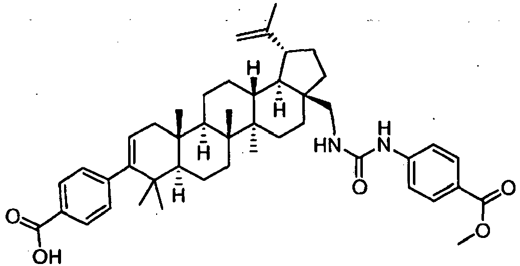

The title compound was prepared following the synthetic route 2 described above using methyl 4-isocyanatobenzoate as the reactant isocyanate. The product was isolated as a white solid (5 mg, 37.7 %). LCMS: m/e 721.5 (MH⁺), 3.07 min (method 3). ¹H NMR (400 MHz, *MeOD)* δ ppm 7.86 - 7.94 (m, 4 H), 7.39 - 7.51 (m, 2 H), 7.15 - 7.26 (m, 2 H), 5.21 - 5.33 (m, 1 H), 4.72 (s, 1 H), 4.59 (s, 1 H), 3.86 (s, 3 H), 3.51 (s, 1 H), 3.03 (d, *J=*13.8 Hz, 1 H), 2.42 - 2.60 (m, 1 H), 2.14 (dd, *J=17.0,* 6.7 Hz, 2 H), 1.61 - 1.76 (m, 8 H), 1.48 (br. s., 6 H), 1.22 - 1.35 (m, 5 H), 1.15 - 1.22 (m, 4 H), 1.14 (br. s., 3 H), 1.04 (s, 3 H), 1.03 (s, 3 H), 0.96 (s, 3 H), 0.93 (s, 3 H).

### EXAMPLE 161. Preparation of 4-((1R,3aS,5aR,5bR,7aR,11aS,11bR,13aR,13bR)-3a-((((2-(1,1-dioxido-4-thiomorpholinyl)ethyl)carbamoyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

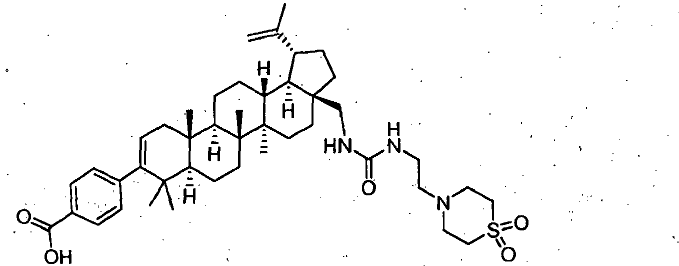

The title compound was prepared following the route 1 described above using N-(2-aminoethyl) thiomorpholine 1,1-dioxide as the reactant amine. The product was isolated as a white solid (8 mg, 20.4 %). LCMS: m/e 748.3 (MH⁺), 2.33 min (method 3). ¹H NMR (400 MHz, *MeOD)* 8 ppm 7.90 (m, *J*=8.3 Hz, 2 H), 7.20 (m, *J*=8.3 Hz, 2 H), 5.28 (d, *J*=4.8 Hz, 1 H), 4.70 (s, 1 H), 4.59 (s, 1 H), 3.71 (br. s., 4 H), 3.35 - 3.56 (m, 7 H), 3.20 (t, *J*=5.3 Hz, 2 H), 2.94 (d, *J*=13.6 Hz, 1 H), 2.37 - 2.57 (m, 1 H), 2.13 (dd, *J*=17.1, 6.3 Hz, 1 H), 1.94 - 2.08 (m, 1 H), 1.80 (br. s., 2 H), 1.61 - 1.77 (m, 8 H), 1.55 (br. s., 2 H), 1.48 (br. s., 4 H), 1.39 (br. s., 2 H), 1.31 (br. s., 1 H), 1.19 - 1.29 (m, 2 H), 1.17 (s, 3 H), 1.06 - 1.12 (m, 2 H), 1.04 (s, 3 H), 1.02 (s, 3 H), 0.95 (s, 3 H), 0.93 (s, 3 H).

### General procedure for the preparation of C28 reversed carbamate derivatives

### Synthetic route 1:

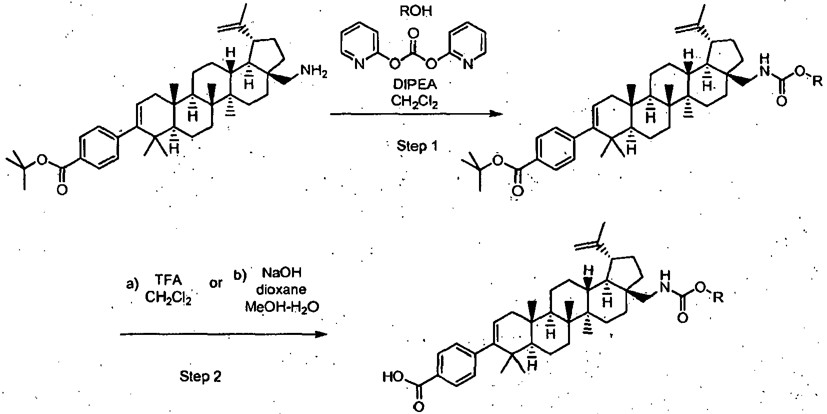

### Step 1: Preparation of Carbamates

To the solution of *tert*-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (100 mg, 0.167 mmol) in DCM (5 ml) at 0 °C was added dipyridin-2-yl carbonate (43.2 mg, 0.200 mmol) followed by DIPEA (0.070 ml, 0.400 mmol). The reaction mixture was stirred for 2 h. To the resulting solution was added 2 - 5 eq: of corresponding alcohol and 3 - 6 eq. of DIPEA at 0 °C. The mixture was stirred for another 18 h. The solvent was removed *in vacuo* and the resulting crude product was used without further purification.

### Step 2: Preparation of benzoic acids

a) Acidic hydrolysis - To a solution of the material from Step 1 in DCM (4 - 5 ml) was added TFA (0.4 - 0.5 ml). The mixture was stirred at rt for 2 - 6 h. The solvent was evaporated under vacuum. The resulting crude product was purified by prep. HPLC to give the desired benzoic acid.
b) Basic hydrolysis - To a solution of the material from Step 1 in dioxane (2 ml) and methanol (2 ml) was added sodium hydroxide (75 mg, 1.875 mmol) and H₂O (0.5 ml). The resulting solution was stirred at 70 °C for 5 - 10 h. The solvent was evaporated under vacuum and the resulting crude product was purified by prep: HPLC to give the desired benzoic acid.

### Synthetic route 2:

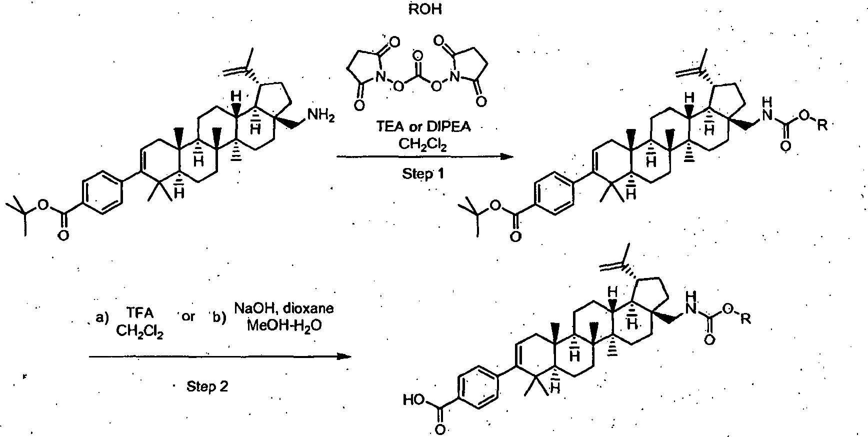

### Step 1. Preparation of the carbamates

To a suspension of bis(2,5-dioxopyrrolidin-1-yl) carbonate (75 mg, 0.293 mmol) and the corresponding alcohol (0.322 mmol) in DCM (2 ml) was added TEA (0.041 ml, 0.293 mmol). The reaction mixture was stirred at rt for 2 - 4 h. To the intermediate solution was added tert-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (1 eq.) in DCM (5 ml) followed by DIPEA (2 eq.). The resulting solution was stirred for two hours. The solvent was evaporated and the resulting crude product was purified by Biotage to give desired carbamates.

### Step 2: Preparation of the benzoic acids

To a solution of the material from Step 3 in dioxane (2 ml) and methanol (2 ml) was added sodium hydroxide (75 mg, 1.875 mmol) and H₂O (0.5 ml). The resulting solution was stirred at 70 °C for 5 - 10 h. The solvent was evaporated under vacuum and the resulting crude product was purified by prep. HPLC to give the desired benzoic acids.

### EXAMPLE 162. 4-((1R,3aS,5aR,5bR,7aR,11aS,11bR,13aR,13bR)-5a,5b,8,8,11a-pentamethyl-3a-(((2-(2-oxopyrrolidin-1-yl)ethoxy)carbonylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

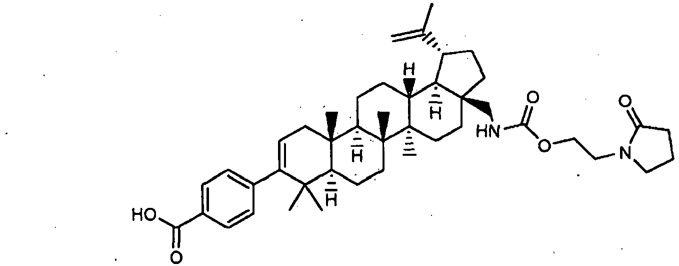

The title compound was prepared in 23 % yield following the synthetic route 2 described above for the preparation of the C28 reversed carbamates, using 1-(2-hydroxyethyl)pyrrolidin-2-one as the reactant alcohol. MS: m/e 699.3 (MH⁺), 1.79 min (method 2). ¹H NMR (400 MHz, *MeOD)* 8 ppm 0.94 (s, 3 H) 0.96 (s, 3 H) 1.03 (s, 3 H) 1.04 (s, 3 H) 1.17 (s, 3 H) 1.70 (s, 3 H) 0.87 -1.96 (m, 20 H) 2.03 (dq, *J*=7.91, 7.65 Hz, 3 H) 2.14 (dd, *J*=17.19, 6.40 Hz, 1 H) 2.36 (t, *J*=8.16 Hz, 2 H) 2.49 (td, *J*=11.04, 5.27 Hz, 1 H) 2.91 (d, *J*=13.05 Hz, 1 H) 3.39 - 3.46 (m, 1 H) 3.48 - 3.57 (m, 4 H) 4.17 (ddd, *J*=5.02, 2.89, 2.64 Hz, 2 H) 4.59 (s, 1 H) 4.71 (d, *J*=2.01 Hz, 1 H) 5.29 (dd, *J*=6.40, 1.88 Hz, 1 H) 7.22 (d, *J*=8.28 Hz, 2 H) 7.91 (d, *J*=8.28 Hz, 2 H).

### EXAMPLE 163. Preparation of 4-((1R,3aS,5aR,5bR,7aR,11aS,11bR,13aR,13bR)-5a,5b,8,8,11a-pentamethyl-3a-(((2-(piperazin-1-yl)ethoxy)carbonylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

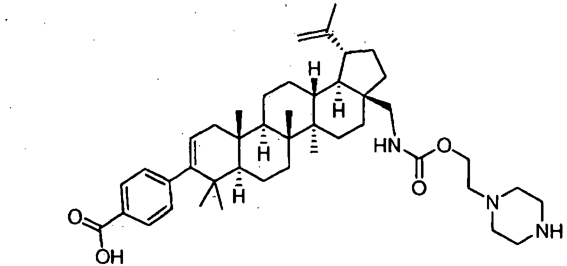

The title compound was prepared following synthetic route 1 described above for the preparation of the C28 reversed carbamates using *tert-*butyl 4-(2-hydroxyethyl)piperazine-1-carboxylate as the reactant alcohol and acid hydrolysis. The product was isolated as a white solid (1.5 mg, 3.82%). LCMS: m/e 700.4 (MH⁺), 2.69 min (method 3).¹H NMR (400 MHz, *MeOD*) δ ppm 7.90 (d; *J*=8.3 Hz, 2 H), 7.20 (d, *J=8.3* Hz, 2 H), 5.19 - 5.37 (m, 1 H), 4.70 (s, 1 H), 4.58 (s, 1 H), 4.18 (t, *J=5.4* Hz, 2 H), 3.77 - 4.02 (m, 2 H), 3.49 - 3.66 (m, 2 H), 3.38 (m, 1H), 3.13 - 3.24 (m, 3 H), 3.03 (s, 1 H), 2.80 - 2.86 (m, 2 H), 2.76 (t, *J*=5.3 Hz, 1 H), 2.43 - 2.55 (m, 1 H), 2.04 - 2.23 (m, 2 H), 1.97 (br. s., 1 H), 1.61 - 1.76 (m, 8 H), 1.52 - 1.61 (m, 2 H), 1.41 - 1.52 (m, 4 H), 1.20 - 1.41 (m, 8 H), 1.16 (s, 3 H), 1.02 (d, *J*=4.0 Hz, 6 H), 0.95 (s, 3 H), 0.93 (s, 3 H).

### EXAMPLE 164. Preparation of 4-((1R,3aS,5aR,5bR,7aR,11aS,11bR,13aR,13bR)-3a-((((3-(1,1-dioxido-4-thiomorpholinyl)propoxy)carbonyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

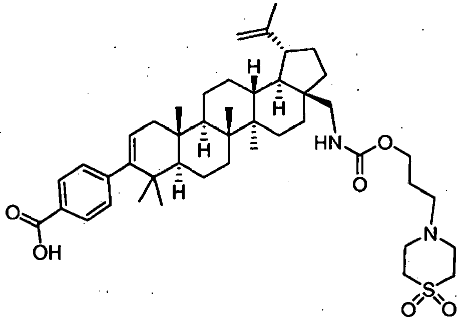

The title compound was prepared following synthetic route 2 described above for the preparation of the C28 reversed carbamates using 4-(3-hydroxypropyl)thiomorpholine 1,1-dioxide as the reactant alcohol. The product was isolated as a white solid (15 mg, 38.2%). LCMS: m/e 763.3 (MH⁺), 2.32 min (method 3). ¹H NMR (500 MHz, *MeOD)* δ ppm 7.88 - 7.99 (m, 2 H), 7.14 - 7.32 (m, 2 H), 5.25 - 5.40 (m, 1 H), 4.74 (s, 1 H), 4.62 (br. s., 1 H), 4.16 (t, *J*=6.1 Hz, 2 H), 3.67 (br. s., 4 H), 3.38 - 3.53 (m, 5 H), 3.14 - 3.28 (m, 2 H), 2.95 (d, *J*=13.7 Hz, 1 H), 2.51 (t, *J*=6.3 Hz, 1 H), 2.17 (dd, *J*=17.2, 6.3 Hz, 1 H), 2.01 - 2.12 (m, 3 H), 1.83 (d, *J*=11.9 Hz, 1 H), 1.64 - 1.80 (m, 8 H), 1.44 - 1.64 (m, 6 H), 1.32 - 1.44 (m, 3 H), 1.22 - 1.32 (m, 3 H), 1.12 - 1.22 (m, 5 H), 1.03 - 1.09 (m, 6 H), 0.99 (s, 3 H), 0.97 (s, 3 H).

### EXAMPLE 165. Preparation of 4-((1R,3aS,5aR,5bR,7aR,11aS,11bR,13aR,13bR)-3a-((((2-(1,1-dioxido-4-thiomorpholinyl)ethoxy)carbonyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

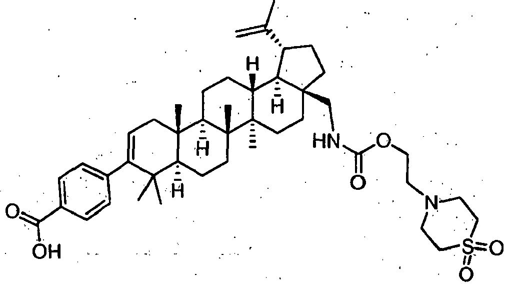

The title compound was prepared following synthetic route 2 described above for the preparation of the C28 reversed carbamates using 4-(2-hydroxyethyl)thiomorpholine 1,2-dioxide as the reactant alcohol. The product was isolated as a white solid (22 mg, 44.9%). LCMS: m/e 749.3 (MH⁺), 2.54 min (method 3). ¹H NMR (400 MHz, *MeOD)* δ ppm 7.94 (m, *J=8.3* Hz, 2 H), 7.24 (m, *J=*8.5 Hz, 2 H), 5.32 (dd, *J*=6.1, 1.6 Hz, 1 H), 4.74 (d, *J=2.0* Hz, 1 H), 4.62 (s, 1 H), 4.18 - 4.40 (m, 2 H), 3.52 (dd, *J*=6.5, 3.8 Hz, 4 H), 3.40 - 3.49 (m, 1 H), 3.25 - 3.38 (m, 6 H), 3.22 (t, *J*=5.0 Hz, 2 H), 2.97 (d, *J*=13.8 Hz, 1 H), 2.53 (td, *J*=11.1, 5.6 Hz, 1 H), 2.17 (dd, *J*=17.2, 6.4 Hz, 1 H), 2.00-2.13 (m, 1 H), 1.84 (dd, *J*=12.2, 3.4 Hz, 2 H), 1.65 - 1.81 (m, 8 H), 1.53 - 1.65 (m, 4 H), 1.50 (d, *J*=11.0 Hz, 3 H), 1.34 - 1.46 (m, 2 H), 1.22 - 1.34 (m, 2 H), 1.19 - 1.22 (m, 2 H), 1.10 - 1.15 (m, 1 H), 1.03 - 1.10 (m, 6 H), 0.99 (s, 3 H), 0.97 (s, 3 H).

### EXAMPLE 166. Preparation of 4-((1R,3aS,5aR,5bR,7aR,11aS,11bR,13aR,13bR)-5a,5b,8,8,11a-pentamethyl-3a-(((3-(2-oxopyrrolidin-1-yl)propoxy)carbonylamino)methyl)-1-(prop-1-en- 2-yl)-2,3,3a,4,5,5a,5b,6,7,7a,8,11a,11b,12,13,13a,13b-octadecahydro-1H-cyclopenta[a]chrysen-9-yl)benzoic acid.

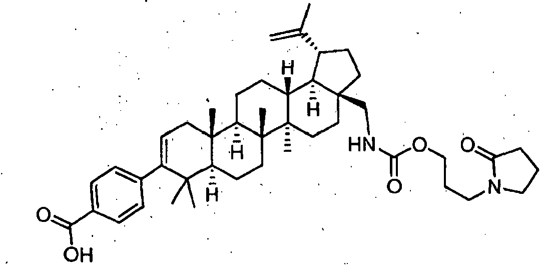

The title compound was prepared following synthetic route 2 described above for the preparation of the C28 reversed carbamates using 1-(3-hydroxypropyl)pyrrolidin-2-one as the reactant alcohol. The product was isolated as a white solid (12 mg, 32.4 %). LCMS: m/e 713.3 (MH⁺), 2.43 min (method 3). ¹H NMR (400 MHz, *MeOD*) δ ppm 7.93 (m, 2 H), 7.24 (m, 2 H), 5.32 (dd, *J*=6.3, 1.8 Hz, 1 H), 4.74 (d, *J*=2.0 Hz, 1 H), 4.62 (s, 1 H), 3.94 - 4.12 (m, 2 H), 3.45 - 3.56 (m, 2 H), 3.34 - 3.45 (m, 3 H), 2.95 (d, *J*=13.8 Hz, 1 H), 2.52 (td, *J*=11.2, 5.6 Hz, 1 H), 2.32 - 2.45 (m, 2 H), 2.17 (dd, *J*=17.2, 6.4 Hz, 1 H), 2.01 - 2.13 (m, 3 H), 1.81 - 2.01 (m, 4 H), 1.64 - 1.81 (m, 8 H), 1.45 - 1.64 (m, 6 H), 1.34 - 1.45 (m, 2 H), 1.23 - 1.34 (m, 2 H), 1.20 (s, 3 H), 1.15 (dt, *J*=8.6, 4.4 Hz, 1 H), 1.09 - 1.12 (m, 1 H), 1.02 - 1.09 (m, 7 H), 0.99 (s, 3 H), 0.97 (s, 3 H).

### EXAMPLE 167. Preparation of 4-(N-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)sulfamoyl)benzoic acid.

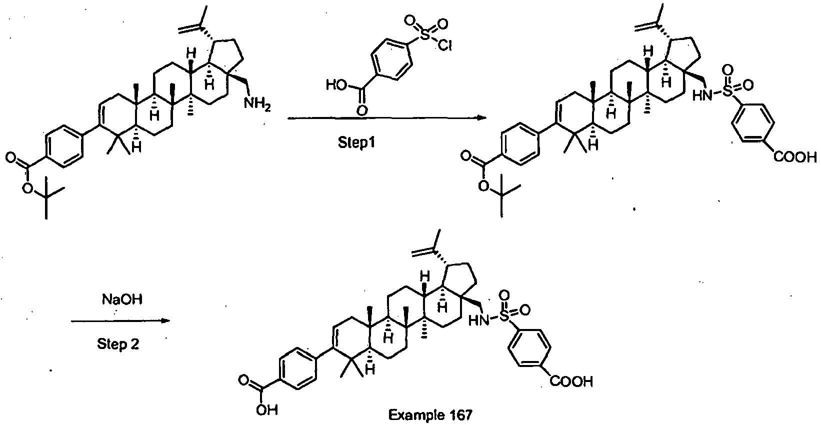

Step 1. Preparation of 4-(N-(((1R,3aS,5aR,5bR,7aR,11aS,11bR,13aR,13bR)-9-(4-(*tert*-butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)sulfamoyl)benzoic acid.

To a solution of *tert*-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (25 mg, 0.042 mmol) in DCM (2 ml) was added 4-(chlorosulfonyl)benzoic acid (9.19 mg, 0.042 mmol) and DIPEA (7.28 µl, 0.042 mmol). The resulting mixture was stirred for 48 h at rt. The mixture was diluted with 7 ml of sat. NaHCO₃ and was extracted with dichloromethane (3 x 7 ml). The combined organic layers were dried with Na₂SO₄. The drying agent was removed by filtration and the filtrate was concentrated under reduced pressure. The crude product was used in the next step with no additional purification.

### Step 2 : Benzoic acid deprotection

4-(N-(((1R,3aS,5aR,5bR,7aR,11aS,11bR,13aR,13bR)-9-(4-(*tert-*butoxycarbonyl)phenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)sulfamoyl)benzoic acid (10 mg, 0.013 mmol) from Step 1 was dissolved in dioxane (1 ml) and MeOH (5 ml) sodium hydroxide (10.20 mg, 0.255 mmol) (powder) was added followed by 5 drops of water. The resulting suspension was stirred at 70°C for 6 h. After the reaction was completed, all volatile material was removed *in vacuo*. The residue was re-dissolved in MeOH and purified by prep. HPLC to afford 4-(N-(((1R,3aS,5aR,5bR,7aR,11aS,11bR,13aR,13bR)-9-(4-carboxyphenyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-3a-yl)methyl)sulfamoyl)benzoic acid as a white solid (1.8 mg, 18.4 %). LCMS: m/e 728.2 (MH⁺), 2.11 min (method 3). ¹H NMR (400 MHz, *MeOD)* δ ppm 8.24 (m, *J*=8.5 Hz, 2 H), 8.01 (m, *J*=8.5 Hz, 2 H), 7.94 (m, *J*=8.3 Hz, 2 H), 7.24 (m, *J*=8.3 Hz, 2 H), 5.31 (d, *J*=4.3 Hz, 1 H), 4.70 (d, *J*=2.3 Hz, 1 H), 4.59 (s, 1 H), 3.05 (s, 1 H), 2.62 (d, *J*=13.1 Hz, 1 H), 2.37 (br. s., 1 H), 2.11 (m, 1 H), 1.76 - 2.01 (m, 4 H), 1.65 - 1.76 (m, 6 H), 1.53 - 1.65 (m, 4 H), 1.44 (br. s., 5 H), 1.31 (d, *J*=2.8 Hz, 6 H), 1.03 (s, 6 H), 1.01 (s, 3 H), 0.99 (s, 3 H), 0.96 (s, 3 H).

### EXAMPLE 168. Preparation of 4-((1R,3aS,5aR,5bR,7aR,11aS,11bR,13aR,13bR)-3a-((1,1-dioxido-4-thiomorpholinyl)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

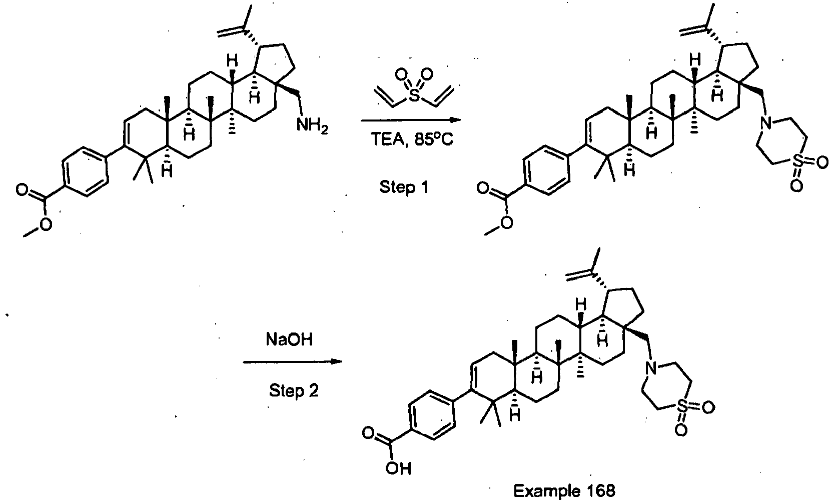

### Step 1. Tandem double Michael addition

To a solution of methyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (120 mg, 0.215 mmol) in dioxane (0.5 ml) and ethanol (0.5 ml) was added TEA (0.09 ml, 0.645 mmol) and vinylsulfonylethene (50.8 mg, 0.430 mmol), the reaction mixture was heated at 85 °C for 3 h. The solvent was removed *in vacuo,* and the crude solid was used in next step without further purification. LCMS: m/e 676.6 (MH⁺), 3.28 min (method 3).

### Step 2. Hydrolysis of methyl ester

To the solution of the crude material from Step 1 (88 mg, 0.130 mmol) in dioxane (1.5 ml) was added a solution of sodium hydroxide (0.5 ml, 1N, 0.500 mmol) .The reaction mixture was heated to 55°C for 4 h. The solvent was removed *in vacuo,* the resulting solid was purified by prep. HPLC to afford 4-((1R,3aS,5aR,5bR,7aR,11aS,11bR,13aR,13bR)-3a-((1,1-dioxido-4-thiomorpholinyl)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid as a white solid (30 mg, 33.1%). LCMS: m/e 662.5 (MH⁺), 2.98 min (method 3). ¹H NMR (400 MHz, *MeOD*) δ ppm 7.94 (m, *J*=8.3 Hz, 2 H), 7.23 (m, *J=8.3* Hz, 2 H), 5.24 - 5.39 (m, 1 H), 4.75 (s, 1H), 4.65 (s, 1H), 3.64 (br. s., 4 H), 3.42 (br. s., 4 H), 3.17 - 3.25 (m, 1 H), 2.86 (m, 1H), 2.55 (m, 1H), 2.08 - 2.23 (m, 1 H), 1.94 (br. s., 2 H), 1.74 (s, 6 H), 1.61 (br. s., 2 H), 1.55 (br. s., 6H), 1.21 - 1.41 (m, 6 H), 1.15 - 1.21 (m, 4 H), 1.07 - 1.15 (m, 4 H), 1.05 (s, 3 H), 0.98 (s, 3 H), 0.96 (s, 3 H).

### EXAMPLE 169. Preparation of 4-((1R,3aS,5aR,5bR,7aR,11aS,11bR,13aR,13bR)-3a-((3-carboxypropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

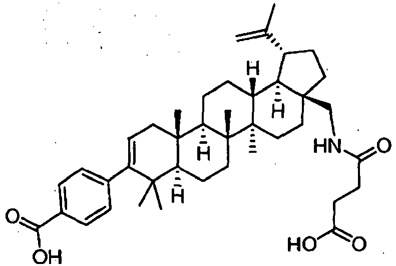

To a solution of 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7;7a,8,11,11a,11b,12,13,13a,13b-octadecahydro-1H-cyclopenta[a]chrysen-9-yl)benzoic acid (20 mg, 0.037 mmol) in DCM (2 ml) was added dihydrofuran-2,5-dione (11.04 mg, 0.110 mmol) followed by DMAP (4.49 mg, 0.037 mmol) and DIPEA (6.42 µl, 0.037 mmol). The mixture was stirred at rt for 18 h. The solvent was removed *in vacuo* and the resulting residue was purified by prep. HPLC. The product was isolated as a white solid (5 mg, 21.1 %). LCMS: m/e 644.5 (MH⁺), 2.80 min (method 3). ¹H NMR (400 MHz, *MeOD*) δ ppm 7.94 (d, *J*=8.5 Hz, 2 H), 7.16 - 7.34 (m, 2 H), 5.32 (dd, *J*=6.4, 1.6 Hz, 1 H), 4.74 (d, *J*=2.0 Hz, 1 H), 4.62 (s, 1 H), 3.54 (d, *J*=13.6 Hz, 1 H), 3.04 (d, *J*=13.8 Hz, 1 H), 2.57 - 2.71 (m, 2 H), 2.44 - 2.57 (m, 2 H), 2.17 (dd, *J*=17.2, 6.4 Hz, 1 H), 2.09 (dd, *J*=13.1, 1.8 Hz, 1 H), 1.85 (dd, *J*=12.2, 3.4 Hz, 2 H), 1.65 - 1.82 (m, 8 H), 1.61 (br. s., 2 H), 1.53 (d, *J*=10.5 Hz, 3 H), 1.48 (d, *J*=2.0 Hz, 1 H), 1.35 - 1.45 (m, 3 H), 1.24 - 1.35 (m, 3 H), 1.16 - 1.24 (m, 4 H), 1.02 - 1.16 (m, 8 H), 0.99 (s, 3 H), 0.95 (s, 3 H).

### EXAMPLE 170. Preparation of 4-((1R,3aS,5aR,5bR,7aR,11aS,11bR,13aR,13bR)-3a-((4-methoxy-4-oxobutanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid.

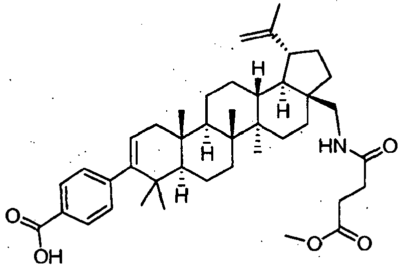

To a solution of 4-((1R,3aS,5aR,5bR,7aR,11aS,11bR,13aR,13bR)-3a-((3-carboxypropanamido)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid (20 mg, 0.037 mmol) in methanol (2 ml) was added TFA to form a 0.1%.v/v TFA-methanol solution. The mixture was stirred at rt for 28 h. The solvent was removed *in vacuo* and the resulting residue was purified by prep. HPLC. The product was isolated as a white solid (1.1 mg, 4.55 %). LCMS: m/e 658.5 (MH⁺), 2.90 min (method 3). ¹H NMR (400 MHz, *MeOD*) δ ppm 7.94 (d, *J=8.5* Hz, 2 H), 7.16 - 7.34 (m, 2 H), 5.32 (dd, *J*=6.4, 1.6 Hz, 1 H), 4.74 (d, *J*=2.0 Hz, 1 H), 4.62 (s, 1 H), 3.68 (s, 3H), 3.54 (d, *J*=13.6 Hz, 1 H), 3.04 (d, *J*=13.8 Hz, 1 H), 2.57-2.71 (m, 2 H), 2.44 - 2.57 (m, 2 H), 2.17 (dd, *J*=17.2, 6.4 Hz, 1 H), 2.09 (dd, *J*=13.1, 1.8 Hz, 1 H), 1.85 (dd, *J*=12.2, 3.4 Hz, 2 H), 1.65 - 1.82 (m, 8 H), 1.61 (br. s., 2 H), 1.53 (d, *J*=10.5 Hz, 3 H), 1.48 (d, *J=2.0* Hz, 1 H), 1.35 - 1.45 (m, 3 H), 1.24 - 1.35 (m, 3 H), 1.16 - 1.24 (m, 4 H), 1.02 - 1.16 (m, 8 H), 0.99 (s, 3 H), 0.95 (s, 3 H).

### EXAMPLE 171. Preparation of N-(dimethylsulfamoyl)-4-((1R,3aS,5aR,5bR,7aR,11aS.11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzamide.

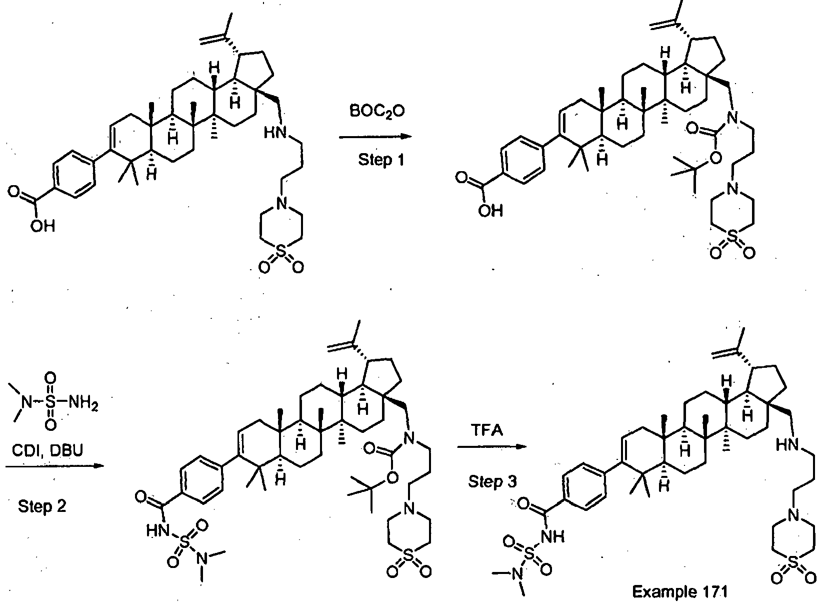

### Step 1. BOC protection of the secondary amine

To a solution of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid (57 mg, 0.079 mmol) in DCM (2 ml) was added di*-tert-*butyl dicarbonate (26.0 mg, 0.119 mmol) followed by DMAP (9.68 mg, 0.079 mmol) and DIPEA (0.014 ml, 0.079 mmol). The mixture was stirred at rt for 18 h. The solvent was removed *in vacuo,* and the resulting solid was carried to the next step without further purification. LCMS: m/e 819.3 (MH⁺), 2.65 min (method 3).

### Step 2: Coupling

To a solution of the material from Step 1 (57 mg, 0.070 mmol) in THF (2 ml) was added carbonyl diimidazole (19.36 mg, 0.119 mmol). The mixture was stirred at rt for 2 h. *N,N*-dimethylsulfamide (24.11 mg, 0.199 mmol) was added followed by DBU (0.030 ml, 0.199 mmol). The resulting mixture was stirred for 12 h at rt. The reaction was quenched by 1N HCl, and extracted with ethyl acetate (3 X 10 ml). The organic layers were collected and dried over sodium sulfate. The material obtained was used in next step without further purification. LCMS: m/e 925.6 (MH⁺), 2.56 min (method 3).

### Step 3. De-BOC hydrolysis

To a solution of the material from Step 2 (5 mg, 0.0054 mmol) in DCM (5 ml) was added TFA (0.5 ml, 6.49 mmol). The mixture was stirred at room temperature for 16 h. The mixture was concentrated under reduced pressure. The residue was dissolved in dioxane and MeOH and was purified by prep. HPLC to afford N-(dimethylsulfamoyl)-4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzamide as a white solid (1.3 mg, 29.2 %). LCMS: m/e 825.5 (MH⁺), 2.41 min (method 3). ¹H NMR (400 MHz, *MeOD*) δ ppm 7.81 (m, *J*=8.3 Hz, 2 H), 7.28 (m, *J*=8.5 Hz, 2 H), 5.27 - 5.40 (m, 1 H), 4.78 (s, 1H), 4.68 (s, 1 H), 3.23 - 3.31 (m, 1 H), 3.13 - 3.23 (m, 6 H), 3.09 (br. s., 4 H), 3.00 (s, 6 H), 2.88 (s, 1 H), 2.74 (t, *J*=6.7 Hz, 2 H), 2.54 (br. s., 1 H), 2.17 (s, 1 H), 1.96 (d, *J*=8.5 Hz, 2 H), 1.76 (s, 8 H), 1.54 (br. s., 8 H), 1.39 (d, *J*=3.5 Hz, 3 H), 1.31 (br. s., 4 H), 1.17 - 1.23 (m, 4 H), 1.11 (s, 3 H), 1.07 (s, 3 H), 1.00 (s, 3 H), 0.98 (s, 3 H)

### EXAMPLE 172. Preparation of benzamide, N-(cyclopropylsulfonyl)-4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11 a,11b,12,13,13a,13b-octadecahydro-1H-cyclopenta[a]chrysen-9-yl)benzamide.

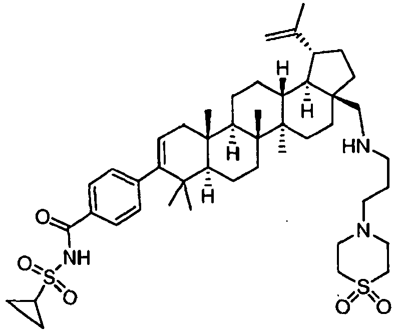

The title compound was prepared following the procedure described above for the preparation of *N*-(dimethylsulfamoyl)-4-((1R,3aS,5aR,5bR,7aR,11aS,11bR,13aR,13bR)-3a-(((3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzamide, example 181, using cyclopropanesulfonamide as the reactant amine. The product was isolated as a white solid (32 mg, 68.5 %). LCMS: m/e 822.5 (MH⁺), 2.38 min (method 3). ¹H NMR (400 MHz, *MeOD*) δ ppm 7.84 (m, 2 H), 7.29 (m, 2 H), 5.22 - 5.38 (m, 1 H), 4.78 (d, *J*=1.5 Hz, 1 H), 4.66 (s, 1 H), 3.49 - 3.65 (m, 4 H), 3.36 - 3.46 (m, 4 H), 3.19 - 3.31 (m, 3 H), 3.14 - 3.19 (m, 1 H), 3.11 (t, *J*=7.4 Hz, 2 H), 2.89 (d, *J=*13.1 Hz, 1 H), 2.53 (td, *J*=10.5, 5.6 Hz, 1 H), 2.00 - 2.24 (m, 4 H), 1.66 - 1.91 (m, 10 H), 1.43 - 1.66 (m, 8 H), 1.28 - 1.43 (m, 5 H), 1.12 - 1.28 (m, 7 H), 1.09 (s, 3 H), 1.05 (s, 3 H), 0.99 (s, 3 H), 0.97 (s, 3 H).

### EXAMPLE 173. Preparation of 4-((1R,3aS,5aR,5bR,7aR,11aS,11bR,13aR,13bR)-3a-(((4-((cyclopropylsulfonyl)amino)-4-oxobutanoyl)(3-(1,1-dioxido-4-thiomorpholinyl)propyl)amino)methyl)-1-isopropenyl-5a,5b,8,8,11a-pentamethyl-2,3,3a,4,5,5a,5b,6,7,7a,8,11,11a,11b,12,13,13a,13b-octadecahydro-1H-cyclopenta[a]chrysen-9-yl)benzoic acid.

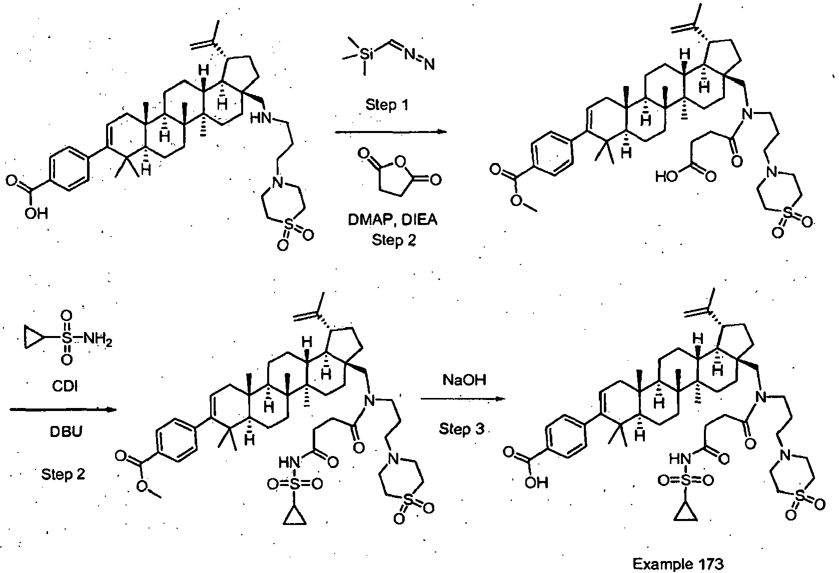

### Step 1. Methyl ester formation

To a solution of 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoic acid (300 mg, 0.42 mmol) in CH₂Cl₂ (50 ml) / MeOH (5 ml) was added (diazomethyl)trimethylsilane (2.053 ml, 4.11 mmol). The resulting solution was stirred at rt for 2 h under nitrogen. LC/MS showed no SM remaining. The reaction mixture was concentrated to give (100%) crude product. LCMS: m/e 733.3 (MH⁺), 2.52 min (method 2).

### Step 2. Acylation

To a solution of methyl, 4-((1R,3aS,5aR,5bR,7aR,11aS,11bR,13aR,13bR)-5a,5b,8,8,11a-pentamethyl-1-(prop-1-en-2-yl)-3a-((3-(1-dioxo-thiomorpholino)propylamino)methyl)-2,3,3a,4,5,5a,5b,6,7,7a,8,11,11a,11b,12,13,13a,13b-octadecahydro-1H-cyclopenta[a]chrysen-9-yl)benzoate (16 mg, 0.022 mmole) in CH₂Cl₂ (0.5 ml) was added dihydrofuran-2,5-dione (3.28 mg, 0.033 mmol) followed by DMAP (3.07 mg, 0.022 mmol) and DIPEA (0.011 ml, 0.065 mmol) . The mixture was stirred at rt for 18 h. The solvent was removed *in vacuo* and the resulting residue was used in next step without further purification. LCMS: m/e 833.3 (MH⁺), 2.45 min (method 3).

### Step 3. Sulfonamide formation

To a solution of material from Step 2 (20 mg, 0.024 mmol) in THF (2 ml) was added CDI (4.67 mg, 0.029 mmol). The mixture was stirred at rt for two hours. To the resulting solution was added cyclopropanesulfonamide (5.82 mg, 0.048 mmol), followed by DBU (7.24 µl, 0.048 mmol). The reaction mixture was stirred for 6 h at rt. The solvent was removed under reduced pressure, the residue was redissolved into CH₂Cl₂ and the solution was washed with aqueous sodium bicarbonate. The organic layer was collected and dried over sodium sulfate. The material obtained was used in the next saponification step without further purification. LCMS: m/e 936.6 (MH⁺), 2.60 min (Method 3).

### Step 4. Alkaline hydrolysis of methyl ester

To a solution of material from Step 3 (18 mg, 0.019 mmol) in dioxane (1.5 ml) was added sodium hydroxide (0.5 ml, 1 N, 500 mmol) .The resulting solution was stirred at 63 °C for 12 h the solvent was removed *in vacuo* and the resulting residue was purified by prep. HPLC to afford the title compound as a white solid (8 mg, 42 %). LCMS: m/e 922.5 (MH⁺), 2.42 min (method 3), ¹H NMR (400 MHz, *MeOD*) δ ppm 7.94 (m, *J*=8.0 Hz, 2 H), 7.24 (m, *J*=8.0 Hz, 2 H), 5.33 (d, *J*=6.3 Hz, 1 H), 4.76 (s, 1H), 4.68 (s, 1H), 3.84 (br. s., 2 H), 3.59 - 3.75 (m, 4 H), 3.48 - 3.59 (m, 3 H), 3.40-3.48 (m, 2 H), 3.06 - 3.21 (m, 1 H), 2.84 - 3.06 (m, 2 H), 2.75 - 2.84 (m, 2 H), 2.54-2.75 (m, 3 H), 2.03 - 2.27 (m, 4 H), 1.91 (d, J=16.6 Hz, 2 H), 1.81 (br. s., 2 H), 1.65-1.79 (m, 6 H), 1.52 - 1.65 (m, 5 H), 1.48 (br. s., 2 H), 1.35 - 1.45 (m, 2 H), 1.17 - 1.35 (m, 8 H), 1.10 - 1.17 (m, 4 H), 1.02 - 1.10 (m, 6 H), 0.91 - 1.02 (m, 6 H).

The title compound was prepared using the procedure described previously for the preparation of intermediate 3, using ketone intermediate B2 as starting material (29%). ¹H NMR (400 MHz, *CHLOROFORM-d*) δ ppm 0.65 (s, 3 H), 0.91 (s, 3 H), 0.94 (s, 3 H), 0.97 (s, 3 H), 1.04 (s, 3 H), 1.05 - 1.12 (m, 1 H), 1.14 (s, 6 H), 1.16 - 1.28 (m, 3 H), 1.28 - 1.42 (m, 2 H), 1.42 - 1.54 (m, 2 H), 1.57 - 1.65 (m, 2 H), 1.68 (d, *J*=14.56 Hz, 2 H), 1.73 (d, *J*=4.52 Hz, 1 H), 1.78 - 1.84 (m, 2 H), 1.86 (dd, *J*=5.90, 4.14 Hz, 1 H), 1.90 - 1.97 (m, 1 H), 1.98 - 2.04 (m, 1 H), 2.12 (dd, *J*=17.07, 6.78 Hz, 1 H), 2.93 (dd, *J*=13.93, 4.14 Hz, 1 H), 5.03 - 5.14 (m, 3 H), 5.33 (t, *J*=3.51 Hz, 1 H), 5.58 (dd, *J*=6.78, 2.01 Hz, 1 H), 7.34 - 7.38 (m, 5 H); ¹⁹F NMR (376.46 MHz, *CHLOROFORM-d*) δ ppm -74.84.

### Example 174. Preparation of 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

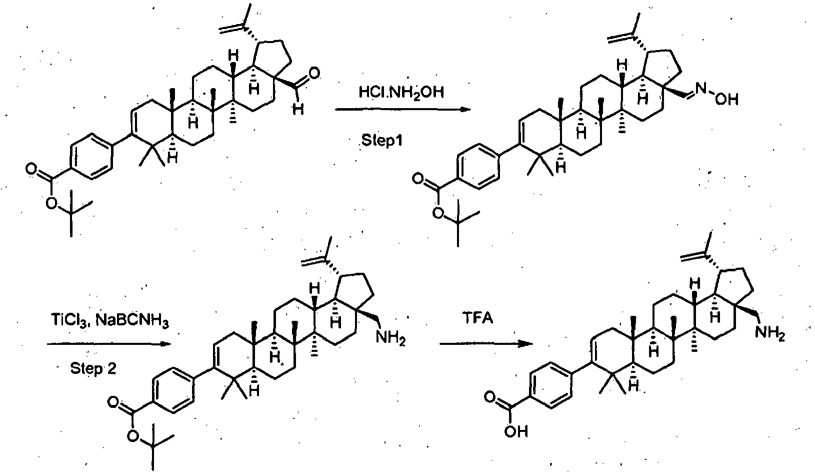

### Step 1. Preparation of tert-butyl 4-((1R,3aS,5aR,5bR,7aR,11aS,11bR,13aR,13bR)-3a-((E)-(hydroxyimino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

To a suspension of *tert*-butyl 4-((1R,3aS,5aR,5bR,7aR,11aS,11bR,13aR,13bR)-3a-formyl-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenfa[a]chrysen-9-yl)benzoate (200 mg, 0.334 mmol) in EtOH (20 mL) was added hydroxylamine hydrochloride (186 mg, 2.67 mmol) and potassium carbonate (369 mg, 2.67mmol) and the mixture was stirred overnight at room temperature. LC/MS showed the mass of the expected product. The mixture was diluted with 7 mL of sat. NaHCO₃ and was extracted with dichloromethane (3 x 7 mL). The combined organic layers were dried with Na₂SO₄. The drying agent was removed by filtration and the filtrate was concentrated under reduced pressure. The crude product as an off white foam (∼100%) was used in the next step with no additional purification. LCMS: m/e 614.53 (MH⁺), 3.82 min (method 3). 1H NMR (400 MHz, *CHLOROFORM-d*) δ ppm 7.90 (2 H, m, *J*=8.3 Hz), 7.60 (1 H, s), 7.19 (2 H, m, *J*=8.3 Hz), 5.23 - 5.35 (1 H, m), 4.76 (1 H, d, *J*=1.8 Hz), 4.64 (1 H, s), 2.56 (1 H, td, *J*=11.0, 5.4 Hz), 2.05 - 2.19 (1 H, m), 1.92 - 2.05 (2 H, m), 1.83 - 1.92 (2 H, m), 1.64 - 1.82 (7 H, m), 1.58 - 1.64 (9 H, m), 1.38 - 1.57 (9 H, m), 1.22 - 1.31 (2 H, m), 1.10 - 1.20 (2 H, m), 1.07 (3 H, s), 1.04 (3 H, s), 0.97 - 1.01 (3 H, m), 0.85 - 0.97 (6 H, m)

### Step 2. Preparation of tert-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

To the crude product from above containing *tert*-butyl 4-((1R,3aS,5aR,5bR,7aR,11aS,11bR,13aR,13bR)-3a-((hydroxyimino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (100 mg, 0.163 mmol) in a 100 mL pear shape flask was added EtOH (12 mL) to form a clear solution. To this was added excess of NH₄Cl (400 mg, 5.19 mmol) and sodium cyanoborohydre (300 mg, 4.77 mmol). The mixture was stirred in an ice bath until cold. To this suspension was added titanium(III) chloride (4 mL, 0.163 mmol) 20% solution. The resulting mixture was blanketed with nitrogen. The ice bath was removed and the stirring was continued at rt for an hour. LCMS show the reaction was complete. At this point, the mixture was dark greenish-blue-purple. A solution of sodium hydroxide (3 mL, 10N in 25 mL water) was added into the reaction mixture, along with methylene chloride (30 mL).

The mixture was stirred vigorously until the dark blue phase was floating on top of the organic phase. The mixture was filtered through a plug of paper to afford a clear filtrate. The two-phase filtrate was tested for pH of the top phase (pH), and LCMS of the bottom phase was taken. The organic layer was collected and the aqueous layer was extracted with CH₂Cl₂ (2 x 20 mL). The organic layers were combined and dried over NaSO₄ to afford an off white solid (95mg, 97.0%) which was used without further purification in the next step). LCMS: m/e 600.58 (MH⁺), 3.03 min (method 3). 1H NMR (400 MHz, *CHLOROFORM-d)* δ ppm 7.90 (2 H, m, *J*=8.3 Hz), 7.19 (2 H, m, *J*=8.3 Hz), 5.29 (1 H, dd, *J*=6.2, 1.6 Hz), 4.71 (1 H, d, *J*=2.3 Hz), 4.61 (1 H, s), 2.89 (1 H, d, *J*=12.8 Hz), 2.44 (1 H, td, *J=*11.0, 5.7 Hz), 2.36 (1 H, d, *J*=13.3 Hz), 2.11 (1 H, dd, *J*=17.1, 6.5 Hz), 1.75 - 1.99 (2 H, m), 1.71 (5 H, s), 1.57 - 1.70 (9 H, m), 1.36 - 1.57 (9 H, m), 1.18 - 1.32 (5H, m), 1.06 - 1.18 (6 H, m), 1.01 - 1.06 (3 H, m), 1.00 (3 H, s), 0.90 - 0.97 (6 H, m).

### Step 3. Preparation of 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

To a solution of *tert*-butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (15 mg, 0.025 mmol) in DCM (4 mL) was added TFA (0.4 ml, 5.19 mmol) and the mixture was stirred at room temperature. The color of the solution turned pinkish. After 6 h, the solvent was removed in vacuo and the crude was purified by reverse phase prep. HPLC to afford the title compound as a white foam (12 mg, 80%). LCMS: m/e 544.49 (MH⁺), 2.60 min (method 3). 1H NMR (500 MHz, *MeOD)* δ ppm 7.94 (2 H, d; *J*=8.5 Hz), 7.24 (2 H, d, *J*=8.5 Hz), 5.32 (1 H, dd, *J*=6.3, 1.7 Hz), 4.77 (1 H, d, *J*=1.8 Hz), 4.66 (1 H, s), 3.10 - 3.24 (1 H, m), 2.73 - 2.84 (1 H, m), 2.50 (1 H, td, *J*=10.6, 5.6 Hz), 2.17 (1 H, dd, *J*=17.1, 6.4 Hz), 1.98 - 2.11 (1 H, m), 1.67 - 1.89 (8 H, m), 1.54 - 1.66 (3 H, m), 1.44 - 1.56 (5 H, m), 1.33 - 1.45 (2 H, m), 1.27 - 1.34 (3 H, m), 1.23 - 1.28 (1 H, m), 1.15 - 1.24 (4 H, m), 1.11 (3 H, s), 1.06 (3 H, s), 0.92 - 1.02 (6 H, m).

### Example 175. Preparation of 4-((1R,3aS,5aR,5bR,7aR,11aS,11bR,13aR,13bR)-3a-((isopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

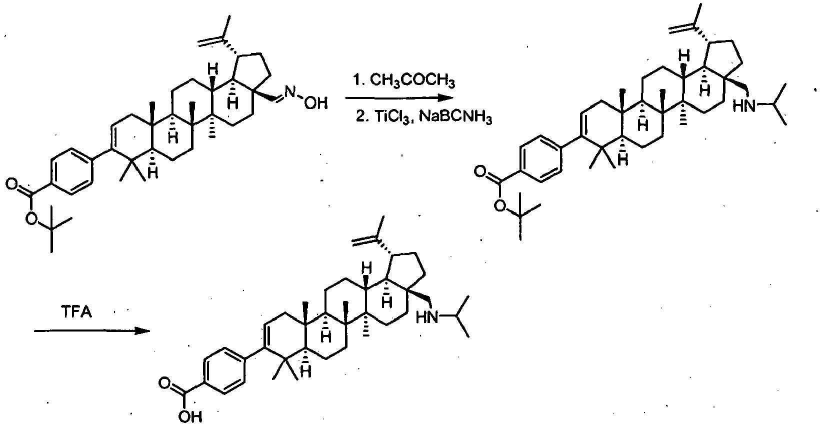

During the purification in silica gel chromatography of *tert*-butyl 4-((1R,3aS,5aR,5bR,7aR,11aS,11bR,13aR,13bR)-3a-((E)-(hydroxyimino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate, acetone was used. As a result, a small amount of the corresponding acetone aldimine was formed. This compound was treated with TiCl₃, NaBCNH₃ as described above to afford (*tert*-butyl 4-((1R,3aS,5aR,5bR,7aR,11aS,11bR,13aR,13bR)-3a-((isopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate). LCMS: m/e 642.69 (M+H)⁺, 2.611 min (method 9 Start%B = 75%). ¹H NMR (400MHz, CHLOROFORM-d) δ 7.90 (d, *J*=8.3 Hz, 2H), 7.19 (d, *J*=8.3 Hz, 2H), 5.30 (dd, *J*=6.3, 1.8 Hz, 1H), 4.72 (d, *J*=2.3 Hz, 1H), 4.61 (dd, *J*=2.1, 1.4 Hz, 1H), 2.82 - 2.75 (m, *J*=12.8 Hz, 1H), 2.79 - 2.71 (m, 1H), 2.79 - 2.71 (m, 1H), 2.48 (td, *J=*11.0*,* 5.8 Hz, 1H), 2.21 (d, *J=*11.5 Hz, 1H), 2.12 (dd, *J*=17.2, 6.4 Hz, 1H), 2.02 - 1.75 (m, 5H), 1.75 - 1.63 (m, 7H), 1.72 (s, 3H), 1.61 (s, 9H), 1.60-1.53 (m, 3H), 1.51 - 1.35 (m, 6H), 1.12 (s, 3H), 1.11 (d, *J*=6.3 Hz, 3H), 1.10 (d, *J*=6.3 Hz, 3H), 1.02 (s, 3H), 1.00 (s, 3H), 0.94 (s, 6H). ¹³C NMR (101MHz, CHLOROFORM-d) δ 165.6, 159.8, 150.3, 147.9, 145.9, 129.55, 129.37, 128.0, 123.6, 109.2, 80.4, 52.4, 49.0, 48.6, 47.0, 42.3, 41.3, 40.4, 38.9, 37.1, 36.8, 35.9, 33.7, 33.1, 29.4, 29.1, 28.9, 27.9, 26.7, 24.9, 20.9, 20.7, 19.4, 18.9, 16.1, 15.3, 14.4.

Subsequent treatment with TFA to remove the protective group as described above rendered the title compound. LCMS: m/e 586.64 (M+H)⁺, 2.14 min (method 9). Partial ¹H NMR (400MHz, CHLOROFORM-d) δ 7.99 (d, *J*=8.3 Hz, 2H), 7.23 (d, *J*=8.3 Hz, 2H), 5.35 - 5.28 (m, 1H), 4.73 (d, *J*=1.5 Hz, 1H), 4.66 - 4.62 (m, 1H), 1.72 (s, 3H), 1.51 (s, 3H), 1.49 (s, 3H), 1.12 (s, 3H), 1.03 (s, 3H), 1.00 (s, 3H), 0.95 (s, 6H).

### Example 176. Preparation of 4-((1R,3aS,5aR,5bR,7aR,11aS,11bR,13aR,13bR)-3a-((3-methoxy-3-oxopropylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

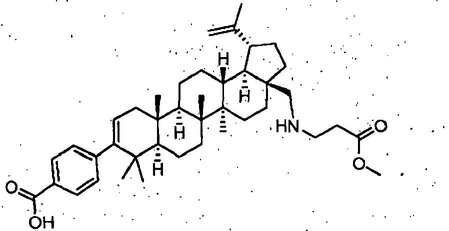

The title compound was prepared following the general procedures described above for the C-28 amine formation and hydrolysis using methyl 3-aminopropanoate as the reactant amine. The product was isolated as a white solid (26 mg, 27.5 %). LCMS: m/e 630.3 (MH⁺), 2.39 min (method 9). ¹H NMR (400MHz, CHLOROFORM-d) δ 8.00 (d, *J*=8.0 Hz, 2H), 7.24 (d, *J*=8.3 Hz, 2H), 5.31 (d, *J*=4.5 Hz, 1H), 4.73 (s, 1H), 4.65 (s, 1H), 3.76 (s, 3H), 3.52 - 3.27 (m, 3H), 2.90 (m, 2H), 2.81 (br. s., 1H), 2.47 - 2.33 (m, 1H), 2.18 - 1.95 (m, 2H), 1.93 - 1.79 (m, 2H), 1.77 - 1.61 (m, 2H), 1.59 - 1.38 (m, 7H), 1.37 - 1.17 (m, 7H), 1.10 (s, 4H), 1.02 (s, 3 H), 0.99 (s, 3 H), 0.96 (s, 3 H), 0.87 - 0.95 (s, 3 H)

### Example 177. Preparation of 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

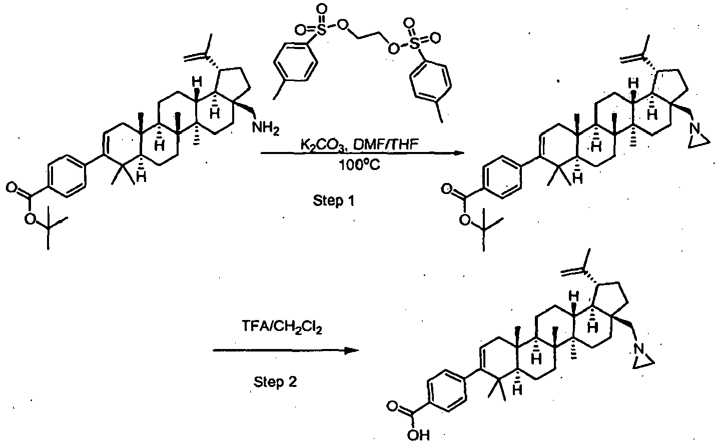

### Step 1. Preparation of tert-butyl 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

*tert-*butyl 4-((1R,3aS,5aR,5bR,7aR,11aS,11bR,13aR,13bR)-3a-(aminomethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (200 mg, 0.667 mmol) was dissolved in DMF (10.00 mL) and THF (10.00 mL), ethane-1,2-diyl bis(4-methylbenzenesulfonate) (247 mg, 0.667 mmol) was added, followed by K₂CO₃ (184 mg, 1.333 mmol) and the mixture was stirred at 100°C until no starting material was detected by LCMS. An M+1=626.7 was observed by LC-MS. The reaction mixture was redissolved in CH₂Cl₂ (100 mL), washed by water (2 x 50 mL) and the organic layer was dried over sodium sulfate. The solvent was removed in vacuo and the resultant residue was purified by silica gel chromatography using a mixture of ethyl acetate/hexanes. A white foam was obtained (130 mg, 65%) LCMS: m/e 426.7 (MH⁺), 4.393 min (method 8).¹H NMR (400MHz, CHLOROFORM-d) δ 7.90 (d, *J*=8.3 Hz, 2H), 7.24 - 7.11 (m, 2H), 5.30 (d, *J*=1.8 Hz, 1H), 4.70 (d, *J*=2.0 Hz, 1H), 4.60 (dd, *J*=2.3, 1.3 Hz, 1H), 2.56 (d, *J*=21.1 Hz, 1H), 2.44 - 2.33 (m, 1H), 2.26 - 2.05 (m, 3H), 2.04 - 1.89 (m, 1H), 1.86 (d, *J*=12.0 Hz, 1H), 1.82 - 1.58 (m, 18H), 1.56 - 1.37 (m, 7H), 1.35 - 1.21 (m, 6H), 1.19 - 1.06 (m, 6H), 1.03 (s, 3 H), 0.99 (s, 3 H), 0.93 (m, 6H).

### Step 2. Preparation of 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

To a solution of *tert*-butyl 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (62 mg, 0.099 mmol) in DCM (4 mL) was added TFA (0.4 ml, 5.19 mmol). The mixture was stirred at rt for 6 h. The solvents were removed in vacuo and the residue was purified by reverse phase prep. HPLC to afford the title compound as a white foam (15 mg, 26.6%) LCMS: m/e 570.36 (MH⁺), 2.363 min (method 9). ¹H NMR (400MHz, METHANOL-d₄) δ 8.02 - 7.84 (m, 2H), 7.24 (d, *J*=8.3 Hz, 2H), 5.33 (s, 1H), 4.78 (s, 1H), 4.66 (s, 1H), 3.28 - 3.14 (m, 1H), 2.93 - 2.72 (m, 1H), 2.59 - 2.41 (m, 1H), 2.24 - 1.95 (m, 2H), 1.92 - 1.68 (m, 12H), 1.56 (br. s., 8H), 1.31 (m, 5H), 1.19 (m, 5H) 1.10 (s, 3 H), 1.06 (s, 3 H), 0.99 (s, 3 H), 0.97 (s, 3H).

### Example 178. Preparation of 4-((1R,3aS,5aR,5bR,7aR,11aS,11bR,13aR,13bR)-3a-((2-hydroxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

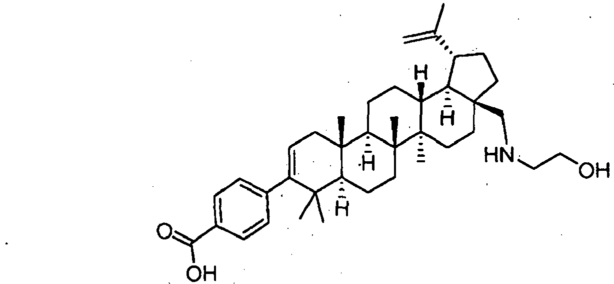

The title compound was prepared following the general procedures described above for the synthesis of 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid using 2-bromoethanol as the alkylating reagent in step 1. The product was isolated as a white solid (21.9 mg, 79.9 %). LCMS: m/e 588.9 (MH⁺), 2.373 min (method 9). ¹H NMR (500MHz, DMSO-d₆) δ 7.88 (d, *J*=8.2 Hz, 2H), 7.23 (d, *J*=8.2 Hz, 2H), 5.25 (d, *J*=4.9 Hz, 1H), 4.72 (s, 1H), 4.61 (s, 1H), 3.74 (m, 2H), 3.14 (br. s., 1H), 3.08 (m, 2H), 2.80 - 2.68 (m, 1H), 2.45 (m, 1H), 2.12 - 1.94 (m, 2H), 1.93 - 1.79 (m, 2H), 1.74 - 1.58 (m, 8H), 1.57 - 1.29 (m, 8H), 1.28 - 1.13 (m, 4H), 1.08 (s, 4H) 1.00 (3 H, s), 0.96 (3 H, s), 0.91 (6 H, s).

### Example 179. Preparation of 4-((1R,3aS,5aR,5bR,7aR,11aS,11bR,13aR,13bR)-5a,5b,8,8,11a-pentamethyl-3a-((2-(4-(methylsulfonyl)piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

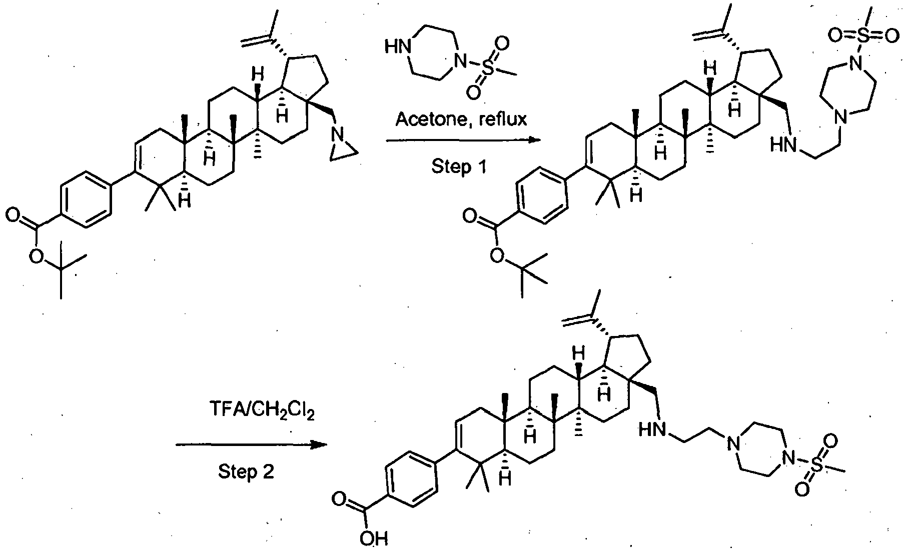

### Step 1. Preparation of tert-butyl 4-((1R,3aS,5aR,5bR,7aR,11aS,11bR,13aR,13bR)-5a,5b,8,8,11a-pentamethyl-3a-((2-(4-(methylsulfonyl)piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

A mixture of *tert*-butyl 4-((1R,3aS,5aR,5bR,7aR,11aS,11bR,13aR,13bR)-3a-(aziridin-1-ylmethyl)-5a,5b,8,8,11a-pentamethyl-1-(prop-2-en-2-yl)-2,3,3a,4,5,5a,5b,6,7,7a,8,11,11a,11b,12,13,13a,13b-octadecahydro-1H-cyclopenta[a]chrysen-9-yl)benzoate (30 mg, 0.048 mmol) and 1-(methylsulfonyl)piperazine (78.7 mg, 0.48 mmol) in acetone (5 mL) was heated at reflux in a sealed tube for 18 h. The desired compound was detected by LCMS (M+1=790.7, 2.96 min, method 9).

### Step 2. Preparation of 4-((1R,3aS,5aR,5bR,7aR,11aS,11bR,13aR,13bR)-5a,5b,8,8,11a-pentamethyl-3a-((2-(4-(methylsulfonyl)piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

The title compound was prepared following the general procedures described above for the synthesis of 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b12)13,13a,13b-octadecahydro-1H-cyclopenta[a]chrysen-9-yl)benzoic acid, step 2. The product was isolated as a white solid (48 mg, 47.5 %). LCMS: m/e 734.5 (MH⁺), 2.35 min (method 9). ¹H NMR (400MHz, METHANOL-d₄) δ 7.95 (d, *J*=8.3 Hz, 2H), 7.24 (d, *J*=8.3 Hz, 2H), 5.36-5.27 (m, 1H), 4.78 (s, 1H), 4.67 (s, 1H), 3.56 - 3.42 (m, 6H), 3.30 (m, 3H), 3.24 - 3.09 (m, 4H), 3.06 - 2.87 (m, 4H), 2.58 - 2.50 (m, 1H), 2.23 - 1.99 (m, 2H), 1.94 - 1.68 (m, 10H), 1.65 - 1.46 (m, 8H), 1.42 - 1.24 (m, 4H) 1.19 (m, 4H) 1.10 (3 H, s), 1.06 (3 H, s), 0.99 (3 H, s), 0.97 (3 H, s).

### Example 180. Preparation of 4-((1R,3aS,5aR,5bR,7aR,11aS,11bR,13aR,13bR)-3a-((2-methoxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

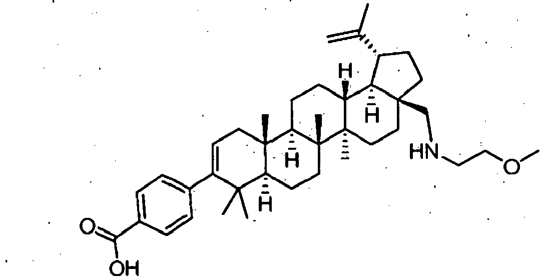

The title compound was prepared from *tert*-butyl 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate following the procedure described above for the synthesis of 4-((1R,3aS,5aR,5bR,7aR,11aS.11bR,13aR,13bR)-5a,5b,8,8,11a-pentamethyl-3a-((2-(4-(methylsulfonyl)piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid, using methanol as reagent in step 1. The product was isolated as a white solid (1.6 mg, 5.2 %). LCMS: m/e 602.49 (MH⁺), 2.40 min (method 9). ¹H NMR (500MHz, METHANOL-d₄) δ 7.85 (d, *J*=7.6 Hz, 2H), 7.12 (d, *J*=7.6 Hz, 2H), 5.29 (s, 1H), 4.79 - 4.76 (s, 1H), 4.66 (s, 1H), 3.67 - 3.64 (m, 2H), 3.43 (s, 3H), 3.21 - 3.15 (m, 3H), 2.69 - 2.62 (m, 1H), 2.52 (m, 1H), 2.19 - 2.10 (m, 1H), 2.07 - 2.00 (m, 1H), 1.79 (m, 8H), 1.57 (m, 8H), 1.40 - 1.24 (m, 7H), 1.18 (m, 4H), 1.09 (3 H, s), 1.05 (3 H, s), 0.97 (3 H, s), 0.96 (3 H, s).

### Example 181. Preparation of 4-((1R,3aS,5aR,5bR,7aR,11aS,11bR,13aR,13bR)-3a-((2-(4-(hydroxymethyl)piperidin-1-yl)ethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

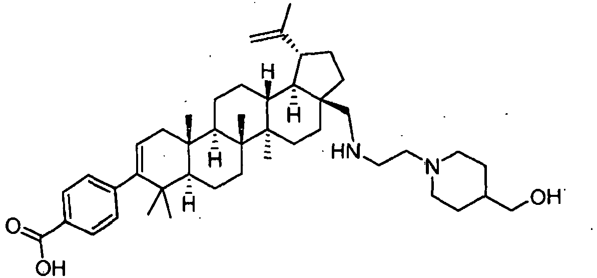

The title compound was prepared from *tert*-butyl 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate following the procedure described above for the synthesis of 4-((1R,3aS,5aR,5bR,7aR,11aS,11bR,13aR,13bR)-5a,5b,8,8,11a-pentamethyl-3a-((2-(4-(methylsulfonyl)piperazin-1-yl)ethylamino)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid, using piperidin-4-ylmethanol as reagent in step 1. The product was isolated as a white solid (5 mg, 16.7 %). LCMS: m/e 685.5 (MH⁺), 2.33 min (method 9). ¹H NMR (400MHz, METHANOL-d₄) δ 7.90 (d, *J=8.0* Hz, 2H), 7.20 (d, *J=8.0* Hz, 2H), 5.28 (d, *J=*4.5 Hz, 1H), 4.74 (br. s., 1H), 4.63 (br. s., 1H), 3.74 - 3.38 (m, 8H), 3.07 (br. s., 2H), 2.98 - 2.85 (m, 2H), 2.48 (br. s., 1H), 2.21 - 1.93 (m, 4H), 1.90 - 1.64 (m, 11H), 1.63 - 1.40 (m, 10H), 1.39 - 1.24 (m, 4H), 1.23 - 1.09 (m, 4H), 1.04 (m, 6H), 0.94 (m, 6H).

### Example 182. Preparation of 4-((1R,3aS,5aR,5bR,7aR,11aS,11bR,13aR,13bR)-5a,5b,8,8,11a-pentamethyl-3a-((2-oxooxazolidin-3-yl)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

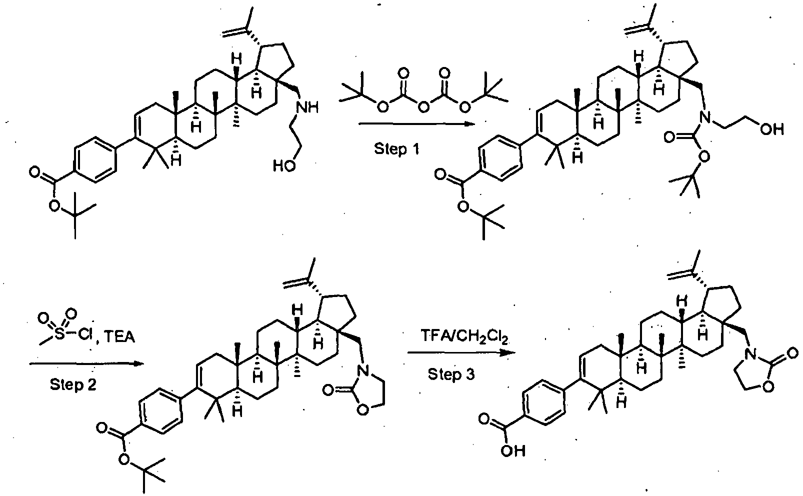

### Step 1. Preparation of tert-butyl 4-((1R,3aS,5aR,5bR,7aR,11aS,11bR,13aR,13bR)-3a-((tert-butoxycarbonyl(2-hydroxyethyl)amino)methyl)-5a,5b,8,8,11a-pentamethyl-1-(prop-1-en-2-yl)-2,3,3a,4,5,5a,5b,6,7,7a,8,11,11 a,11b,12,13,13a,13b-octadecahydro-1H-cyclopenta[a]chrysen-9-yl)benzoate

*tert*-butyl 4-((1R,3aS,5aR,5bR,7aR,11aS,11br,13aR,13bR)-3a-((2-hydroxyethylamino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (600 mg, 0.932 mmol) was dissolved in CH₂Cl₂ (10 mL), BOC₂O (0.260 mL, 1.118 mmol) was added followed by Hunig's Base (0.163 mL, 0.932 mmol) to form a colorless solution that was stirred at 0 °C for 6 hrs. LCMS indicated formation of desired product (M+23=767). The solvent was removed in vacuo and the resultant white solid was used as is in the next step.

### Step 2. Preparation of tert-butyl 4-((1R,3aS,5aR,5bR,7aR,11aS,11bR,13aR,13bR)-3a-((tert-butoxycarbonyl(2-hydroxyethyl)amino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate

*tert*-butyl 4-((1R,3aS,5aR,5bR,7aR,11aS,11bR,13aR,13bR)-3a-(((tert-butoxycarbonyl)(2-hydroxyethyl)amino)methyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoate (133 mg, 0.179 mmol) was dissolved in CH₂Cl₂ (5 mL) and the solution was cooled at 0°C. Methanesulfonyl chloride (30.7 mg, 0.268 mmol) was added followed by TEA (0.050 mL, 0.357 mmol) and the mixture was stirred for 18 h. The solvent was removed and the resultant residue was purified by silica gel chromatography to afford the title compound as a white solid (100 mg, 83.3 %). ¹H NMR (400MHz, CHLOROFORM-d) δ 7.95 - 7.86 (m, *J*=8.3 Hz, 2H), 7.24-7.10 (m, *J=*8.3 Hz, 2H), 5.36 - 5.24 (m, 1H), 4.82 - 4.68 (m, 1H), 4.62 (s, 1H), 4.39 - 4.26 (m, 2H), 3.75 - 3.59 (m, 2H), 3.46 (d, *J*=13.8 Hz, 1H), 3.10 (d, *J*=14.3 Hz, 1H), 2.58 - 2.39 (m, 1H), 2.24 - 2.04 (m, 2H), 1.98 - 1.69 (m, 8H), 1.69 - 1.58 (m, 12H), 1.57 - 1.39 (m, 8H), 1.36 - 1.22 (m, 4H), 1.21 - 1.07 (m, 5H), 1.07 - 0.97 (m, 6H), 0.97 - 0.80 (m, 6H).

### Step 3. Preparation of 4-((1R,3aS,5aR,5bR,7aR,11aS,11bR,13aR,13bR)-5a,5b,8,8,11a-pentamethyl-3a-((2-oxooxazolidin-3-yl)methyl)-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid

The title compound was prepared following step 2 of the procedure described above for the synthesis of 4-((1R,3aS,5aR,5bR,7aR,11aS,11bR,13aR,13bR)-3a-(aziridin-1-ylmethyl)-5a,5b,8,8,11a-pentamethyl-1-(prop-1-en-2-yl)-2,3,3a,4,5,5a,5b,6,7,7a,8,11,11a,11b,12,13,13a,13b-octadecahydro-1H-cyclopenta[a]chrysen-9-yl)benzoic acid. The product was isolated as a white solid (15 mg, 14.7%). LCMS: m/e 614.5 (MH⁺), 2.47 min (method 8). ¹H NMR (400MHz, CHLOROFORM-d) δ 8.02 (d, *J*=8.3 Hz, 2H), 7.24 (d, *J*=8.3 Hz, 2H), 5.32 (d, *J*=4.5 Hz, 1H), 4.74 (s, 1H), 4.62 (s, 1H), 4.34 (t, *J*=7.9 Hz, 2H), 3.81 - 3.59 (m, 2H), 3.47 (m, 2H), 3.20 - 3.00 (m, 1H), 2.51 (td, *J*=11.2, 5.5 Hz, 1H), 2.26 - 2.06 (m, 2H), 1.97 - ' 1.66 (m, 10H), 1.50 - 1.22 (m, 10H), 1.15 (s, 4H), 1.06 - 0.83 (m, 13H)

### Biology Data for the Examples

- "µM" means micromolar;
- "mL" means milliliter;
- "µl" means microliter;
- "mg" means milligram;
- "µg" means microgram;

The materials and experimental procedures used to obtain the results reported in Tables 1-2 are described below.

*HIV cell culture assay -* MT-2 cells and 293T cells were obtained from the NIH AIDS Research and Reference Reagent Program. MT-2 cells were propagated in RPMI 1640 media supplemented with 10% heat inactivated fetal bovine serum, 100 µg/ml penicillin G and up to 100 units/ml streptomycin. The 293T cells were propagated in DMEM media supplemented with 10% heat inactivated fetal bovine serum (FBS), 100 units/ml penicillin G and 100 µg/ml streptomycin. The proviral DNA clone of NL₄₋₃ was obtained from the NIH AIDS Research and Reference Reagent Program. A recombinant NL₄₋₃ virus, in which a section of the nef gene from NL4-3 was replaced with the *Renilla* luciferase gene, was used as a reference virus. In addition, residue Gag P373 was converted to P373S. Briefly, the recombinant virus was prepared by transfection of the altered proviral clone of NL₄₋₃. Transfections were performed in 293T cells using LipofectAMINE PLUS from Invitrogen (Carlsbad, CA), according to manufacturer's instruction. The virus was titered in MT-2 cells using luciferase enzyme activity as a marker. Luciferase was quantitated using the Dual Luciferase kit from Promega (Madison, WI), with modifications to the manufacturer's protocol. The diluted Passive Lysis solution was pre-mixed with the re-suspended Luciferase Assay Reagent and the re-suspended Stop & Glo Substrate (2:1:1 ratio). Fifty (50) µL of the mixture was added to each aspirated well on assay plates and luciferase activity was measured immediately on a Wallac TriLux (Perkin-Elmer). Antiviral activities of inhibitors toward the recombinant virus were quantified by measuring luciferase activity in cells infected for 4-5 days with NLRluc recombinants in the presence serial dilutions of the inhibitor. The EC₅₀ data for the compounds is shown in Table 2. Table 1 is the key for the data in Table 2.

### Results

**Table 1. Biological Data Key for EC₅₀**

| Compounds with EC₅₀ >0.1 µM | Compounds with EC₅₀ < 0.1 µM |
|---|---|
| "B" | Group "A" |

**Table 2**

| Examples # | Structure | EC₅₀ |
|---|---|---|
| 1 | 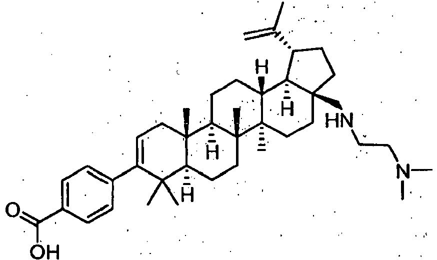 | A |
| 2 | 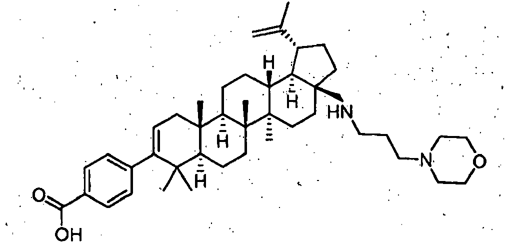 | A |
| 3 | 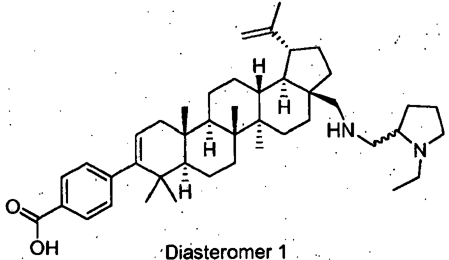 | 0.03 |
| 4 | 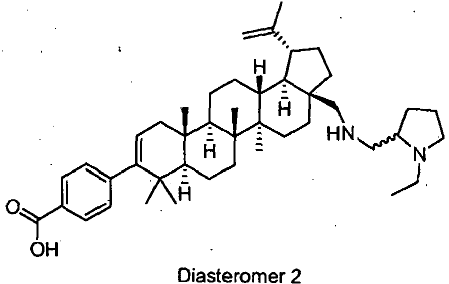 | A |
| 5 | 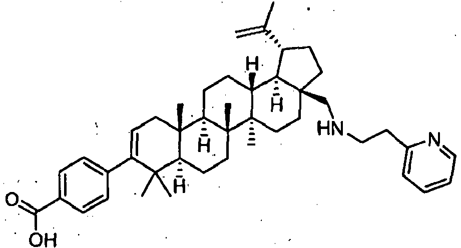 | 0.02 |
| 6 | 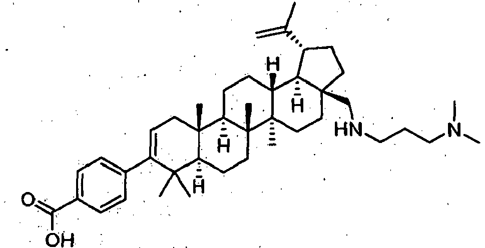 | 0.03 |
| 7 | 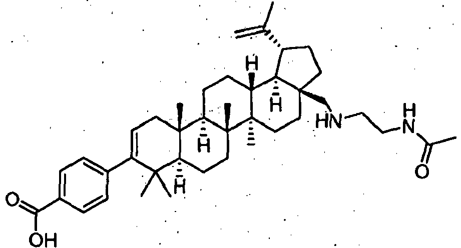 | A |
| 8 | 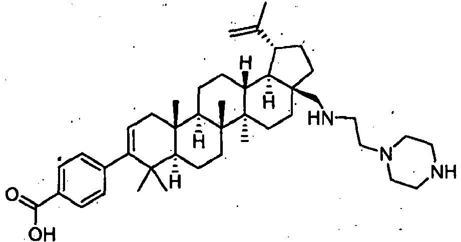 | A |

| Example # | Structure | EC₅₀ |
|---|---|---|
| 9 | 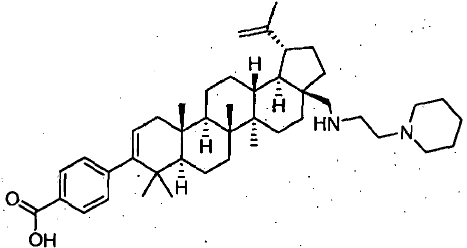 | A |
| 10 | 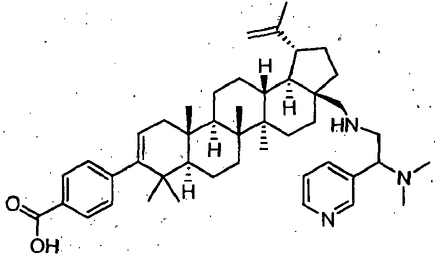 | 0.004 |
| 11 | 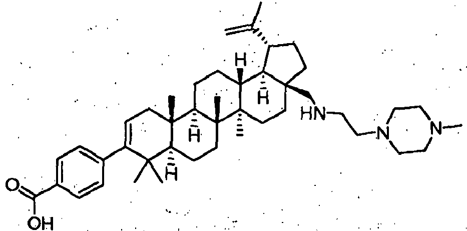 | A |
| 12 | 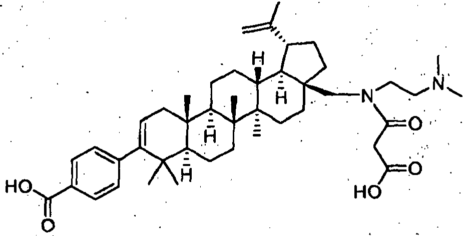 | A |
| 13 | 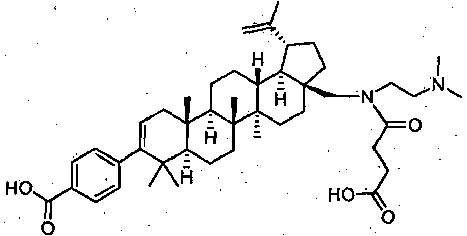 | A |

| Example # | Structure | ECso |
|---|---|---|
| 14 | 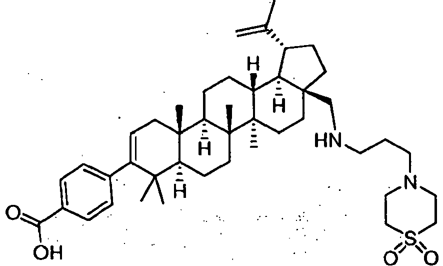 | A |
| 15 | 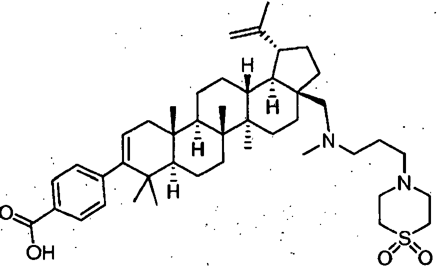 | A |
| 16 | 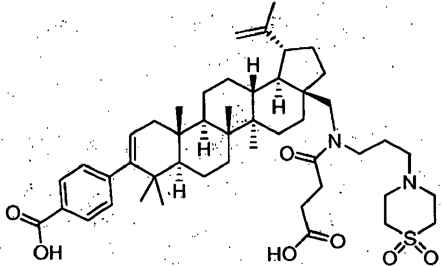 | A |
| 17 | 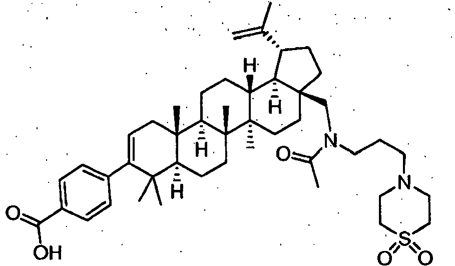 | A |

| Example # | Structure | EC₅₀ |
|---|---|---|
| 18 | 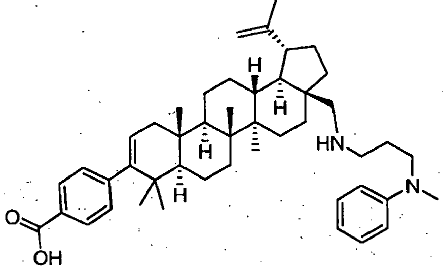 | A |
| 19 | 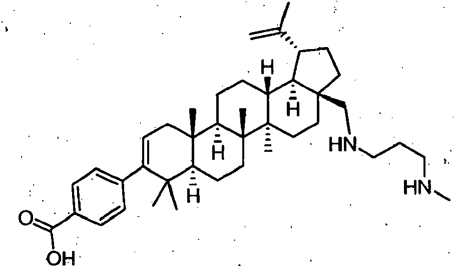 | A |
| 20 | 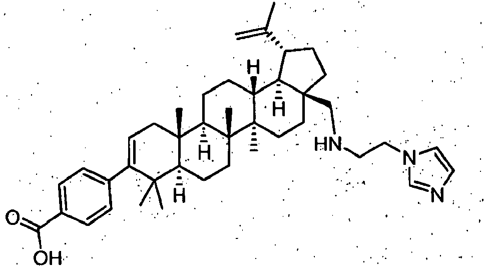 | A |
| 21 | 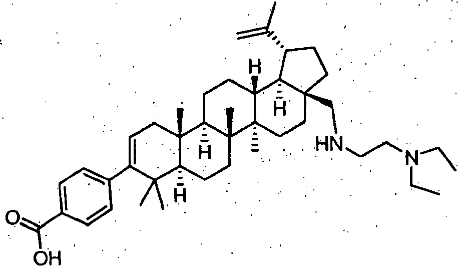 | A |
| 22 | 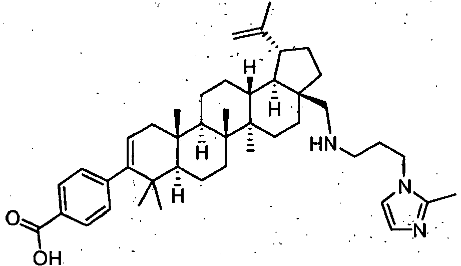 | A |
| 23 | 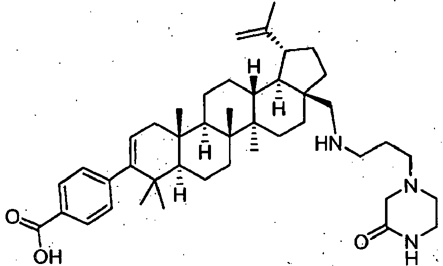 | A |
| 24 | 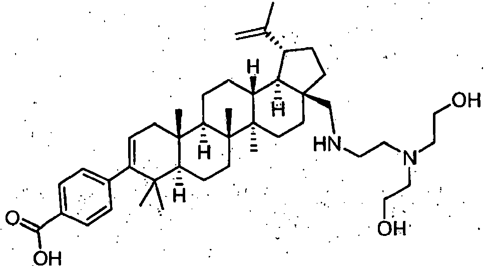 | A |
| 25 | 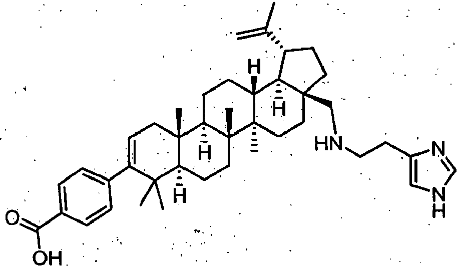 | A |
| 26 | 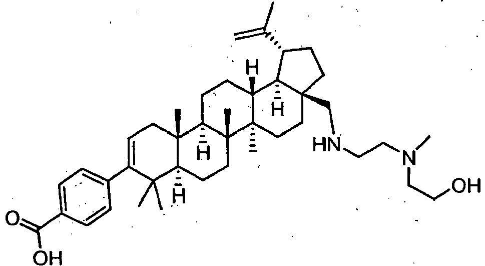 | A |
| 27 | 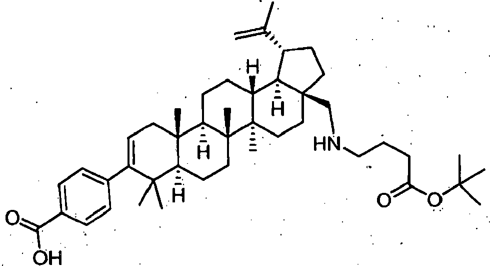 | A |
| 28 | 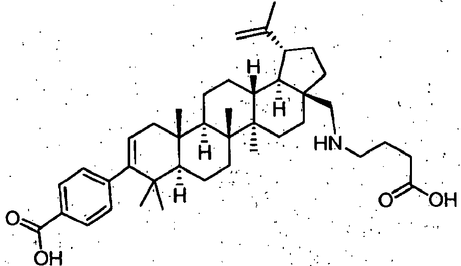 | 0.002 |
| 29 | 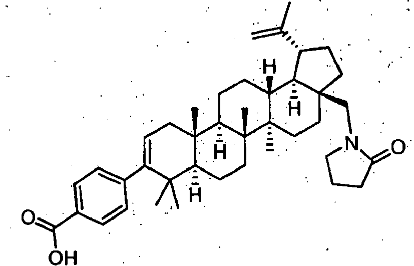 | 1.0 |
| 30 | 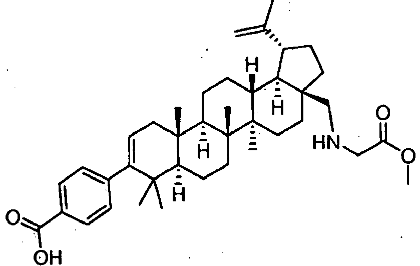 | A |
| 31 | 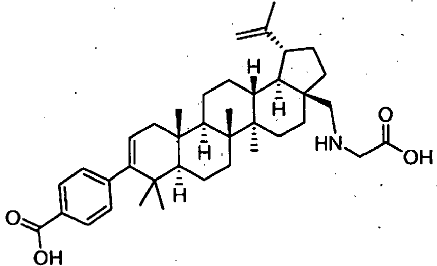 | A |
| 32 | 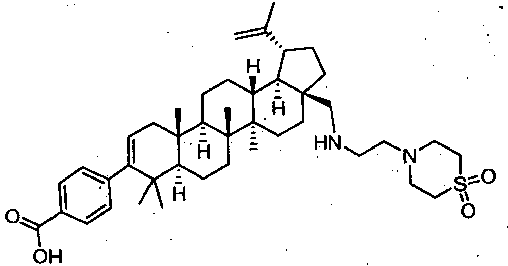 | A |
| 33 | 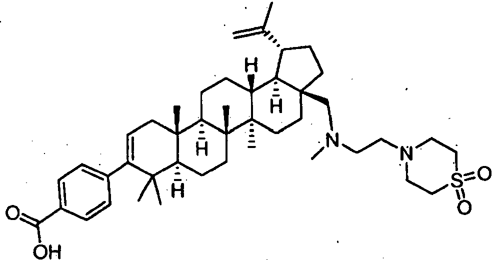 | A |
| 34 | 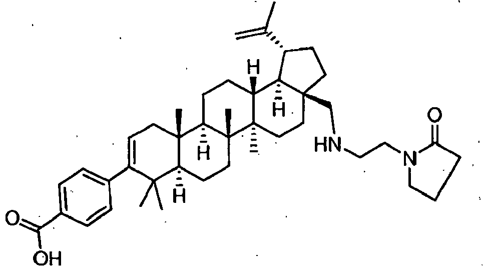 | A |
| 35 | 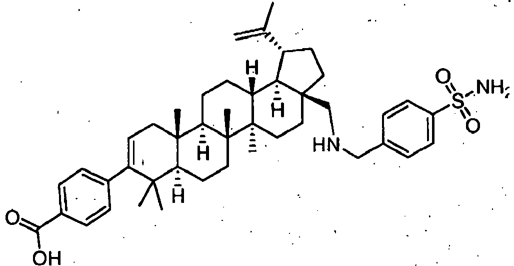 | A |
| 36 | 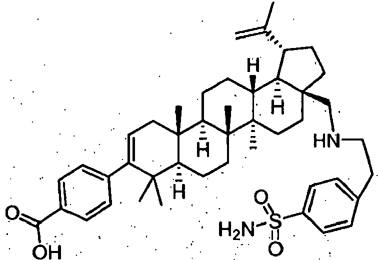 | A |
| 37 | 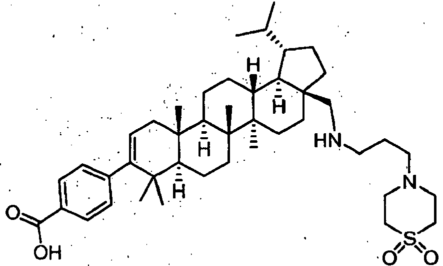 | A |
| 38 | 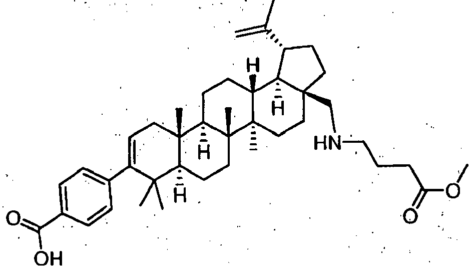 | A |
| 39 | 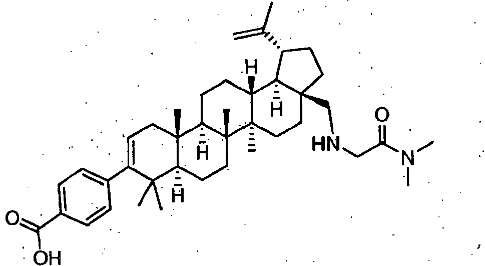 | A |
| 40 | 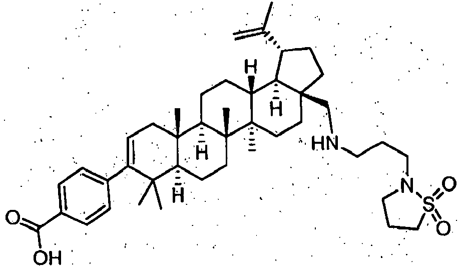 | A |
| 41 | 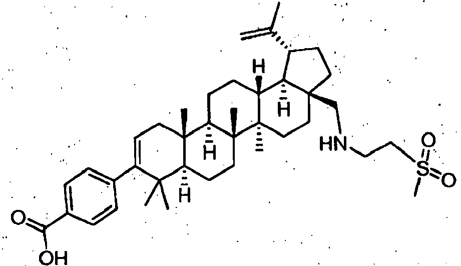 | A |
| 42 | 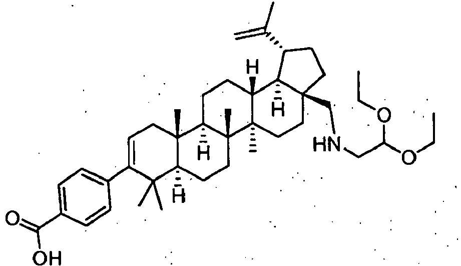 | A |
| 43 | 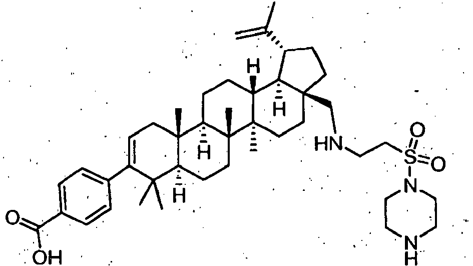 | A |
| 44 | 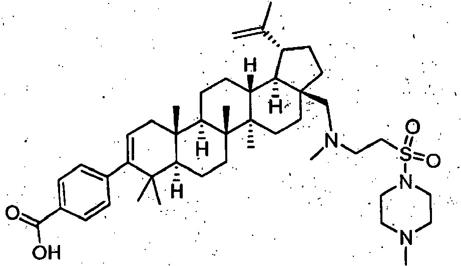 | 0.02 |
| 45 | 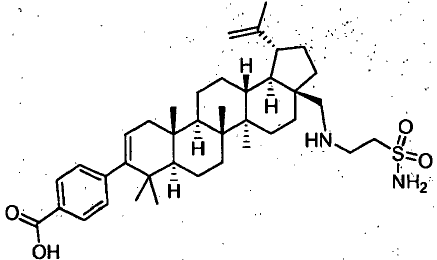 | A |
| 46 | 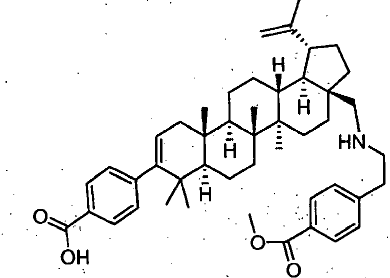 | A |
| 47 | 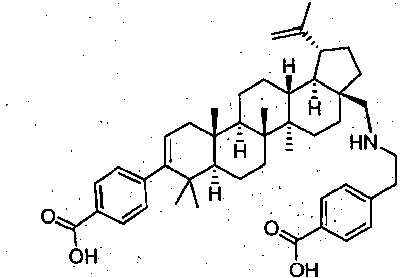 | A |
| 48 | 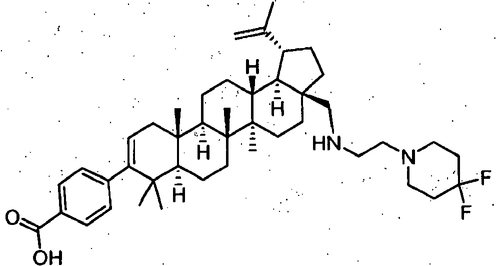 | A |
| 49 | 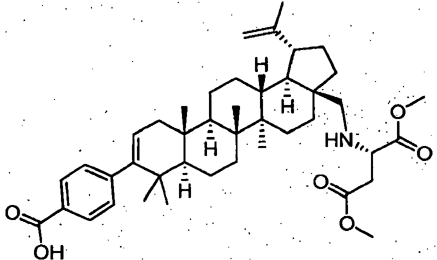 | A |
| 50 | 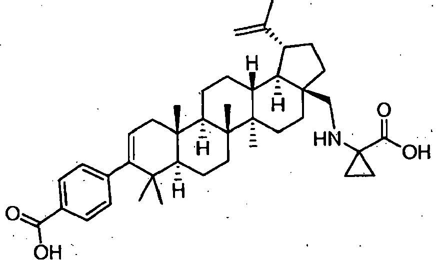 | A |
| 51 | 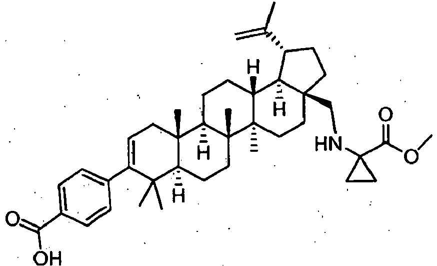 | A |
| 52 | 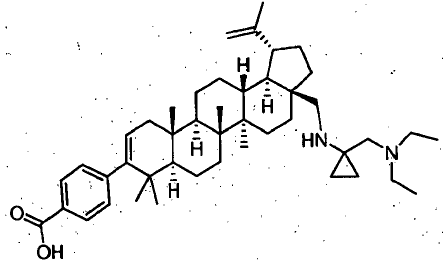 | A |
| 53 | 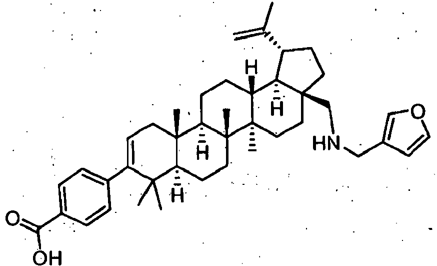 | A |
| 54 | 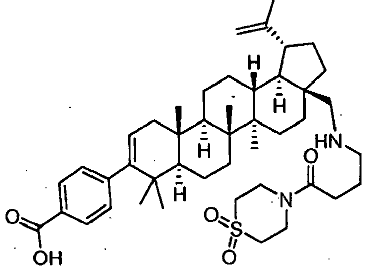 | A |
| 55 | 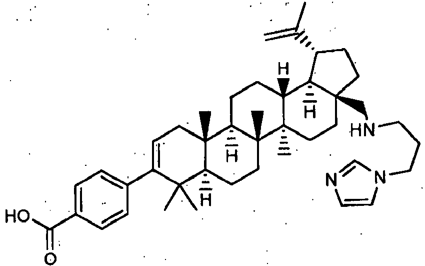 | A |
| 56 | 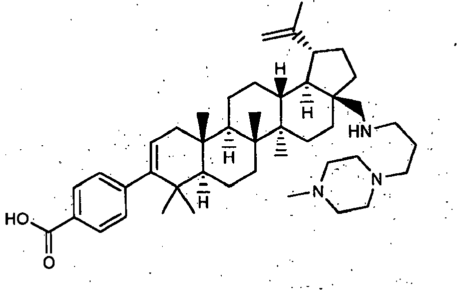 | A |
| 57 | 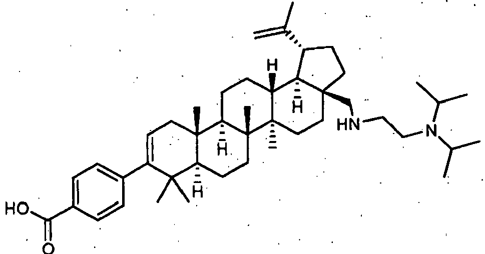 | A |
| 58 | 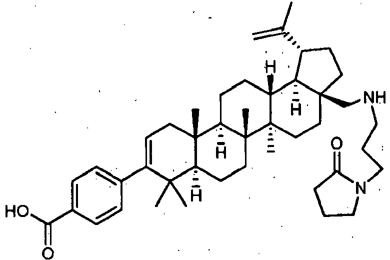 | A |
| 59 | 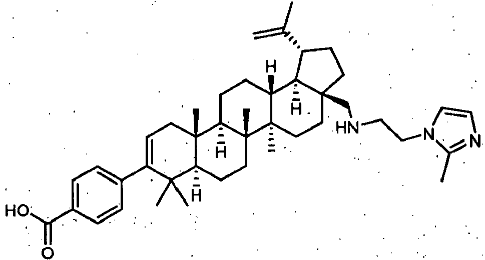 | A |
| 60 | 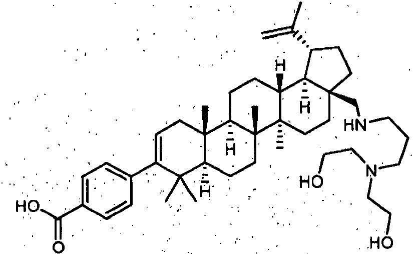 | A |
| 61 | 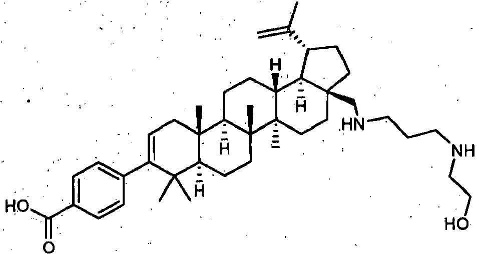 | A |
| 62 | 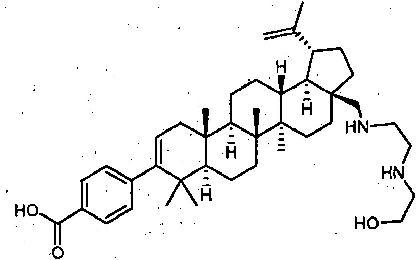 | A |
| 63 | 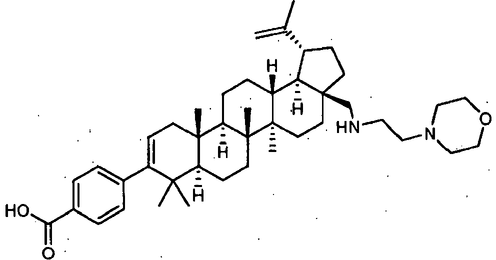 | A |
| 64 | 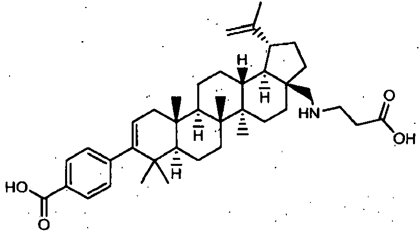 | A |
| 65 | 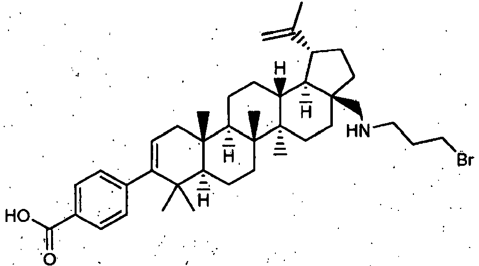 | 0.001 |
| 66 | 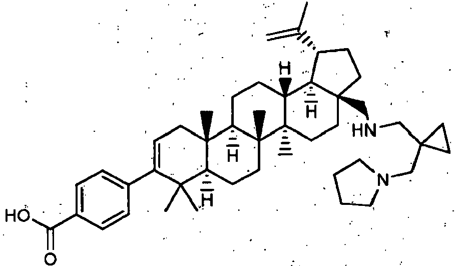 | A |
| 67 | 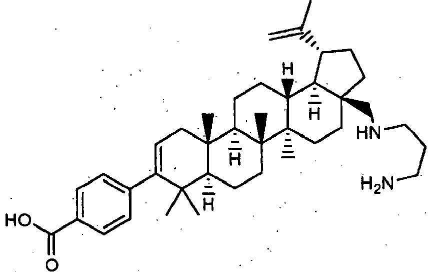 | A |
| 68 | 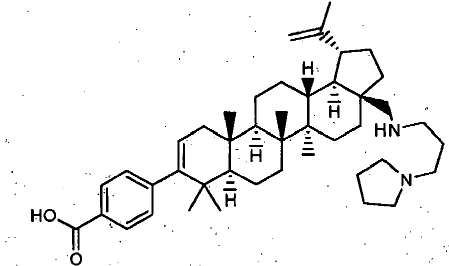 | A |
| 69 | 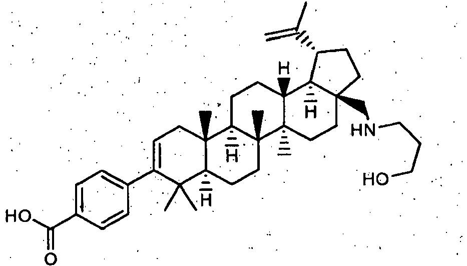 | A |
| 70 | 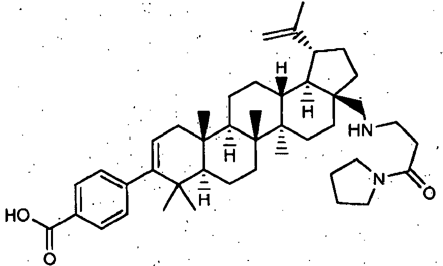 | A |
| 71 | 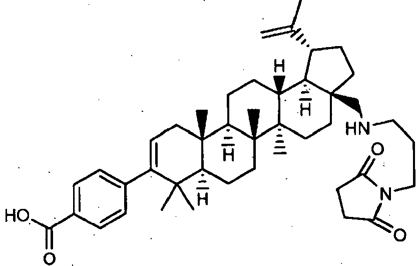 | A |
| 72 | 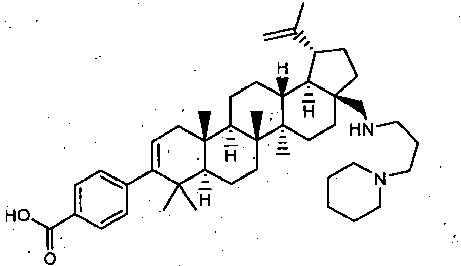 | A |
| 73 | 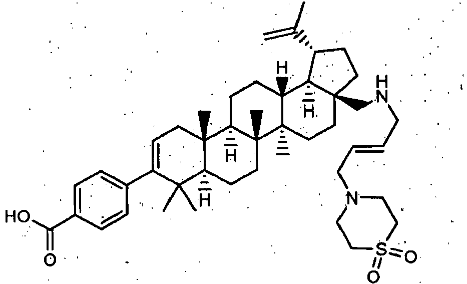 | A |
| 74 | 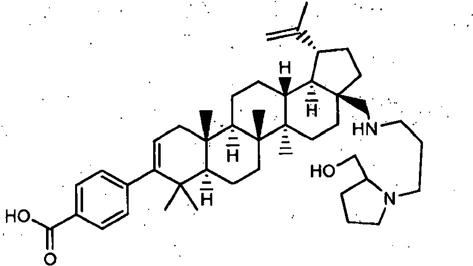 | A |
| 75 | 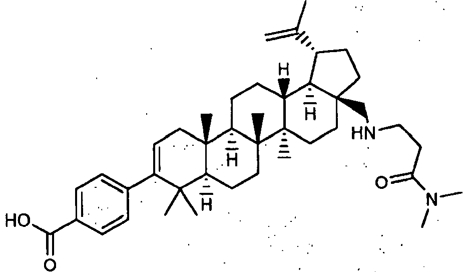 | A |
| 76 | 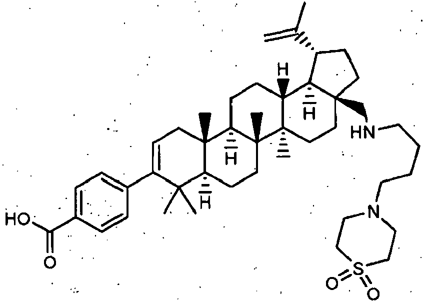 | A |
| 77 | 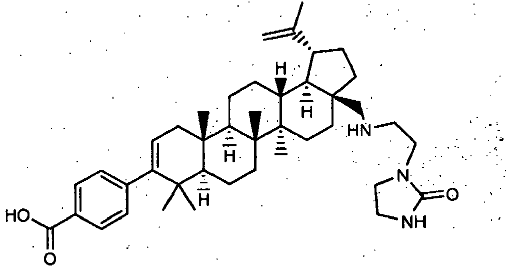 | A |
| 78 | 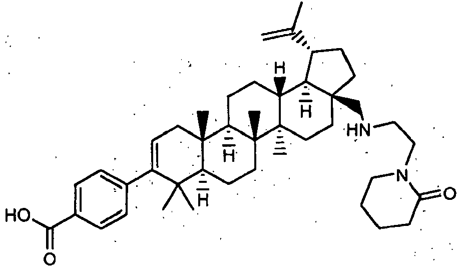 | A |
| 79 | 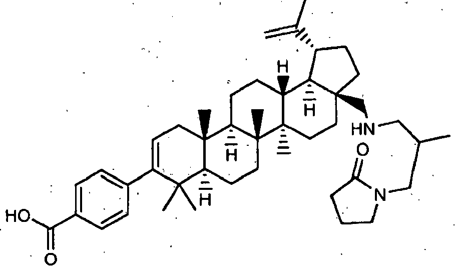 | A |
| 80 | 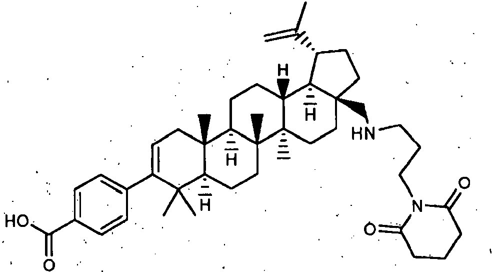 | A |
| 81 | 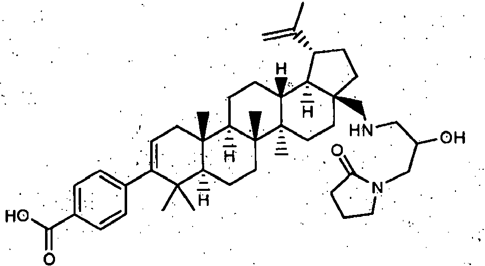 | A |
| 82 | 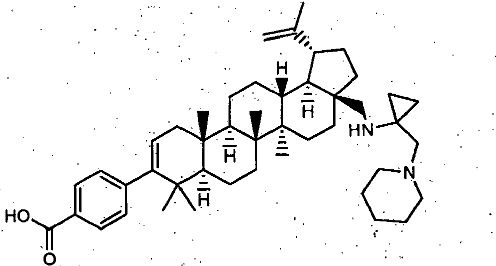 | A |
| 83 | 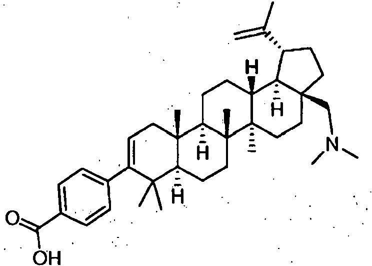 | A |
| 84 | 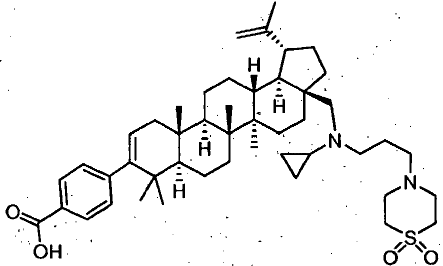 | A |
| 85 | 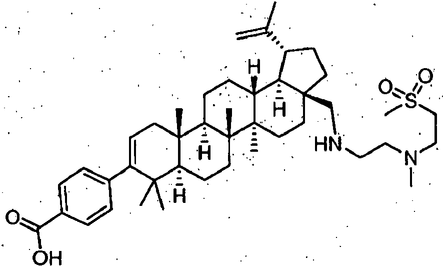 | A |
| 86 | 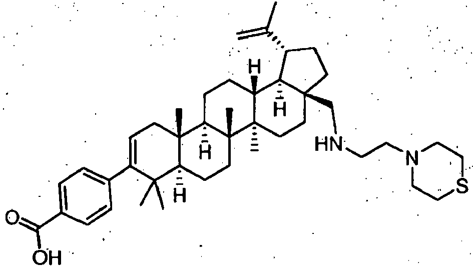 | A |
| 87 | 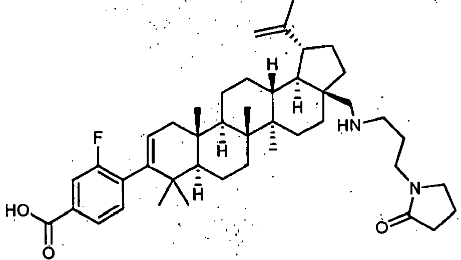 | A |
| 88 | 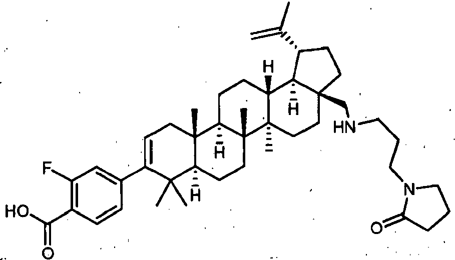 | A |
| 89 | 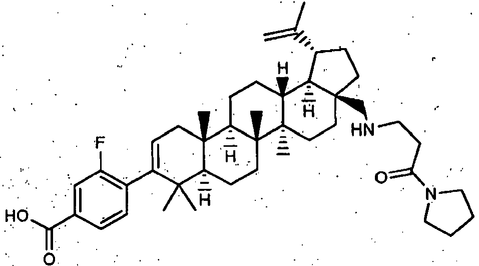 | A |
| 90 | 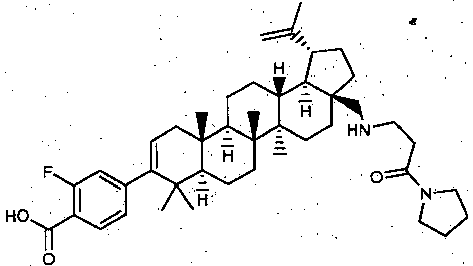 | A |
| 91 | 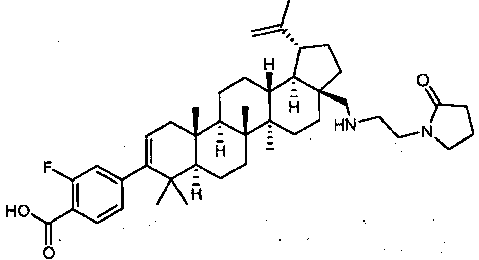 | A |
| 92 | 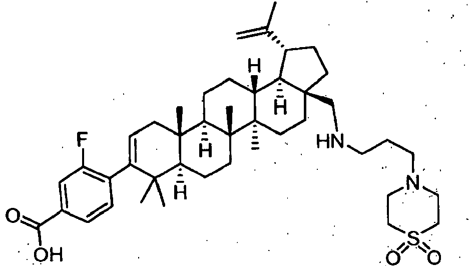 | A |
| 93 | 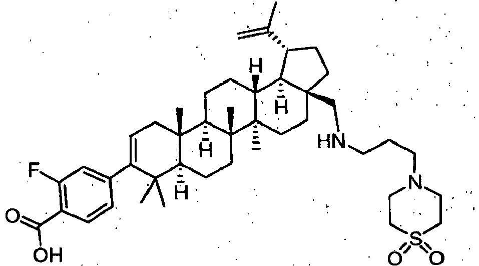 | A |
| 94 | 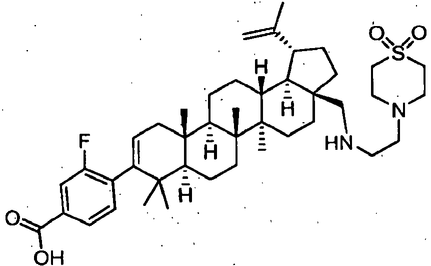 | A |
| 95 | 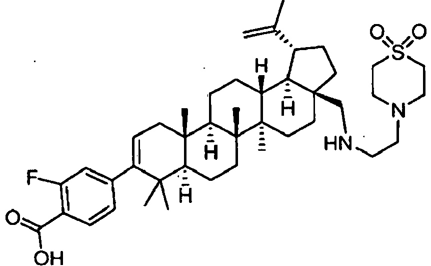 | A |
| 96 | 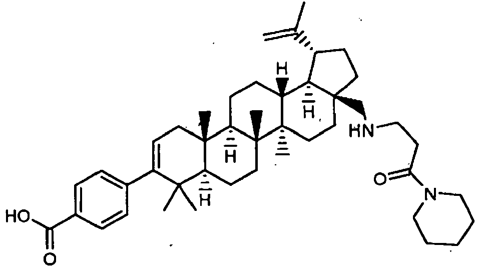 | A |
| 97 | 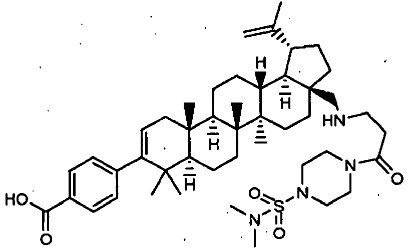 | A |
| 98 | 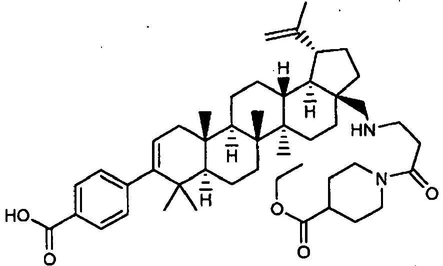 | A |
| 99 | 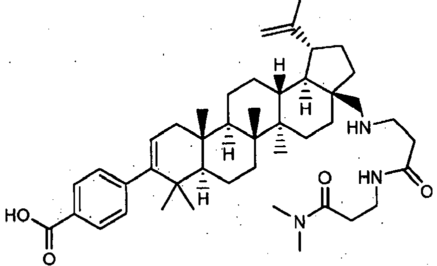 | A |
| 100 | 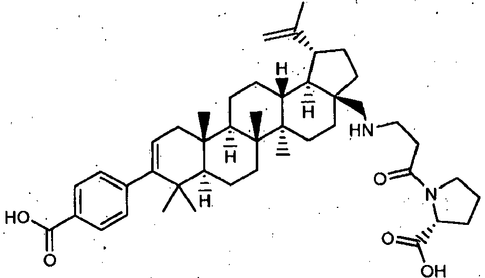 | A |
| 101 | 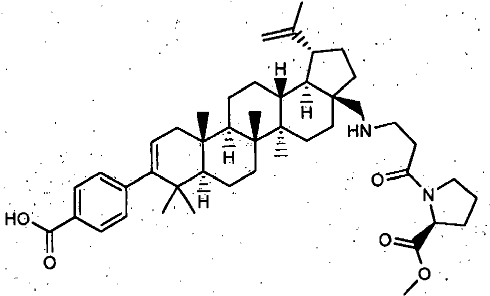 | A |
| 102 | 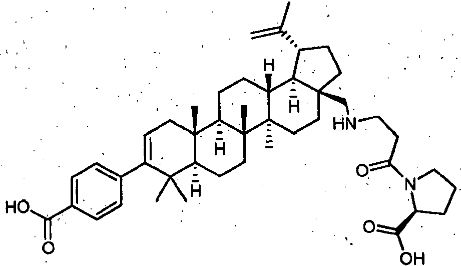 | A |
| 103 | 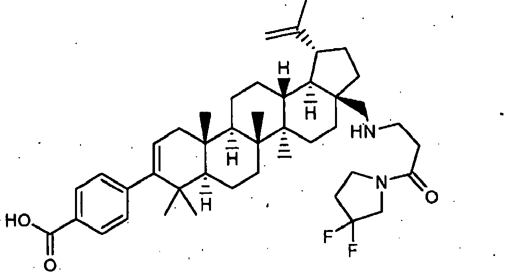 | A |
| 104 | 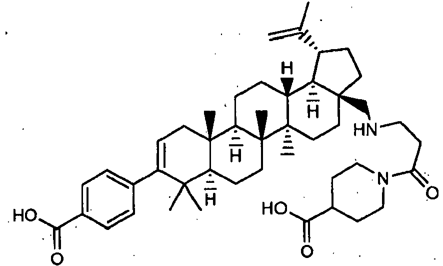 | A |
| 105 | 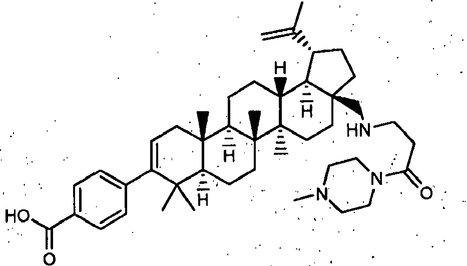 | A |
| 106 | 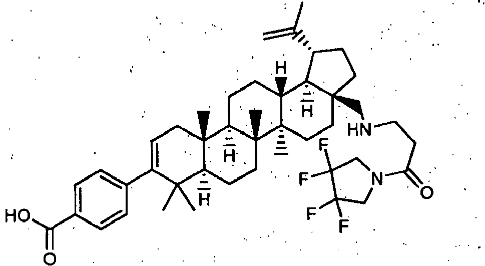 | A |
| 107 | 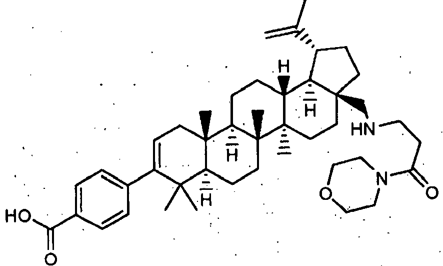 | A |
| 108 | 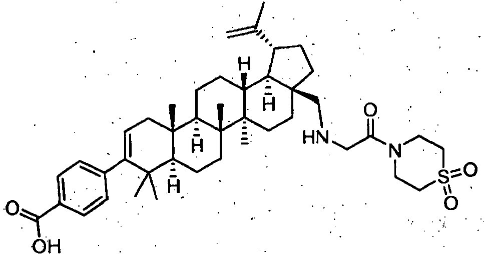 | A |
| 109 | 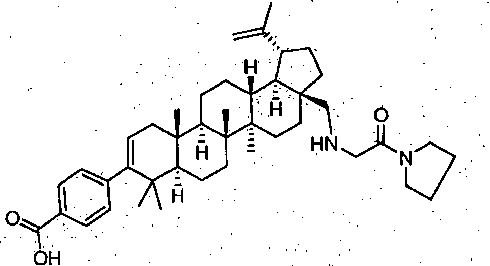 | A |
| 110 | 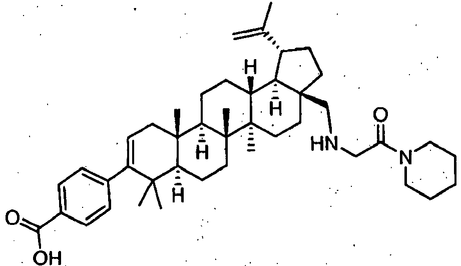 | A |
| 111 | 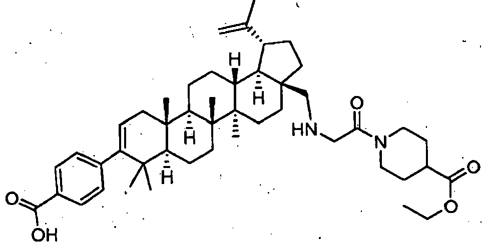 | A |
| 112 | 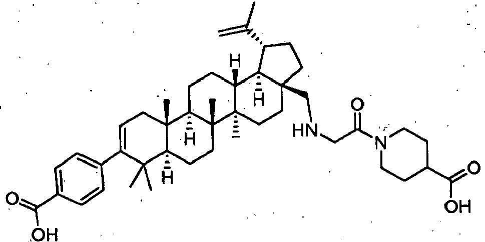 | A |
| 113 | 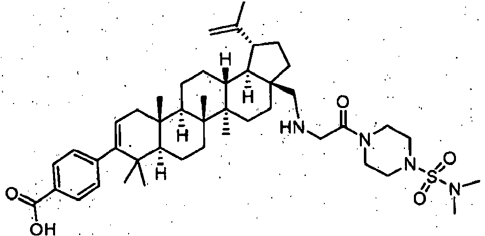 | A |
| 114 | 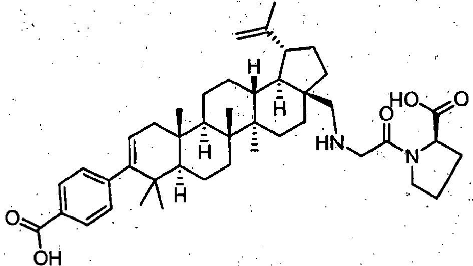 | A |
| 115 | 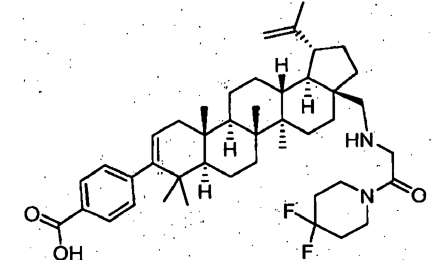 | 0.003 |
| 116 | 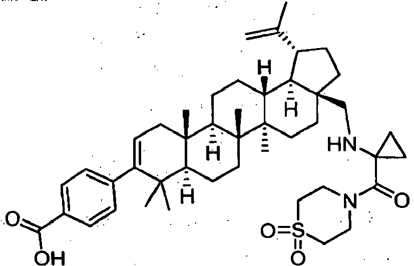 | A |
| 117 | 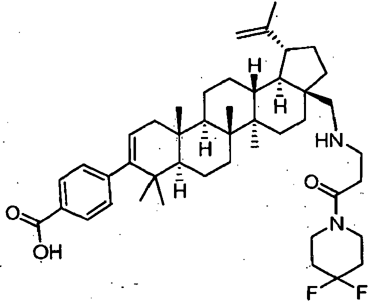 | A |
| 118 | 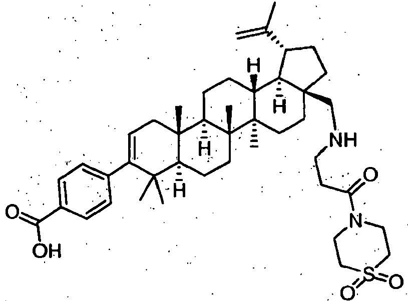 | A |
| 119 | 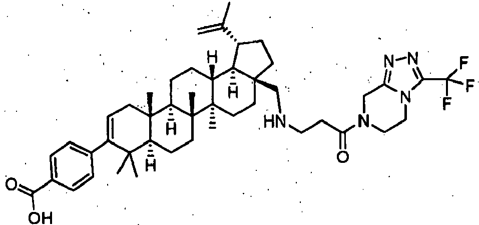 | A |
| 120 | 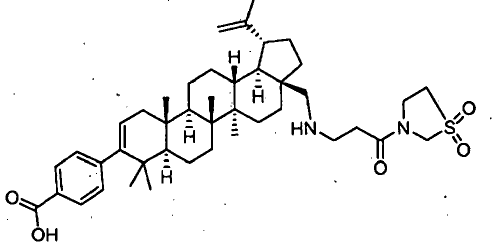 | A |
| 121 | 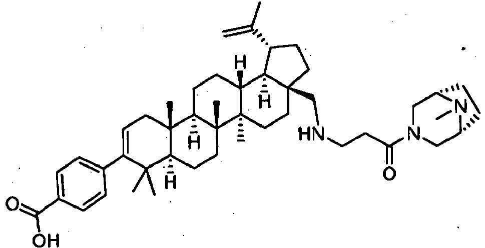 | A |
| 122 | 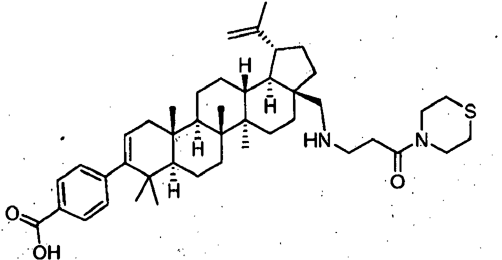 | A |
| 123 | 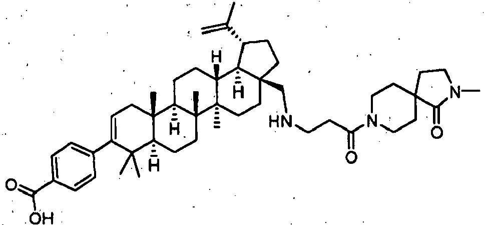 | A |
| 124 | 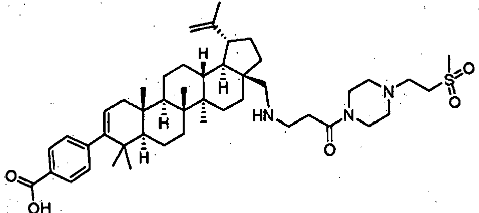 | A |
| 125 | 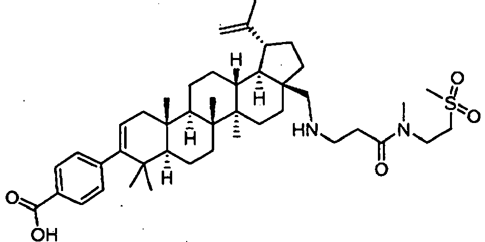 | A |
| 126 | 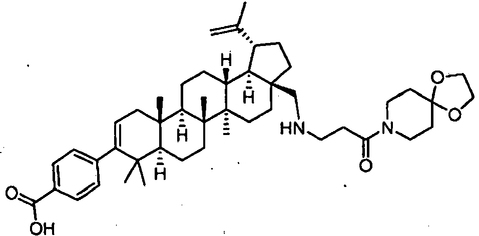 | 0.001 |
| 127 | 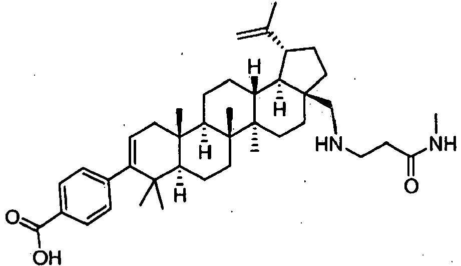 | A |
| 128 | 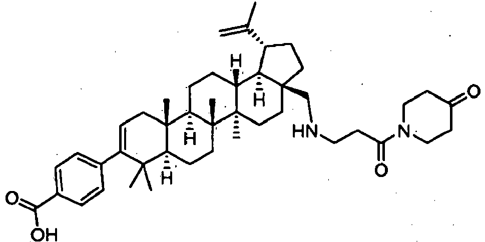 | A |
| 129 | 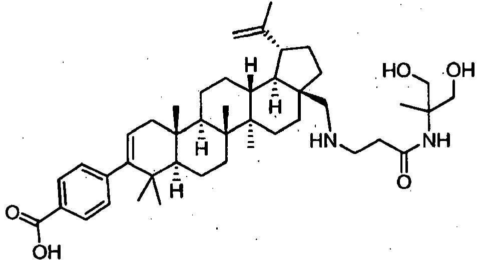 | A |
| 130 | 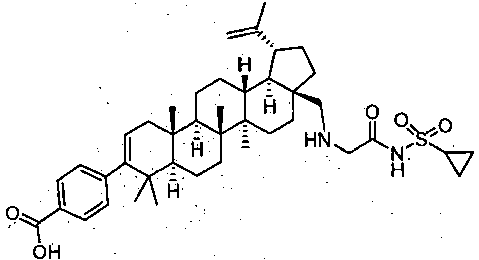 | A |
| 131 | 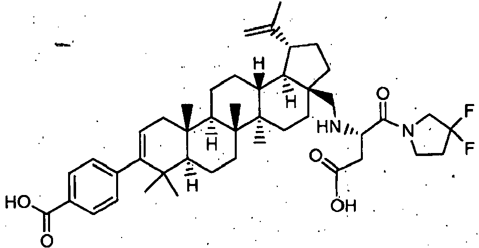 | A |
| 132 | 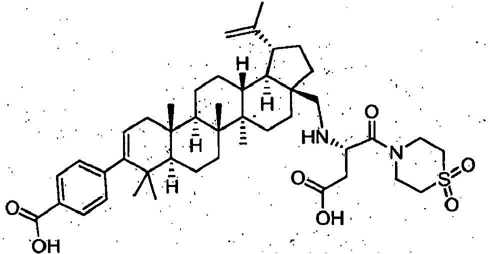 | A |
| 133 | 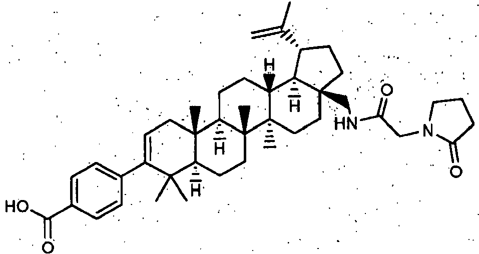 | A |
| 134 | 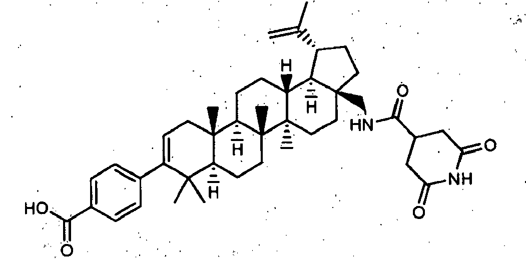 | A |
| 135 | 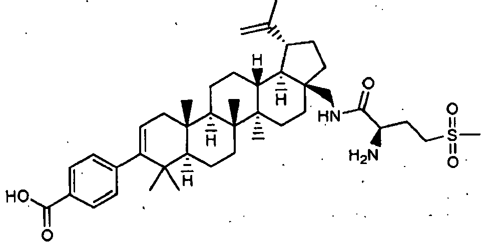 | A |
| 136 | 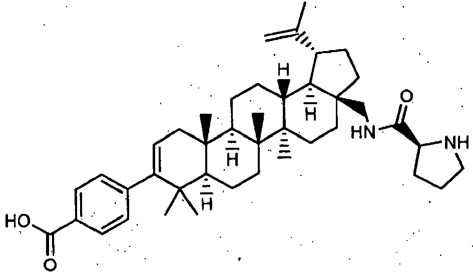 | A |
| 137 | 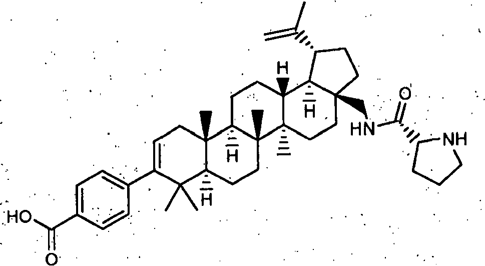 | A |
| 138 | 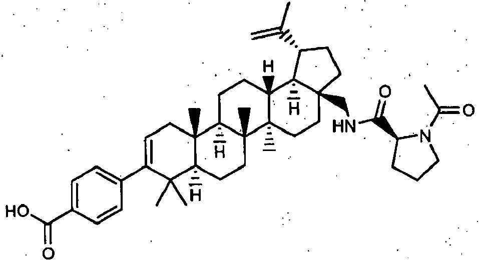 | A |
| 139 | 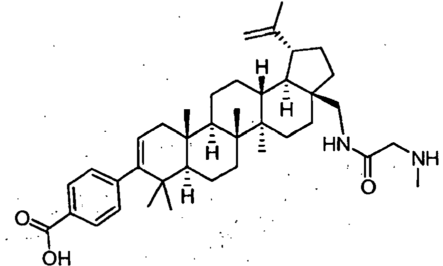 | A |
| 140 | 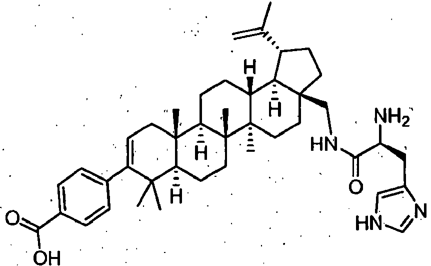 | A |
| 141 | 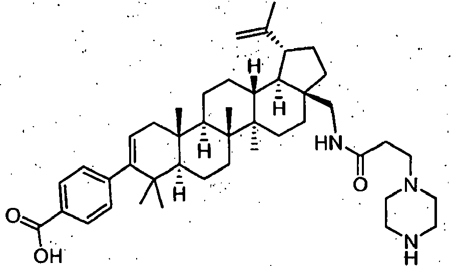 | A |
| 142 | 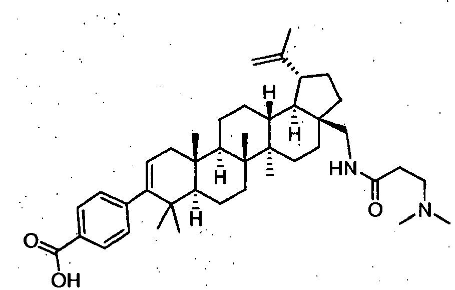 | A |
| 143 | 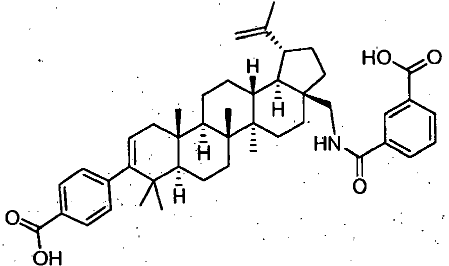 | A |
| 144 | 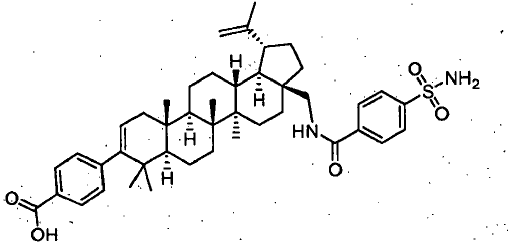 | A |
| 145 | 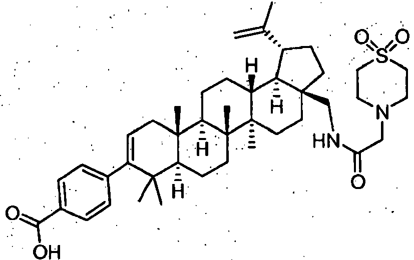 | A |
| 146 | 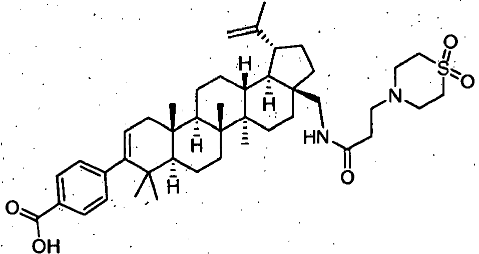 | A |
| 147 | 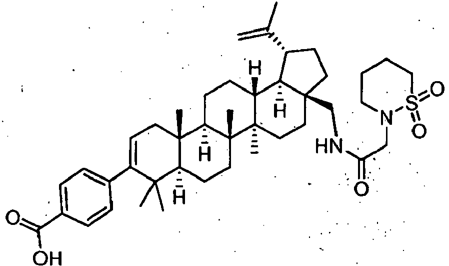 | A |
| 148 | 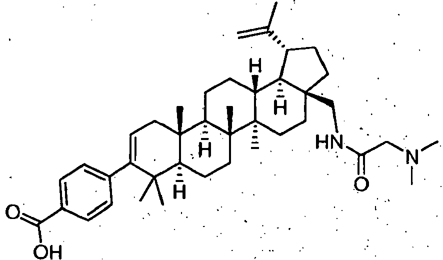 | A |
| 149 | 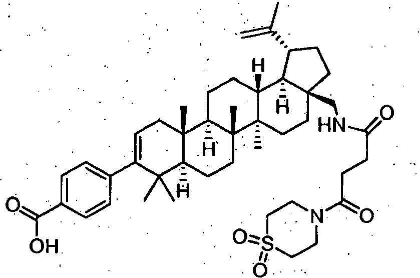 | A |
| 150 | 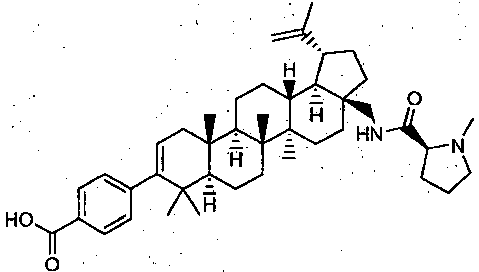 | A |
| 151 | 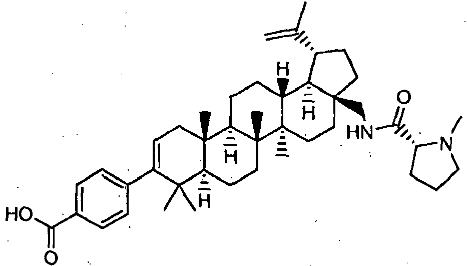 | A |
| 152 | 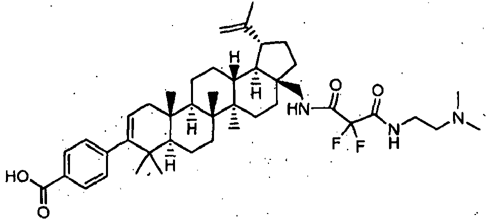 | A |
| 153 | 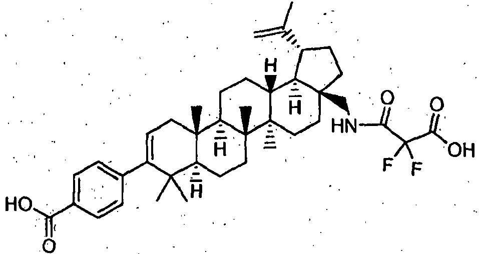 | B |
| 154 | 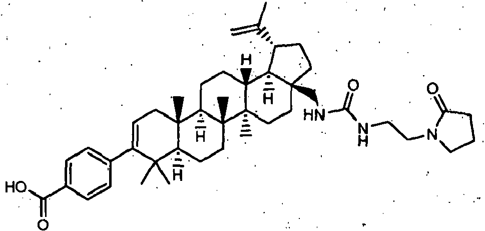 | A |
| 155 | 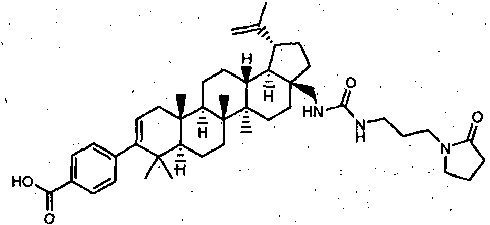 | A |
| 156 | 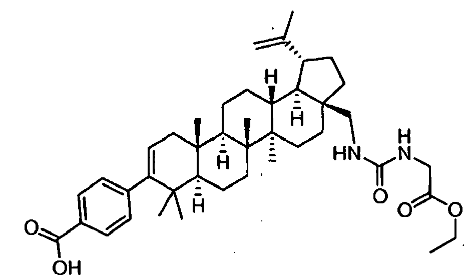 | A |
| 157 | 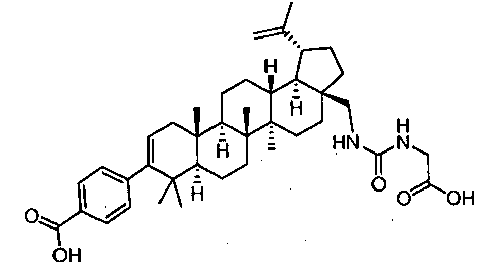 | 0.04 |
| 158 | 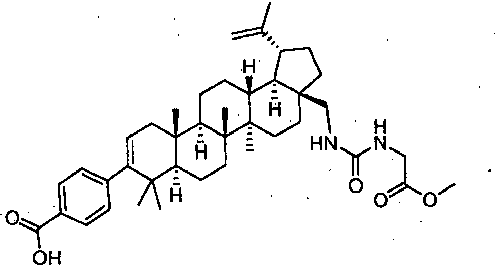 | A |
| 159 | 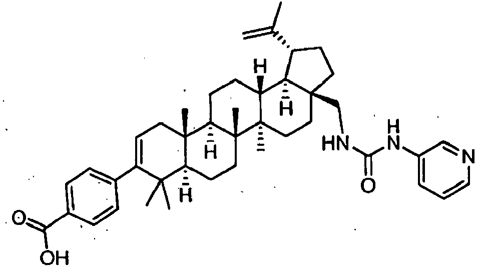 | 0.01 |
| 160 | 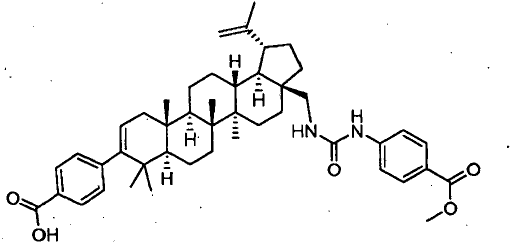 | 0.26 |
| 161 | 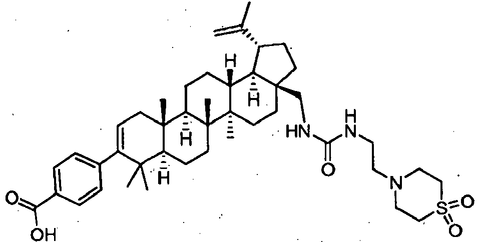 | A |
| 162 | 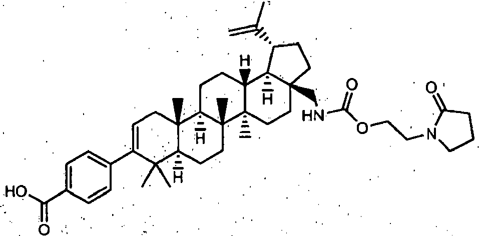 | A |
| 163 | 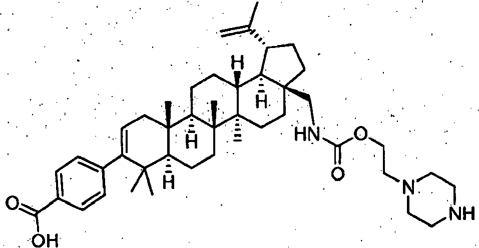 | A |
| 164 | 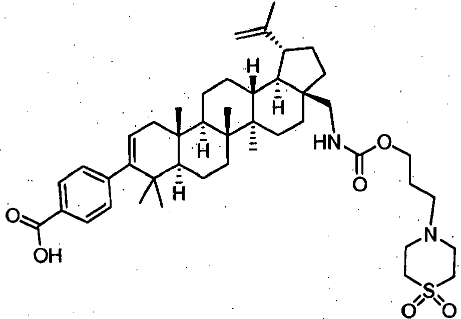 | A |
| 165 | 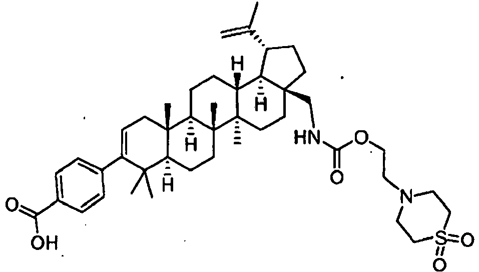 | A |
| 166 | 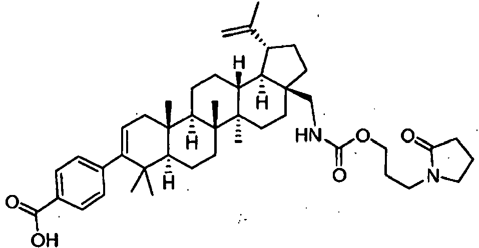 | A |
| 167 | 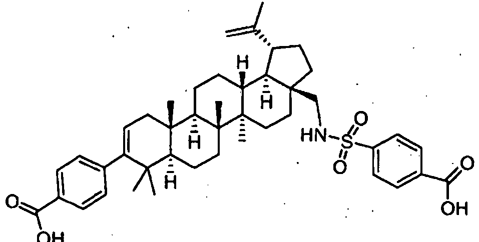 | A |
| 168 | 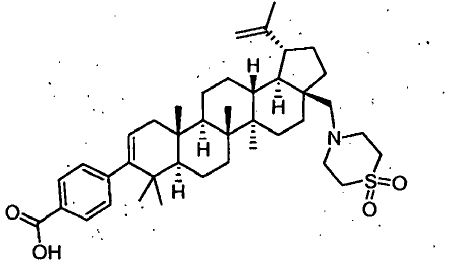 | A |
| 169 | 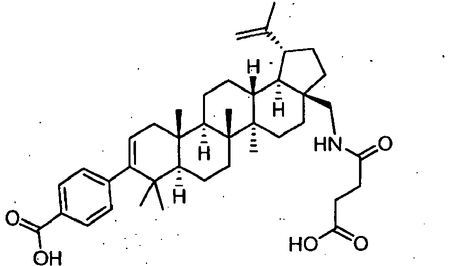 | A |
| 170 | 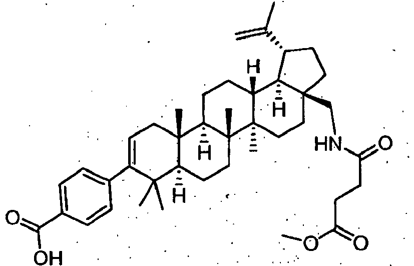 | A |
| 171 | 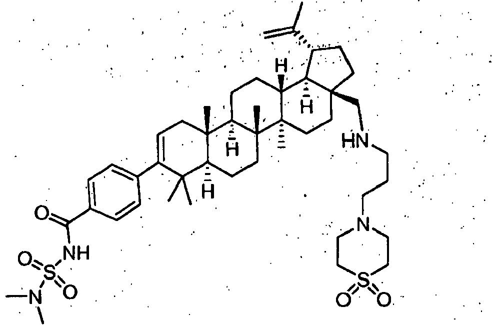 | A |
| 172 | 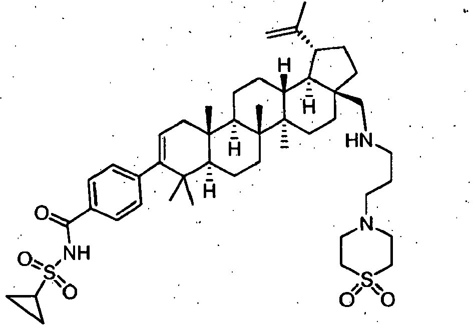 | A |
| 173 | 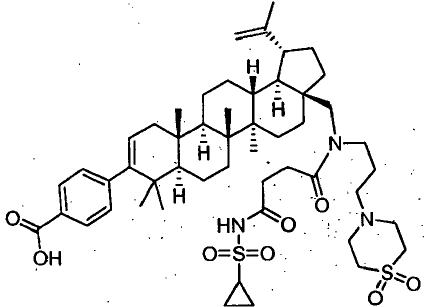 | A |
| 174 | 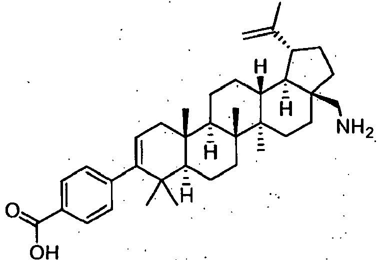 | A |
| 175 | 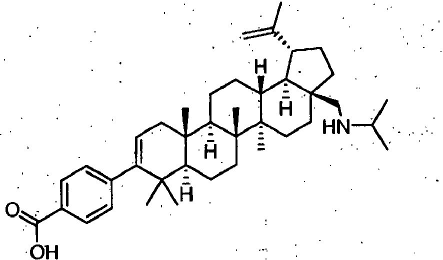 | A |
| 176 | 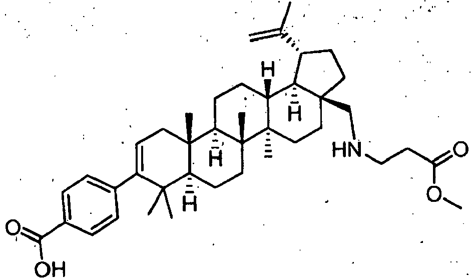 | 0.014 |
| 177 | 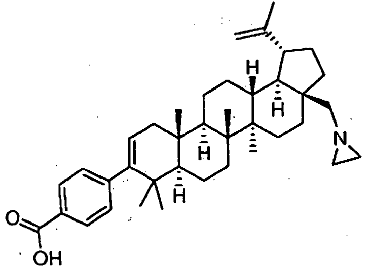 | A |
| 178 | 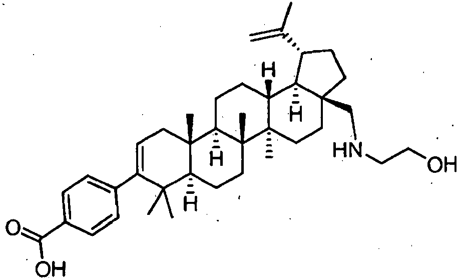 | A |
| 179 | 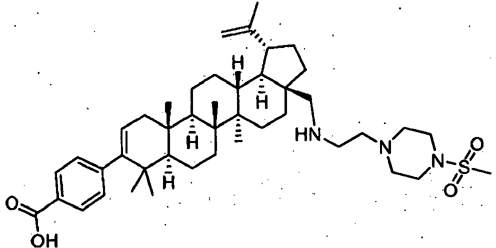 | A |
| 180 | 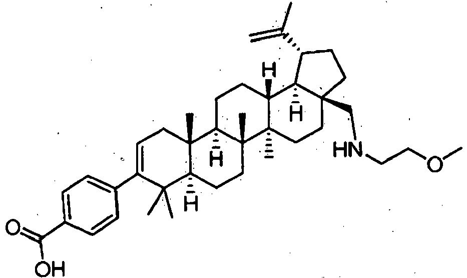 | A |
| 181 | 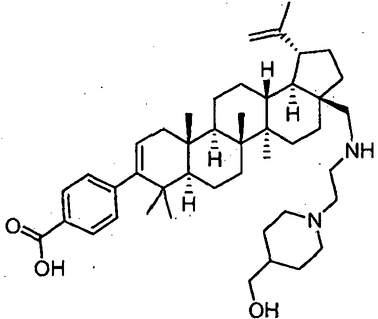 | 0.001 |
| 182 | 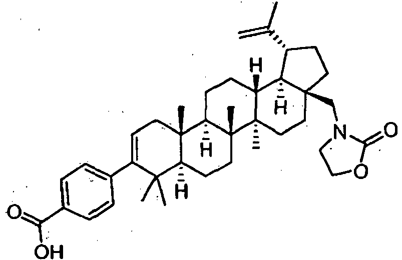 | A |

## Claims

1. A compound, including pharmaceutically acceptable salts thereof, which is selected from the group of:
a compound of formula I 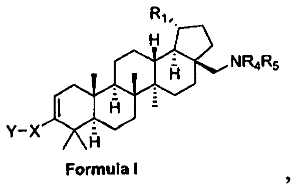
a compound of formula II 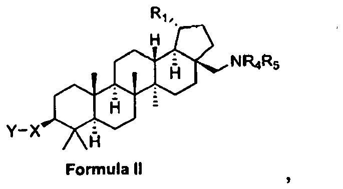 a compound of formula III 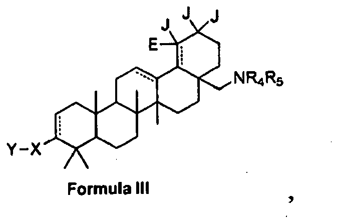 wherein R₁ is isopropenyl or isopropyl;
J and E are independently -H or -CH₃ and E is absent when the double bond is present;
X is a phenyl or heteroaryl ring substituted with A, wherein A is at least one member selected from the group of -H, -halo, -hydroxyl, -C₁₋₆ alkyl, -C₁₋₆ alkoxy, and -COOR₂;
R₂ is -H, -C₁₋₆ alkyl or -alkylsubstituted C₁₋₆ alkyl or -arylsubstituted C₁₋₆ alkyl;
Y is selected from the group of -COOR₂, -C(O)NR₂SO₂R₃, -C(O)NHSO₂NR₂R₂, -NR₂SO₂R₂, -SO₂NR₂R₂, -C₃₋₆ cycloalkyl-COOR₂, -C₁₋₆ alkenyl-COOR₂, -C₁₋₆ alkynyl-COOR₂, -C₁₋₆ alkyl-COOR₂, -NHC(O)(CH₂)ₙ-COOR₂, -SO₂NR₂C(O)R₂, -tetrazole, and -CONHOH, wherein n=1-6;
R₃ is -C₁₋₆ alkyl or alkylsubstituted C₁₋₆alkyl;
R₄ is selected from the group of H, -C₁₋₆ alkyl, -C₃₋₆ cycloalkyl, -C₁₋₆ substituted alkyl, -C₁₋₆ alkyl-heteroaryl, -C₁₋₆ alkyl-substitutedheteroaryl, -C₁₋₆alkyl-NR₆R₇, - C₁₋₆ alkyl-CONR₈R₉, - C₃₋₆ cycloalkyl-CONR₈R₉, -C₃₋₆ cycloalkyl-(CH₂)₁₋₃-NR₆R₇, -(CH₂)₁₋₃-C₃₋₆ cycloalkyl-NR₆R₇; -(CH₂)₁₋₃-C₃₋₆ cycloalkyl-(CH₂)₁₋₃-NR₆R₇; -C₁₋₆alkyl-Q₁, C₃₋₆ cycloalkyl-Q₁, -COR₁₀,-SO₂R₃ and -SO₂NR₂R₂;
Q₁ = -hydroxy, -COOR₂, -halo, -SO₂Rₐ;
Rₐ= C₁₋₆alkyl, NR₂R₂, 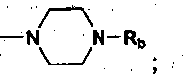
R_{b}= -H, -C₁₋₆ alkyl, -COR₃, -SO₂R₃, -SONR₃R₃;
R₄ can also be selected from the group of : 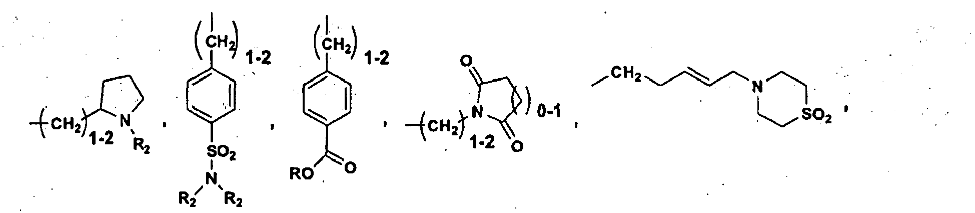 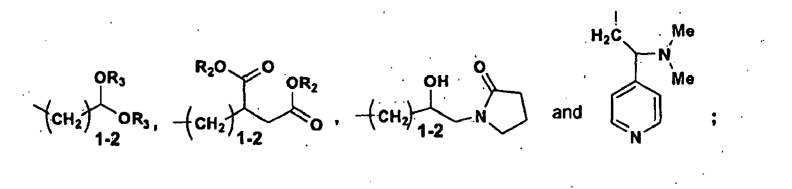
R₅ is selected from the group of -H, -C₁₋₆ alkyl, -C₃₋₆ cycloalkyl, -C₁₋₆ alkylsubstituted alkyl, - COR₁₀,-SO₂R₃ and -SO₂NR₂R₂;
with the proviso that only one of R₄ or R₅ can be selected from the group of -COR₁₀,-SO₂R₃ and -SO₂NR₂R₂;
or R₄ and R₅ are taken together with the adjacent N to form a cycle such as 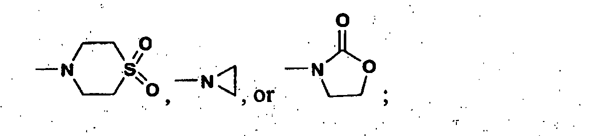
R₁₀ is selected from the group of -H, -C₁₋₆ alkyl, -C₁₋₆ alkyl-NR₆R₇, -NR₁₁R₁₂, -OR₁₃, -C₁₋₆ alkyl-Q₂, -C₃₋₆ cycloalkyl-Q₂, aryl-Q₂, wherein n =1-6,
wherein Q₂ = hydroxy, -COOR₂, -halo, SO₂Rₐ, -CONHSO₂R₃, -CONHSO₂NR₂R₂;
R₁₀ can also be selected from the group of: 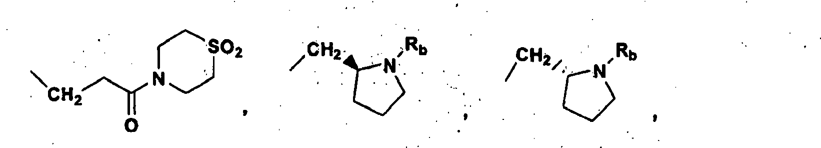 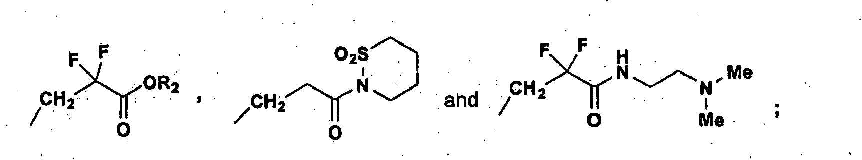
R₆ and R₇ are independently selected from the group of -H, -C₁₋₆ alkyl, -C₁₋₆ substituted alkyl, aryl, heteroaryl, substituted aryl, substituted heteroaryl, and -C₁₋₆ alkyl-Q₁,
or R₆ and R₇ are taken together with the adjacent N to form a cycle selected from the group of 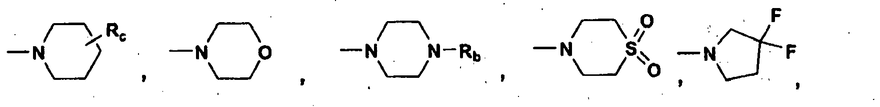 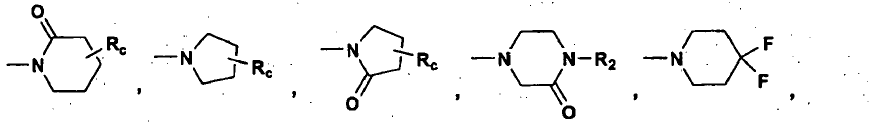 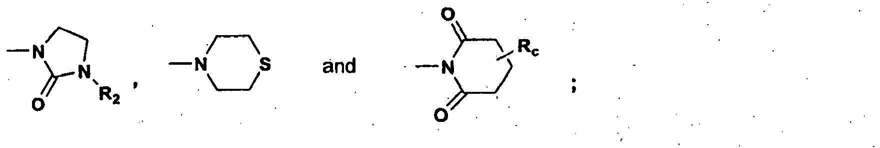
R_{c} = C₁₋₆ alkyl, NR₂R₂, -COOR₃;
R₈ and R₉ are independently selected from the group of -H, -C₁₋₆ alkyl, -C₃₋₆ cycloalkyl, -C₁₋₆ substituted alkyl, -C₁₋₆alkyl-heteroaryl, -C₁₋₆ alkyl-substitutedheteroaryl, -C₁₋₆ alkyl-NR₂R₂, C₁₋₆ alkyl-CONR₂R₂, -C₁₋₆ alkyl-Q₁, C₃₋₆ cycloalkyl-Q₁,
or R₈ and R₉ can also be independently selected from the group of 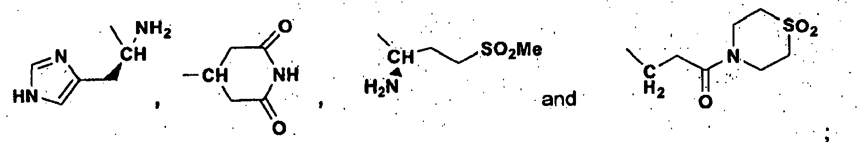
or R₈ and R₉ are taken together with the adjacent N to form a cycle selected from the group of: 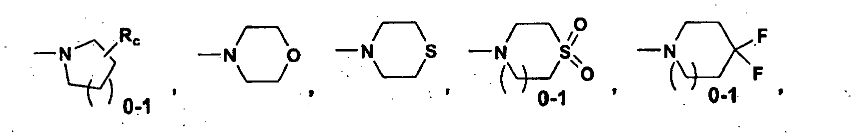 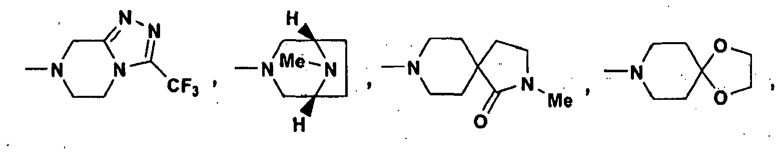 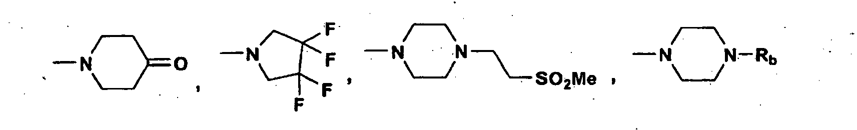 and 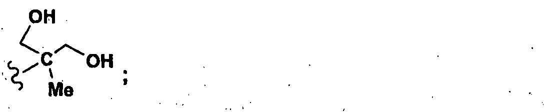
and R₁₁ and R₁₂ are independently selected from the group of -H, -C₁₋₆ alkyl, -C₃₋₆ cycloalkyl, and -C₁₋₆ alkylsubstituted alkyl;
or R₁₁ and R₁₂ are taken together with the adjacent N to form a cycle selected from the group of 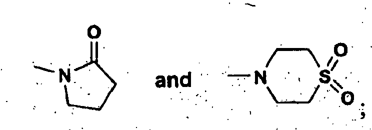 and
R₁₃ is selected from the group of -H, C₁₋₆ alkyl, -C₁₋₆ alkylsubstituted alkyl, and -C₁₋₆ alkyl NR₁₄R₁₅, wherein
R₁₄ and R₁₅ are independently selected from the group of -H,-C₁₋₆ alkyl, and -C₁₋₆ alkylsubstituted alkyl, or R₁₄ and R₁₅ are taken together with the adjacent N to form a cycle selected from the group of

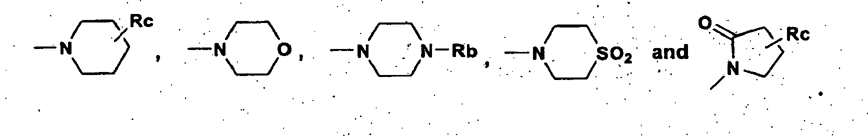

2. The compound as claimed in claim 1, wherein said compound has the Formula I.

3. The compound as claimed in claim 2, wherein X is a phenyl ring, and Y is in the para position.

4. The compound as claimed in claim 3, wherein X is a substituted phenyl ring.

5. The compound as claimed in 4, wherein said phenyl ring is substituted with A, and A is at least one member selected from the group of -H, -OH and -F.

6. The compound as claimed in claim 5, wherein Y is -COOH.

7. The compound as claimed in claim 4, wherein X is a phenyl ring and Y is -COOH in the para position according to Formula Ia:

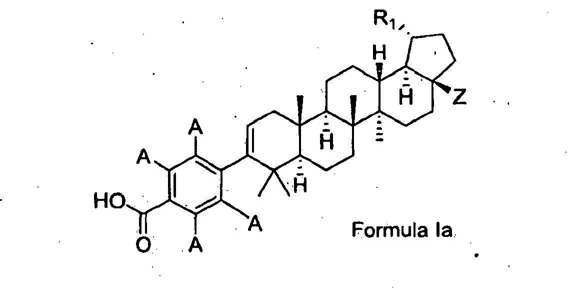

8. The compound as claimed in claim 7, wherein A is at least one member selected from the group of -H, -OH and -F.

9. The compound as claimed in claim 8, wherein A is -H or -F.

10. A compound of claim 1, including pharmaceutically acceptable salts thereof, which is selected from the group consisting of: 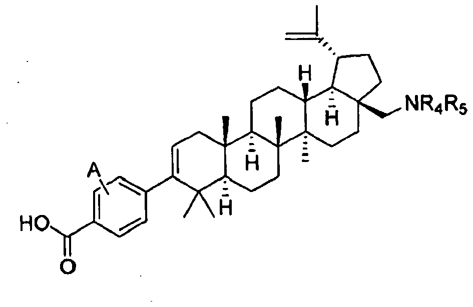 and

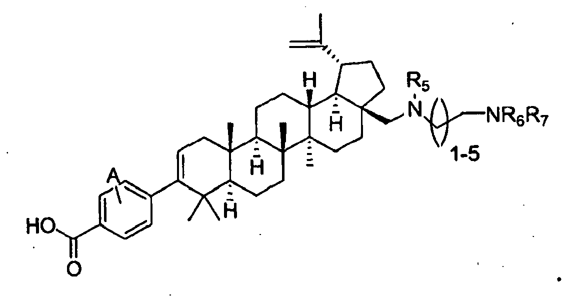

11. A compound of claim 1 which is selected from the group consisting of: 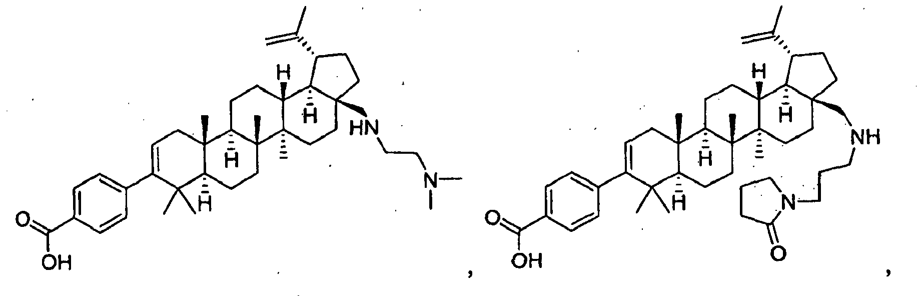 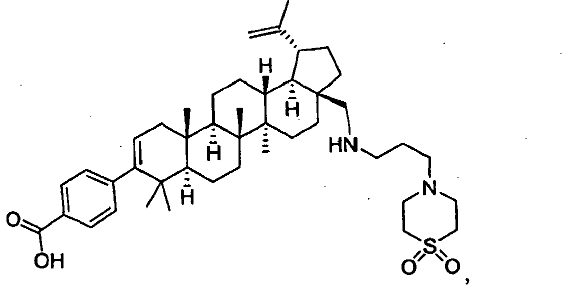 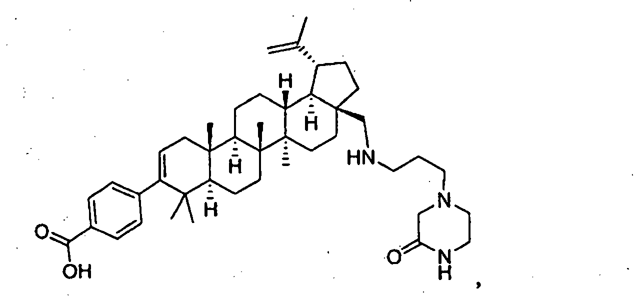 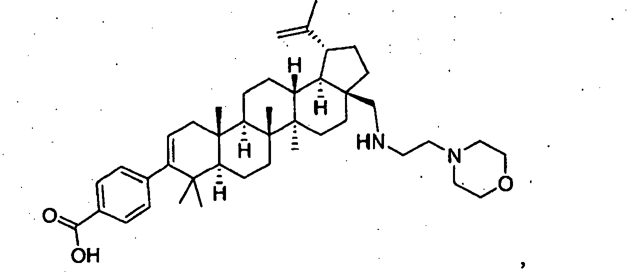 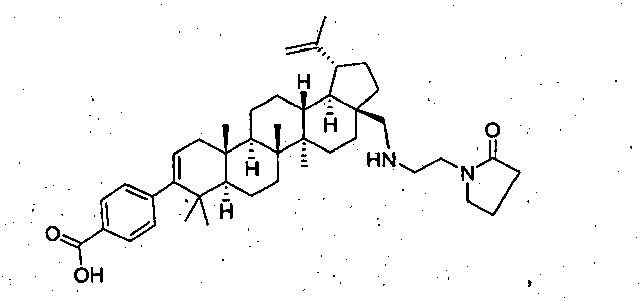 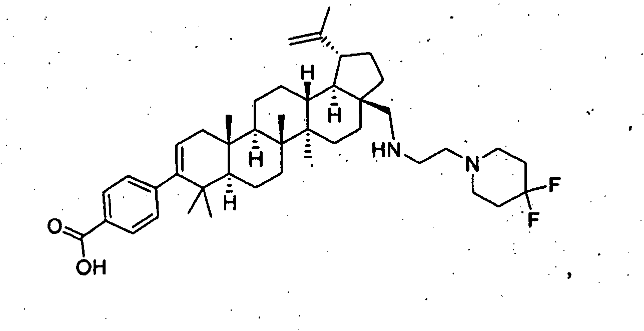 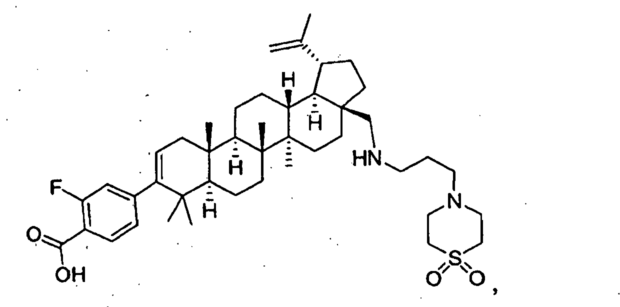 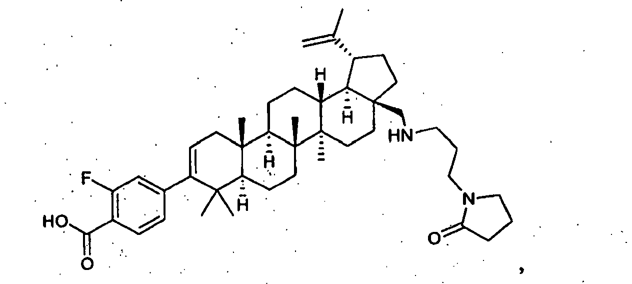 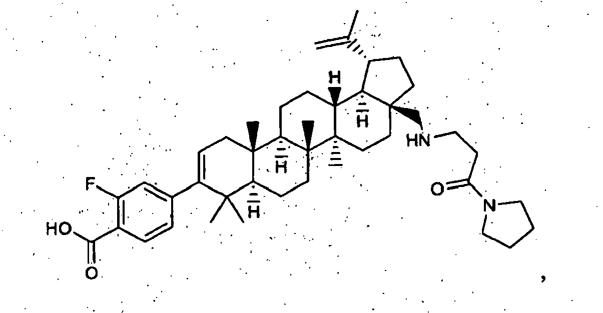 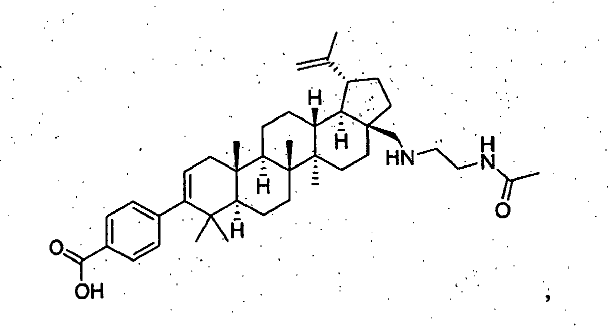 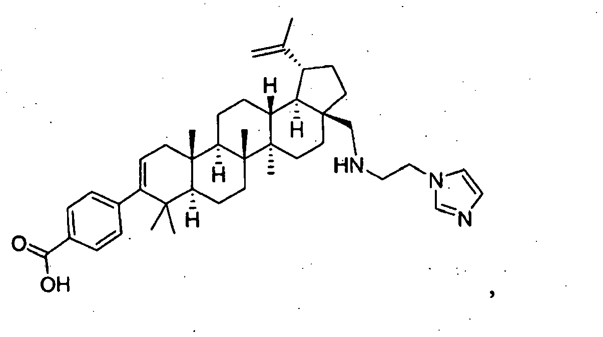 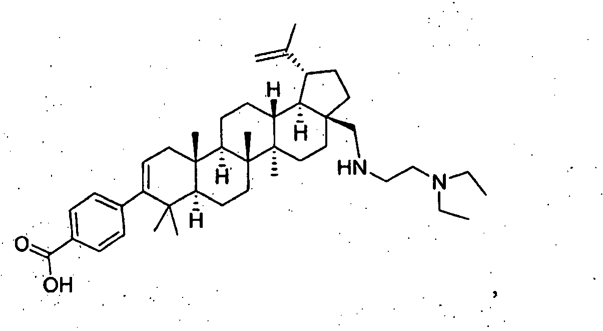 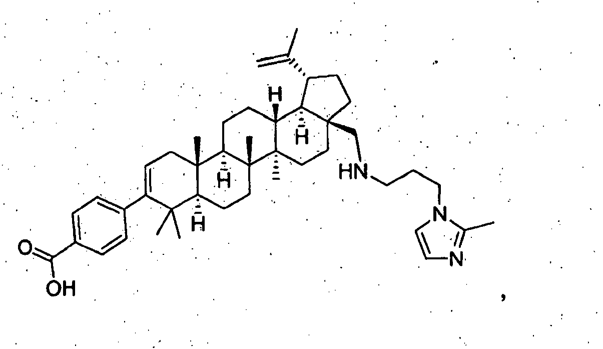 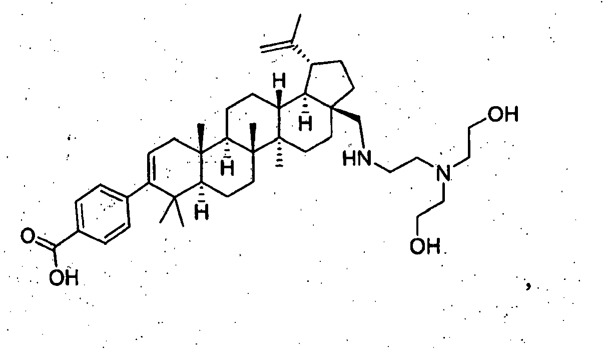 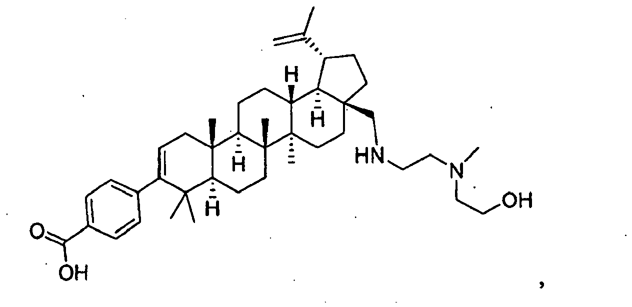 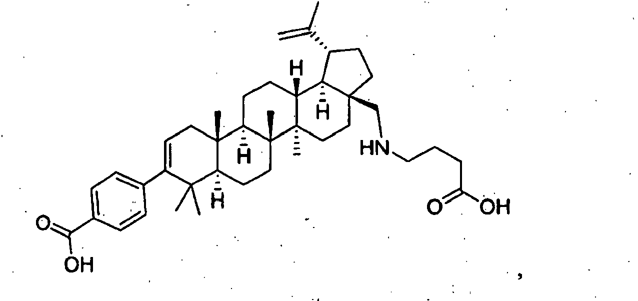 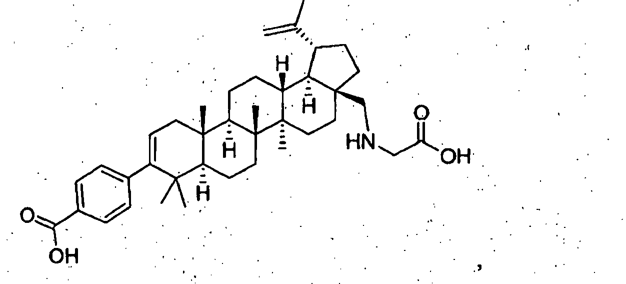 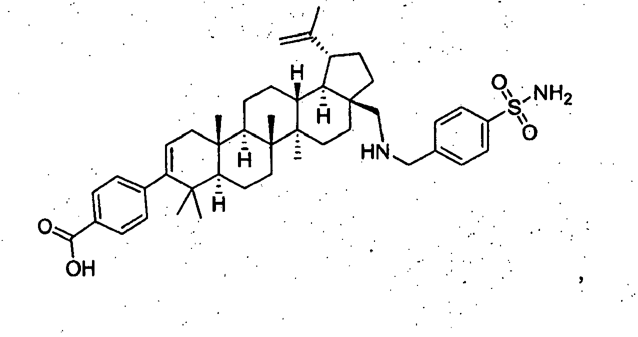 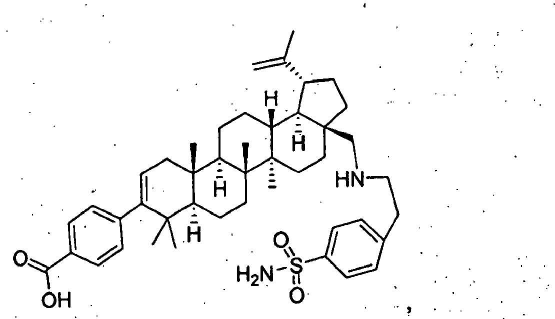 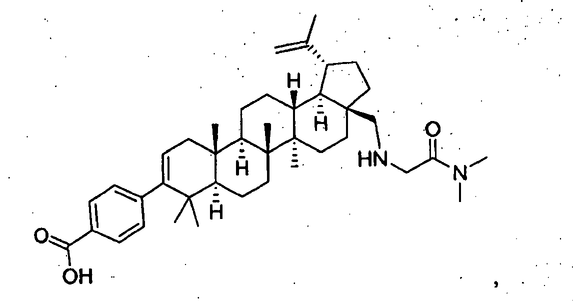 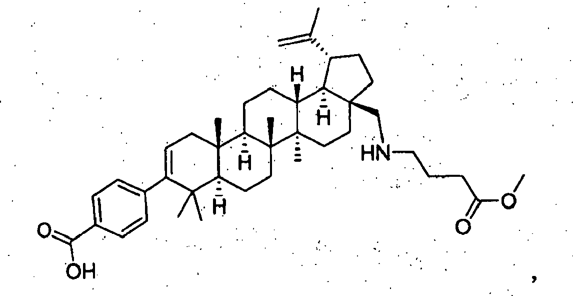 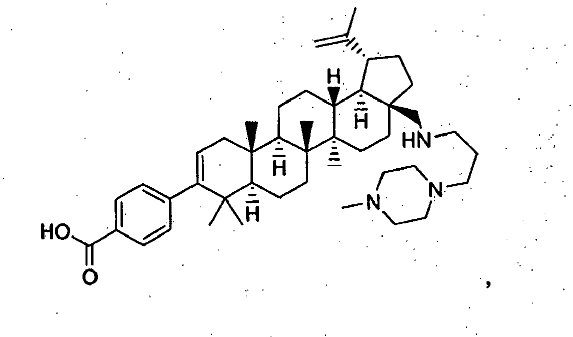 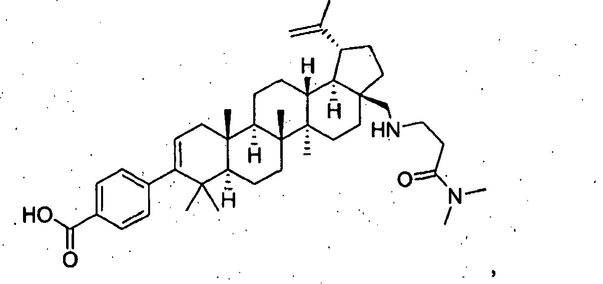 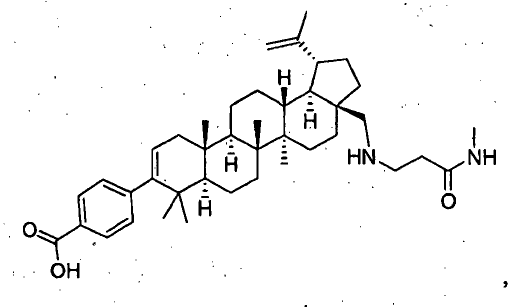 and

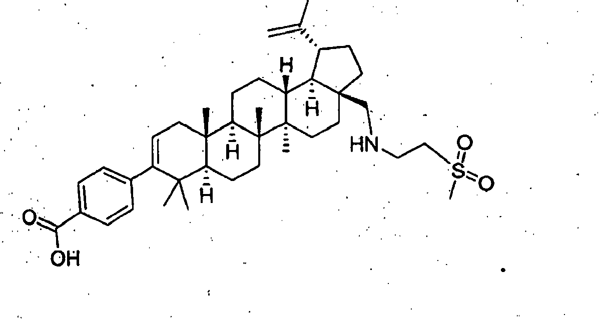

12. A pharmaceutical composition which comprises an antiviral effective amount of one or more of the compounds as claimed in any one of claims 1 to 11, together with one or more pharmaceutically acceptable carriers, excipients or diluents.

13. The pharmaceutical composition of claim 12, useful for treating infection by HIV, which additionally comprises an antiviral effective amount of an AIDS treatment agent selected from the group consisting of:
(a) an AIDS antiviral agent;
(b) an anti-infective agent;
(c) an immunomodulator; and
(d) another HIV entry inhibitor.

14. A compound as claimed in any one of claims 1 to 11 for use in a method for treating a mammal infected with the HIV virus.

15. An intermediate compound which is selected from the group of: 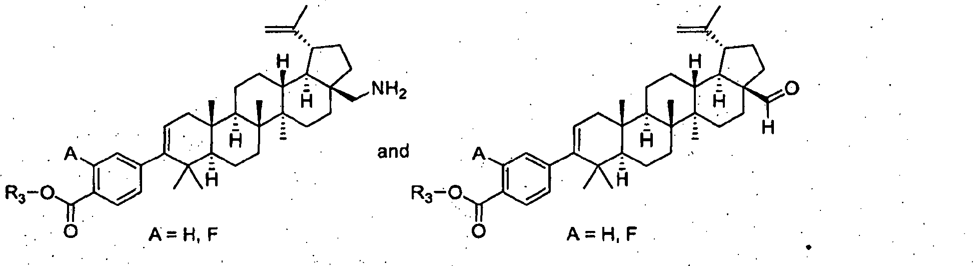 wherein R₃ is as defined in claim 1

## Patentansprüche

1. Verbindung, einschließlich pharmazeutisch verträglicher Salze davon, ausgewählt aus:
einer Verbindung der Formel I 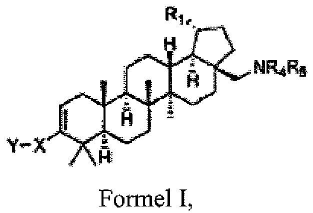
einer Verbindung der Formel II 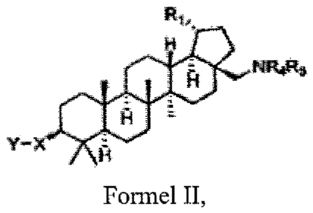 einer Verbindung der Formel III, 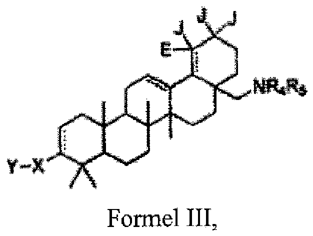
wobei R₁ Isopropenyl oder Isopropyl ist;
J und E unabhängig -H oder -CH₃ sind und E nicht vorhanden ist, wenn die Doppelbindung vorhanden ist;
X ein Phenyl- oder Heteroarylring ist, der mit A substituiert ist, wobei A zumindest ein Element ist, das ausgewählt ist aus -H, -Halo, -Hydroxyl, -C₁₋₆-Alkyl, -C₁₋₆-Alkoxy und -COOR₂;
R₂ -H, -C₁₋₆-Alkyl oder -alkylsubstituiertes C₁₋₆-Alkyl oder arylsubstituiertes C₁₋₆-Alkyl ist;
Y ausgewählt ist aus -COOR₂, -C(O)NR₂SO₂R₃, -C(O)NHSO₂NR₂R₂, - NR₂SO₂R₂, -SO₂NR₂R₂, -C₃₋₆-Cycloalkyl-COOR₂, -C₁₋₆-Alkenyl-COOR₂, -C₁₋₆-Alkynyl-COOR₂, -C₁₋₆-Alkyl-COOR₂, -NHC(O)(CH₂)ₙ-COOR₂, -SO₂NR₂C(O)R₂, -Tetrazol und -CONHOH, wobei n 1 bis 6 ist;
R₃ -C₁₋₆-Alkyl oder alkylsubstituiertes C₁₋₆-Alkyl ist;
R₄ ausgewählt ist aus H, -C₁₋₆-Alkyl, -C₃₋₆-Cycloalkyl, -C₁₋₆-substituiertem Alkyl, -C₁₋₆-Alkylheteroaryl, -C₁₋₆-alkylsubstituiertem Heteroaryl, -C₁₋₆-Alkyl-NR₆R₇, -C₁₋₆-Alkyl-CONR₈R₉, -C₃₋₆-Cycloalkyl-CONR₈R₉, -C₃₋₆-Cycloalkyl-(CH₂)₁₋₃-NR₆R₇, -(CH₂)₁₋₃-C₃₋₆-Cycloalkyl-NR₆R₇, -(CH₂)₁₋₃-C₃₋₆-Cycloalkyl-(CH₂)₁₋₃-NR₆R₇, -C₁₋₆-Alkyl-Q₁, C₃₋₆-Cycloalkyl-Q₁, -COR₁₀, -SO₂R₃ und -SO₂NR₂R₂;
Q₁ -Hydroxy, -COOR₂, -Halo, -SO₂Rₐ ist;
Rₐ C₁₋₆-Alkyl, NR₂R₂, 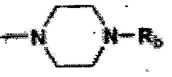 ist;
R_{b}-H, -C₁₋₆-Alkyl, -COR₃, -SO₂R₃, -SONR₃R₃ ist;
R₄ auch ausgewählt sein kann aus: 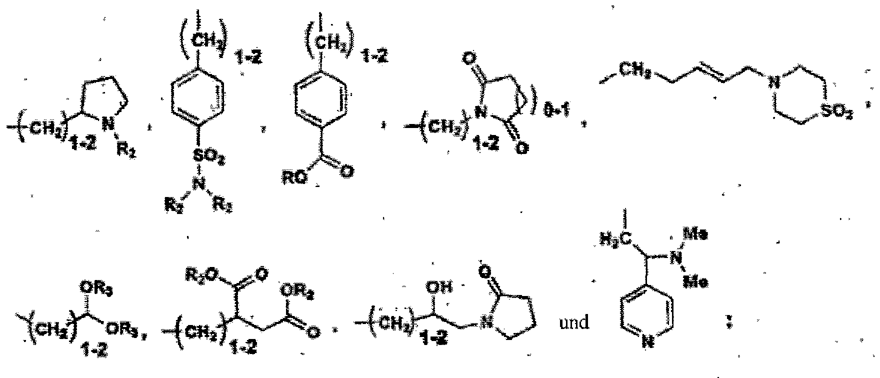
R₅ ausgewählt ist aus -H, -C₁₋₆-Alkyl, -C₃₋₆-Cycloalkyl, -C₁₋₆-alkylsubstiuiertem Alkyl, -COR₁₀, -SO₂R₃ und -SO₂NR₂R₂;
mit der Maßgabe, dass nur eines von R₄ oder R₅ ausgewählt sein kann aus-COR₁₀, -SO₂R₃ und -SO₂NR₂R₂;
oder R₄ und R₅ mit dem benachbarten N zusammengenommen sind, um einen Cyclus wie 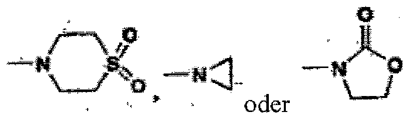 zu bilden;
R₁₀ ausgewählt ist aus -H, -C₁₋₆-Alkyl, -C₁₋₆-Alkyl-NR₆R₇, -NR₁₁R₁₂, -OR₁₃, -C₁₋₆-Alkyl-Q₂, -C₃₋₆-Cycloalkyl-Q₂, Aryl-Q₂, wobei n 1 bis 6 ist,
wobei Q₂ Hydroxy, -COOR₂, -Halo, SO₂Rₐ, -CONHSO₂R₃, -CONHSO₂NR₂R₂ ist;
R₁₀ auch ausgewählt sein kann aus: 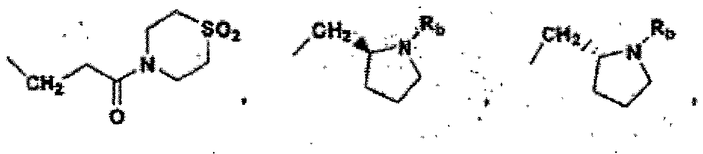 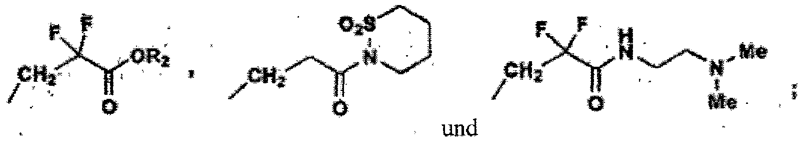
R₆ und R₇ unabhängig ausgewählt sind aus -H, -C₁₋₆-Alkyl, -substituiertem Alkyl, Aryl, Heteroaryl, substituiertem Aryl, substituiertem Heteroaryl und -C₁₋₆-Alkyl-Q₁,
oder R₆ und R₇ mit dem benachbarten N zusammengenommen sind, um einen Cyclus zu bilden, der ausgewählt ist aus: 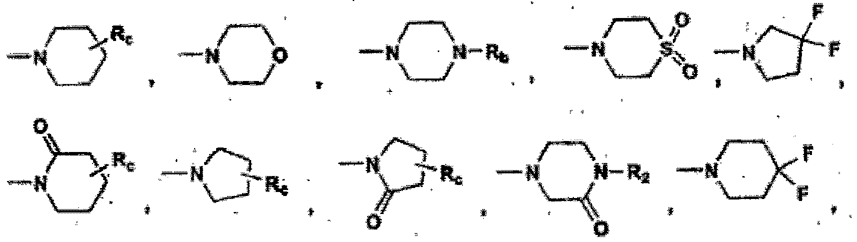 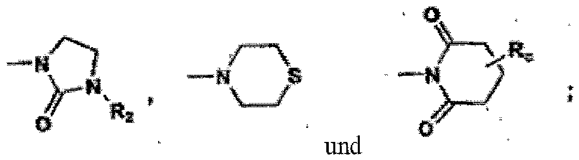
R_{c} C₁₋₆-Alkyl, NR₂R₂, -COOR₃;
R₈ und R₉ unabhängig ausgewählt sind aus -H, -C₁₋₆-Alkyl, -C₃₋₆-Cycloalkyl, -C₁₋₆-substituiertem Alkyl, -C₁₋₆-Alkylheteroaryl, -C₁₋₆-alkylsubstituiertern Heteroaryl, -C₁₋₆-Alkyl-NR₂R₂, -C₁₋₆-Alkyl-CONR₂R₂, -C₁₋₆-Alkyl-Q₁, C₃₋₆-Cycloalkyl-Q₁,
oder R₈ und R₉ auch unabhängig ausgewählt sein aus: 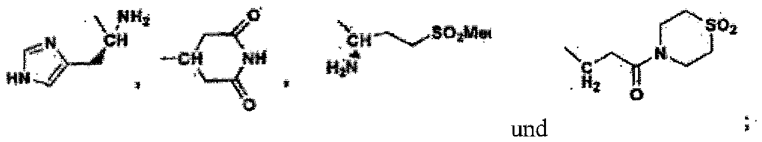
oder R₈ und R₉ mit dem benachbarten N zusammengenommen sind, um einen Cyclus zu bilden, der ausgewählt ist aus: 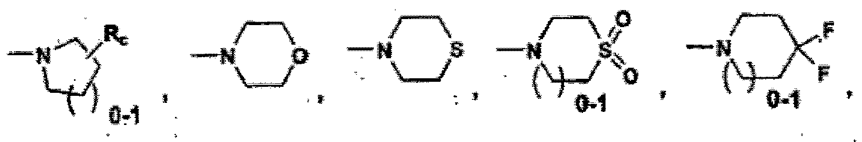 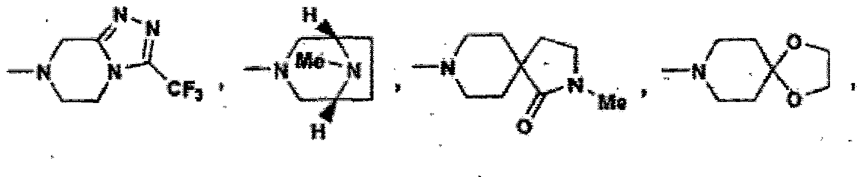 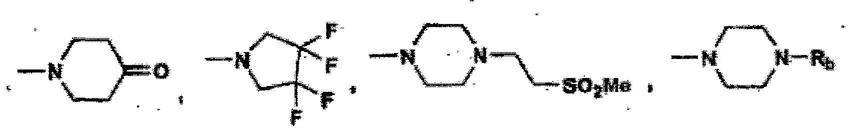 und 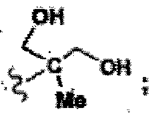
und R₁₁ und R₁₂ unabhängig ausgewählt sind aus -H, -C₁₋₆-Alkyl, -C₃₋₆-Cycloalkyl und -C₁₋₆-alkylsubstituiertem Alkyl;
oder R₁₁ und R₁₂ mit dem benachbarten N zusammengenommen sind, um einen Cyclus zu bilden, der ausgewählt ist aus: 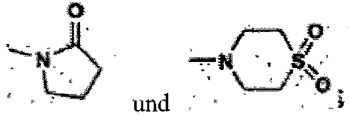 und
R₁₃ ausgewählt ist aus -H, C₁₋₆-Alkyl, -C₁₋₆-alkylsubstituiertem Alkyl und -C₁₋₆-Alkyl-NR₁₄R₁₅, wobei
R₁₄ und R₁₅ unabhängig ausgewählt sind aus -H, -C₁₋₆-Alkyl und -C₁₋₆-alkylsubstituiertem Alkyl oder R₁₄ und R₁₅ mit dem benachbarten N zusammengenommen sind, um einen Cyclus zu bilden, der ausgewählt ist aus:

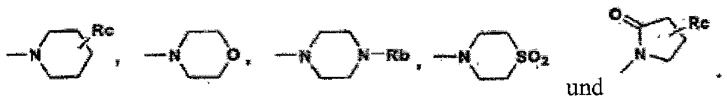

2. Verbindung nach Anspruch 1, wobei die Verbindung Formel I aufweist.

3. Verbindung nach Anspruch 2, wobei X ein Phenylring ist und Y in der para-Position vorliegt.

4. Verbindung nach Anspruch 3, wobei X ein substituierter Phenylring ist.

5. Verbindung nach Anspruch 4, wobei der Phenylring mit A substituiert ist und A zumindest ein Element ausgewählt aus -H, -OH und -F ist.

6. Verbindung nach Anspruch 5, wobei Y -COOH ist.

7. Verbindung nach Anspruch 4, wobei X ein Phenylring ist und Y -COOH in der para-Position gemäß Formel Ia ist:

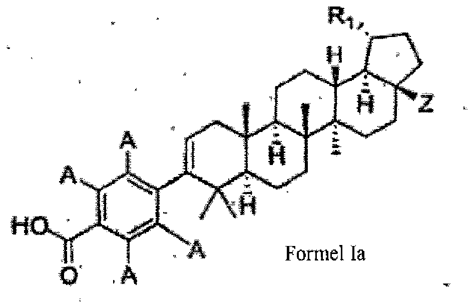

8. Verbindung nach Anspruch 7, wobei A zumindest ein Element ausgewählt aus -H, -OH und -F ist.

9. Verbindung nach Anspruch 8, wobei A -H oder -F ist.

10. Verbindung nach Anspruch 1, einschließlich pharmazeutisch verträglicher Salze davon, die ausgewählt ist aus: 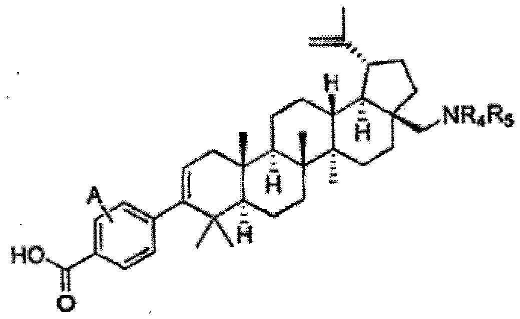 und

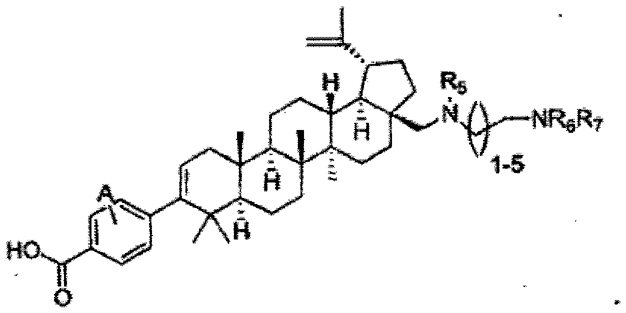

11. Verbindung nach Anspruch 1, die ausgewählt ist aus: 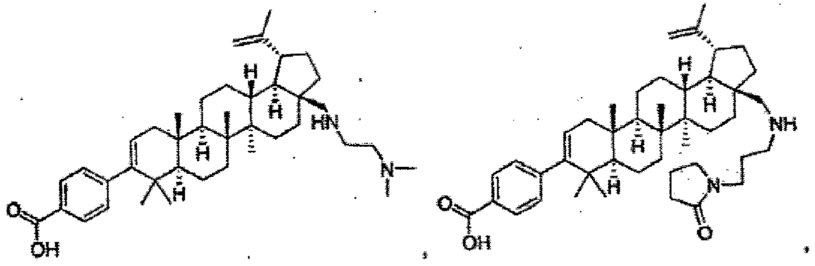 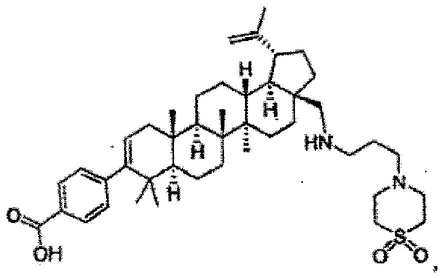 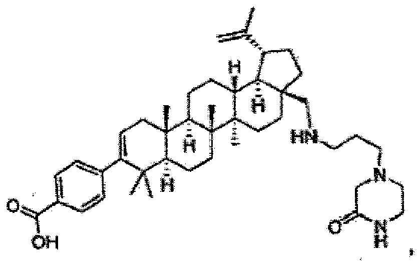 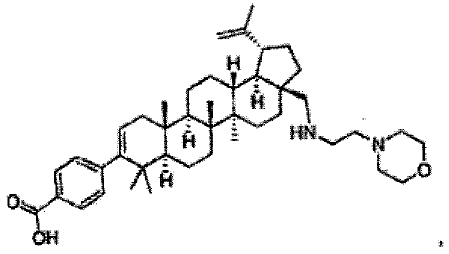 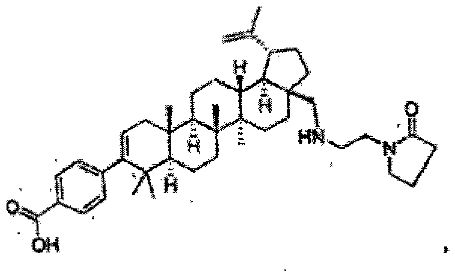 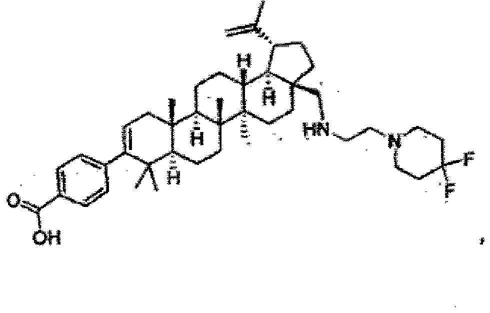 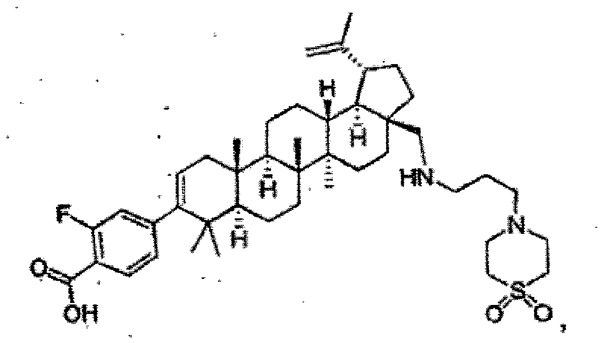 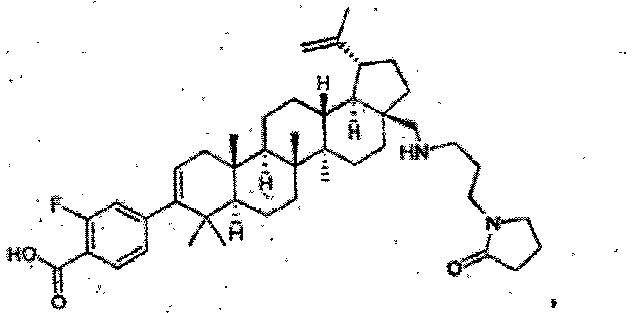 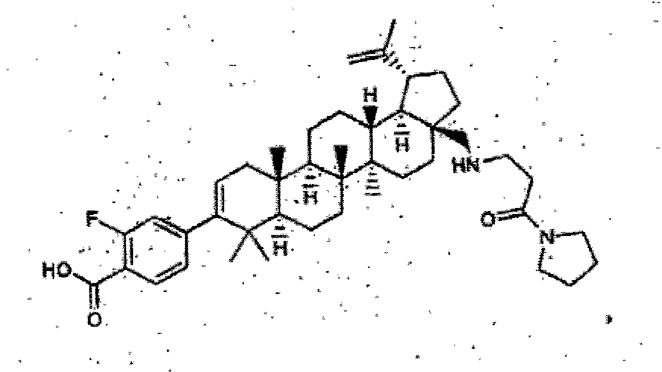 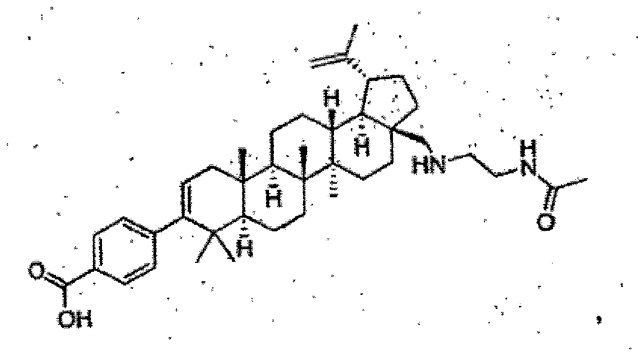 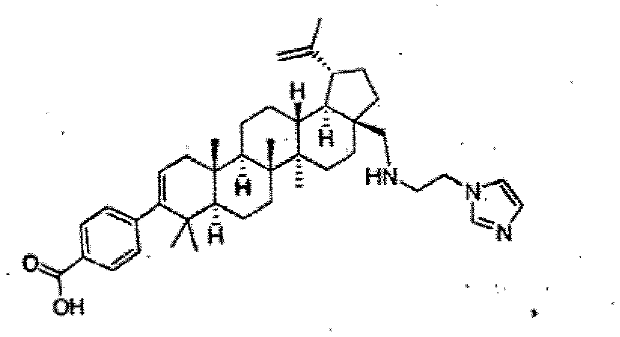 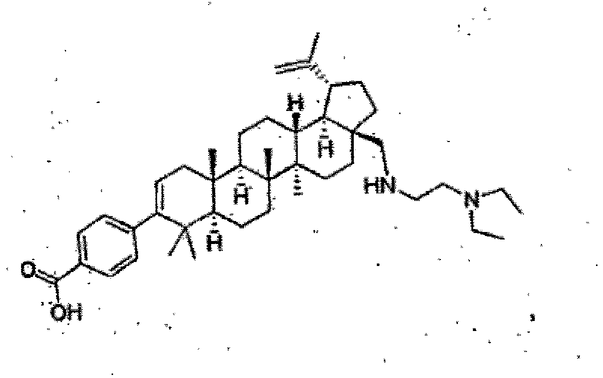 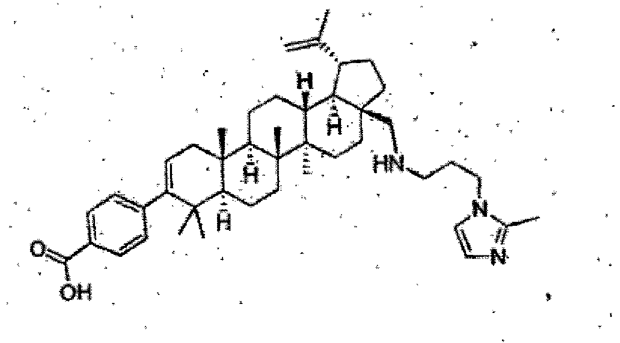 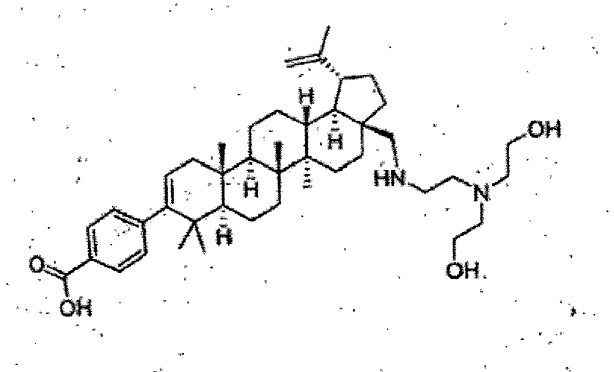 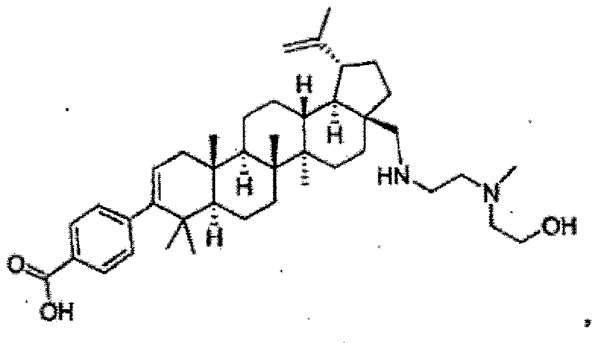 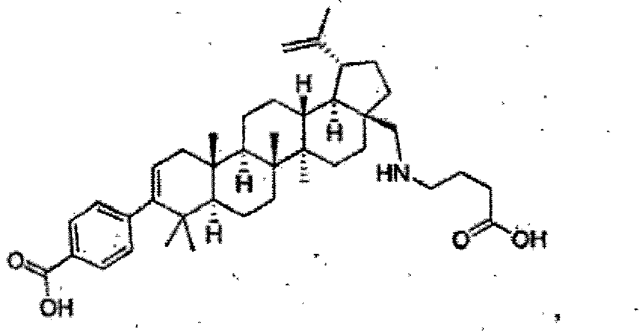 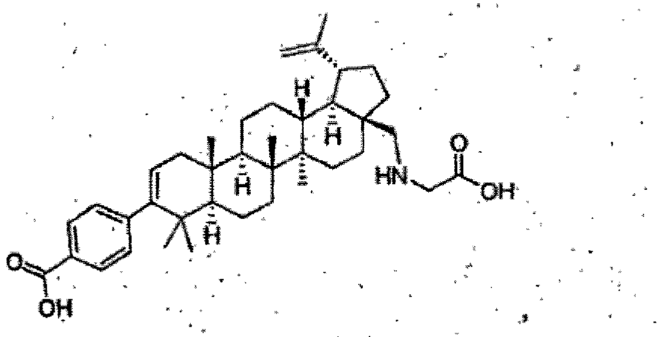 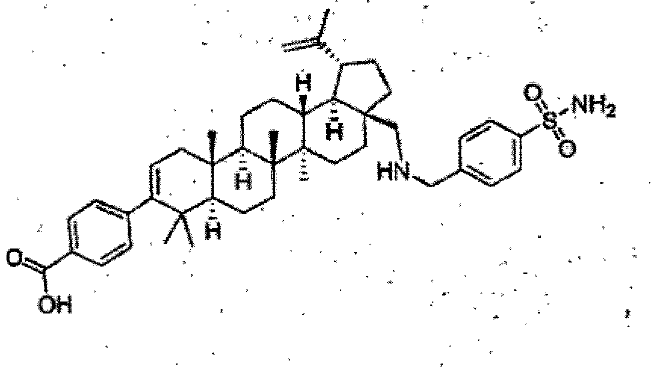 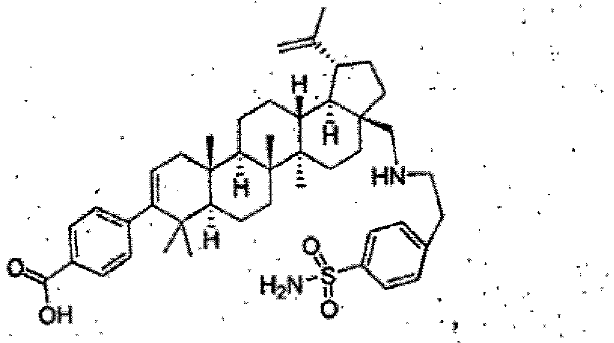 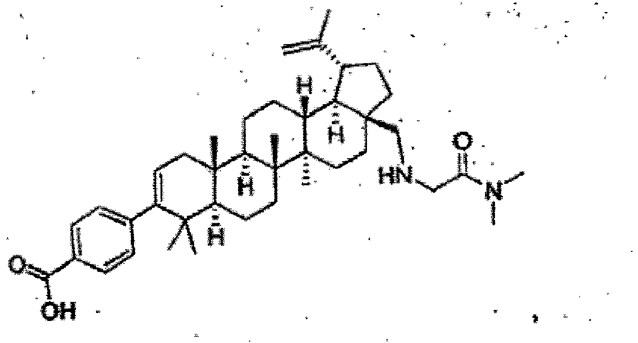 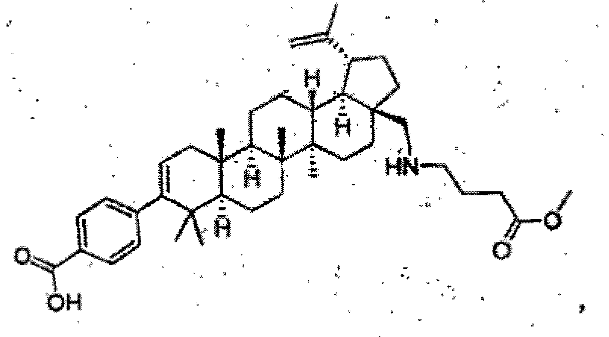 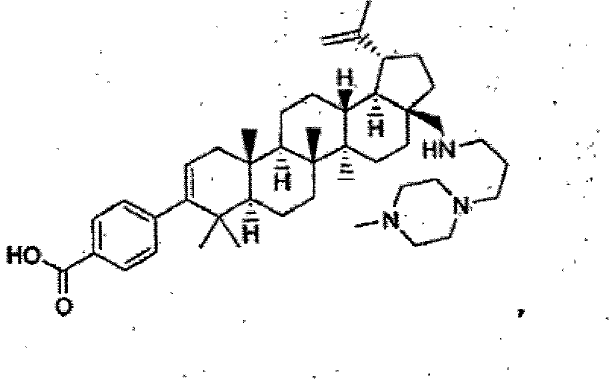 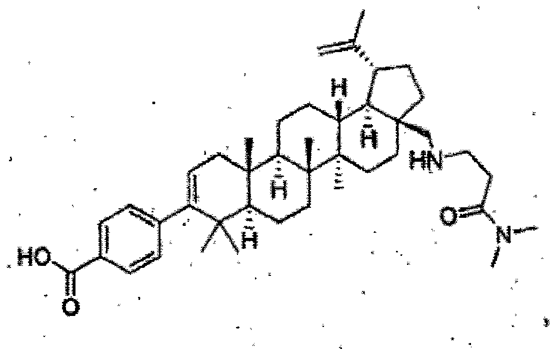 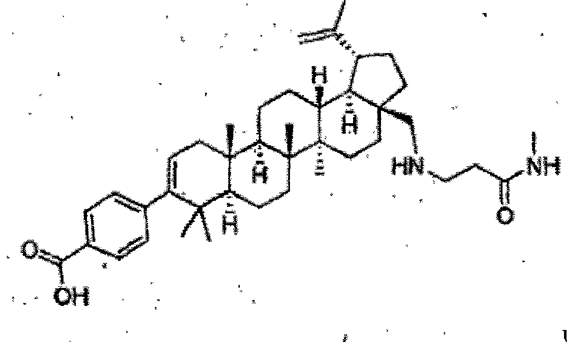 und

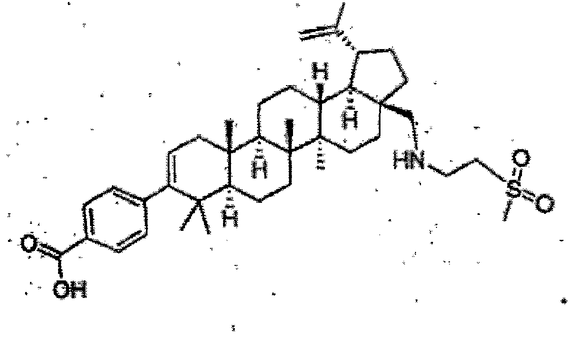

12. Pharmazeutische Zusammensetzung, die eine antivirale wirksame Menge einer oder mehrerer der Verbindungen nach einem der Ansprüche 1 bis 11 zusammen mit einem oder mehreren pharmazeutisch verträglichen Trägern, Exzipienten oder Verdünnungsmitteln umfasst.

13. Pharmazeutische Zusammensetzung nach Anspruch 12, die bei der Behandlung einer HIV-Infektion nützlich ist, die darüber hinaus eine antivirale wirksame Menge eines AIDS-Behandlungsmittels umfasst, das ausgewählt ist aus:
(a) einem antiviralen AIDS-Mittel;
(b) einem infektionshemmenden Mittel;
(c) einem Immunmodulator; und
(d) einem weiteren HIV-Eintrittshemmer.

14. Verbindung nach einem der Ansprüche 1 bis 11 zur Verwendung bei einem Verfahren zur Behandlung eines mit dem HI-Virus infizierten Säugetiers.

15. Zwischenverbindung, die ausgewählt ist aus: 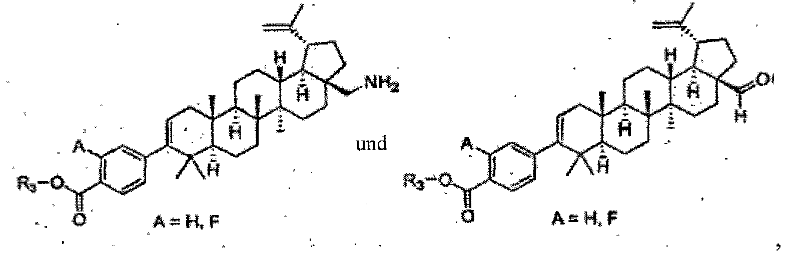 wobei R₃ wie in Anspruch 1 definiert ist.

## Revendications

1. Composé, y compris des sels pharmaceutiquement acceptables de celui-ci, qui est sélectionné parmi le groupe de:
composé de la formule I 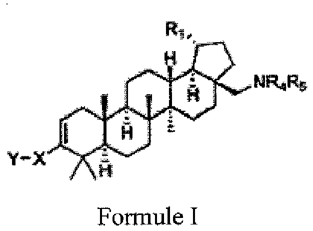
composé de la formule II 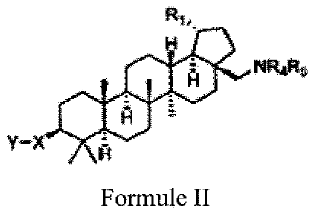 composé de la formule III 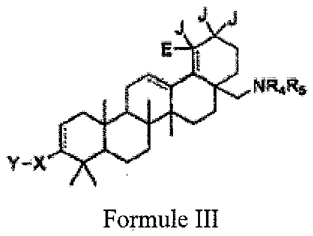
où R₁ est un isopropényle ou un isopropyle;
J et E sont indépendamment -H ou -CH₃ et E est absent lorsque la double liaison est présente;
X est un cycle phényle ou hétéroaryle substitué par A, où A est au moins un chaînon sélectionné parmi le groupe de -H, -halo, -hydroxyle, -C₁₋₆ alkyle, -C₁₋₆ alcoxy et -COOR₂;
R₂ est -H, -C₁₋₆ alkyle ou -C₁₋₆ alkyle substitué alkyle ou -C₁₋₆ alkyle substitué aryle;
Y est sélectionné parmi le groupe de -COOR₂, -C(O)NR₂SO₂R₃,-C(O)NHSO₂NR₂R₂, -NR₂SO₂R₂, -SO₂NR₂R₂, -C₃₋₆ cycloalkyle-COOR₂, -C₁₋₆ alcényle-COOR₂, -C₁₋₆ alcynyle-COOR₂, -C₁₋₆alkyle-COOR₂, -NHC(O)(CH₂)ₙ-COOR₂,-SO₂NR₂C(O)R₂, -tétrazole et -CONHOH,
où n = 1-6;
R₃ est un -C₁₋₆ alkyle ou un C₁₋₆ alkyle substitué alkyle;
R₄ est sélectionné parmi le groupe de H, -C₁₋₆ alkyle, -C₃₋₆ cycloalkyle, -C₁₋₆ alkyle substitué, -C₁₋₆ alkyle-hétéroaryle, -C₁₋₆ alkyle-hétéroaryle substitué, -C₁₋₆ alkyle-NR₆R₇, -C₁₋₆ alkyle-CONR₈R₉, -C₃₋₆ cycloalkyle-CONR₈R₉, -C₃₋₆ cycloalkyle-(CH₂)₁₋₃-NR₆R₇, -(CH₂)₁₋₃-C₃₋₆ cycloalkyle-NR₆R₇, -(CH₂)₁₋₃C₃₋₆ cycloalkyle-(CH₂)₁₋₃-NR₆R₇,-C₁₋₆ alkyle-Q₁, C₃₋₆ cycloalkyle-Q₁, -COR₁₀, -SO₂R₃ et -SO₂NR₂R₂;
Q₁ = hydroxy, -COOR₂, -halo, -SO₂Rₐ;
Rₐ = C₁₋₆ alkyle, NR₂R₂, 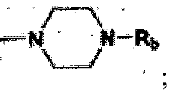
R_{b} = -H, -C₁₋₆ alkyle, -COR₃, -SO₂R₃, -SONR₃R₃;
R₄ peut également être sélectionné parmi le groupe de: 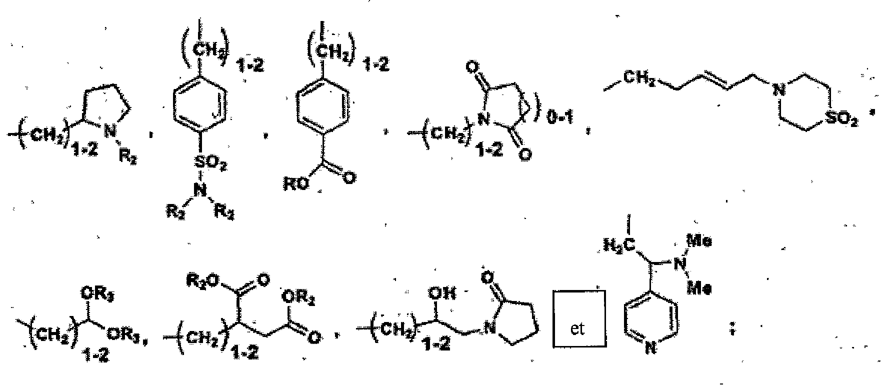
R₅ est sélectionné parmi le groupe de -H, -C₁₋₆ alkyle, -C₃₋₆ cycloalkyle, -alkyle substitué C₁₋₆ alkyle, -COR₁₀, -SO₂R₃ et -SO₂NR₂R₂;
à condition qu'un seul d'entre R₄ ou R₅ puisse être sélectionné parmi le groupe de - COR₁₀, -SO₂R₃ et -SO₂NR₂R₂;
ou bien R₄ et R₅ pris avec le N adjacent forment un cycle tel que 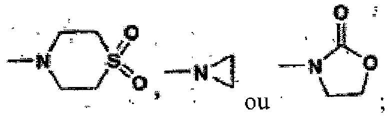
R₁₀ est sélectionné parmi le groupe de -H, -C₁₋₆ alkyle, -C₁₋₆ alkyle-NR₆R₇, - NR₁₁R₁₂, -OR₁₃, -C₁₋₆ alkyle-Q₂, -C₃₋₆ cycloalkyle-Q₂, aryle-Q₂, où n = 1-6,
où Q₂ = hydroxy, -COOR₂, -halo, SO₂Rₐ, -CONHSO₂R₃, -CONHSO₂NR₂R₂;
R₁₀ peut également être sélectionné parmi le groupe de: 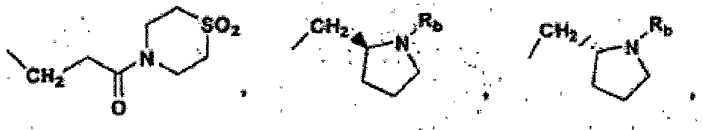 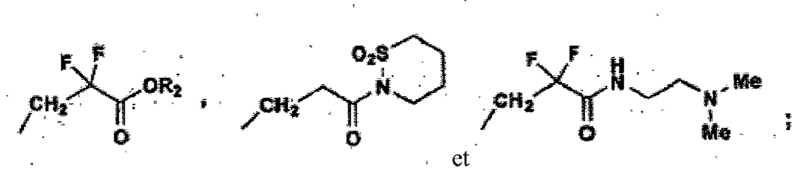
R₆ et R₇ sont sélectionnés indépendamment parmi le groupe de -H, -C₁₋₆ alkyle, - C₁₋₆ alkyle substitué, aryle, hétéroaryle, aryle substitué, hétéroaryle substitué et -C₁₋₆ alkyle-Q₁,
ou bien R₆ et R₇ sont pris avec le N adjacent pour former un cycle sélectionné parmi le groupe de: 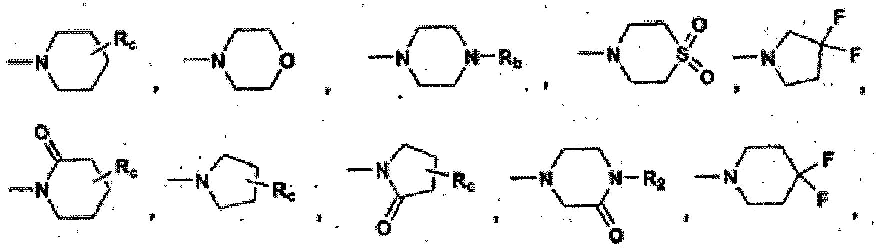 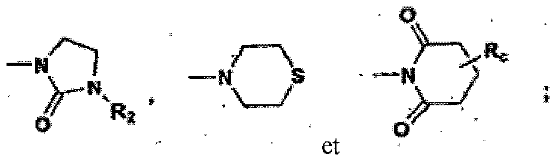
R_{c} = C₁₋₆ alkyle, NR₂R₂, -COOR₃;
R₈ et R₉ sont sélectionnés indépendamment parmi le groupe de -H, C₁₋₆ alkyle, - C₃₋₆ cycloalkyle, -C₁₋₆ alkyle substitué, -C₁₋₆ alkyle-hétéroaryle, -C₁₋₆ alkyle-hétéroaryle substitué, -C₁₋₆ alkyle-NR₂R₂, -C₁₋₆ alkyle-CONR₂R₂, -C₁₋₆ alkyle-Q₁, C₃₋₆ cycloalkyle-Q₁,
ou bien R₈ et R₉ peuvent également être sélectionnés indépendamment parmi le groupe de: 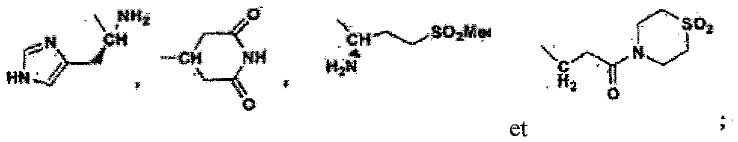
ou bien R₈ et R₉ sont pris avec le N adjacent pour former un cycle sélectionné parmi le groupe de: 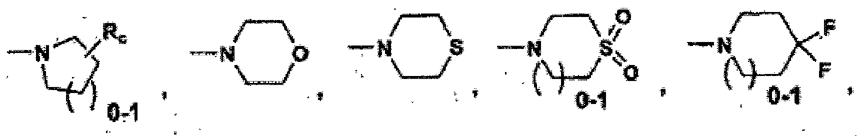 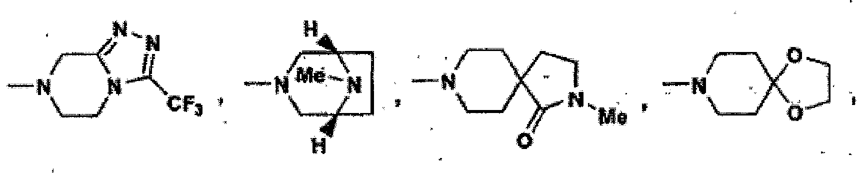 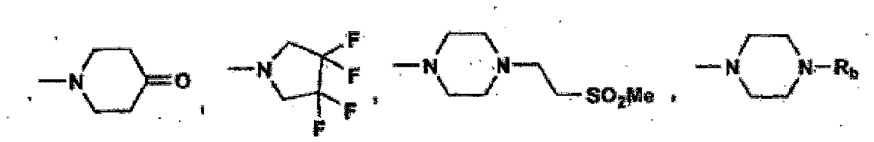 et 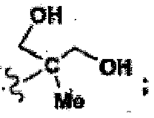
et R₁₁ et R₁₂ sont sélectionnés indépendamment parmi le groupe de -H, C₁₋₆ alkyle, -C₃₋₆ cycloalkyle et -alkyle substitué C₁₋₆ alkyle;
ou bien R₁₁ et R₁₂ sont pris avec le N adjacent pour former un cycle sélectionné parmi le groupe de: 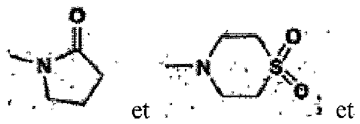 R₁₃ est sélectionné parmi le groupe de -H, C₁₋₆ alkyle, -alkyle substitué C₁₋₆ alkyle et -C₁₋₆ alkyle-NR₁₄R₁₅, où
R₁₄ et R₁₅ sont sélectionnés indépendamment parmi le groupe de -H, -C₁₋₆ alkyle et -alkyle substitué C₁₋₆ alkyle, ou bien R₁₄ et R₁₅ sont pris avec le N adjacent pour former un cycle sélectionné parmi le groupe de:

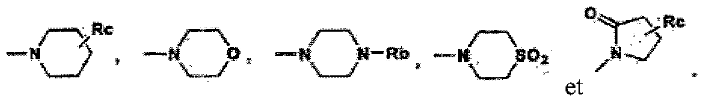

2. Composé selon la revendication 1, où ledit composé a la Formule I.

3. Composé selon la revendication 2, dans lequel X est un cycle phényle et Y est à la position para.

4. Composé selon la revendication 3, dans lequel X est un cycle phényle substitué.

5. Composé selon la revendication 4, dans lequel ledit cycle phényle est substitué par A, et A est au moins un chaînon sélectionné parmi le groupe de -H, -OH et -F.

6. Composé selon la revendication 5, dans lequel Y est -COOH.

7. Composé selon la revendication 4, dans lequel X est un cycle phényle et Y est - COOH à la position para selon la Formule Ia:

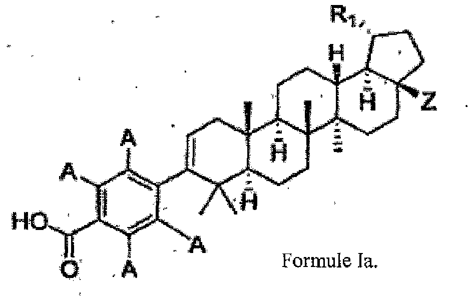

8. Composé selon la revendication 7, dans lequel A est au moins un chaînon sélectionné parmi le groupe de -H, -OH et -F.

9. Composé selon la revendication 8, dans lequel A est -H ou -F.

10. Composé selon la revendication 1, y compris des sels pharmaceutiquement acceptables de celui-ci, qui est sélectionné parmi le groupe consistant en: 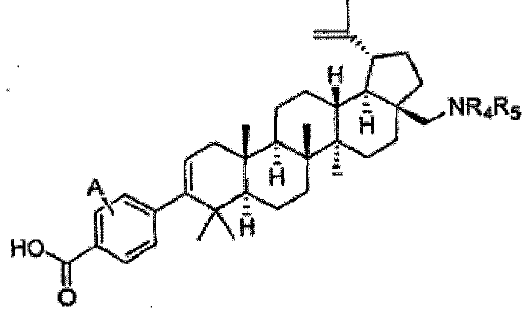 et

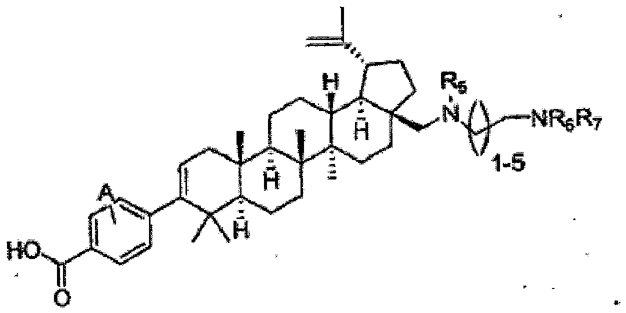

11. Composé selon la revendication 1, qui est sélectionné parmi le groupe consistant en: 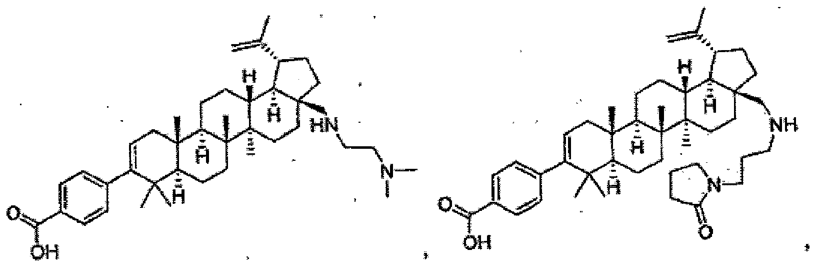 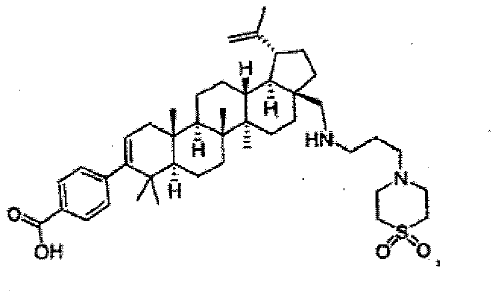 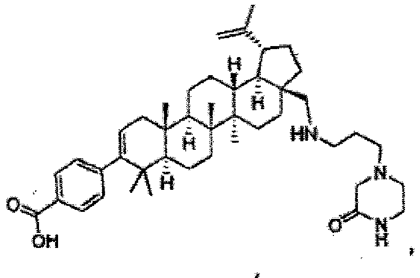 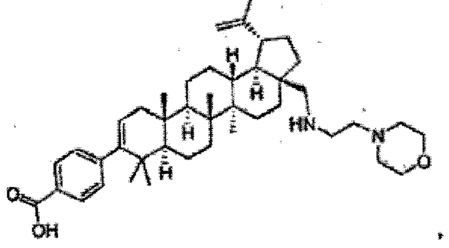 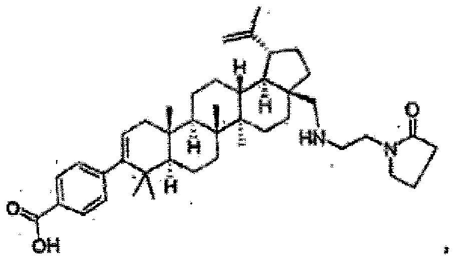 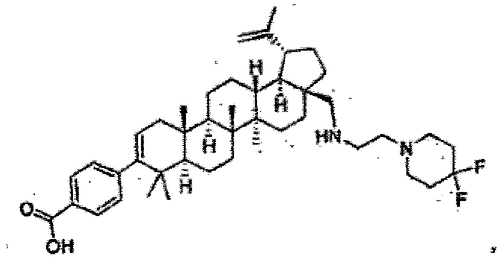 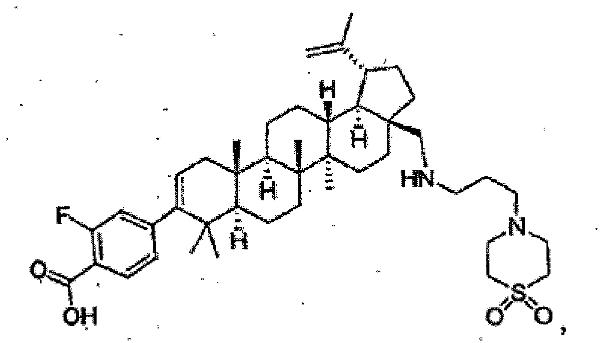 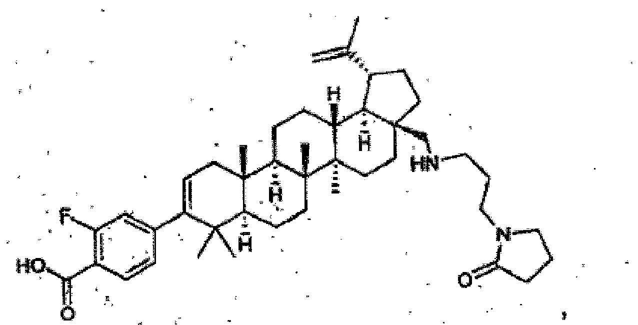 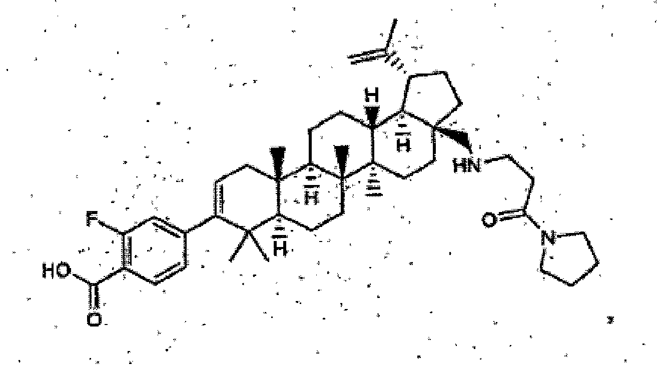 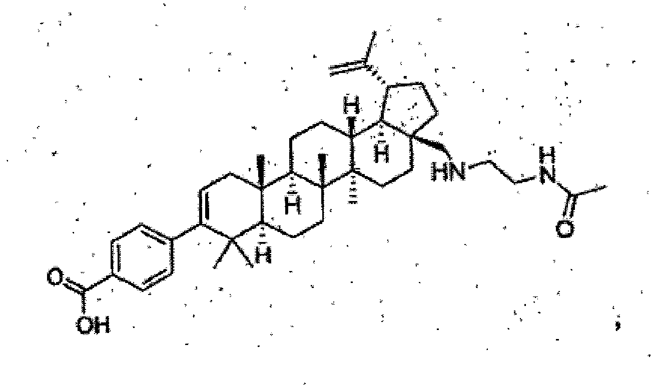 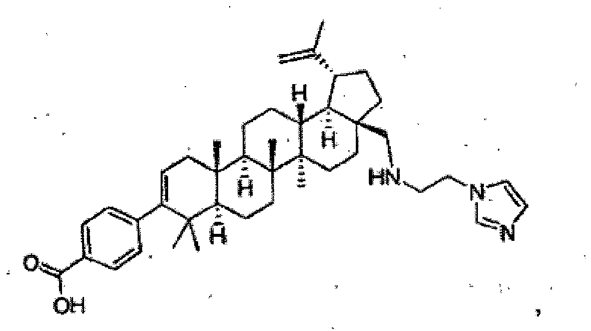 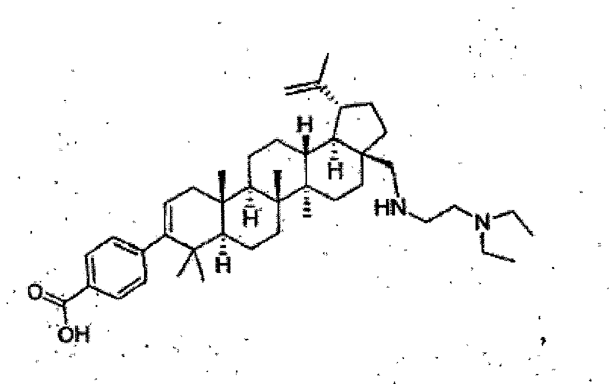 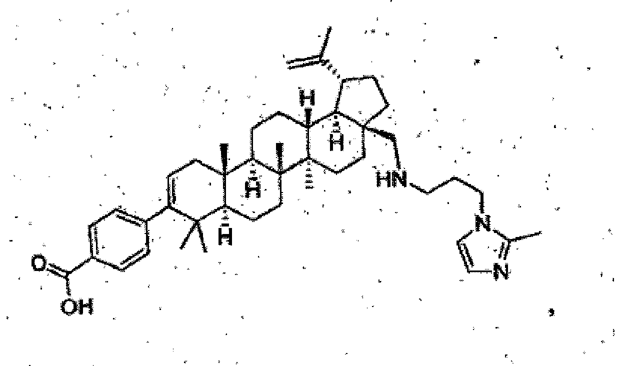 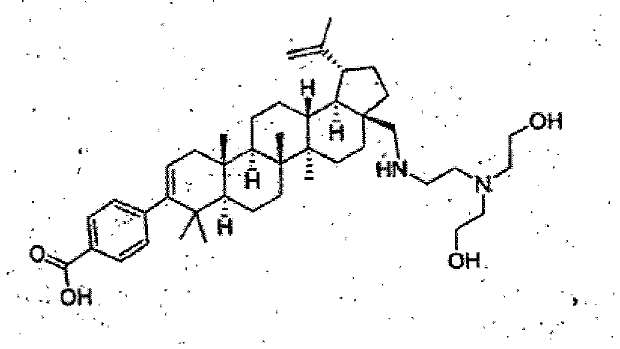 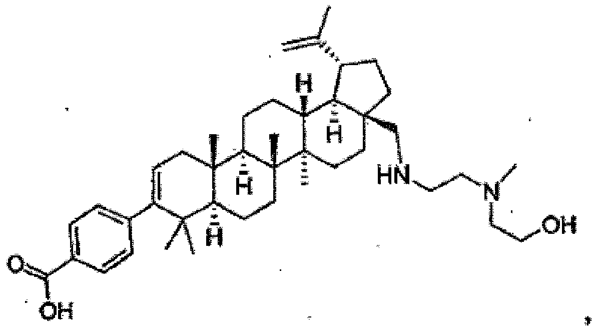 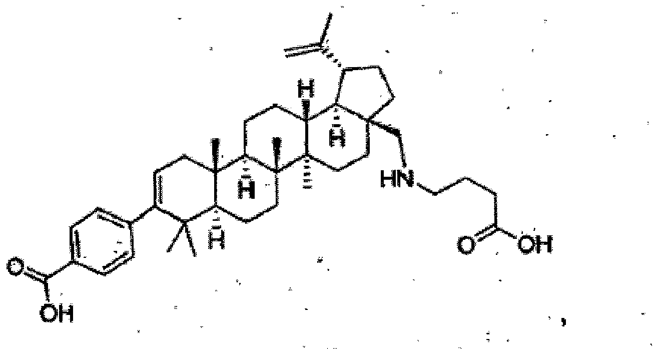 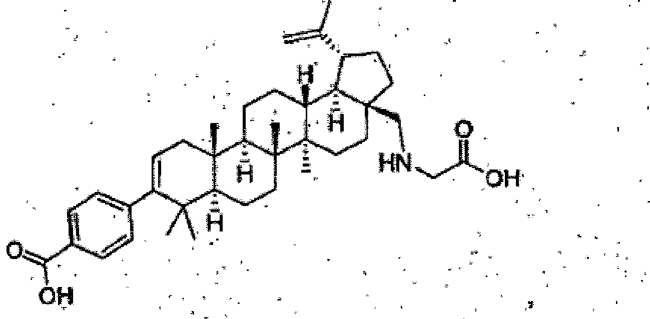 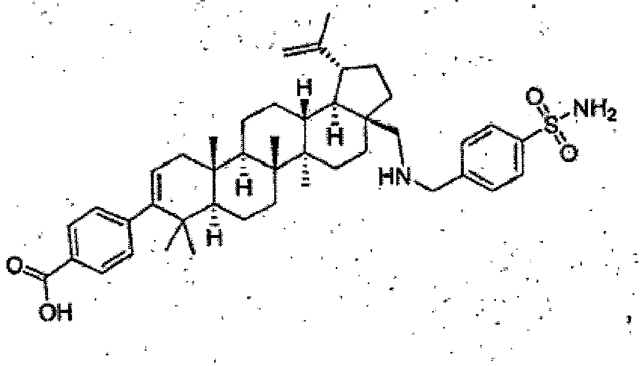 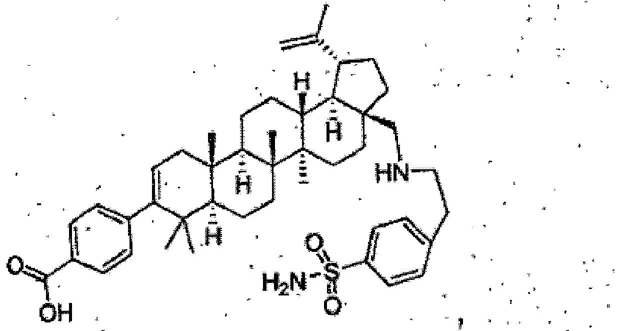 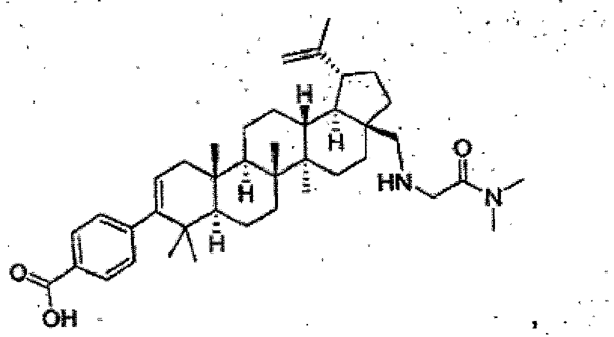 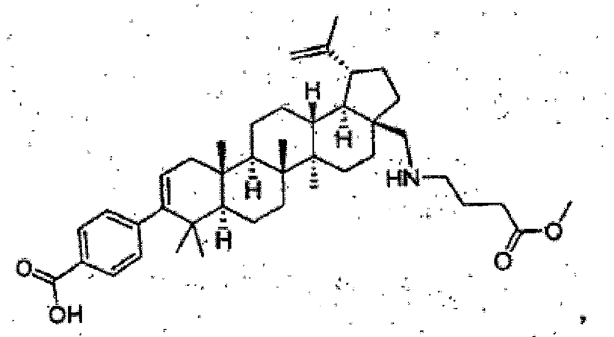 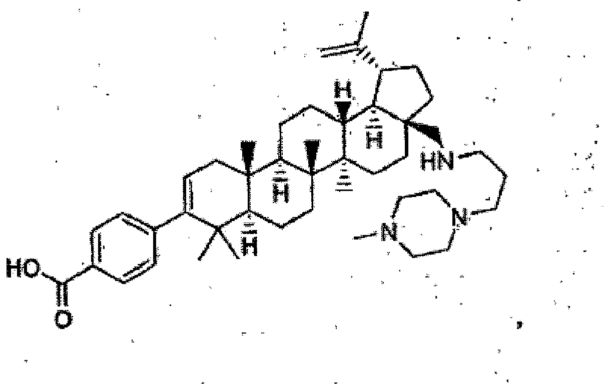 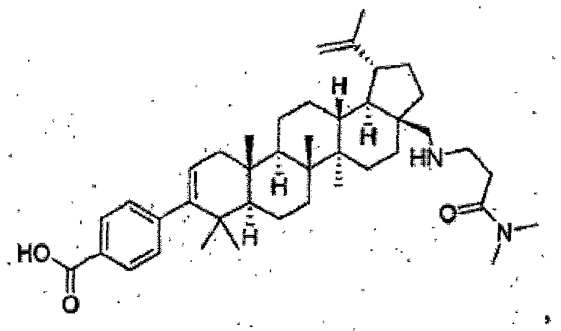 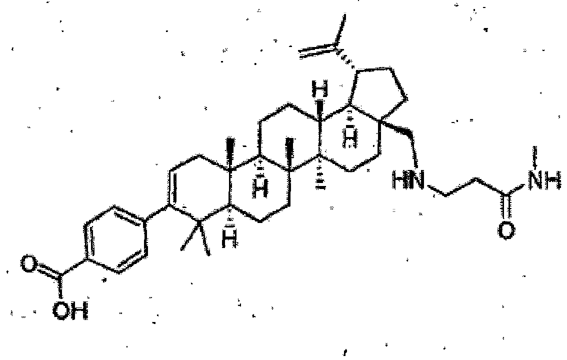 et

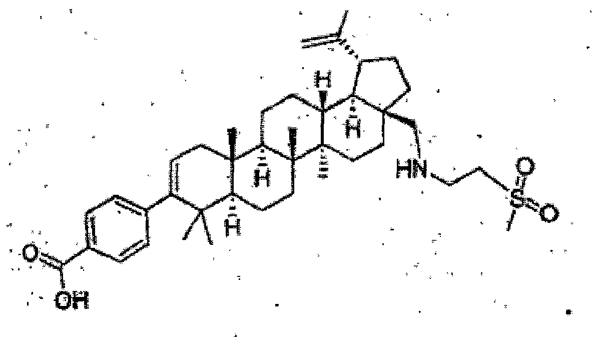

12. Composition pharmaceutique qui comprend une quantité antivirale effective d'un ou de plusieurs des composés selon l'une quelconque des revendications 1 à 11, ainsi qu'un ou plusieurs véhicules, excipients ou diluants pharmaceutiquement acceptables.

13. Composition pharmaceutique selon la revendication 12, utile pour traiter une infection par le VIH, laquelle comprend en plus une quantité antivirale effective d'un agent pour le traitement du SIDA sélectionné parmi le groupe consistant en:
(a) un agent antiviral contre le SIDA;
(b) un agent anti-infectieux;
(c) un immunomodulateur; et
(d) un autre inhibiteur d'entrée du VIH.

14. Composé selon l'une quelconque des revendications 1 à 11, à utiliser dans une méthode de traitement d'un mammifère infecté par le virus VIH.

15. Composé intermédiaire qui est sélectionné parmi le groupe de: 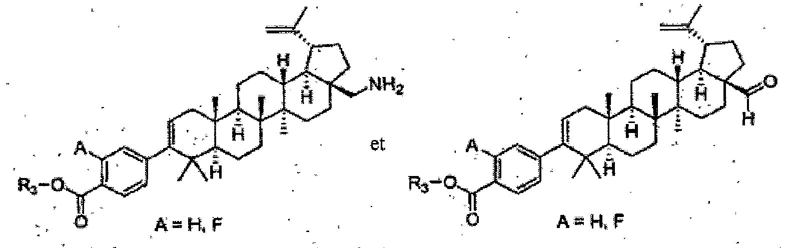 où R₃ est tel que défini dans la revendication 1.
